# EUROPEAN PATENT APPLICATION

(11) **EP 3 632 926 A1**
(43) Date of publication of application: **08.04.2020**
(21) Application number: 17912073.8
(22) Date of filing: 06.12.2017
(51) Int. Cl.: C07K 14/465, A23L 15/00, A61K 39/35, A61P 37/08, G01N 33/53

(54) **ANTIGEN OF ALLERGY AND EPITOPE THEREOF**

(30) Priority: 02.06.2017 WO PCT/JP2017/020654
(71) Applicant: Hoyu Co., Ltd., Nagoya-shi Aichi, 461-8650 (JP)
(72) Inventor: MATSUNAGA, Kayoko, Toyoake-shi Aichi 470-1192 (JP); YAGAMI, Akiko, Toyoake-shi Aichi 470-1192 (JP); OHNO, Fumiaki, Nagakute-shi Aichi 480-1136 (JP); AOKI, Yuji, Nagakute-shi Aichi 480-1136 (JP)
(74) Representative: Mewburn Ellis LLP
(86) International application number: PCT/JP2017/043877
(87) International publication number: WO 2018/220889

(57) **Abstract**

The present invention provides novel antigens of an allergy to egg, methods and kits for diagnosing an allergy to egg, pharmaceutical compositions comprising such an antigen, eggs or processed products of egg in which such an antigen is eliminated or reduced, birds that deliver such eggs or are born from such eggs, a method for producing processed products of egg in which such an antigen is eliminated or reduced, and a tester for determining the presence or absence of an egg antigen in an object of interest. The present invention also relates to polypeptides comprising an epitope of an antigen, kits, compositions and methods for diagnosing an allergy, comprising such a polypeptide, pharmaceutical compositions comprising such a polypeptide, and raw materials or processed products in which an antigen comprising such a polypeptide is eliminated or reduced. The present invention further relates to a tester for determining the presence or absence of an antigen in an object of interest.

## Description

### TECHNICAL FIELD

The present invention relates to a novel antigen of an allergy to eggs. The present invention also relates to a kit, a composition, and a method for diagnosing allergy to eggs. The present invention also relates to a pharmaceutical composition comprising such an antigen, egg or processed products of egg in which such an antigen is eliminated or reduced, and bird that delivers such egg or is born from such egg. The present invention further relates to a method for producing a processed product of egg in which such an antigen is eliminated or reduced. The present invention further relates to a tester for determining the presence or absence of an egg antigen in an object of interest.

The present invention also relates to a polypeptide comprising an epitope of an antigen. The present invention also relates to a kit, a composition and a method for diagnosing an allergy, comprising such a polypeptide. The present invention also relates to a pharmaceutical composition comprising such a polypeptide, and a raw material or a processed product in which such a polypeptide is eliminated or reduced. The present invention further relates to a method for producing a processed product in which such a polypeptide is eliminated or reduced. The present invention further relates to a tester for determining the presence or absence of an antigen comprising such a polypeptide in an object of interest.

### BACKGROUND ART

In sera and tissues of allergic patients, IgE antibodies specific to particular antigens (hereinafter also referred to as allergens) are produced. Physiological consequences caused by interaction between such IgE antibodies and such particular antigens elicit allergic reactions. The antigens refer to food or cooking ingredients, etc. that cause allergic symptoms in a broad sense, and refer to proteins (hereinafter also referred to as allergen components) contained in food or cooking ingredients, etc. to which specific IgE antibodies bind in a narrow sense.

In the process of production of conventional allergy testing agents, antigen reagents are commonly prepared simply by grinding a candidate allergenic food, cooking ingredients or the like (Patent Literature 1). For this reason, the only case where conventional allergy tests have permitted detection of a positive allergic reaction is when a conventional antigen reagent contains an allergen component in an amount exceeding a threshold that allows determination of a positive reaction for binding to an IgE antibody. Thus, diagnosis efficiency is not sufficiently high.

Egg allergens shown in the table below, etc. are currently known (Non Patent Literature 1).

**[Table 1]**

| **Species** | **Allergen** | **Biochemical name** | **MW(SDS-PAGE)** | **Food Allergen** | **Entry Date** | **Modified Date** |
|---|---|---|---|---|---|---|
| ***Gallus domesticus* (chicken)** | | | | | | |
| | Gal d 1 | Ovomucoid | 28 | Yes | 2016-04-04 | 2010-04-29 |
| | Gal d 2 | Ovalbumin | 44 | Yes | 2016-04-04 | 2010-04-29 |
| | Gal d 3 | Ovotransferrin | 78 | Yes | 2016-04-04 | 2010-04-29 |
| | Gal d 4 | Lysozyme C | 14 | Yes | 2016-04-04 | 2010-04-29 |
| | Gal d 5 | Serum albumin | 69 | Yes | 2016-04-04 | 2015-12-19 |
| | Gal d 6 | YGP42 | 35 kDa | Yes | 2016-04-04 | 2011-08-04 |
| | Gal d 7 | Myosin light chain 1f | 22 kDa | Yes | 2016-04-04 | 2015-08-17 |
| | Gal d 8 | alpha-parvalbumin | 11.8 kDa | Yes | 2016-04-04 | 2016-02-22 |
| | Gal d 9 | Enolase | 50 kDa | Yes | 2016-04-04 | 2016-02-22 |
| | Gal d 10 | Aldolase | | Yes | 2016-04-04 | 2016-02-22 |

Some allergen components have been suggested for allergen candidate food or cooking ingredients, and have also been commercialized as testing kits. However, while it is necessary to exhaustively identify allergen components in order to enhance the reliability of allergy tests, the patient detection rate by the measurement of such allergenic components is far from sufficient. Identification of novel allergens in eggs is very important not only for increasing the precision of diagnosis, but also for determining targets of therapeutic agents, low allergenic foods, and low allergenic cooking ingredients.

Meanwhile, as for protein separation and purification, in recent years, a method for separating and purifying many different proteins from a small amount of sample has been used, which is more specifically a two-dimensional electrophoresis consisting of isoelectric focusing in the first dimension, followed by SDS-PAGE (sodium dodecyl sulfate-polyacrylamide gel electrophoresis) in the second dimension. The present applicant has conventionally developed some 2D electrophoresis methods with high separation ability (Patent Literature 2-5).

Allergen-specific IgE antibodies recognize and bind to epitopes that are particular amino acid sequences in allergen components. However, only a slight number of analyses have been made on epitopes as to the allergen components (Non Patent Literature 2), but such analyses are still totally quite rare. Furthermore, any kit for diagnosing an allergy using a polypeptide comprising an epitope has not yet emerged in the market.

### CITATION LIST

### PATENT LITERATURE

PTL1: Japanese Patent Application Publication No. JP 2002-286716
PTL2: Japanese Patent Application Publication No. JP 2011-33544
PTL3: Japanese Patent Application Publication No. JP 2011-33546
PTL4: Japanese Patent Application Publication No. JP 2011-33547
PTL5: Japanese Patent Application Publication No. JP 2011-33548

### NON PATENT LITERATURE

NPL 1: Allergen Nomenclature, WHO/IUIS Allergen Nomenclature Sub-Committee, [searched on May 31, 2016], Internet <URL: http://www.allergen.org/search.php?allergenname=&allergensource=gallus+domesticus&Tax Source=&TaxOrder=&foodallerg=all&bioname=>
NPL 2: Matsuo, H., et al., J. Biol. Chem., (2004), Vol.279, No.13, pp.12135-12140

### SUMMARY OF INVENTION

### TECHNICAL PROBLEM

The present invention provides novel antigens of an allergy to eggs. The present invention also provides methods and kits for diagnosing allergy to eggs. The present invention also provides a pharmaceutical composition comprising such an antigen, egg or processed products of egg in which such an antigen is eliminated or reduced, and bird that delivers such egg or is born from such egg. The present invention further provides testers for determining the presence or absence of an egg antigen in an object of interest.

The present invention also provides polypeptides comprising an epitope of an antigen. The present invention also provides kits, compositions and methods for diagnosing an allergy including such polypeptides. The present invention also provides pharmaceutical compositions comprising such a polypeptide, and raw materials or processed products in which an antigen comprising such a polypeptide is eliminated or reduced. The present invention further relates to methods for producing a processed product in which such an antigen is eliminated or reduced. The present invention further relates to testers for determining the presence or absence of an antigen comprising such a polypeptide in an object of interest.

### SOLUTION TO PROBLEM

In order to solve the aforementioned problems, the present inventors had made intensive studies to identify causative antigens of an allergy to eggs. As a result, the inventors succeeded in identifying novel antigens to which an IgE antibody in the serum of an egg allergic patient specifically binds. The present invention has been completed based on this finding.

Thus, in one embodiment, the present invention can be as defined below.
[1] A kit for diagnosing an allergy to an egg, the kit comprising, as an antigen, at least one of proteins as defined below in any one of (4) to (10):
   (4)
      (4A) a protein comprising vitellogenin-3 or a variant thereof, which is an antigen of an allergy to an egg and is defined below in any of (4A-a) to (4A-e):
         (4A-a) a protein comprising an amino acid sequence with deletion, substitution, insertion or addition of one or several amino acids in SEQ ID NO: 47;
         (4A-b) a protein comprising an amino acid sequence having at least 70% identity to the amino acid sequence of SEQ ID NO: 47;
         (4A-c) a protein comprising an amino acid sequence encoded by a nucleotide sequence with deletion, substitution, insertion or addition of one or several nucleotides in SEQ ID NO: 46;
         (4A-d) a protein comprising an amino acid sequence encoded by a nucleotide sequence having at least 70% identity to the nucleotide sequence of SEQ ID NO: 46; or
         (4A-e) a protein comprising an amino acid sequence encoded by a nucleic acid that hybridizes under stringent conditions with a nucleic acid having a nucleotide sequence complementary to the nucleotide sequence of SEQ ID NO: 46; or
      (4B) a protein comprising at least one amino acid sequence selected from the group consisting of SEQ ID NOs: 47-57;
   (5)
      (5A) a protein comprising vitellogenin-2 precursor (hereinafter also referred to as vitellogenin-2) or a variant thereof, which is an antigen of an allergy to an egg and is defined below in any of (5A-a) to (5A-e):
         (5A-a) a protein comprising an amino acid sequence with deletion, substitution, insertion or addition of one or several amino acids in SEQ ID NO: 59;
         (5A-b) a protein comprising an amino acid sequence having at least 70% identity to the amino acid sequence of SEQ ID NO: 59;
         (5A-c) a protein comprising an amino acid sequence encoded by a nucleotide sequence with deletion, substitution, insertion or addition of one or several nucleotides in SEQ ID NO: 58;
         (5A-d) a protein comprising an amino acid sequence encoded by a nucleotide sequence having at least 70% identity to the nucleotide sequence of SEQ ID NO: 58; or
         (5A-e) a protein comprising an amino acid sequence encoded by a nucleic acid that hybridizes under stringent conditions with a nucleic acid having a nucleotide sequence complementary to the nucleotide sequence of SEQ ID NO: 58; or
      (5B) a protein comprising at least one amino acid sequence selected from the group consisting of SEQ ID NOs: 59-104;
   (6)
      (6A) vitellogenin-2 precursor (hereinafter also referred to as vitellogenin-2) or a variant thereof, which is an antigen of an allergy to an egg and is defined below in any of (6A-a) to (6A-e):
         (6A-a) a protein comprising an amino acid sequence with deletion, substitution, insertion or addition of one or several amino acids in SEQ ID NO: 106;
         (6A-b) a protein comprising an amino acid sequence having at least 70% identity to the amino acid sequence of SEQ ID NO: 106;
         (6A-c) a protein comprising an amino acid sequence encoded by a nucleotide sequence with deletion, substitution, insertion or addition of one or several nucleotides in SEQ ID NO: 105;
         (6A-d) a protein comprising an amino acid sequence encoded by a nucleotide sequence having at least 70% identity to the nucleotide sequence of SEQ ID NO: 105; or
         (6A-e) a protein comprising an amino acid sequence encoded by a nucleic acid that hybridizes under stringent conditions with a nucleic acid having a nucleotide sequence complementary to the nucleotide sequence of SEQ ID NO: 105; or
      (6B) a protein comprising at least one amino acid sequence selected from the group consisting of SEQ ID NOs: 106-150;
   (7)
      (7A) apolipoprotein B precursor (hereinafter also referred to as apolipoprotein B) or a variant thereof, which is an antigen of an allergy to an egg and is defined below in any of (7A-a) to (7A-e):
         (7A-a) a protein comprising an amino acid sequence with deletion, substitution, insertion or addition of one or several amino acids in SEQ ID NO: 152;
         (7A-b) a protein comprising an amino acid sequence having at least 70% identity to the amino acid sequence of SEQ ID NO: 152;
         (7A-c) a protein comprising an amino acid sequence encoded by a nucleotide sequence with deletion, substitution, insertion or addition of one or several nucleotides in SEQ ID NO: 151;
         (7A-d) a protein comprising an amino acid sequence encoded by a nucleotide sequence having at least 70% identity to the nucleotide sequence of SEQ ID NO: 151; or
         (7A-e) a protein comprising an amino acid sequence encoded by a nucleic acid that hybridizes under stringent conditions with a nucleic acid having a nucleotide sequence complementary to the nucleotide sequence of SEQ ID NO: 151; or
      (7B) a protein comprising at least one amino acid sequence selected from the group consisting of SEQ ID NOs: 152-235;
   (8)
      (8A) apolipoprotein B precursor (hereinafter also referred to as apolipoprotein B) or a variant thereof, which is an antigen of an allergy to an egg and is defined below in any of (8A-a) to (8A-e):
         (8A-a) a protein comprising an amino acid sequence with deletion, substitution, insertion or addition of one or several amino acids in SEQ ID NO: 237;
         (8A-b) a protein comprising an amino acid sequence having at least 70% identity to the amino acid sequence of SEQ ID NO: 237;
         (8A-c) a protein comprising an amino acid sequence encoded by a nucleotide sequence with deletion, substitution, insertion or addition of one or several nucleotides in SEQ ID NO: 236;
         (8A-d) a protein comprising an amino acid sequence encoded by a nucleotide sequence having at least 70% identity to the nucleotide sequence of SEQ ID NO: 236; or
         (8A-e) a protein comprising an amino acid sequence encoded by a nucleic acid that hybridizes under stringent conditions with a nucleic acid having a nucleotide sequence complementary to the nucleotide sequence of SEQ ID NO: 236; or
      (8B) a protein comprising at least one amino acid sequence selected from the group consisting of SEQ ID NOs: 237-249;
   (9)
      (9A) apolipoprotein B precursor (hereinafter also referred to as apolipoprotein B) or a variant thereof, which is an antigen of an allergy to an egg and is defined below in any of (9A-a) to (9A-e):
         (9A-a) a protein comprising an amino acid sequence with deletion, substitution, insertion or addition of one or several amino acids in SEQ ID NO: 251;
         (9A-b) a protein comprising an amino acid sequence having at least 70% identity to the amino acid sequence of SEQ ID NO: 251;
         (9A-c) a protein comprising an amino acid sequence encoded by a nucleotide sequence with deletion, substitution, insertion or addition of one or several nucleotides in SEQ ID NO: 250;
         (9A-d) a protein comprising an amino acid sequence encoded by a nucleotide sequence having at least 70% identity to the nucleotide sequence of SEQ ID NO: 250; or
         (9A-e) a protein comprising an amino acid sequence encoded by a nucleic acid that hybridizes under stringent conditions with a nucleic acid having a nucleotide sequence complementary to the nucleotide sequence of SEQ ID NO: 250; or
      (9B) a protein comprising at least one amino acid sequence selected from the group consisting of SEQ ID NOs: 251-260;
   (10)
      (10A) vitellogenin-2 precursor (hereinafter also referred to as vitellogenin-2) or a variant thereof, which is an antigen of an allergy to an egg and is defined below in any of (10A-a) to (10A-e):
         (10A-a) a protein comprising an amino acid sequence with deletion, substitution, insertion or addition of one or several amino acids in SEQ ID NO: 262;
         (10A-b) a protein comprising an amino acid sequence having at least 70% identity to the amino acid sequence of SEQ ID NO: 262;
         (10A-c) a protein comprising an amino acid sequence encoded by a nucleotide sequence with deletion, substitution, insertion or addition of one or several nucleotides in SEQ ID NO: 261;
         (10A-d) a protein comprising an amino acid sequence encoded by a nucleotide sequence having at least 70% identity to the nucleotide sequence of SEQ ID NO: 261; or
         (10A-e) a protein comprising an amino acid sequence encoded by a nucleic acid that hybridizes under stringent conditions with a nucleic acid having a nucleotide sequence complementary to the nucleotide sequence of SEQ ID NO: 261; or
      (10B) a protein comprising at least one amino acid sequence selected from the group consisting of SEQ ID NOs: 262-271;
[2] A composition for diagnosing an allergy to eggs, comprising, as an antigen, at least one of proteins as defined above in any of (4) to (10) of [1].
[3] A method for providing an indicator for diagnosing an allergy to eggs in a subject, the method comprising the steps of:
   (i) contacting a sample obtained from the subject with an antigen, wherein the sample is a solution comprising an IgE antibody;
   (ii) detecting binding between the IgE antibody present in the sample from the subject and the antigen; and
   (iii) when the binding between the IgE antibody in the subject and the antigen is detected, an indicator of the fact that the subject is allergic to eggs is provided;
   wherein the antigen is at least one of proteins as defined above in any of (4) to (10) of [1].
[4] A pharmaceutical composition comprising at least one of proteins as defined above in any of (4) to (10) of [1].
[5] The pharmaceutical composition as set forth in [4], wherein the pharmaceutical composition is intended for the treatment of an allergy to eggs.
[6] Egg or processed products of egg in which an antigen is eliminated or reduced, or bird that delivers the egg or is born from the egg, wherein the antigen is at least one of proteins as defined above in any of (4) to (10) of [1].
[7] A tester for determining the presence or absence of an egg antigen in an object of interest, comprising an antibody that binds to at least one of proteins as defined above in any of (4) to (10) of [1].
[8] A tester for determining the presence or absence of an antigen of an allergy to eggs in an object of interest, comprising a primer having a nucleotide sequence complementary to at least one of the nucleotide sequences of SEQ ID NOs: 46, 58, 105, 151, 236, 250 and 261.
[9] A method for producing a processed product of egg in which an antigen is eliminated or reduced, the method comprising the step of confirming that the antigen is eliminated or reduced, in a production process of the processed product, wherein the antigen is at least one of proteins as defined above in any of (4) to (10) of [1].
[10] An egg-derived antigen which is at least one of proteins as defined above in any of (4) to (10) of [1] and is causative of an allergy to eggs.
   The present inventors also succeeded in finding epitopes as to egg-derived antigens including the antigens described above.
   Since the epitopes have a relatively short amino acid sequence, the IgE antibodies are capable of binding to different allergen components if the same amino acid sequence is present in the different allergen components. Since different allergen components have a common epitope so that IgE antibodies from allergic patients bind to both of them, the antigens have cross-reactivity. Thus, the epitopes defined in the present invention enable diagnosis or treatment of an allergy including cross-reactivity, and detection of a plurality of allergen components comprising the epitopes, etc.
   The present invention has been completed based on this finding. Thus, in another embodiment, the present invention can be as defined below.
[11] A polypeptide specifically binding to an IgE antibody from an allergic patient, the polypeptide being any one of the following:
   (1β) a polypeptide comprising at least one amino acid sequence selected from the group consisting of SEQ ID NOs: 272-295 and 319-525;
   (2β) a polypeptide comprising at least one amino acid sequence selected from the group consisting of SEQ ID NOs: 296-303 and 526-552;
   (3β) a polypeptide comprising at least one amino acid sequence selected from the group consisting of SEQ ID NOs: 304-311 and 553-625; and
   (4β) a polypeptide comprising at least one amino acid sequence selected from the group consisting of SEQ ID NOs: 312-318 and 626-682.
[12] The polypeptide according to the above [11], wherein the number of amino acid residues is 500 or less.
[13] A kit for diagnosing an allergy, comprising at least one of polypeptides according to the above [11] or [12].
[14] A composition for diagnosing an allergy, the composition comprising at least one of polypeptides according to the above [11] or [12] as an antigen.
[15] A method for providing an indicator for diagnosing an allergy in a subject, the method comprising the steps of:
   (i) contacting a sample obtained from the subject with an antigen, wherein the sample is a solution comprising an IgE antibody;
   (ii) detecting binding between the IgE antibody present in the sample obtained from the subject and the antigen; and
   (iii) when the binding between the IgE antibody in the subject and the antigen is detected, an indicator of the fact that the subject is allergic is provided;
   wherein the antigen is at least one of polypeptides according to any one of the above [11] or [12].
[16] A pharmaceutical composition comprising at least one of polypeptides according to the above [11] or [12].
[17] The pharmaceutical composition according to the above [16], wherein the pharmaceutical composition is intended for the treatment of an allergy.
[18] A tester for determining the presence or absence of an antigen in an object of interest, the tester comprising an antibody that binds to at least one of polypeptides according to the above [11] or [12].
[19] A tester for determining the presence or absence of an antigen in an object of interest, the tester comprising any of the following primers:
   (a) a primer comprising a portion of the nucleotide sequence of a nucleic acid encoding a polypeptide according to the above [11] or [12], and/or a complementary strand thereof; and
   (b) a primer which is a portion of at least one of the nucleotide sequences of SEQ ID NOs: 46, 58, 105, 151, 236, 250 and 261 and/or a primer which is a portion of a sequence complementary to at least one of the nucleotide sequences of SEQ ID NOs: 46, 58, 105, 151, 236, 250 and 261.
[20] A raw material or a processed product in which an antigen is eliminated or reduced, wherein the antigen is at least one of polypeptides according to the above [11] or [12].
[21] A method for producing a processed product in which an antigen is eliminated or reduced, the method comprising the step of confirming that the antigen is eliminated or reduced in a production process of the processed product, wherein the antigen is at least one of polypeptides according to the above [11] or [12].

### ADVANTAGEOUS EFFECTS OF INVENTION

The present invention can provide novel antigens of an allergy to eggs. Since the novel antigens (allergen components) that trigger an egg allergy were identified according to this invention, this invention can provide highly sensitive methods and kits for diagnosing an allergy to egg, pharmaceutical compositions comprising such an antigen, egg or processed products of egg in which such an antigen is eliminated or reduced, bird that delivers such egg or is born from such egg, a method for producing processed products of egg in which such an antigen is eliminated or reduced, and testers for determining the presence or absence of an egg antigen in an object of interest.

The present invention can provide novel polypeptides comprising an epitope of an antigen. Use of the polypeptide of the present invention enables provision of highly sensitive kits, compositions and methods for diagnosing an allergy, pharmaceutical compositions comprising such a polypeptide, testers for determining the presence or absence of an antigen comprising such a polypeptide in an object of interest, raw materials or processed products in which such a polypeptide is eliminated or reduced, and a method for producing the processed products.

### BRIEF DESCRIPTION OF DRAWINGS

[Fig. 1] Fig. 1 is a photograph of a gel showing a protein electrophoretic pattern in two-dimensional electrophoresis of proteins contained in chicken egg (raw egg) yolk. The bands at the left of the photograph are bands of molecular weight markers, and the numeric values at the left of the photograph are respective molecular weights (KDa) of the molecular weight markers. The numeric values at the top of the photograph represent isoelectric points.
[Fig. 2] Fig. 2 is a photograph of an immunoblot of a two-dimensional electrophoretic pattern of proteins contained in chicken egg (raw egg) yolk using the serum of egg-allergic patient 1.
[Fig. 3] Fig. 3 is a photograph of a gel showing a two-dimensional electrophoretic pattern of proteins contained in chicken egg (raw egg) yolk. Spots 1, 2 and 3 on which the serum of egg-allergic patient 1 specifically reacted are each boxed.
[Fig. 4] Fig. 4 is a photograph of an immunoblot of a two-dimensional electrophoretic pattern of proteins contained in chicken egg (raw egg) yolk using the serum of egg-allergic patient 2. Spots 4 to 8 on which the serum of egg-allergic patient 2 specifically reacted are each boxed.
[Fig. 5] Fig. 5 is a photograph of an immunoblot of a two-dimensional electrophoretic pattern of proteins contained in chicken egg (raw egg) yolk using the serum of egg-allergic patient 3. Spots 9 and 10 on which the serum of egg-allergic patient 3 specifically reacted are each boxed.
[Fig. 6] Fig. 6 is a photograph of an immunoblot of a two-dimensional electrophoretic pattern of proteins contained in chicken egg (raw egg) yolk using the serum of egg-allergic patient 4. Spot 11 on which the serum of egg-allergic patient 4 specifically reacted are each boxed.

### DESCRIPTION OF EMBODIMENTS

The present invention will be described in detail below, but the present invention is not limited to them.

Unless otherwise defined herein, all scientific and technical terms used in relation to the present invention shall have meanings commonly understood by those skilled in the art.

As referred to herein, the "allergy" refers to the state in which, when a certain antigen enters the body of a living individual sensitized to said antigen, the living individual shows a hypersensitive reaction detrimental to him/her. An allergic reaction can be produced upon contact with an antigen or consumption of the antigen. Here, the contact refers to touch to an object and, particularly, as for the human body, refers to attachment to the skin, the mucosa (eyes, lips, etc.) or the like. The consumption refers to incorporation into the body and refers to incorporation by inhalation, through an oral route, or the like. In general, allergic reactions caused by consumption of foods are particularly referred to as food allergies. In a preferred embodiment,embodiment, the allergy may be a food allergy. In blood and tissues of individuals with many food-allergic diseases, IgE antibodies specific to antigens are produced. IgE antibodies bind to mast cells or basophils. When an antigen specific to such an IgE antibody enters again the body of a patient with an allergic disease, said antigen combines with the IgE antibody bound to mast cells or basophils, and the IgE antibody crosslinks said antigen on the cell surface, resulting in physiological effects of IgE antibody-antigen interaction. Examples of such physiological effects include release of histamine, serotonin, heparin, eosinophil chemotactic factors, leucotrienes, or the like. These released substances provoke an allergic reaction resulting from the combination of an IgE antibody with particular antigens. Specifically, IgE antibodies recognize and bind to epitopes that are particular amino acid sequences in particular antigens. Allergic reactions caused by such antigens occur through the aforementioned pathway.

In the present invention, the allergy of interest is not particularly limited as long as it is an allergy to an allergen comprising an epitope to be used. Such an allergy may include allergies to plants of the family *Oleaceae*, the family *Compositae*, the family *Poaceae*, the family *Bromeliaceae*, the family *Juglandaceae*, the family *Cucurbitaceae*, and the family *Leguminosae*, allergies to animals of the family *Phasianidae* and the family *Bovidae*, allergies to seafood of the family *Carangidae*, the family *Sparidae*, the family *Salmonidae*, and the family *Penaeidae.* Examples of the plant of the family *Oleaceae* include olive (scientific name: *Olea europaea*)*.* Examples of the plant of the family *Compositae* include artichoke (scientific name: *Cynara scolymus*)*.* Examples of the plant of the family *Poaceae* include bread wheat (scientific name: *Triticum aestivum*) and Tausch's goatgrass (scientific name: *Aegilops tauschii*). Examples of the plant of the family *Bromeliaceae* include pineapple (scientific name: *Ananas comosus*). Examples of the plant of the family *Juglandaceae* include walnut (scientific name: *Juglans regia*). Examples of the plant of the family *Cucurbitaceae* include squash (scientific name: *Cucurbita moschata*)*.* Examples of the plant of the family *Leguminosae* include soybean (scientific name: *Glycine max*). Examples of the animal of the family *Phasianidae* include Japanese quail (scientific name: *Coturnix japonica*) and wild turkey (scientific name: *Meleagris gallopavo*)*.* Examples of the animal of the family *Bovidae* include cattle (scientific name: *Bos taurus*). Examples of the seafood of the family *Carangidae* include greater amberjack (scientific name: *Seriola dumerili*)*.* Examples of the seafood of the family *Sparidae* include red seabream (scientific name: *Pagrus major*). Examples of the seafood of the family *Salmonidae* include rainbow trout (scientific name: *Oncorhynchus mykiss*)*.* Examples of the seafood of the family *Penaeidae* include whiteleg shrimp (scientific name: *Litopenaeus vannamei*).

As referred to herein, the egg means an egg of a bird, preferably a chicken egg, unless otherwise specified. As referred to herein, an egg of a fish is referred to as "fish egg" and is discriminated from the "egg", which typically means an egg of a bird.

As referred to herein, the allergy to eggs refers to the state in which an individual has an allergic reaction caused by proteins, etc. present in eggs which act as an antigen. The allergy to eggs can produce an allergic reaction upon contact with an antigen present in eggs or consumption of the antigen. In general, allergic reactions caused by consumption of foods are particularly referred to as food allergies. The allergy to eggs may be a food allergy.

As referred to herein, the "antigen" refers to a substance that provokes an allergic reaction, and is also referred to as an "allergen component". The antigen is preferably a protein.

As referred to herein, the "protein" refers to a molecule having a structure in which naturally occurring amino acids are joined together by peptide bond. The number of amino acids present in a protein is not particularly limited. As referred to herein, the term "polypeptide" also means a molecule having a structure in which naturally occurring amino acids are joined together by peptide bond. The number of amino acids present in a polypeptide is not particularly limited. The "polypeptide" conceptually includes the "protein". Also, polypeptides having about 2 to 50 amino acids joined together by peptide bond are in some cases called "peptides", especially. In the case where amino acids can form different enantiomers, the amino acids are understood to form an L-enantiomer, unless otherwise indicated. The amino acid sequences of proteins, polypeptides, or peptides as used herein are represented by one-letter symbols of amino acids in accordance with standard usage and the notational convention commonly used in the art. The leftward direction represents the amino-terminal direction, and the rightward direction represents the carboxy-terminal direction. In the one-letter symbols of amino acids, X can be any substance having an amino group and a carboxyl group that can bind to amino acids at both ends, and particularly represents that any of 20 types of naturally occurring amino acids are acceptable. The residue of X is an amino acid residue at a site where binding activity against IgE antibodies from allergic patients was maintained even after substitution by alanine in alanine scanning described in Example 6. It is well known to those skilled in the art that even when such a site is substituted by any other amino acids, it is highly probable that this binding activity against IgE antibodies is maintained.

### Identification of antigens

Proteins contained in eggs were analyzed by the aforementioned technique to identify antigens of an allergy to eggs. To be specific, raw chicken eggs were separated into yolk and albumen, and egg proteins contained in each of them were subjected to two-dimensional electrophoresis under the conditions described below.

The electrophoresis in the first dimension was isoelectric focusing, which was performed using isoelectric focusing gels with a gel-strip length of 5 to 10 cm and a gel pH range of 3 to 10. The pH gradient of the gels in the direction of electrophoresis was as follows: when the total gel-strip length is taken as 1, the gel-strip length up to pH 5 is taken as "a", the gel-strip length from pH 5 to 7 is taken as "b", and the gel-strip length above pH 7 is taken as "c", "a" is in the range of 0.15 to 0.3, "b" is in the range of 0.4 to 0.7, and "c" is in the range of 0.15 to 0.3. More specifically, the isoelectric focusing was performed using the IPG gels, Immobiline Drystrip (pH3-10NL), produced by GE Healthcare Bio-Sciences Corporation (hereinafter abbreviated as "GE"). The electrophoresis system used was IPGphor produced by GE. The maximum current of the electrophoresis system was limited to 75 µA per gel strip. The voltage program adopted to perform the first-dimensional isoelectric focusing was as follows: (1) a constant voltage step was performed at a constant voltage of 300 V until the volt-hours reached 750 Vhr (the current variation width during electrophoresis for 30 minutes before the end of this step was 5 µA); (2) the voltage was increased gradually to 1000 V for 300 Vhr; (3) the voltage was further increased gradually to 5000 V for 4500 Vhr; and then (4) the voltage was held at a constant voltage of 5000 V until the total Vhr reached 12000.

The electrophoresis in the second dimension was SDS-PAGE, which was performed using polyacrylamide gels whose gel concentration at the distal end in the direction of electrophoresis was set to 3 to 6% and whose gel concentration at the proximal end was set to a higher value than that at the distal end. More specifically, the SDS-PAGE was performed using NuPAGE 4-12% Bris-Tris Gels (IPG well, Mini, 1 mm) produced by Life Technologies. The electrophoresis system used was XCell SureLock Mini-Cell produced by Life Technologies. The electrophoresis was run at a constant voltage of 200 V for about 45 minutes using an electrophoresis buffer composed of 50 mM MOPS, 50 mM Tris base, 0.1% (w/v) SDS and 1 mM EDTA.

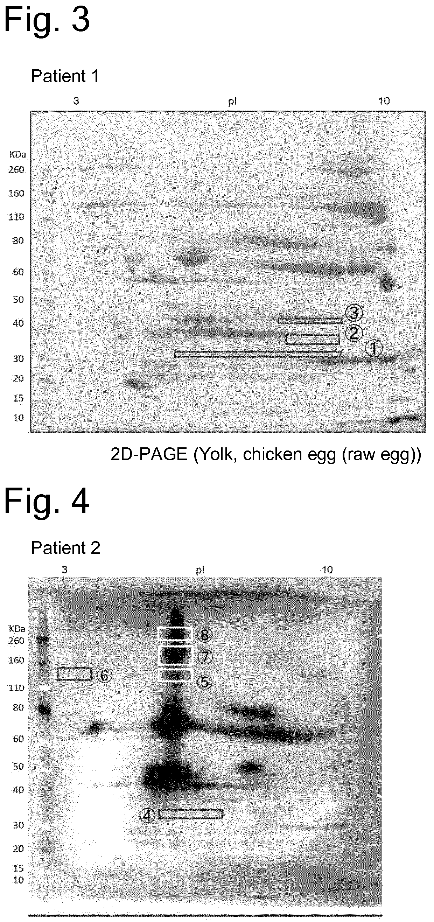

As a result, antigens in spots 1 to 3 in a two-dimensional electrophoresis gel run under the conditions described above for proteins in chicken egg have been revealed to specifically bind to IgE antibodies from egg-allergic patients diagnosed with food-dependent exercise-induced anaphylaxis (Fig. 3). Further, antigens in spots 4 to 11 have been revealed to specifically bind to IgE antibodies from other egg-allergic patients (Figs. 4 to 6).

### Antigen

### (4) Antigen in spot 4

As the result of sequence identification of the antigen in spot 4 by mass spectroscopy, the amino acid sequences of SEQ ID NOs: 48-57 were detected.

Also, the mass spectroscopic data (SEQ ID NOs: 48-57) obtained for spot 4 on a mass spectrometer was analyzed by comparing the data against the NCBI protein data, and as a result, vitellogenin-3 (amino acid sequence: SEQ ID NO: 47, encoding nucleotide sequence: SEQ ID NO: 46) was identified.

Accordingly, the antigen in spot 4 in the present invention can be any of below in (4A-a) to (4A-e), and (4B):
(4A-a) a protein comprising an amino acid sequence with deletion, substitution, insertion or addition of one or several amino acids in SEQ ID NO: 47;
(4A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 47;
(4A-c) a protein comprising an amino acid sequence encoded by a nucleotide sequence with deletion, substitution, insertion or addition of one or several nucleotides in SEQ ID NO: 46;
(4A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 46;
(4A-e) a protein comprising an amino acid sequence encoded by a nucleic acid that hybridizes under stringent conditions with a nucleic acid having a nucleotide sequence complementary to the nucleotide sequence of SEQ ID NO: 46;
(4B) a protein comprising at least one amino acid sequence selected from the group consisting of SEQ ID NOs: 47-57, preferably a protein comprising at least 2, 3, 4, 5, 6, 7, 8, 9, or 10 types or all sequences of the amino acid sequences. More preferably a protein comprising at least one amino acid sequence selected from the group consisting of SEQ ID NOs: 47, 50, 51, 53, and 54, preferably a protein comprising at least 2, 3, or 4 types or all sequences of the amino acid sequences. As referred to above, the amino acid sequence of any of SEQ ID NOs: 47 to 57 may be an amino acid sequence derived therefrom by deletion, substitution, insertion or addition of one or several amino acids.

The proteins as defined above in (4A-a) to (4A-e) and (4B) can be proteins that are found in a protein spot with a molecular weight of 20 to 50 kDa, preferably around 25 to 40 kDa, more preferably 30 to 35 kDa, and an isoelectric point of 3.0 to 8.0, preferably 3.5 to 7.0, more preferably 4.0 to 6.0 on gels used in the two-dimensional electrophoresis performed under the conditions described above in the subsection titled "Identification of antigens".

Epitopes of the aforementioned antigens (4A-a) to (4A-e) and (4B) reside in amino acids 14-28 (SEQ ID NO: 296), amino acids 164-178 (SEQ ID NO: 297), amino acids 204-218 (SEQ ID NO: 298), amino acids 214-228 (SEQ ID NO: 299), amino acids 224-238 (SEQ ID NO: 300), amino acids 244-258 (SEQ ID NO: 301), amino acids 254-268 (SEQ ID NO: 302), and amino acids 284-298 (SEQ ID NO: 303) of SEQ ID NO: 47. When the antigen in spot 4 contains a variation in the amino acid sequence of SEQ ID NO: 47, amino acids corresponding to at least one of these epitopes may retain the sequence in SEQ ID NO: 47.

From the viewpoint that binding activity against IgE antibodies such as sensitivity (a degree to which a patient can be diagnosed as being positive) or specificity (a degree to which a healthy subject is not diagnosed as being positive) remains even if the amino acid sequence is varied, the antigen in spot 4 may be the following variant.

In the epitope having the sequence of SEQ ID NO: 296, sites of amino acids other than X in SEQ ID NO: 526 or 527 are important for binding to IgE antibodies from allergic patients. Thus, in another preferred embodiment,embodiment, when the antigen in spot 4 contains a variation in the amino acid sequence of SEQ ID NO: 47, the antigen may have an amino acid sequence of SEQ ID NO: 47 in which amino acids 14-28 are SEQ ID NO: 526 or 527.

In the epitope having the sequence of SEQ ID NO: 297, sites of amino acids other than X in SEQ ID NO: 529, 530 or 532 are important for binding to IgE antibodies from allergic patients. Thus, in another preferred embodiment,embodiment, when the antigen in spot 4 contains a variation in the amino acid sequence of SEQ ID NO: 47, the antigen may have an amino acid sequence of SEQ ID NO: 47 in which amino acids 164-178 are SEQ ID NO: 529, 530 or 532.

In the epitope having the sequence of SEQ ID NO: 298, sites of amino acids other than X in SEQ ID NO: 536, 537, 545 or 546 are important for binding to IgE antibodies from allergic patients. Thus, in another preferred embodiment,embodiment, when the antigen in spot 4 contains a variation in the amino acid sequence of SEQ ID NO: 47, the antigen may have an amino acid sequence of SEQ ID NO: 47 in which amino acids 204-218 are SEQ ID NO: 536, 537, 545 or 546.

In the epitope having the sequence of SEQ ID NO: 299, sites of amino acids other than X in SEQ ID NO: 547, 548 or 549 are important for binding to IgE antibodies from allergic patients. Thus, in another preferred embodiment,embodiment, when the antigen in spot 4 contains a variation in the amino acid sequence of SEQ ID NO: 47, the antigen may have an amino acid sequence of SEQ ID NO: 47 in which amino acids 214-228 are SEQ ID NO: 547, 548 or 549.

In the epitope having the sequence of SEQ ID NO: 301, sites of amino acids other than X in SEQ ID NO: 552 are important for binding to IgE antibodies from allergic patients. Thus, in another preferred embodiment,embodiment, when the antigen in spot 4 contains a variation in the amino acid sequence of SEQ ID NO: 47, the antigen may have an amino acid sequence of SEQ ID NO: 47 in which amino acids 244-258 are SEQ ID NO: 552.

### (5) Antigen in spots 5 and 6

As the result of sequence identification of the antigen in spots 5 and 6 by mass spectroscopy, the amino acid sequences of SEQ ID NOs: 60-104 were detected.

Also, the mass spectroscopic data (SEQ ID NOs: 60-104) obtained for spots 5 and 6 on a mass spectrometer was analyzed by comparing the data against the NCBI protein data, and as a result, Vitellogenin-2 precursor (amino acid sequence: SEQ ID NO: 59, encoding nucleotide sequence: SEQ ID NO: 58) was identified.

Accordingly, the antigens in spots 5 and 6 in the present invention can be any of below in (5A-a) to (5A-e) and (5B):
(5A-a) a protein comprising an amino acid sequence with deletion, substitution, insertion or addition of one or several amino acids in SEQ ID NO: 59;
(5A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 59;
(5A-c) a protein comprising an amino acid sequence encoded by a nucleotide sequence with deletion, substitution, insertion or addition of one or several nucleotides in SEQ ID NO: 58;
(5A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 58;
(5A-e) a protein comprising an amino acid sequence encoded by a nucleic acid that hybridizes under stringent conditions with a nucleic acid having a nucleotide sequence complementary to the nucleotide sequence of SEQ ID NO: 58;
(5B) a protein comprising at least one amino acid sequence selected from the group consisting of SEQ ID NOs: 59-104, preferably a protein comprising at least 2, 3, 4, 5, 6, 7, 8, 9, 10, 15, 20, 25, 30, 35, 40 or 45 types or all sequences of the amino acid sequences. More preferably a protein comprising at least one amino acid sequence selected from the group consisting of SEQ ID NOs: 59, 60, 63, 65, 68, 69, 71, 73, 75-79, 81, 83-88, 91-94, 96-99, and 101-103, preferably a protein comprising at least 2, 3, 4, 5, 6, 7, 8, 9, 10, 15, 20, 25, or 30 types or all sequences of the amino acid sequences. As referred to above, the amino acid sequence of any of SEQ ID NOs: 59 to 104 may be an amino acid sequence derived therefrom by deletion, substitution, insertion or addition of one or several amino acids.

The proteins as defined above in (5A-a) to (5A-e) and (5B) can be proteins that are found in a protein spot with a molecular weight of 80 to 260 kDa, preferably around 90 to 200 kDa, more preferably 110 to 160 kDa, and an isoelectric point of 1.0 to 8.0, preferably 2.0 to 7.0, more preferably 3.0 to 6.0 on gels used in the two-dimensional electrophoresis performed under the conditions described above in the subsection titled "Identification of antigens".

Epitopes of the aforementioned antigens (5A-a) to (5A-e) and (5B) reside in amino acids 71-85 (SEQ ID NO: 272), amino acids 141-155 (SEQ ID NO: 273), amino acids 471-485 (SEQ ID NO: 274), amino acids 521-535 (SEQ ID NO: 275), amino acids 611-625 (SEQ ID NO: 276), amino acids 651-665 (SEQ ID NO: 277), amino acids 661-675 (SEQ ID NO: 278), amino acids 711-725 (SEQ ID NO: 279), amino acids 741-755 (SEQ ID NO: 280), amino acids 751-765 (SEQ ID NO: 281), amino acids 881-895 (SEQ ID NO: 282), amino acids 1011-1025 (SEQ ID NO: 283), amino acids 1071-1085 (SEQ ID NO: 284), and amino acids 1101-1115 (SEQ ID NO: 285) of SEQ ID NO: 59. When the antigen in spot 5 or 6 contains a variation in the amino acid sequence of SEQ ID NO: 59, amino acids corresponding to at least one of these epitopes may retain the sequence in SEQ ID NO: 59.

From the viewpoint that binding activity against IgE antibodies such as sensitivity or specificity remains even if the amino acid sequence is varied, the antigen in spots 5 and 6 may be the following variant.

In the epitope having the sequence of SEQ ID NO: 272, sites of amino acids other than X in SEQ ID NO: 320 or 321 are important for binding to IgE antibodies from allergic patients. Thus, in another preferred embodiment,embodiment, when the antigen in spot 5 or 6 contains a variation in the amino acid sequence of SEQ ID NO: 59, the antigen may have an amino acid sequence of SEQ ID NO: 59 in which amino acids 71-85 are SEQ ID NO: 320 or 321.

In the epitope having the sequence of SEQ ID NO: 273, sites of amino acids other than X in SEQ ID NO: 328 are important for binding to IgE antibodies from allergic patients. Thus, in another preferred embodiment,embodiment, when the antigen in spot 5 or 6 contains a variation in the amino acid sequence of SEQ ID NO: 59, the antigen may have an amino acid sequence of SEQ ID NO: 59 in which amino acids 141-155 are SEQ ID NO: 328.

In the epitope having the sequence of SEQ ID NO: 274, sites of amino acids other than X in SEQ ID NO: 332 or 337 are important for binding to IgE antibodies from allergic patients. Thus, in another preferred embodiment,embodiment, when the antigen in spot 5 or 6 contains a variation in the amino acid sequence of SEQ ID NO: 59, the antigen may have an amino acid sequence of SEQ ID NO: 59 in which amino acids 471-485 are SEQ ID NO: 332 or 337.

In the epitope having the sequence of SEQ ID NO: 275, sites of amino acids other than X in SEQ ID NO: 341, 348 or 352 are important for binding to IgE antibodies from allergic patients. Thus, in another preferred embodiment,embodiment, when the antigen in spot 5 or 6 contains a variation in the amino acid sequence of SEQ ID NO: 59, the antigen may have an amino acid sequence of SEQ ID NO: 59 in which amino acids 521-535 are SEQ ID NO: 341, 348 or 352.

In the epitope having the sequence of SEQ ID NO: 276, sites of amino acids other than X in SEQ ID NO: 358 are important for binding to IgE antibodies from allergic patients. Thus, in another preferred embodiment,embodiment, when the antigen in spot 5 or 6 contains a variation in the amino acid sequence of SEQ ID NO: 59, the antigen may have an amino acid sequence of SEQ ID NO: 59 in which amino acids 611-625 are SEQ ID NO: 358.

In the epitope having the sequence of SEQ ID NO: 277, sites of amino acids other than X in SEQ ID NO: 361, 361, 370 or 371 are important for binding to IgE antibodies from allergic patients. Thus, in another preferred embodiment, embodiment, when the antigen in spot 5 or 6 contains a variation in the amino acid sequence of SEQ ID NO: 59, the antigen may have an amino acid sequence of SEQ ID NO: 59 in which amino acids 651-665 are SEQ ID NO: 361, 361, 370 or 371.

In the epitope having the sequence of SEQ ID NO: 278, sites of amino acids other than X in SEQ ID NO: 376 are important for binding to IgE antibodies from allergic patients. Thus, in another preferred embodiment,embodiment, when the antigen in spot 5 or 6 contains a variation in the amino acid sequence of SEQ ID NO: 59, the antigen may have an amino acid sequence of SEQ ID NO: 59 in which amino acids 661-675 are SEQ ID NO: 376.

In the epitope having the sequence of SEQ ID NO: 279, sites of amino acids other than X in SEQ ID NO: 382, 383, 394, 395 or 403 are important for binding to IgE antibodies from allergic patients. Thus, in another preferred embodiment,embodiment, when the antigen in spot 5 or 6 contains a variation in the amino acid sequence of SEQ ID NO: 59, the antigen may have an amino acid sequence of SEQ ID NO: 59 in which amino acids 711-725 are SEQ ID NO: 382, 383, 394, 395 or 403.

In the epitope having the sequence of SEQ ID NO: 280, sites of amino acids other than X in SEQ ID NO: 408 are important for binding to IgE antibodies from allergic patients. Thus, in another preferred embodiment,embodiment, when the antigen in spot 5 or 6 contains a variation in the amino acid sequence of SEQ ID NO: 59, the antigen may have an amino acid sequence of SEQ ID NO: 59 in which amino acids 741-755 are SEQ ID NO: 408.

In the epitope having the sequence of SEQ ID NO: 281, sites of amino acids other than X in SEQ ID NO: 415 or 416 are important for binding to IgE antibodies from allergic patients. Thus, in another preferred embodiment, embodiment, when the antigen in spot 5 or 6 contains a variation in the amino acid sequence of SEQ ID NO: 59, the antigen may have an amino acid sequence of SEQ ID NO: 59 in which amino acids 751-765 are SEQ ID NO: 415 or 416.

In the epitope having the sequence of SEQ ID NO: 282, sites of amino acids other than X in SEQ ID NO: 420, 421, 427 or 428 are important for binding to IgE antibodies from allergic patients. Thus, in another preferred embodiment, when the antigen in spot 5 or 6 contains a variation in the amino acid sequence of SEQ ID NO: 59, the antigen may have an amino acid sequence of SEQ ID NO: 59 in which amino acids 881-895 are SEQ ID NO: 420, 421, 427 or 428.

In the epitope having the sequence of SEQ ID NO: 285, sites of amino acids other than X in SEQ ID NO: 431 or 432 are important for binding to IgE antibodies from allergic patients. Thus, in another preferred embodiment,embodiment, when the antigen in spot 5 or 6 contains a variation in the amino acid sequence of SEQ ID NO: 59, the antigen may have an amino acid sequence of SEQ ID NO: 59 in which amino acids 1101-1115 are SEQ ID NO: 431 or 432.

### (6) Antigen in spot 7

As the result of sequence identification of the antigen in spot 7 by mass spectroscopy, the amino acid sequences of SEQ ID NOs: 107-150 were detected.

Also, the mass spectroscopic data (SEQ ID NOs: 107-150) obtained for spot 7 on a mass spectrometer was analyzed by comparing the data against the NCBI protein data, and as a result, Vitellogenin-2 precursor (amino acid sequence: SEQ ID NO: 106, encoding nucleotide sequence: SEQ ID NO: 105) was identified.

Accordingly, the antigen in spot 7 in the present invention can be any of below in (6A-a) to (6A-e), and (6B):
(6A-a) a protein comprising an amino acid sequence with deletion, substitution, insertion or addition of one or several amino acids in SEQ ID NO: 106;
(6A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 106;
(6A-c) a protein comprising an amino acid sequence encoded by a nucleotide sequence with deletion, substitution, insertion or addition of one or several nucleotides in SEQ ID NO: 105;
(6A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 105;
(6A-e) a protein comprising an amino acid sequence encoded by a nucleic acid that hybridizes under stringent conditions with a nucleic acid having a nucleotide sequence complementary to the nucleotide sequence of SEQ ID NO: 105;
(6B) a protein comprising at least one amino acid sequence selected from the group consisting of SEQ ID NOs: 106-150, preferably a protein comprising at least 2, 3, 4, 5, 6, 7, 8, 9, 10, 15, 20, 25, 30, 35, 40 or 44 types or all sequences of the amino acid sequences. More preferably a protein comprising at least one amino acid sequence selected from the group consisting of SEQ ID NOs: 106, 108, 110, 111, 113, 114, 116, 117, 120-124, 126-131, 133, 134, 136-138, 140-143, 145-147 and 149, preferably a protein comprising at least 2, 3, 4, 5, 6, 7, 8, 9, 10, 15, 20, 25, 30, or 31 types or all sequences of the amino acid sequences. As referred to above, the amino acid sequence of any of SEQ ID NOs: 106 to 150 may be an amino acid sequence derived therefrom by deletion, substitution, insertion or addition of one or several amino acids.

The proteins as defined above in (6A-a) to (6A-e) and (6B) can be proteins that are found in a protein spot with a molecular weight of 110 to 300 kDa, preferably around 130 to 280 kDa, more preferably 160 to 260 kDa, and an isoelectric point of 3.0 to 8.0, preferably 3.5 to 7.0, more preferably 4.0 to 6.0 on gels used in the two-dimensional electrophoresis performed under the conditions described above in the subsection titled "Identification of antigens".

Epitopes of the aforementioned antigens (6A-a)-(6A-e) and (6B) reside in amino acids 71-85 (SEQ ID NO: 272), amino acids 141-155 (SEQ ID NO: 273), amino acids 471-485 (SEQ ID NO: 274), amino acids 521-535 (SEQ ID NO: 275), amino acids 611-625 (SEQ ID NO: 276), amino acids 651-665 (SEQ ID NO: 277), amino acids 661-675 (SEQ ID NO: 278), amino acids 711-725 (SEQ ID NO: 279), amino acids 741-755 (SEQ ID NO: 280), amino acids 751-765 (SEQ ID NO: 281), amino acids 881-895 (SEQ ID NO: 282), amino acids 1011-1025 (SEQ ID NO: 283), amino acids 1071-1085 (SEQ ID NO: 284), amino acids 1101-1115 (SEQ ID NO: 285), amino acids 1207-1221 (SEQ ID NO: 286), amino acids 1337-1351 (SEQ ID NO: 287), amino acids 1417-1431 (SEQ ID NO: 288), amino acids 1537-1551 (SEQ ID NO: 289), amino acids 1587-1601 (SEQ ID NO: 290), amino acids 1627-1641 (SEQ ID NO: 291), amino acids 1687-1701 (SEQ ID NO: 292), amino acids 1727-1741 (SEQ ID NO: 293), amino acids 1757-1771 (SEQ ID NO: 294), and amino acids 1797-1811 (SEQ ID NO: 295) of SEQ ID NO: 106. When the antigen in spot 7 contains a variation in the amino acid sequence of SEQ ID NO: 106, amino acids corresponding to at least one of these epitopes may retain the sequence in SEQ ID NO: 106.

From the viewpoint that binding activity against IgE antibodies such as sensitivity or specificity remains even if the amino acid sequence is varied, the antigen in spot 7 may be the following variant.

In the epitope having the sequence of SEQ ID NO: 272, sites of amino acids other than X in SEQ ID NO: 320 or 321 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 71-85 are SEQ ID NO: 320 or 321.

In the epitope having the sequence of SEQ ID NO: 273, sites of amino acids other than X in SEQ ID NO: 328 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 141-155 are SEQ ID NO: 328.

In the epitope having the sequence of SEQ ID NO: 274, sites of amino acids other than X in SEQ ID NO: 332 or 337 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 471-485 are SEQ ID NO: 332 or 337.

In the epitope having the sequence of SEQ ID NO: 275, sites of amino acids other than X in SEQ ID NO: 341, 348 or 352 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 521-535 are SEQ ID NO: 341, 348 or 352.

In the epitope having the sequence of SEQ ID NO: 276, sites of amino acids other than X in SEQ ID NO: 358 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 611-625 are SEQ ID NO: 358.

In the epitope having the sequence of SEQ ID NO: 277, sites of amino acids other than X in SEQ ID NO: 361, 361, 370 or 371 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 651-665 are SEQ ID NO: 361, 361, 370 or 371.

In the epitope having the sequence of SEQ ID NO: 278, sites of amino acids other than X in SEQ ID NO: 376 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 661-675 are SEQ ID NO: 376.

In the epitope having the sequence of SEQ ID NO: 279, sites of amino acids other than X in SEQ ID NO: 382, 383, 394, 395 or 403 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 711-725 are SEQ ID NO: 382, 383, 394, 395 or 403.

In the epitope having the sequence of SEQ ID NO: 280, sites of amino acids other than X in SEQ ID NO: 408 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 741-755 are SEQ ID NO: 408.

In the epitope having the sequence of SEQ ID NO: 281, sites of amino acids other than X in SEQ ID NO: 415 or 416 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 751-765 are SEQ ID NO: 415 or 416.

In the epitope having the sequence of SEQ ID NO: 282, sites of amino acids other than X in SEQ ID NO: 420, 421, 427 or 428 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 881-895 are SEQ ID NO: 420, 421, 427 or 428.

In the epitope having the sequence of SEQ ID NO: 285, sites of amino acids other than X in SEQ ID NO: 431 or 432 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 1101-1115 are SEQ ID NO: 431 or 432.

In the epitope having the sequence of SEQ ID NO: 286, sites of amino acids other than X in SEQ ID NO: 437, 441 or 442 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 1207-1221 are SEQ ID NO: 437, 441 or 442.

In the epitope having the sequence of SEQ ID NO: 287, sites of amino acids other than X in SEQ ID NO: 450 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 1337-1351 are SEQ ID NO: 450.

In the epitope having the sequence of SEQ ID NO: 288, sites of amino acids other than X in SEQ ID NO: 459 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 1417-1431 are SEQ ID NO: 459.

In the epitope having the sequence of SEQ ID NO: 290, sites of amino acids other than X in SEQ ID NO: 467 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 1587-1601 are SEQ ID NO: 467.

In the epitope having the sequence of SEQ ID NO: 291, sites of amino acids other than X in SEQ ID NO: 474, 475, 477, 478, 488 or 489 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 1627-1641 are SEQ ID NO: 474, 475, 477, 478, 488 or 489.

In the epitope having the sequence of SEQ ID NO: 292, sites of amino acids other than X in SEQ ID NO: 496, 501, 502 or 510 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 1687-1701 are SEQ ID NO: 496, 501, 502 or 510.

In the epitope having the sequence of SEQ ID NO: 293, sites of amino acids other than X in SEQ ID NO: 513 or 514 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 1727-1741 are SEQ ID NO: 513 or 514.

In the epitope having the sequence of SEQ ID NO: 294, sites of amino acids other than X in SEQ ID NO: 524 or 525 are important for binding to IgE antibodies from allergic patients. Thus, in another preferred embodiment,embodiment, when the antigen in spot 7 contains a variation in the amino acid sequence of SEQ ID NO: 106, the antigen may have an amino acid sequence of SEQ ID NO: 106 in which amino acids 1757-1771 are SEQ ID NO: 524 or 525.

### (7) Antigen in spot 8

As the result of sequence identification of the antigen in spot 8 by mass spectroscopy, the amino acid sequences of SEQ ID NOs: 153-235 were detected.

Also, the mass spectroscopic data (SEQ ID NOs: 153-235) obtained for spot 8 on a mass spectrometer was analyzed by comparing the data against the NCBI protein data, and as a result, Apolipoprotein B precursor (amino acid sequence: SEQ ID NO: 152, encoding nucleotide sequence: SEQ ID NO: 151) was identified.

Accordingly, the antigen in spot 8 in the present invention can be any of the proteins as defined below in (7A-a) to (7A-e) and (7B).
(7A-a) a protein comprising an amino acid sequence with deletion, substitution, insertion or addition of one or several amino acids in SEQ ID NO: 152;
(7A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 152;
(7A-c) a protein comprising an amino acid sequence encoded by a nucleotide sequence with deletion, substitution, insertion or addition of one or several nucleotides in SEQ ID NO: 151;
(7A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 151;
(7A-e) a protein comprising an amino acid sequence encoded by a nucleic acid that hybridizes under stringent conditions with a nucleic acid having a nucleotide sequence complementary to the nucleotide sequence of SEQ ID NO: 151;
(7B) a protein comprising at least one amino acid sequence selected from the group consisting of SEQ ID NOs: 152-235, preferably a protein comprising at least 2, 3, 4, 5, 6, 7, 8, 9, 10, 15, 20, 25, 30, 35, 40, 45, 50, 55, 60, 65, 70, 75, 80 or 83 or all sequences of the amino acid sequences. More preferably a protein comprising at least one amino acid sequence selected from the group consisting of SEQ ID NOs: 152, 154, 157-160, 163, 164, 167-172, 174, 175, 177, 178, 180, 182-185, 187, 189, 190, 192, 194-196, 198-200, 203-207, 209, 211, 212, 214-216, 220-229, 231 and 232, preferably a protein comprising at least 2, 3, 4, 5, 6, 7, 8, 9, 10, 15, 20, 25, 30, 35, 40, 45, 50, or 55 types or all sequences of the amino acid sequences. As referred to above, the amino acid sequence of any of SEQ ID NOs: 152 to 235 may be an amino acid sequence derived therefrom by deletion, substitution, insertion or addition of one or several amino acids.

The proteins as defined above in (7A-a) to (7A-e) and (7B) can be proteins that are found in a protein spot with a molecular weight of 160 to 550 kDa, preferably around 180 to 400 kDa, more preferably 200 to 300 kDa, and an isoelectric point of 3.0 to 8.0, preferably 3.5 to 7.0, more preferably 4.0 to 6.0 on gels used in the two-dimensional electrophoresis performed under the conditions described above in the subsection titled "Identification of antigens".

Epitopes of the aforementioned antigens (7A-a)-(7A-e) and (7B) reside in amino acids 2556-2570 (SEQ ID NO: 304), amino acids 131-145 (SEQ ID NO: 305), amino acids 631-645 (SEQ ID NO: 306), amino acids 681-695 (SEQ ID NO: 307), amino acids 841-855 (SEQ ID NO: 308), amino acids 1131-1145 (SEQ ID NO: 309), amino acids 1201-1215 (SEQ ID NO: 310), and amino acids 1571-1585 (SEQ ID NO: 311) of SEQ ID NO: 152. When the antigen in spot 8 contains a variation in the amino acid sequence of SEQ ID NO: 152, amino acids corresponding to at least one of these epitopes may retain the sequence in SEQ ID NO: 152.

From the viewpoint that binding activity against IgE antibodies such as sensitivity or specificity remains even if the amino acid sequence is varied, the antigen in spot 8 may be the following variant.

In the epitope having the sequence of SEQ ID NO: 304, sites of amino acids other than X in SEQ ID NO: 558 are important for binding to IgE antibodies from allergic patients. Thus, in another preferred embodiment,embodiment, when the antigen in spot 8 contains a variation in the amino acid sequence of SEQ ID NO: 152, the antigen may have an amino acid sequence of SEQ ID NO: 152 in which amino acids 2556-2570 are SEQ ID NO: 558.

In the epitope having the sequence of SEQ ID NO: 305, sites of amino acids other than X in SEQ ID NO: 563, 564, 574 or 575 are important for binding to IgE antibodies from allergic patients. Thus, in another preferred embodiment,embodiment, when the antigen in spot 8 contains a variation in the amino acid sequence of SEQ ID NO: 152, the antigen may have an amino acid sequence of SEQ ID NO: 152 in which amino acids 131-145 are SEQ ID NO: 563, 564, 574 or 575.

In the epitope having the sequence of SEQ ID NO: 306, sites of amino acids other than X in SEQ ID NO: 582 are important for binding to IgE antibodies from allergic patients. Thus, in another preferred embodiment,embodiment, when the antigen in spot 8 contains a variation in the amino acid sequence of SEQ ID NO: 152, the antigen may have an amino acid sequence of SEQ ID NO: 152 in which amino acids 631-645 are SEQ ID NO: 582.

In the epitope having the sequence of SEQ ID NO: 308, sites of amino acids other than X in SEQ ID NO: 590, 596 or 603 are important for binding to IgE antibodies from allergic patients. Thus, in another preferred embodiment,embodiment, when the antigen in spot 8 contains a variation in the amino acid sequence of SEQ ID NO: 152, the antigen may have an amino acid sequence of SEQ ID NO: 152 in which amino acids 841-855 are SEQ ID NO: 590, 596 or 603.

In the epitope having the sequence of SEQ ID NO: 309, sites of amino acids other than X in SEQ ID NO: 610, 611 or 612 are important for binding to IgE antibodies from allergic patients. Thus, in another preferred embodiment,embodiment, when the antigen in spot 8 contains a variation in the amino acid sequence of SEQ ID NO: 152, the antigen may have an amino acid sequence of SEQ ID NO: 152 in which amino acids 1131-1145 are SEQ ID NO: 610, 611 or 612.

In the epitope having the sequence of SEQ ID NO: 310, sites of amino acids other than X in SEQ ID NO: 620 or 621 are important for binding to IgE antibodies from allergic patients. Thus, in another preferred embodiment,embodiment, when the antigen in spot 8 contains a variation in the amino acid sequence of SEQ ID NO: 152, the antigen may have an amino acid sequence of SEQ ID NO: 152 in which amino acids 1201-1215 are SEQ ID NO: 620 or 621.

In the epitope having the sequence of SEQ ID NO: 311, sites of amino acids other than X in SEQ ID NO: 625 are important for binding to IgE antibodies from allergic patients. Thus, in another preferred embodiment,embodiment, when the antigen in spot 8 contains a variation in the amino acid sequence of SEQ ID NO: 152, the antigen may have an amino acid sequence of SEQ ID NO: 152 in which amino acids 1571-1585 are SEQ ID NO: 625.

### (8) Antigen in spot 9

As the result of sequence identification of the antigen in spot 9 by mass spectroscopy, the amino acid sequences of SEQ ID NOs: 238-249 were detected.

Also, the mass spectroscopic data (SEQ ID NOs: 238-249) obtained for spot 9 on a mass spectrometer was analyzed by comparing the data against the NCBI protein data, and as a result, Apolipoprotein B precursor (amino acid sequence: SEQ ID NO: 237, encoding nucleotide sequence: SEQ ID NO: 236) was identified.

Accordingly, the antigen in spot 9 in the present invention can be any of the proteins as defined below in (8A-a) to (8A-e) and (8B).
(8A-a) a protein comprising an amino acid sequence with deletion, substitution, insertion or addition of one or several amino acids in SEQ ID NO: 237;
(8A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 237;
(8A-c) a protein comprising an amino acid sequence encoded by a nucleotide sequence with deletion, substitution, insertion or addition of one or several nucleotides in SEQ ID NO: 236;
(8A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 236;
(8A-e) a protein comprising an amino acid sequence encoded by a nucleic acid that hybridizes under stringent conditions with a nucleic acid having a nucleotide sequence complementary to the nucleotide sequence of SEQ ID NO: 236;
(8B) a protein comprising at least one amino acid sequence selected from the group consisting of SEQ ID NOs: 237-249, preferably a protein comprising at least 2, 3, 4, 5, 6, 7, 8, 9, 10, 11 or 12 or all sequences of the amino acid sequences. More preferably a protein comprising at least one amino acid sequence selected from the group consisting of SEQ ID NOs: 237-239, 241, 242, 244, and 246-249, preferably a protein comprising at least 2, 3, 4, 5, 6, 7, 8, or 9 types or all sequences of the amino acid sequences. As referred to above, the amino acid sequence of any of SEQ ID NOs: 237 to 249 may be an amino acid sequence derived therefrom by deletion, substitution, insertion or addition of one or several amino acids.

The proteins as defined above in (8A-a) to (8A-e) and (8B) can be proteins that are found in a protein spot with a molecular weight of 20 to 50 kDa, preferably around 25 to 45 kDa, more preferably 30 to 40 kDa, and an isoelectric point of 7.0 to 11.0, preferably 8.0 to 10.0, more preferably 9.0 to 10.0 on gels used in the two-dimensional electrophoresis performed under the conditions described above in the subsection titled "Identification of antigens".

Epitopes of the aforementioned antigens (8A-a) to (8A-e) and (8B) reside in amino acids 131 to 145 (SEQ ID NO: 305) of SEQ ID NO: 237. When the antigen in spot 9 contains a variation in the amino acid sequence of SEQ ID NO: 237, amino acids corresponding to at least one of these epitopes may retain the sequence in SEQ ID NO: 237.

From the viewpoint that binding activity against IgE antibodies such as sensitivity or specificity remains even if the amino acid sequence is varied, the antigen in spot 9 may be the following variant.

In the epitope having the sequence of SEQ ID NO: 305, sites of amino acids other than X in SEQ ID NO: 563, 564, 574 or 575 corresponding to the positions of the amino acids at positions 5-13 in SEQ ID NO: 198 are important for binding to IgE antibodies from allergic patients. Thus, in another preferred embodiment,embodiment, when the antigen in spot 9 contains a variation in the amino acid sequence of SEQ ID NO: 237, the antigen may have an amino acid sequence of SEQ ID NO: 237 in which amino acids 131-145 are SEQ ID NO: 563, 564, 574 or 575.

### (9) Antigen in spot 10

As the result of sequence identification of the antigen in spot 10 by mass spectroscopy, the amino acid sequences of SEQ ID NOs: 252-260 were detected.

Also, the mass spectroscopic data (SEQ ID NOs: 252-260) obtained for spot 10 on a mass spectrometer was analyzed by comparing the data against the NCBI protein data, and as a result, apolipoprotein B precursor (amino acid sequence: SEQ ID NO: 251, encoding nucleotide sequence: SEQ ID NO: 250) was identified.

Accordingly, the antigen in spot 10 in the present invention can be any of below in (9A-a) to (9A-e), and (9B):
(9A-a) a protein comprising an amino acid sequence with deletion, substitution, insertion or addition of one or several amino acids in SEQ ID NO: 251;
(9A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 251;
(9A-c) a protein comprising an amino acid sequence encoded by a nucleotide sequence with deletion, substitution, insertion or addition of one or several nucleotides in SEQ ID NO: 250;
(9A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 250;
(9A-e) a protein comprising an amino acid sequence encoded by a nucleic acid that hybridizes under stringent conditions with a nucleic acid having a nucleotide sequence complementary to the nucleotide sequence of SEQ ID NO: 250;
(9B) a protein comprising at least one amino acid sequence selected from the group consisting of SEQ ID NOs: 251-260, preferably a protein comprising at least 2, 3, 4, 5, 6, 7, 8, or 9 types or all sequences of the amino acid sequences. More preferably a protein comprising at least one amino acid sequence selected from the group consisting of SEQ ID NOs: 251-254, 256, 257 and 260, preferably a protein comprising at least 2, 3, 4, 5, or 6 types or all sequences of the amino acid sequences. As referred to above, the amino acid sequence of any of SEQ ID NOs: 251 to 260 may be an amino acid sequence derived therefrom by deletion, substitution, insertion or addition of one or several amino acids.

The proteins as defined above in (9A-a) to (9A-e) and (9B) can be proteins that are found in a protein spot with a molecular weight of 20 to 50 kDa, preferably around 25 to 45 kDa, more preferably 30 to 40 kDa, and an isoelectric point of 7.0 to 11.0, preferably 8.0 to 10.0, more preferably 9.0 to 10.0 on gels used in the two-dimensional electrophoresis performed under the conditions described above in the subsection titled "Identification of antigens".

### (10) Antigen in spot 11

As the result of sequence identification of the antigen in spot 11 by mass spectroscopy, the amino acid sequences of SEQ ID NOs: 263-271 were detected.

Also, the mass spectroscopic data (SEQ ID NOs: 263-271) obtained for spot 11 on a mass spectrometer was analyzed by comparing the data against the NCBI protein data, and as a result, vitellogenin-2 precursor (amino acid sequence: SEQ ID NO: 262, encoding nucleotide sequence: SEQ ID NO: 261) was identified.

Accordingly, the antigen in spot 11 in the present invention can be any of below in (10A-a) to (10A-e), and (10B):
(10A-a) a protein comprising an amino acid sequence with deletion, substitution, insertion or addition of one or several amino acids in SEQ ID NO: 262;
(10A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 262;
(10A-c) a protein comprising an amino acid sequence encoded by a nucleotide sequence with deletion, substitution, insertion or addition of one or several nucleotides in SEQ ID NO: 261; (10A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 261;
(10A-e) a protein comprising an amino acid sequence encoded by a nucleic acid that hybridizes under stringent conditions with a nucleic acid having a nucleotide sequence complementary to the nucleotide sequence of SEQ ID NO: 261;
(10B) a protein comprising at least one amino acid sequence selected from the group consisting of SEQ ID NOs: 262-271, preferably a protein comprising at least 2, 3, 4, 5, 6, 7, 8, or 9 types or all sequences of the amino acid sequences, more preferably a protein comprising at least one amino acid sequence selected from the group consisting of SEQ ID NOs: 262-265, 268 and 269, preferably a protein comprising at least 2, 3, 4, or 5 types or all sequences of the amino acid sequences. As referred to above, the amino acid sequence of any of SEQ ID NOs: 262 to 271 may be an amino acid sequence derived therefrom by deletion, substitution, insertion or addition of one or several amino acids.

The proteins as defined above in (10A-a) to (10A-e) and (10B) can be proteins that are found in a protein spot with a molecular weight of 15 to 40 kDa, preferably around 17 to 35 kDa, more preferably 20 to 30 kDa, and an isoelectric point of 6.0 to 11.0, preferably 7.0 to 10.0, more preferably 8.0 to 10.0 on gels used in the two-dimensional electrophoresis performed under the conditions described above in the subsection titled "Identification of antigens".

Epitopes of the aforementioned antigens (10A-a) to (10A-e) and (10B) reside in amino acids 41-55 (SEQ ID NO: 288), amino acids 161-175 (SEQ ID NO: 289), and amino acids 211-255 (SEQ ID NO: 290) of SEQ ID NO: 262. When the antigen in spot 11 contains a variation in the amino acid sequence of SEQ ID NO: 262, amino acids corresponding to at least one of these epitopes may retain the sequence in SEQ ID NO: 262.

From the viewpoint that binding activity against IgE antibodies such as sensitivity or specificity remains even if the amino acid sequence is varied, the antigen in spot 11 may be the following variant.

In the epitope having the sequence of SEQ ID NO: 288, sites of amino acids other than X in SEQ ID NO: 459 are important for binding to IgE antibodies from allergic patients. Thus, in another preferred embodiment,embodiment, when the antigen in spot 11 contains a variation in the amino acid sequence of SEQ ID NO: 262, the antigen may have an amino acid sequence of SEQ ID NO: 262 in which amino acids 41-55 are SEQ ID NO: 459.

In the epitope having the sequence of SEQ ID NO: 290, sites of amino acids other than X in SEQ ID NO: 467 are important for binding to IgE antibodies from allergic patients. Thus, in another preferred embodiment,embodiment, when the antigen in spot 11 contains a variation in the amino acid sequence of SEQ ID NO: 262, the antigen may have an amino acid sequence of SEQ ID NO: 262 in which amino acids 211-255 are SEQ ID NO: 467.

The proteins that are the aforementioned antigens (4) to (10) include those proteins whose amino acid residues are modified by phosphorylation, sugar chain modification, aminoacylation, ring-opening, deamination or the like.

The proteins that are the aforementioned antigens (4) to (10) are preferably antigens of an allergy to egg.

By stating herein "deletion, substitution, insertion or addition of one or several amino acids" in relation to amino acid sequence, it is meant that in an amino acid sequence of interest, one or several amino acids (e.g., 30%, preferably 25%, 20%, 15%, 10%, 5%, 3%, 2% or 1%, of amino acids with respect to the total length of the amino acid sequence) are deleted, one or several amino acids are substituted by any other amino acids, any other amino acids are inserted, and/or any other amino acids are added.

Among the aforementioned modifications, substitution is preferably conservative substitution. The "conservative substitution" refers to the substitution of a certain amino acid residue by a different amino acid residue having similar physicochemical characteristics, and can be any type of substitution as long as it does not substantially change the characteristics of the structure of the original sequence for example, any type of substitution is acceptable as long as any substituted amino acids do not disrupt the helical structure of the original sequence or other secondary structures that characterize the original sequence. The following gives examples of separate groups of amino acid residues that are conservatively substitutable with each other, but substitutable amino acid residues are not limited to the examples given below.
Group A: leucine, isoleucine, valine, alanine, methionine
Group B: aspartic acid, glutamic acid
Group C: asparagine, glutamine
Group D: lysine, arginine
Group E: serine, threonine
Group F: phenylalanine, tyrosine

In the case of non-conservative substitution, one member belonging to one of the aforementioned groups can be replaced with a member belong to any other group. For example, in order to eliminate the possibility of unwanted sugar-chain modification, amino acids of group B, D or E as listed above may be substituted by those of any other group. Also, cysteines may be deleted or substituted by any other amino acids to prevent them from being folded into a protein in its tertiary structure. Also, in order to maintain the balance between hydrophilicity and hydrophobicity or to increase hydrophilicity for the purpose of facilitating synthesis, any amino acids may be substituted in consideration of the hydropathy scales of amino acids, which are a measure of the hydrophilic and hydrophobic properties of amino acids (J. Kyte and R. Doolittle, J. Mol. Biol., Vol. 157, p. 105-132, 1982).

In another embodiment,embodiment, substitution of the original amino acid by an amino acid with less steric hindrance, for example, substitution of group F by group A, B, C, D or E; or substitution of an amino acid having an electric charge by an amino acid having no electric charge, for example, substitution of group B by group C, may be performed. This may improve binding activity against IgE antibodies.

As referred to herein, the percent identity between two amino acid sequences can be determined by visual inspection and mathematical calculation. Alternatively, the percent identity can be determined using a computer program. Examples of such computer programs include BLAST and ClustalW. In particular, various conditions (parameters) for identity searches with the BLAST program are described in Altschul, et al. (Nucl. Acids. Res., 25, p.3389-3402, 1997), and are publicly available on the websites of the National Center for Biotechnology Information (NCBI) and DNA Data Bank of Japan (DDBJ) (Altschul, et al., BLAST Handbook, Altschul, et al., NCB/NLM/NIH Bethesda, MD 20894). Also, the percent identity can be determined using a genetic information processing software program, such as GENETYX Ver.7 (Genetyx Corporation), DNASIS Pro (Hitachi Software Engineering Co., Ltd.), or Vector NTI (Infomax Inc.).

By stating herein "deletion, substitution, insertion or addition of one or several nucleotides" in relation to nucleotide sequence, it is meant that in a nucleotide sequence of interest, one or several nucleotides (e.g., 30%, preferably 25%, 20%, 15%, 10%, 5%, 3%, 2% or 1%, of amino acid with respect to the total length of the nucleotide sequence, or e.g., 1, 5, 10, 15, 20, 25 or 30 nucleotides) are deleted, one or several nucleotides are substituted by any other nucleotides, any other nucleotides are inserted, and/or any other nucleotides are added. It is preferable that such a nucleotide deletion, substitution, insertion or addition should not give rise to a frame shift in an amino acid coding sequence.

As referred to herein, the percent identity between two nucleotide sequences can be determined by visual inspection and mathematical calculation. Alternatively, the percent identity can be determined using a computer program. The BLASTN program (Altschul et al., (1990) J. Mol. Biol. 215: 403-10) available on the website: https://blast.ncbi.nlm.nih.gov/Blast.cgi of the National Library of Medicine can be used as such a sequence comparison computer program, and the percent identity can be determined using default parameters.

As referred to herein, "under stringent conditions" means that hybridization takes place under moderately or highly stringent conditions. To be specific, the moderately stringent conditions can be easily determined by those having ordinary skill in the art on the basis of, for example, the length of DNA. Basic conditions are described in Sambrook, et al., Molecular Cloning: A Laboratory Manual, 3rd ed., ch.6-7, Cold Spring Harbor Laboratory Press, 2001. The moderately stringent conditions include hybridization under the conditions of preferably 1×SSC to 6×SSC at 42°C to 55°C, more preferably 1×SSC to 3×SSC at 45°C to 50°C, most preferably 2×SSC at 50°C. In the case of using a hybridization solution containing, for example, about 50% formamide, a temperature around 5 to 15°C lower than the foregoing should be adopted. Washing is also carried out under the conditions of 0.5×SSC to 6×SSC at 40°C to 60°C. In the process of hybridization and washing, generally 0.05% to 0.2% SDS, preferably about 0.1% SDS, may be added. Likewise, the highly stringent conditions can be easily determined by those having ordinary skill in the art on the basis of, for example, the length of DNA. Generally, the highly stringent (high stringent) conditions include hybridization and/or washing at a higher temperature and/or a lower salt concentration than those adopted under the moderately stringent conditions. For example, hybridization is carried out under the conditions of 0.1×SSC to 2×SSC at 55°C to 65°C, more preferably 0.1×SSC to 1×SSC at 60°C to 65°C, most preferably 0.2×SSC at 63°C. Washing is carried out under the conditions of 0.2×SSC to 2×SSC at 50°C to 68°C, more preferably 0.2×SSC at 60 to 65°C.

Antigens may be obtained by separating and purifying them from eggs (preferably chicken eggs) using a combination of protein purification methods well known to those skilled in the art. Also, antigens may be obtained by expressing them as recombinant proteins using a genetic recombination technique well known to those skilled in the art and by separating and purifying them using protein purification methods well known to those skilled in the art.

Exemplary protein purification methods include: solubility-based purification methods such as salt precipitation and solvent precipitation; purification methods based on molecular weight difference, such as dialysis, ultrafiltration, gel filtration and SDS-PAGE; charge-based purification methods such as ion exchange chromatography and hydroxylapatite chromatography; specific affinity-based purification methods such as affinity chromatography; purification methods based on hydrophobicity difference, such as reverse-phase high-performance liquid chromatography; and purification methods based on isoelectric point difference, such as isoelectric focusing.

Preparation of a protein by a genetic recombination technique is carried out by preparing an expression vector comprising an antigen-encoding nucleic acid, introducing the expression vector into appropriate host cells by gene transfer or genetic transformation, culturing the host cells under suitable conditions for expression of a recombinant protein, and recovering the recombinant protein expressed in the host cells.

The "vector" refers to a nucleic acid that can be used to introduce a nucleic acid attached thereto into host cells. The "expression vector" is a vector that can induce the expression of a protein encoded by a nucleic acid introduced therethrough. Exemplary vectors include plasmid vectors and viral vectors. Those skilled in the art can select an appropriate expression vector for the expression of a recombinant protein depending on the type of host cells to be used.

The "host cells" refers to cells that undergo gene transfer or genetic transformation by a vector. The host cells can be appropriately selected by those skilled in the art depending on the type of the vector to be used. The host cells can be derived from prokaryotes such as E. coli. When prokaryotic cells like E. coli are used as host cells, the antigen of the present invention may be designed to contain an N-terminal methionine residue in order to facilitate the expression of a recombinant protein in the prokaryotic cells. The N-terminal methionine can be cleaved from the recombinant protein after expression. Also, the host cells may be cells derived from eukaryotes, such as single-cell eukaryotes like yeast, plant cells and animal cells (e.g., human cells, monkey cells, hamster cells, rat cells, murine cells or insect cells) or silkworm.

Gene transfer or genetic transformation of an expression vector into host cells can be carried out as appropriate by following a technique known to those skilled in the art. Those skilled in the art can make possible the expression of a recombinant protein by selecting suitable conditions for the expression of the recombinant protein as appropriate depending on the type of host cells and culturing the host cells under the selected conditions. Then, those skilled in the art can homogenize the host cells having the expressed recombinant protein, and separate and purify an antigen expressed as the recombinant protein from the resulting homogenate by using an appropriate combination of such protein purification methods as mentioned above.

### Diagnosis kit and method (1)

The present invention provides a method for providing an indicator for diagnosing an allergy to eggs in a subject, the method comprising the steps of:
(i) contacting a sample obtained from the subject with an antigen, wherein the sample is a solution comprising an IgE antibody;
(ii) detecting binding between an IgE antibody present in the sample from the subject and the antigen; and
(iii) when the binding between the IgE antibody in the subject and the antigen is detected, an indicator of the fact that the subject is allergic to eggs is provided;
wherein the antigen is at least one of proteins as defined above in any of (4) to (10).

The sample obtained from a subject is a solution containing an IgE antibody, as collected from the subject. Examples of such solutions include blood, saliva, sputum, snivel, urine, sweat, and tear. The sample obtained from the subject may be subjected to pretreatment for increasing the concentration of an IgE antibody in the sample before being contacted with an antigen. The pretreatment of a sample may involve, for example, collection of the serum or the plasma from the blood. Furthermore, a Fab moiety that is an antigen-binding moiety may be purified. In a particularly preferred embodiment,embodiment, the step (i) mentioned above is carried out by contacting an IgE antibody present in the serum obtained from a subject with an antigen.

The IgE antibody may be the IgE antibody itself or may be mast cells or the like bound to IgE antibodies.

Detection of contact and binding between the sample obtained from a subject and an antigen can be carried out by using a known method. Examples of such methods that can be used include detection by ELISA (Enzyme-Linked Immunosorbent Assay), sandwich immunoassay, immunoblotting, immunoprecipitation, or immunochromatography. These are all techniques for detecting the binding between an antigen and an IgE antibody from a subject by contacting and binding the IgE antibody from a subject with the antigen, allowing an enzymatically labelled secondary antibody to act on the IgE antibody specifically bound to the antigen, and adding an enzyme substrate (generally, chromogenic or luminescent reagent) to detect an enzymatic reaction product. Alternatively, they are methods of detecting a fluorescently labeled secondary antibody. Alternatively, detection by a measurement method capable of evaluating binding between an antigen and an IgE antibody, such as surface plasmon resonance (SPR), can also be used. A plurality of antigen-specific IgE antibodies may be mixed.

The antigen may be provided as an isolated antigen in a state immobilized on a carrier. In this case, the steps (i) and (ii) mentioned above can be carried out using ELISA, sandwich immunoassay, immunochromatography, surface plasmon resonance, or the like. Also, the step (i) mentioned above can be carried out by contacting the sample obtained from a subject with an antigen-immobilized surface. The isolated antigen may be obtained by separating and purifying it from eggs (preferably chicken eggs) using a combination of protein purification methods well known to those skilled in the art, or by preparing it using a genetic recombination technique. Alternatively, the antibody may be used in a state immobilized on a carrier.

The antigen may be in a state unimmobilized on a carrier. In this case, flow cytometry or the like can be used in the aforementioned steps (i) and (ii), and the presence of the antigen bound to the antibody can be confirmed with laser beam. Examples of this method include a basophil activation test (BAT). Another example includes a histamine release test (HRT) which examines whether histamine is released by further contacting the antigen with blood cells in a sample.

The antigen may be detected by immunoblotting method by transferring from a state separated by two-dimensional electrophoresis. The two-dimensional electrophoresis is a technique for separating a protein sample by performing isoelectric focusing in the first dimension and performing SDS-PAGE (SDS-polyacrylamide gel electrophoresis) in the second dimension. The conditions for two-dimensional electrophoresis are not particularly limited as long as the conditions permit the separation of the antigen of the present invention. For example, the conditions for two-dimensional electrophoresis as described above in the subsection titled "Identification of antigens" can be adopted. Also, the electrophoresis conditions may be defined by reference to the descriptions in Patent Literatures 1 to 4 mentioned above. For example, two-dimensional electrophoresis can be carried out under the conditions that satisfy at least one selected from the group consisting of the following requirements:
(A) the isoelectric focusing gels used in the first dimension should have a gel-strip length of 5 to 10 cm and a gel pH range of 3 to 10, and the pH gradient of the gels in the direction of electrophoresis should be as follows: where the gel-strip length up to pH 5 is taken as "a", that length from pH 5 to 7 as "b", and that length above pH 7 as "c", the relations "a < b" and "b > c" are satisfied;
(B) in the case of (A), when the total gel-strip length is taken as 1, "a" should be in the range of 0.15 to 0.3, "b" should be in the range of 0.4 to 0.7, and "c" should be in the range of 0.15 to 0.3;
(C) in the first dimensional isoelectric focusing, a constant voltage step should be performed by applying a constant voltage ranging from 100 V to 600 V per gel strip containing a sample, and after the electrophoresis variation width during electrophoresis for 30 minutes falls within the range of 5 µA, a voltage-increasing step should be started at which the voltage is increased from the aforementioned constant voltage;
(D) in the case of (C), the final voltage at the voltage-increasing step should be in the range of 3000 V to 6000 V;
(E) the isoelectric focusing gels used in the first dimension should have a longitudinal gel-strip length of 5 to 10 cm, and the electrophoresis gels used in the second dimension should have a gel concentration at the distal end in the direction of electrophoresis, which is in the range of 3 to 6%; and
(F) in the case of (E), the electrophoresis gels used in the second dimension should have a gel concentration at the proximal end in the direction of electrophoresis, which is set to a higher value than that at the distal end in the direction of electrophoresis.

The aforementioned antigens (4) to (10) are antigens that specifically bind to IgE antibodies from patients with allergy to eggs. Therefore, when binding between an IgE antibody from a subject and the antigen is detected, an indicator of the fact that the subject is allergic to eggs is provided.

The present invention further provides a kit for diagnosing an allergy to eggs, comprising at least one of the aforementioned antigens (4) to (10). The diagnosis kit of this invention may be used in the aforementioned method for providing an indicator for diagnosing an allergy to eggs or in a diagnosis method as described later. The diagnosis kit of this invention may comprise not only the at least one of the aforementioned antigens (4) to (10), but also an anti-IgE antibody labeled with an enzyme and a chromogenic or luminescent substrate serving as a substrate for the enzyme. Also, a fluorescent-labeled anti-IgE antibody may be used. In the diagnosis kit of this invention, the antigen may be provided in a state immobilized on a carrier. The diagnosis kit of this invention may also be provided together with instructions on the procedure for diagnosis or a package containing said instructions.

In another embodiment,embodiment, the aforementioned diagnosis kit comprises a companion diagnostic agent for an allergy to eggs. The companion diagnostic agent is used for identifying patients expected to respond to pharmaceutical products or identifying patients having the risk of severe adverse reactions to pharmaceutical products, or for studying the reactivity of pharmaceutical products in order to optimize treatment using the pharmaceutical products. Here, the optimization of treatment includes, for example, determination of dosage and administration, judgment regarding discontinuation of administration, and confirmation of an allergen component that is used to cause immunological tolerance.

The present invention further provides a composition for diagnosing an allergy to eggs, comprising at least one of the aforementioned antigens (4) to (10). The diagnosis composition of this invention can be used in a diagnosis method as described below. The diagnosis composition of this invention may further comprise a pharmaceutically acceptable carrier and/or additives commonly used with the antigen of this invention depending on the need.

In one embodiment,embodiment, the present invention provides a method for diagnosing an allergy to eggs in a subject, the method comprising:
(i) contacting a sample obtained from the subject with an antigen;
(ii) detecting binding between an IgE antibody present in the sample from the subject and the antigen; and
(iii) when the binding between the IgE antibody in the subject and the antigen is detected, diagnosing the subject as being allergic to eggs;
wherein the antigen is at least one of proteins as defined above in any of (4) to (10). In this method, the steps (i) and (ii) are performed as described above regarding the corresponding steps of the method for providing an indicator for diagnosing an allergy to eggs.

In another embodiment,embodiment, the present invention provides a method for diagnosing an allergy to eggs in a subject, the method comprising administering to the subject at least one of the aforementioned antigens (4) to (10). This method may be performed in the form of a skin test characterized by applying the antigen onto the skin. Examples of the skin test include various forms of tests, such as: a prick test in which a diagnosis composition is applied onto the skin and then a tiny prick to such an extent as not to provoke bleeding is made in the skin to allow an antigen to penetrate the skin, thereby observing a skin reaction; a scratch test in which a diagnosis composition is applied onto the skin and then the skin is lightly scratched to observe a reaction; a patch test in which a diagnosis composition in the form of cream, ointment, etc. is applied onto the skin to observe a reaction; and an intracutaneous test in which an antigen is administered intracutaneously to observe a reaction. If a skin reaction such as swelling occurs in a skin portion to which the antigen has been applied, the subject of interest is diagnosed as having an allergy to eggs. The amount of the antigen to be applied to the skin in such tests can be, for example, not more than 100 µg per dose.

In the process of allergy diagnosis, a load test aiming to identify an antigen is often adopted. At least one of the aforementioned antigens (4) to (10) can be used as an active ingredient for a load test to diagnose an allergy to eggs. Here, the antigen protein to be used in the load test may be a protein that has been expressed and purified and may be a protein that has been expressed in food or cooking ingredients, such as rice-based vaccine expressing pollen allergens which are obtained by transforming rice with a gene of a cedar pollen antigen and expressing the antigen protein in the rice.

In yet another embodiment,embodiment, the present invention provides at least one of the aforementioned antigens (4) to (10), intended for use in the diagnosis of an allergy to eggs. This also includes the provision of at least one of the aforementioned antigens (4) to (10) mixed with a known antigen.

In still another embodiment,embodiment, the present invention provides use of at least one of the aforementioned antigens (4) to (10) for the production of a diagnostic agent for an allergy to eggs.

### Pharmaceutical composition and treatment method (1)

The present invention provides a pharmaceutical composition comprising at least one of the aforementioned antigens (4) to (10).

In one embodiment,embodiment, the aforementioned pharmaceutical composition is used for the treatment and diagnosis of an allergy to eggs. The treatment of an allergy increases the limit amount of an antigen in which the allergy does not develop even if the antigen is incorporated into the body, and finally aims for the state where the allergy does not develop by the common amount of the antigen to be consumed (remission).

The present invention also provides a method for treating an allergy to eggs, the method comprising administering at least one of the aforementioned antigens (4) to (10) to a patient in need of a treatment for an allergy to eggs.

In another embodiment,embodiment, the present invention provides at least one of the aforementioned antigens (4) to (10), intended for use in the treatment for an allergy to eggs. In yet another embodiment, embodiment, the present invention provides use of at least one of the aforementioned antigens (4) to (10) for the production of a therapeutic agent for an allergy to eggs.

In the process of allergy treatment, a hyposensitization therapy aiming to induce immunological tolerance by administering an antigen to a patient is often adopted. The at least one of the aforementioned antigens (4) to (10) can be used as an active ingredient for a hyposensitization therapy for an allergy to eggs. Here, the antigen protein to be used in the hyposensitization therapy may be a protein that has been expressed and purified and may be a protein that has been expressed in food or cooking ingredients, such as rice-based vaccine expressing pollen allergens which are obtained by transforming rice with a gene of a cedar pollen antigen and expressing the antigen protein in the rice.

The pharmaceutical composition of the present invention can be administered by common administration routes. Examples of common administration routes include oral, sublingual, percutaneous, intracutaneous, subcutaneous, intravascular, intranasal, intramuscular, intraperitoneal, and intrarectal administrations.

The pharmaceutical composition of the present invention can be used as a pharmaceutical composition to which a commonly used pharmaceutically acceptable adjuvant or excipient or any other additives (e.g., stabilizer, solubilizer, emulsifier, buffer, preservative, colorant) are added by a conventional method together with the antigen of this invention depending on the need. The dosage form of the pharmaceutical composition can be selected by those skilled in the art as appropriate depending on the administration route. The pharmaceutical composition can be in the form of, for example, tablet, capsule, troche, sublingual tablet, parenteral injection, intranasal spray, poultice, solution, cream, lotion, or suppository. The administration dose, frequency and/or period of the pharmaceutical composition of this invention can be selected by a physician as appropriate depending on the administration route and the patient's condition and characteristics such as age and body weight. For example, the pharmaceutical composition may be administered to an adult patient at a dose of not more than 100 µg per dose. The administration interval can be, for example, once daily, once weekly, twice weekly, once per three months or so. The administration period can be, for example, several weeks to several years. The pharmaceutical composition may be administered in such a manner that the dose is increased in incremental steps over the administration period.

### Tester (1)

The present invention provides a tester comprising an antibody for at least one of the aforementioned antigens (4) to (10).

The antibody can be prepared by a conventional method. For example, the antibody may be prepared by immunizing a mammal such as rabbit with one of the aforementioned antigens (4) to (10). The antibody may be an IgE antibody, a polyclonal antibody, a monoclonal antibody, or an antigen-binding fragment thereof (e.g., Fab, F(ab')₂, Fab').

Further, in the aforementioned tester, the antibody may be provided in a form bound to a carrier. The type of the carrier is not particularly limited as long as it is usable for detection of binding between an antibody and an antigen. Any given carrier known to those skilled in the art can be used.

Examples of a method for determining the presence or absence of an antigen include the following methods:
a method in which a prepared tester comprising an IgE antibody is contacted with a sample obtained from a food, a cooking ingredient, etc., ELISA or the like method is used to detect whether there is a binding between the IgE antibody and an antigen in the sample, and if the binding between the IgE antibody and the antigen is detected, it is determined that the antibody remains in the food, the cooking ingredient, etc. of interest; and
a method in which filter paper is impregnated with a food or a cooking ingredient and reacted with an antibody solution so as to detect an antigen contained therein.

Another embodiment of the present invention includes a tester for determining the presence or absence of an antigen of an allergy to eggs in an object of interest, the tester comprising a primer having a nucleotide sequence complementary to a portion of at least one of the nucleotide sequences of SEQ ID NOs: 46, 58, 105, 151, 236, 250 and 261. The primer has, for example, but not limited to, a nucleotide sequence complementary to, preferably, 12 residues, 15 bases, 20 bases, or 25 bases, in a 3'-terminal portion or central sequence in a sequence of at least one of the nucleotide sequences of SEQ ID NOs: 46, 58, 105, 151, 236, 250 and 261. Particularly, when mRNA is of interest, the tester has a complementary primer of a poly-A tail. In a preferred embodiment,embodiment, the tester comprising the primer mentioned above may further comprise a primer comprising a 5'-terminal nucleotide sequence, preferably a nucleotide sequence of 12 bases, 15 bases, 20 bases, or 25 bases, of at least one of the nucleotide sequences of SEQ ID NOs: 46, 58, 105, 151, 236, 250 and 261.

For example, DNA or cDNA is amplified by PCR (Polymerase Chain Reaction) including RT-PCR using templated DNA or mRNA obtained from an egg or a bird and the aforementioned complementary primer, and the sequence of the amplified cDNA is compared with the nucleotide sequence of SEQ ID NO: 46, 58, 105, 151, 236, 250 or 261 to determine the presence or absence of the antigen. Amplification methods by PCR can be exemplified by RACE. In this respect, even if there exists a point mutation encoding the same amino acid in the comparison of the amplified cDNA with the nucleotide sequence of SEQ ID NO: 46, 58, 105, 151, 236, 250 or 261, or even if the nucleotide sequence of the amplified cDNA has insertion, deletion, substitution or addition of bases in the nucleotide sequence of SEQ ID NO: 46, 58, 105, 151, 236, 250 or 261, it is determined that the antigen is present when the amino acid sequence encoded by the cDNA has at least 70%, preferably at least 80, 90, 95, 98, or 99% identity to the amino acid sequence of SEQ ID NO: 47, 59, 106, 152, 237, 251 or 262.

In one embodiment,embodiment, the aforementioned tester is used to determine the presence or absence of an antigen in cooking ingredients (an egg or a bird) or in products of interest in a food production line. The tester may also be used for quality inspection of production lines and pre-shipment products by manufacturers, or may be used for self-checking of the presence or absence of an antigen in a food or cooking ingredients of interest by consumers.

### Antigen-free food and the like (1)

The present invention provides an egg or a processed product of egg in which at least one of the aforementioned antigens (4) to (10) is eliminated or reduced, or a bird that delivers such egg or is born from such egg.

The method for eliminating or reducing the antigen of the present invention in an egg or processed products of egg, a bird that delivers such egg or is born from such egg is not limited. The elimination or reduction of the antigen may be conducted by any method, as long as the method permits the elimination or reduction of the antigen.

For example, the egg of the present invention whose antigen is eliminated or reduced may be obtained by preparing an egg in which the expression of the antigen of the present invention is knocked out, using a gene knock-out technique.

Any technique known to those skilled in the art can be used as the gene knock-out technique. For example, Oishi, et al. (Scientific Reports, Vol. 6, Article number: 23980, 2016, doi:10.1038/srep23980) states that individuals with ovomucoid gene deletion are obtained by applying a genome editing technique CRISPER/Cas9 to primordial germ cells of chickens. The egg of the present invention whose antigen is eliminated may be obtained through the use of a similar technique. The bird or the egg of the present invention whose antigen is eliminated or reduced may be obtained by mating through artificial insemination with birds or eggs containing no antigen or containing the antigen in a small amount. The artificial crossing of birds or eggs can be performed by a conventional method.

The processed products of egg of the present invention in which an antigen is eliminated or reduced may be a processed product using the egg of the present invention in which an antigen is eliminated or reduced as a source ingredient. In the case of using an ordinary egg as a source ingredient, a treatment for removing or reducing the antigen of this invention is performed before or after preparation of a processed product of egg. The methods for removing or reducing the antigen of the present invention in the processed products of egg which assumed an ordinary egg raw material include a method to remove protein component in food or a cooking ingredient such as a high pressure treatment and elution with the neutral salt solution or the high temperature steam, and a method to perform hydrolysis, denaturation, or amino acid alteration (chemical modification, elimination, or the like of a side chain) by heat treatment and acid treatment.

The bird of the present invention whose antigen is eliminated or reduced may be a bird grown from hatched eggs in which the aforementioned antigen of the present invention is eliminated or reduced. The bird includes each stage of growth of baby bird, child bird, young bird, adult bird and aged bird. As referred to herein, the bird means an animal belonging to avian species, preferably a chicken.

### Method for producing antigen-free processed product (1)

The present invention provides a method for producing a processed product of egg in which an antigen is eliminated or reduced, the method comprising the step of confirming that the antigen is eliminated or reduced, in a production process of the processed product, wherein the antigen is at least one of the aforementioned antigens (4) to (10).

The step of confirming that the antigen is eliminated or reduced, in a production process of the processed product of egg in which an antigen is eliminated or reduced may be performed by confirming the presence or absence of an antigen by the method described above in the subsection titled "Tester (1)".

The production of the processed product of egg in which an antigen is eliminated or reduced may be performed by the method described above in the subsection titled "Antigen-free food and the like (1)".

### Epitope of antigen

Epitopes and amino acids important for binding activity against IgE antibodies from allergic patients within the epitopes were identified as shown in Example 6 as to antigens identified as shown in Examples 2 to 5.

The present invention provides polypeptides of the following (1β) to (4β) as polypeptides comprising an amino acid sequence specifically binding to an IgE antibody from an allergic patient.

### (1β) Epitope of vitellogenin-2

In the present invention, the polypeptide (1β) can be any polypeptide selected from the group consisting of (1β-1) to (1β-21) as defined below:
(1β-1) a polypeptide comprising at least one amino acid sequence selected from the group consisting of SEQ ID NOs: 272-295, 319, 323, 324, 327, 330, 331, 333, 335, 336, 339, 340, 342, 343, 344, 346, 347, 350, 351, 353, 355, 356, 357, 359, 360, 363, 366, 369, 374, 375, 377, 379, 380, 381, 384, 385, 388, 391, 392, 393, 397, 400, 401, 402, 404, 405, 406, 407, 409, 412, 414, 419, 422, 425, 426, 430, 434, 436, 440, 444, 446, 449, 451, 452, 455, 458, 461, 462, 464, 465, 466, 470, 471, 473, 476, 479, 480, 482, 483, 486, 487, 491, 492, 494, 495, 497, 498, 500, 503, 505, 507, 509, 510, 512, 515, 516, 517, 520, 522 and 523.

(1β-2) a polypeptide comprising the amino acid sequence of XXXXXQXEEYNGXWP (SEQ ID NO: 320), preferably a polypeptide comprising the amino acid sequence of XXXSPQVEEYNGVWP (SEQ ID NO: 321), more preferably a polypeptide comprising an amino acid sequence in which any 1, 2, 3, 4, 5, 6 or 7 of the amino acids at positions 1-5, 7 and 13 of SEQ ID NO: 272 are substituted by other amino acids, preferably alanine, further preferably a polypeptide comprising an amino acid sequence in which any 1, 2 or 3 of the amino acids at positions 1-3 of SEQ ID NO: 272 are substituted by other amino acids, preferably alanine.

In another embodiment,embodiment, a polypeptide comprising the amino acid sequence of XEEYNG (SEQ ID NO: 322), preferably a polypeptide comprising an amino acid sequence in which the amino acid at position 1 of SEQ ID NO: 323 is substituted by another amino acid, preferably alanine.

(1β-3) a polypeptide comprising the amino acid sequence of XXXXXXXXNVYEXXX (SEQ ID NO: 328), preferably a polypeptide comprising an amino acid sequence in which any 1, 2, 3, 4, 5, 6, 7, 8, 9, 10 or 11 of the amino acids at positions 1-8 and 13-15 of SEQ ID NO: 273 are substituted by other amino acids, preferably alanine.

In another embodiment,embodiment, a polypeptide comprising the amino acid sequence of XXXXXXXXNV (SEQ ID NO: 325), preferably a polypeptide comprising the amino acid sequence of XXXIKXXXNV (SEQ ID NO: 326), preferably a polypeptide comprising an amino acid sequence in which any 1, 2, 3, 4, 5, 6, 7 or 8 of the amino acids at positions 1-8 of SEQ ID NO: 327 are substituted by other amino acids, preferably alanine, more preferably a polypeptide comprising an amino acid sequence in which any 1, 2, 3, 4, 5 or 6 of the amino acids at positions 1-3 and 6-8 of SEQ ID NO: 327 are substituted by other amino acids, preferably alanine.

(1β-4) a polypeptide comprising the amino acid sequence of RXRXXKXXXXXXXXX (SEQ ID NO: 332), preferably a polypeptide comprising an amino acid sequence in which any 1, 2, 3, 4, 5, 6, 7, 8, 9, 10, 11 or 12 of the amino acids at positions 2, 4, 5 and 7-15 of SEQ ID NO: 274 are substituted by other amino acids, preferably alanine.

In another embodiment,embodiment, a polypeptide comprising the amino acid sequence of RXXXXKXXXALXXXX (SEQ ID NO: 337), preferably a polypeptide comprising an amino acid sequence in which any 1, 2, 3, 4, 5, 6, 7, 8, 9 or 10 of the amino acids at positions 2-5, 7-9 and 12-15 of SEQ ID NO: 274 are substituted by other amino acids, preferably alanine.

In yet another embodiment,embodiment, a polypeptide comprising the amino acid sequence of RXRXXKXX (SEQ ID NO: 329), preferably a polypeptide comprising an amino acid sequence in which any 1, 2, 3, 4 or 5 of the amino acids at positions 2, 4, 5, 7 and 8 of SEQ ID NO: 330 are substituted by other amino acids, preferably alanine.

In yet another embodiment,embodiment, a polypeptide comprising the amino acid sequence of KXXXALXXXX (SEQ ID NO: 334), preferably a polypeptide comprising an amino acid sequence in which any 1, 2, 3, 4, 5, 6 or 7 of the amino acids at positions 2-4 and 7-10 of SEQ ID NO: 335 are substituted by other amino acids, preferably alanine.

(1β-5) a polypeptide comprising the amino acid sequence of AXXKIAXXXPKTVXX (SEQ ID NO: 341), preferably a polypeptide comprising an amino acid sequence in which any 1, 2, 3, 4, 5, 6 or 7 of the amino acids at positions 2, 3, 7-9, 14 and 15 of SEQ ID NO: 275 are substituted by other amino acids, preferably alanine.

In another embodiment,embodiment, a polypeptide comprising the amino acid sequence of AXXXXAXXXXXTVQX (SEQ ID NO: 348), preferably a polypeptide comprising an amino acid sequence in which any 1, 2, 3, 4, 5, 6, 7, 8, 9 or 10 of the amino acids at positions 2-5, 7-11 and 15 of SEQ ID NO: 275 are substituted by other amino acids, preferably alanine.

In yet another embodiment,embodiment, a polypeptide comprising the amino acid sequence of AXXXXAXKXXXXXQG (SEQ ID NO: 352), preferably a polypeptide comprising an amino acid sequence in which any 1, 2, 3, 4, 5, 6, 7, 8, 9 or 10 of the amino acids at positions 2-5, 7 and 9-13 of SEQ ID NO: 275 are substituted by other amino acids, preferably alanine.

In yet another embodiment,embodiment, a polypeptide comprising the amino acid sequence of AXXKIAXXXP (SEQ ID NO: 338), preferably a polypeptide comprising an amino acid sequence in which any 1, 2, 3, 4 or 5 of the amino acids at positions 2, 3 and 7-9 of SEQ ID NO: 339 are substituted by other amino acids, preferably alanine.

In yet another embodiment,embodiment, a polypeptide comprising the amino acid sequence of AWKDPKXXXG (SEQ ID NO: 345), preferably a polypeptide comprising an amino acid sequence in which any 1, 2 or 3 of the amino acids at positions 7-9 of SEQ ID NO: 346 are substituted by other amino acids, preferably alanine.

In yet another embodiment, a polypeptide comprising the amino acid sequence of AXKXXXXX (SEQ ID NO: 349), preferably a polypeptide comprising an amino acid sequence in which any 1, 2, 3, 4, 5 or 6 of the amino acids at positions 2 and 4-8 of SEQ ID NO: 350 are substituted by other amino acids, preferably alanine.

(1β-6) a polypeptide comprising the amino acid sequence of XALXXLSPKLDSMSY (SEQ ID NO: 358), preferably a polypeptide comprising an amino acid sequence in which any 1, 2 or 3 of the amino acids at positions 1, 4 and 5 of SEQ ID NO: 276 are substituted by other amino acids, preferably alanine.

In another embodiment, a polypeptide comprising the amino acid sequence of XLSPKLDS (SEQ ID NO: 354), preferably a polypeptide comprising an amino acid sequence in which the amino acid at position 1 of SEQ ID NO: 655 is substituted by another amino acid, preferably alanine.

(1β-7) a polypeptide comprising the amino acid sequence of VXXXRTMFPXAXISK (SEQ ID NO: 361), preferably a polypeptide comprising the amino acid sequence of VNXXRTMFPSAXISK (SEQ ID NO: 362), more preferably a polypeptide comprising an amino acid sequence in which any 1, 2, 3, 4 or 5 of the amino acids at positions 2-4, 10 and 12 of SEQ ID NO: 277 are substituted by other amino acids, preferably alanine, further preferably a polypeptide comprising an amino acid sequence in which any 1, 2 or 3 of the amino acids at positions 3, 4 and 12 of SEQ ID NO: 277 are substituted by other amino acids, preferably alanine.

In another embodiment, a polypeptide comprising the amino acid sequence of XXSXXXMXPXAXXXK (SEQ ID NO: 370), preferably a polypeptide comprising the amino acid sequence of VNSPRTMXPXAXXXK (SEQ ID NO: 371), more preferably a polypeptide comprising an amino acid sequence in which any 1, 2, 3, 4, 5, 6, 7, 8, 9 or 10 of the amino acids at positions 1, 2, 4-6, 8, 10 and 12-14 of SEQ ID NO: 277 are substituted by other amino acids, preferably alanine, further preferably a polypeptide comprising an amino acid sequence in which any 1, 2, 3, 4 or 5 of the amino acids at positions 8, 10 and 12-14 of SEQ ID NO: 277 are substituted by other amino acids, preferably alanine.

In yet another embodiment, a polypeptide comprising the amino acid sequence of SXXXMXPXAX (SEQ ID NO: 364), preferably a polypeptide comprising the amino acid sequence of SPRTMXPXAX (SEQ ID NO: 365), more preferably a polypeptide comprising an amino acid sequence in which any 1, 2, 3, 4, 5 or 6 of the amino acids at positions 2-4, 6, 8 and 10 of SEQ ID NO: 366 are substituted by other amino acids, preferably alanine, further preferably a polypeptide comprising an amino acid sequence in which any 1, 2 or 3 of the amino acids at positions 3, 4 and 12 of SEQ ID NO: 366 are substituted by other amino acids, preferably alanine.

In yet another embodiment, a polypeptide comprising the amino acid sequence of XMXPXAXXXK (SEQ ID NO: 367), preferably a polypeptide comprising the amino acid sequence of TMXPXAXXXK (SEQ ID NO: 368), more preferably a polypeptide comprising an amino acid sequence in which any 1, 2, 3, 4, 5 or 6 of the amino acids at positions 1, 3, 5 and 7-9 of SEQ ID NO: 369 are substituted by other amino acids, preferably alanine, further preferably a polypeptide comprising an amino acid sequence in which any 1, 2, 3, 4 or 5 of the amino acids at positions 3, 5 and 7-9 of SEQ ID NO: 369 are substituted by other amino acids, preferably alanine.

(1β-8) a polypeptide comprising the amino acid sequence of AIISKLXAXXAXSXA (SEQ ID NO: 376), preferably a polypeptide comprising an amino acid sequence in which any 1, 2, 3, 4 or 5 of the amino acids at positions 7, 9, 10, 12 and 14 of SEQ ID NO: 278 are substituted by other amino acids, preferably alanine.

In another embodiment, a polypeptide comprising the amino acid sequence of IXKLXAXXA (SEQ ID NO: 372), preferably a polypeptide comprising the amino acid sequence of ISKLXAXXA (SEQ ID NO: 373), more preferably a polypeptide comprising an amino acid sequence in which any 1, 2, 3 or 4 of the amino acids at positions 2, 5, 7 and 8 of SEQ ID NO: 374 are substituted by other amino acids, preferably alanine, further preferably a polypeptide comprising an amino acid sequence in which any 1, 2 or 3 of the amino acids at positions 5, 7 and 8 of SEQ ID NO: 374 are substituted by other amino acids, preferably alanine.

(1β-9) a polypeptide comprising the amino acid sequence of GKALQXXKELPTXTP (SEQ ID NO: 382), preferably a polypeptide comprising the amino acid sequence of GKALQXXKELPTETP (SEQ ID NO: 383), more preferably a polypeptide comprising an amino acid sequence in which any 1, 2 or 3 of the amino acids at positions 6, 7 and 13 of SEQ ID NO: 279 are substituted by other amino acids, preferably alanine, further preferably a polypeptide comprising an amino acid sequence in which any 1 or 2 of the amino acids at positions 6 and 7 of SEQ ID NO: 279 are substituted by other amino acids, preferably alanine.

In another embodiment, a polypeptide comprising the amino acid sequence of XXAXXXXXEXXXXXP (SEQ ID NO: 394), preferably a polypeptide comprising the amino acid sequence of XXALXXXXEXXXXXP (SEQ ID NO: 395), more preferably a polypeptide comprising an amino acid sequence in which any 1, 2, 3, 4, 5, 6, 7, 8, 9, 10, 11 or 12 of the amino acids at positions 1, 2, 4-8 and 10-14 of SEQ ID NO: 279 are substituted by other amino acids, preferably alanine, further preferably a polypeptide comprising an amino acid sequence in which any 1, 2, 3, 4, 5, 6, 7, 8, 9, 10 or 11 of the amino acids at positions 1, 2, 5-8 and 10-14 of SEQ ID NO: 279 are substituted by other amino acids, preferably alanine.

In yet another embodiment, a polypeptide comprising the amino acid sequence of GKAXXXXKEXXXXXX (SEQ ID NO: 403), preferably a polypeptide comprising an amino acid sequence in which any 1, 2, 3, 4, 5, 6, 7, 8, 9 or 10 of the amino acids at positions 4-7 and 10-15 of SEQ ID NO: 279 are substituted by other amino acids, preferably alanine.

In yet another embodiment, a polypeptide comprising the amino acid sequence of KALQXXKELP (SEQ ID NO: 378), preferably a polypeptide comprising an amino acid sequence in which any 1 or 2 of the amino acids at positions 5 and 6 of SEQ ID NO: 379 are substituted by other amino acids, preferably alanine.

In yet another embodiment, a polypeptide comprising the amino acid sequence of XAXXXXXEX (SEQ ID NO: 386), preferably a polypeptide comprising the amino acid sequence of XALXXXXEX (SEQ ID NO: 387), more preferably a polypeptide comprising an amino acid sequence in which any 1, 2, 3, 4, 5, 6 or 7 of the amino acids at positions 1, 3-7 and 9 of SEQ ID NO: 388 are substituted by other amino acids, preferably alanine, further preferably a polypeptide comprising an amino acid sequence in which any 1, 2, 3, 4, 5 or 6 of the amino acids at positions 1, 4-7 and 9 of SEQ ID NO: 388 are substituted by other amino acids, preferably alanine.

In yet another embodiment, a polypeptide comprising the amino acid sequence of XXXEXXXXXP (SEQ ID NO: 389), preferably a polypeptide comprising the amino acid sequence of XXXEXXXEXP (SEQ ID NO: 390), more preferably a polypeptide comprising an amino acid sequence in which any 1, 2, 3, 4, 5, 6, 7 or 8 of the amino acids at positions 1-3 and 5-9 of SEQ ID NO: 392 are substituted by other amino acids, preferably alanine, further preferably a polypeptide comprising an amino acid sequence in which any 1, 2, 3, 4, 5, 6 or 7 of the amino acids at positions 1-3, 5-7 and 9 of SEQ ID NO: 392 are substituted by other amino acids, preferably alanine.

In yet another embodiment, a polypeptide comprising the amino acid sequence of KAXXXXKEXX (SEQ ID NO: 396), preferably a polypeptide comprising an amino acid sequence in which any 1, 2, 3, 4, 5 or 6 of the amino acids at positions 3-6, 9 and 10 of SEQ ID NO: 397 are substituted by other amino acids, preferably alanine.

In yet another embodiment, a polypeptide comprising the amino acid sequence of XXKEXXXXXX (SEQ ID NO: 398), preferably a polypeptide comprising the amino acid sequence of GXKEXPXXTX (SEQ ID NO: 399), more preferably a polypeptide comprising an amino acid sequence in which any 1, 2, 3, 4, 5, 6, 7 or 8 of the amino acids at positions 1, 2 and 5-10 of SEQ ID NO: 400 are substituted by other amino acids, preferably alanine, further preferably a polypeptide comprising an amino acid sequence in which any 1, 2, 3, 4 or 5 of the amino acids at positions 2, 5, 7, 8 and 10 of SEQ ID NO: 400 are substituted by other amino acids, preferably alanine.

(1β-10) a polypeptide comprising the amino acid sequence of XXXNKELXQQVMXXV (SEQ ID NO: 408), preferably a polypeptide comprising an amino acid sequence in which any 1, 2, 3, 4, 5 or 6 of the amino acids at positions 1-3, 8, 13 and 14 of SEQ ID NO: 280 are substituted by other amino acids, preferably alanine.

In another embodiment, a polypeptide comprising the amino acid sequence of ELXQQ (SEQ ID NO: 406), preferably a polypeptide comprising an amino acid sequence in which the amino acid at position 3 of SEQ ID NO: 407 is substituted by another amino acid, preferably alanine.

(1β-11) a polypeptide comprising the amino acid sequence of XXXXVVXPADXNAAI (SEQ ID NO: 415), preferably a polypeptide comprising the amino acid sequence of XXXTVVEPADXNAAI (SEQ ID NO: 416), more preferably a polypeptide comprising an amino acid sequence in which any 1, 2, 3, 4, 5 or 6 of the amino acids at positions 1-4, 7 and 11 of SEQ ID NO: 281 are substituted by other amino acids, preferably alanine, further preferably a polypeptide comprising an amino acid sequence in which any 1, 2, 3 or 4 of the amino acids at positions 1-3 and 11 of SEQ ID NO: 281 are substituted by other amino acids, preferably alanine.

In another embodiment, a polypeptide comprising the amino acid sequence of XXXVVXPA (SEQ ID NO: 410), preferably a polypeptide comprising the amino acid sequence of XXTVVEPA (SEQ ID NO: 411), more preferably a polypeptide comprising an amino acid sequence in which any 1, 2, 3 or 4 of the amino acids at positions 1-3 and 6 of SEQ ID NO: 412 are substituted by other amino acids, preferably alanine.

In yet another embodiment, a polypeptide comprising the amino acid sequence of ADXNA (SEQ ID NO: 413), preferably a polypeptide comprising an amino acid sequence in which the amino acid at position 3 of SEQ ID NO: 413 is substituted by another amino acid, preferably alanine.

(1β-12) a polypeptide comprising the amino acid sequence of KXXAKXXXXXKNXXX (SEQ ID NO: 420), preferably a polypeptide comprising the amino acid sequence of KXXAKXDVXXKNLXX (SEQ ID NO: 421), more preferably a polypeptide comprising an amino acid sequence in which any 1, 2, 3, 4, 5, 6, 7, 8, 9 or 10 of the amino acids at positions 2, 3, 6-10 and 13-15 of SEQ ID NO: 282 are substituted by other amino acids, preferably alanine, further preferably a polypeptide comprising an amino acid sequence in which any 1, 2, 3, 4, 5, 6 or 7 of the amino acids at positions 2, 3, 6, 9, 10, 14 and 15 of SEQ ID NO: 282 are substituted by other amino acids, preferably alanine.

In another embodiment, a polypeptide comprising the amino acid sequence of KFXAKXDXKXKNXKX (SEQ ID NO: 427), preferably a polypeptide comprising the amino acid sequence of KFDAKXDVKXKNXKX (SEQ ID NO: 428), more preferably a polypeptide comprising an amino acid sequence in which any 1, 2, 3, 4, 5 or 6 of the amino acids at positions 3, 6, 8, 10, 13 and 15 of SEQ ID NO: 282 are substituted by other amino acids, preferably alanine, further preferably a polypeptide comprising an amino acid sequence in which any 1, 2, 3 or 4 of the amino acids at positions 6, 10, 13 and 15 of SEQ ID NO: 282 are substituted by other amino acids, preferably alanine.

In yet another embodiment, a polypeptide comprising the amino acid sequence of XXXXKNLX (SEQ ID NO: 417), preferably a polypeptide comprising the amino acid sequence of DVXXKNLX (SEQ ID NO: 418), more preferably a polypeptide comprising an amino acid sequence in which any 1, 2, 3, 4 or 5 of the amino acids at positions 1-4 and 8 of SEQ ID NO: 419 are substituted by other amino acids, preferably alanine, further preferably a polypeptide comprising an amino acid sequence in which any 1, 2 or 3 of the amino acids at positions 3, 4 and 8 of SEQ ID NO: 419 are substituted by other amino acids, preferably alanine.

In yet another embodiment, a polypeptide comprising the amino acid sequence of DXKXKNXKX (SEQ ID NO: 423), preferably a polypeptide comprising the amino acid sequence of DVKXKNXKX (SEQ ID NO: 424), more preferably a polypeptide comprising an amino acid sequence in which any 1, 2, 3 or 4 of the amino acids at positions 2, 4, 7 and 9 of SEQ ID NO: 425 are substituted by other amino acids, preferably alanine, further preferably a polypeptide comprising an amino acid sequence in which any 1, 2 or 3 of the amino acids at positions 4, 7 and 9 of SEQ ID NO: 425 are substituted by other amino acids, preferably alanine.

(1β-13) a polypeptide comprising the amino acid sequence of NXXXKKXNXVXAXXX (SEQ ID NO: 431), preferably a polypeptide comprising the amino acid sequence of NRXXKKXNTVLAXXX (SEQ ID NO: 432), more preferably a polypeptide comprising an amino acid sequence in which any 1, 2, 3, 4, 5, 6, 7, 8 or 9 of the amino acids at positions 2-4, 7, 9, 11 and 13-15 of SEQ ID NO: 285 are substituted by other amino acids, preferably alanine, further preferably a polypeptide comprising an amino acid sequence in which any 1, 2, 3, 4, 5 or 6 of the amino acids at positions 3, 4, 7 and 13-15 of SEQ ID NO: 285 are substituted by other amino acids, preferably alanine.

In another embodiment, a polypeptide comprising the amino acid sequence of XXKKXNTVLA (SEQ ID NO: 429), preferably a polypeptide comprising an amino acid sequence in which any 1, 2 or 3 of the amino acids at positions 1, 2 and 5 of SEQ ID NO: 430 are substituted by other amino acids, preferably alanine.

(1β-14) a polypeptide comprising the amino acid sequence of SSSSSSSXSNSKSSS (SEQ ID NO: 437), preferably a polypeptide comprising an amino acid sequence in which the amino acid at position 8 of SEQ ID NO: 286 is substituted by another amino acid, preferably alanine.

In another embodiment, a polypeptide comprising the amino acid sequence of XXSXSSSXXXSKSSS (SEQ ID NO: 441), preferably a polypeptide comprising the amino acid sequence of XXSSSSSXXNSKSSS (SEQ ID NO: 442), more preferably a polypeptide comprising an amino acid sequence in which any 1, 2, 3, 4, 5 or 6 of the amino acids at positions 1, 2, 4 and 8-10 of SEQ ID NO: 286 are substituted by other amino acids, preferably alanine, further preferably a polypeptide comprising an amino acid sequence in which any 1, 2, 3 or 4 of the amino acids at positions 1, 2, 8 and 9 of SEQ ID NO: 286 are substituted by other amino acids, preferably alanine.

In yet another embodiment, a polypeptide comprising the amino acid sequence of SSSXSNSKS (SEQ ID NO: 433), preferably a polypeptide comprising an amino acid sequence in which the amino acid at position 4 of SEQ ID NO: 434 is substituted by another amino acid, preferably alanine.

In yet another embodiment, a polypeptide comprising the amino acid sequence of SXSNSKSSSS (SEQ ID NO: 435), preferably a polypeptide comprising an amino acid sequence in which the amino acid at position 2 of SEQ ID NO: 436 is substituted by another amino acid, preferably alanine.

In yet another embodiment, a polypeptide comprising the amino acid sequence of XXXSKSS (SEQ ID NO: 438), preferably a polypeptide comprising the amino acid sequence of XXNSKSS (SEQ ID NO: 439), more preferably a polypeptide comprising an amino acid sequence in which any 1, 2 or 3 of the amino acids at positions 1-3 of SEQ ID NO: 440 are substituted by other amino acids, preferably alanine, further preferably a polypeptide comprising an amino acid sequence in which any 1 or 2 of the amino acids at positions 1 and 2 of SEQ ID NO: 400 are substituted by other amino acids, preferably alanine.

(1β-15) a polypeptide comprising the amino acid sequence of XEXXXXKXPXXXAXX (SEQ ID NO: 450), preferably a polypeptide comprising an amino acid sequence in which any 1, 2, 3, 4, 5, 6, 7, 8, 9, 10 or 11 of the amino acids at positions 1, 3-6, 8, 10-12, 14 and 15 of SEQ ID NO: 287 are substituted by other amino acids, preferably alanine.

In another embodiment, a polypeptide comprising the amino acid sequence of XEXXXXKXPX (SEQ ID NO: 443), preferably a polypeptide comprising an amino acid sequence in which any 1, 2, 3, 4, 5, 6 or 7 of the amino acids at positions 1, 3-6, 8 and 10 of SEQ ID NO: 444 are substituted by other amino acids, preferably alanine.

In yet another embodiment, a polypeptide comprising the amino acid sequence of XXXKXPXXXA (SEQ ID NO: 445), preferably a polypeptide comprising an amino acid sequence in which any 1, 2, 3, 4, 5, 6 or 7 of the amino acids at positions 1-3, 5 and 7-9 of SEQ ID NO: 446 are substituted by other amino acids, preferably alanine.

In yet another embodiment, a polypeptide comprising the amino acid sequence of XKXPXXXAXX (SEQ ID NO: 447), preferably a polypeptide comprising the amino acid sequence of XKXPXXXAXS (SEQ ID NO: 448), more preferably a polypeptide comprising an amino acid sequence in which any 1, 2, 3, 4, 5, 6 or 7 of the amino acids at positions 1, 3, 5-7, 9 and 10 of SEQ ID NO: 449 are substituted by other amino acids, preferably alanine, further preferably a polypeptide comprising an amino acid sequence in which any 1, 2, 3, 4, 5 or 6 of the amino acids at positions 1, 3, 5-7 and 9 of SEQ ID NO: 449 are substituted by other amino acids, preferably alanine.

(1β-16) a polypeptide comprising the amino acid sequence of XXTXXXKXXLXADAX (SEQ ID NO: 459), more preferably a polypeptide comprising an amino acid sequence in which any 1, 2, 3, 4, 5, 6, 7, 8 or 9 of the amino acids at positions 1, 2, 4-6, 8, 9, 11 and 15 of SEQ ID NO: 288 are substituted by other amino acids, preferably alanine.

In another embodiment, a polypeptide comprising the amino acid sequence of TXXXKXXLXA (SEQ ID NO: 453), preferably a polypeptide comprising the amino acid sequence of TXXXKXKLCA (SEQ ID NO: 454), more preferably a polypeptide comprising an amino acid sequence in which any 1, 2, 3, 4, 5 or 6 of the amino acids at positions 2-4, 6, 7 and 9 of SEQ ID NO: 455 are substituted by other amino acids, preferably alanine.

In yet another embodiment, a polypeptide comprising the amino acid sequence of KXXLXADAX (SEQ ID NO: 456), preferably a polypeptide comprising the amino acid sequence of KXKLCADAX (SEQ ID NO: 457), more preferably a polypeptide comprising an amino acid sequence in which any 1, 2, 3 or 4 of the amino acids at positions 2, 3, 5 and 9 of SEQ ID NO: 458 are substituted by other amino acids, preferably alanine, further preferably a polypeptide comprising an amino acid sequence in which any 1 or 2 of the amino acids at positions 2 and 9 of SEQ ID NO: 458 are substituted by other amino acids, preferably alanine.

(1β-17) a polypeptide comprising the amino acid sequence of KIKTXNEXXFXXSMP (SEQ ID NO: 467), preferably a polypeptide comprising an amino acid sequence in which any 1, 2, 3, 4 or 5 of the amino acids at positions 5, 8, 9, 11 and 12 of SEQ ID NO: 290 are substituted by other amino acids, preferably alanine.

In another embodiment, a polypeptide comprising the amino acid sequence of KIKTX (SEQ ID NO: 460), preferably a polypeptide comprising an amino acid sequence in which the amino acid at position 5 of SEQ ID NO: 461 is substituted by another amino acid, preferably alanine.

In yet another embodiment, a polypeptide comprising the amino acid sequence of XNEXXFNYSM (SEQ ID NO: 463), preferably a polypeptide comprising an amino acid sequence in which any 1, 2 or 3 of the amino acids at positions 1, 4 and 5 of SEQ ID NO: 464 are substituted by other amino acids, preferably alanine.

(1β-18) a polypeptide comprising the amino acid sequence of XAXXXXAXXIXIGXH (SEQ ID NO: 474), preferably a polypeptide comprising the amino acid sequence of EAXXXXAXXIXIGXH (SEQ ID NO: 475), more preferably a polypeptide comprising an amino acid sequence in which any 1, 2, 3, 4, 5, 6, 7, 8 or 9 of the amino acids at positions 1, 3-6, 8, 9, 11 and 14 of SEQ ID NO: 291 are substituted by other amino acids, preferably alanine, further preferably a polypeptide comprising an amino acid sequence in which any 1, 2, 3, 4, 5, 6, 7 or 8 of the amino acids at positions 3-6, 8, 9, 11 and 14 of SEQ ID NO: 291 are substituted by other amino acids, preferably alanine.

In another embodiment, a polypeptide comprising the amino acid sequence of XAXNXXAXXXKIGXH (SEQ ID NO: 477), preferably a polypeptide comprising the amino acid sequence of XAXNXKAXXXKIGXH (SEQ ID NO: 478), more preferably a polypeptide comprising an amino acid sequence in which any 1, 2, 3, 4, 5, 6, 7 or 8 of the amino acids at positions 1, 3, 5, 6, 8-10 and 14 of SEQ ID NO: 291 are substituted by other amino acids, preferably alanine, further preferably a polypeptide comprising an amino acid sequence in which any 1, 2, 3, 4, 5, 6 or 7 of the amino acids at positions 1, 3, 5, 8-10 and 14 of SEQ ID NO: 291 are substituted by other amino acids, preferably alanine.

In yet another embodiment, a polypeptide comprising the amino acid sequence of XAXXLXAXNIXXGXH (SEQ ID NO: 488), preferably a polypeptide comprising the amino acid sequence of EAXXLKAXNIXXGXH (SEQ ID NO: 489), more preferably a polypeptide comprising an amino acid sequence in which any 1, 2, 3, 4, 5, 6 or 7 of the amino acids at positions 1, 3, 4, 6, 11, 12 and 14 of SEQ ID NO: 291 are substituted by other amino acids, preferably alanine, further preferably a polypeptide comprising an amino acid sequence in which any 1, 2, 3, 4, 5 or 6 of the amino acids at positions 3, 4, 8, 11, 12 and 14 of SEQ ID NO: 291 are substituted by other amino acids, preferably alanine.

In yet another embodiment, a polypeptide comprising the amino acid sequence of XAXNXXAXX (SEQ ID NO: 468), preferably a polypeptide comprising the amino acid sequence of EAXNLXAXX (SEQ ID NO: 469), more preferably a polypeptide comprising an amino acid sequence in which any 1, 2, 3, 4, 5 or 6 of the amino acids at positions 1, 3, 5, 6, 8 and 9 of SEQ ID NO: 470 are substituted by other amino acids, preferably alanine, further preferably a polypeptide comprising an amino acid sequence in which any 1, 2, 3 or 4 of the amino acids at positions 3, 6, 8 and 9 of SEQ ID NO: 470 are substituted by other amino acids, preferably alanine.

In yet another embodiment, a polypeptide comprising the amino acid sequence of XXIXIGXH (SEQ ID NO: 472), preferably a polypeptide comprising an amino acid sequence in which any 1, 2, 3 or 4 of the amino acids at positions 1, 2, 4 and 7 of SEQ ID NO: 473 are substituted by other amino acids, preferably alanine.

In yet another embodiment, a polypeptide comprising the amino acid sequence of AXXLKAXNI (SEQ ID NO: 481), preferably a polypeptide comprising an amino acid sequence in which any 1, 2 or 3 of the amino acids at positions 2, 3 and 7 of SEQ ID NO: 482 are substituted by other amino acids, preferably alanine.

In yet another embodiment, a polypeptide comprising the amino acid sequence of XAXNIXXGX (SEQ ID NO: 484), preferably a polypeptide comprising the amino acid sequence of KAXNIXXGX (SEQ ID NO: 485), more preferably a polypeptide comprising an amino acid sequence in which any 1, 2, 3, 4 or 5 of the amino acids at positions 1, 3, 6, 7 and 9 of SEQ ID NO: 486 are substituted by other amino acids, preferably alanine, further preferably a polypeptide comprising an amino acid sequence in which any 1, 2, 3 or 4 of the amino acids at positions 3, 6, 7 and 9 of SEQ ID NO: 486 are substituted by other amino acids, preferably alanine.

(1β-19) a polypeptide comprising the amino acid sequence of AAPXXGXDKXXXXGK (SEQ ID NO: 496), preferably a polypeptide comprising an amino acid sequence in which any 1, 2, 3, 4, 5, 6 or 7 of the amino acids at positions 4, 5, 7 and 10-13 of SEQ ID NO: 292 are substituted by other amino acids, preferably alanine.

In another embodiment, a polypeptide comprising the amino acid sequence of AAPXXGIDKLXFDGK (SEQ ID NO: 501), preferably a polypeptide comprising the amino acid sequence of AAPXXGIDKLYFDGK (SEQ ID NO: 502), more preferably a polypeptide comprising an amino acid sequence in which any 1, 2 or 3 of the amino acids at positions 4, 5 and 11 of SEQ ID NO: 292 are substituted by other amino acids, preferably alanine, further preferably a polypeptide comprising an amino acid sequence in which any 1 or 2 of the amino acids at positions 4 and 5 of SEQ ID NO: 292 are substituted by other amino acids, preferably alanine.

In yet another embodiment, a polypeptide comprising the amino acid sequence of AAXXXXIDKXXXXXK (SEQ ID NO: 510), preferably a polypeptide comprising an amino acid sequence in which any 1, 2, 3, 4, 5, 6, 7, 8 or 9 of the amino acids at positions 3-6 and 10-14 of SEQ ID NO: 292 are substituted by other amino acids, preferably alanine.

In yet another embodiment, a polypeptide comprising the amino acid sequence of AAPXXG (SEQ ID NO: 490), preferably a polypeptide comprising an amino acid sequence in which any 1 or 2 of the amino acids at positions 4 and 5 of SEQ ID NO: 491 are substituted by other amino acids, preferably alanine.

In yet another embodiment, a polypeptide comprising the amino acid sequence of DKXXXX (SEQ ID NO: 493), preferably a polypeptide comprising an amino acid sequence in which any 1, 2, 3 or 4 of the amino acids at positions 3-6 of SEQ ID NO: 494 are substituted by other amino acids, preferably alanine.

In yet another embodiment, a polypeptide comprising the amino acid sequence of DKLXFDG (SEQ ID NO: 499), preferably a polypeptide comprising an amino acid sequence in which the amino acid at position 4 of SEQ ID NO: 500 is substituted by another amino acid, preferably alanine.

In yet another embodiment, a polypeptide comprising the amino acid sequence of AAXXXXID (SEQ ID NO: 504), preferably a polypeptide comprising an amino acid sequence in which any 1, 2, 3 or 4 of the amino acids at positions 3-6 of SEQ ID NO: 505 are substituted by other amino acids, preferably alanine.

In yet another embodiment, a polypeptide comprising the amino acid sequence of XXXXIDKXXX (SEQ ID NO: 506), preferably a polypeptide comprising an amino acid sequence in which any 1, 2, 3, 4, 5, 6 or 7 of the amino acids at positions 1-4 and 8-10 of SEQ ID NO: 507 are substituted by other amino acids, preferably alanine.

In yet another embodiment, a polypeptide comprising the amino acid sequence of DKXXXXXKT (SEQ ID NO: 508), preferably a polypeptide comprising an amino acid sequence in which any 1, 2, 3, 4 or 5 of the amino acids at positions 3-7 of SEQ ID NO: 509 are substituted by other amino acids, preferably alanine.

(1β-20) a polypeptide comprising the amino acid sequence of XXEXXMXNGYXAKNA (SEQ ID NO: 513), preferably a polypeptide comprising the amino acid sequence of XQEXXMXNGYLAKNA (SEQ ID NO: 514), more preferably a polypeptide comprising an amino acid sequence in which any 1, 2, 3, 4, 5 or 6 of the amino acids at positions 1, 2, 4, 5, 7 and 11 of SEQ ID NO: 293 are substituted by other amino acids, preferably alanine, further preferably a polypeptide comprising an amino acid sequence in which any 1, 2, 3 or 4 of the amino acids at positions 1, 4, 5 and 7 of SEQ ID NO: 293 are substituted by other amino acids, preferably alanine.

In another embodiment, a polypeptide comprising the amino acid sequence of MXNGYLAKNA (SEQ ID NO: 511), preferably a polypeptide comprising an amino acid sequence in which the amino acid at position 2 of SEQ ID NO: 512 is substituted by another amino acid, preferably alanine.

(1β-21) a polypeptide comprising the amino acid sequence of XAXXLXXXFVKLXXX (SEQ ID NO: 524), preferably a polypeptide comprising the amino acid sequence of XAXXLXXSFVKLXXX (SEQ ID NO: 525), more preferably a polypeptide comprising an amino acid sequence in which any 1, 2, 3, 4, 5, 6, 7, 8 or 9 of the amino acids at positions 1, 3, 4, 6-8 and 13-15 of SEQ ID NO: 294 are substituted by other amino acids, preferably alanine, further preferably a polypeptide comprising an amino acid sequence in which any 1, 2, 3, 4, 5, 6, 7 or 8 of the amino acids at positions 1, 3, 4, 6, 7 and 13-15 of SEQ ID NO: 294 are substituted by other amino acids, preferably alanine.

In another embodiment, a polypeptide comprising the amino acid sequence of AXXLXXXFVK (SEQ ID NO: 518), preferably a polypeptide comprising the amino acid sequence of AXXLXXSFVK (SEQ ID NO: 519), more preferably a polypeptide comprising an amino acid sequence in which any 1, 2, 3, 4 or 5 of the amino acids at positions 2, 3 and 5-7 of SEQ ID NO: 520 are substituted by other amino acids, preferably alanine, further preferably a polypeptide comprising an amino acid sequence in which any 1, 2, 3 or 4 of the amino acids at positions 2, 3, 5 and 6 of SEQ ID NO: 520 are substituted by other amino acids, preferably alanine.

In yet another embodiment, a polypeptide comprising the amino acid sequence of XSFVKLX (SEQ ID NO: 521), preferably a polypeptide comprising an amino acid sequence in which any 1 or 2 of the amino acids at positions 1 and 7 of SEQ ID NO: 522 are substituted by other amino acids, preferably alanine.

### (2β) Epitope of vitellogenin-3

In the present invention, the polypeptide (2β) can be any polypeptide selected from the group consisting of (2β-1) to (2β-6) as defined below:
(2β-1) a polypeptide comprising at least one amino acid sequence selected from the group consisting of SEQ ID NOs: 296-303, 528, 531, 533, 534, 535, 539, 541, 542, 544, 550 and 551.

(2β-2) a polypeptide comprising the amino acid sequence of XPRTCDXXXKXXXXX (SEQ ID NO: 526), preferably a polypeptide comprising an amino acid sequence in which any 1, 2, 3, 4, 5, 6, 7, 8 or 9 of the amino acids at positions 1, 7-9 and 11-15 of SEQ ID NO: 296 are substituted by other amino acids, preferably alanine.

In another embodiment, a polypeptide comprising the amino acid sequence of SPRXXXXXLXLPXXX (SEQ ID NO: 527), preferably a polypeptide comprising an amino acid sequence in which any 1, 2, 3, 4, 5, 6, 7, 8 or 9 of the amino acids at positions 4-8, 10 and 13-15 of SEQ ID NO: 296 are substituted by other amino acids, preferably alanine.

(2β-3) a polypeptide comprising the amino acid sequence of SXXXXXXEXXXXXXX (SEQ ID NO: 529), preferably a polypeptide comprising the amino acid sequence of SXXXXSXEXXXXXXX (SEQ ID NO: 530), more preferably a polypeptide comprising an amino acid sequence in which any 1, 2, 3, 4, 5, 6, 7, 8, 9, 10, 11, 12 or 13 of the amino acids at positions 2-7 and 9-15 of SEQ ID NO: 297 are substituted by other amino acids, preferably alanine, further preferably a polypeptide comprising an amino acid sequence in which any 1, 2, 3, 4, 5, 6, 7, 8, 9, 10, 11 or 12 of the amino acids at positions 2-5, 7 and 9-15 of SEQ ID NO: 297 are substituted by other amino acids, preferably alanine.

In another embodiment, a polypeptide comprising the amino acid sequence of XXXLXSXXXXXXXXX (SEQ ID NO: 532), preferably a polypeptide comprising an amino acid sequence in which any 1, 2, 3, 4, 5, 6, 7, 8, 9, 10, 11, 12 or 13 of the amino acids at positions 1-3, 5 and 7-15 of SEQ ID NO: 297 are substituted by other amino acids, preferably alanine.

(2β-4) a polypeptide comprising the amino acid sequence of GKXXXXXXXXXXXKK (SEQ ID NO: 536), preferably a polypeptide comprising the amino acid sequence of GKXXXXXXXXXXEKK (SEQ ID NO: 537), more preferably a polypeptide comprising an amino acid sequence in which any 1, 2, 3, 4, 5, 6, 7, 8, 9, 10 or 11 of the amino acids at positions 3-13 of SEQ ID NO: 298 are substituted by other amino acids, preferably alanine, further preferably a polypeptide comprising an amino acid sequence in which any 1, 2, 3, 4, 5, 6, 7, 8, 9 or 10 of the amino acids at positions 3-12 of SEQ ID NO: 298 are substituted by other amino acids, preferably alanine.

In another embodiment, a polypeptide comprising the amino acid sequence of XXTCXXXXXXXXEKK (SEQ ID NO: 545), preferably a polypeptide comprising the amino acid sequence of XKTCXIXXXXXXEKK (SEQ ID NO: 546), more preferably a polypeptide comprising an amino acid sequence in which any 1, 2, 3, 4, 5, 6, 7, 8, 9 or 10 of the amino acids at positions 1, 2 and 5-12 of SEQ ID NO: 298 are substituted by other amino acids, preferably alanine, further preferably a polypeptide comprising an amino acid sequence in which any 1, 2, 3, 4, 5, 6, 7 or 8 of the amino acids at positions 1, 5 and 7-12 of SEQ ID NO: 298 are substituted by other amino acids, preferably alanine.

In yet another embodiment, a polypeptide comprising the amino acid sequence of CXIXXXXXX (SEQ ID NO: 538), preferably a polypeptide comprising an amino acid sequence in which any 1, 2, 3, 4, 5, 6 or 7 of the amino acids at positions 2 and 4-9 of SEQ ID NO: 539 are substituted by other amino acids, preferably alanine.

In yet another embodiment, a polypeptide comprising the amino acid sequence of IXXXXXXEKK (SEQ ID NO: 540), preferably a polypeptide comprising an amino acid sequence in which any 1, 2, 3, 4, 5 or 6 of the amino acids at positions 2-7 of SEQ ID NO: 541 are substituted by other amino acids, preferably alanine.

In yet another embodiment, a polypeptide comprising the amino acid sequence of XXXEKK (SEQ ID NO: 543), preferably a polypeptide comprising an amino acid sequence in which any 1, 2 or 3 of the amino acids at positions 1-3 of SEQ ID NO: 544 are substituted by other amino acids, preferably alanine.

(2β-5) a polypeptide comprising the amino acid sequence of XAXXXREYQMPSGYL (SEQ ID NO: 547), preferably a polypeptide comprising an amino acid sequence in which any 1, 2, 3 or 4 of the amino acids at positions 1 and 3-5 of SEQ ID NO: 299 are substituted by other amino acids, preferably alanine.

In another embodiment, a polypeptide comprising the amino acid sequence of XAXXXXXXQMPSXXL (SEQ ID NO: 548), preferably a polypeptide comprising an amino acid sequence in which any 1, 2, 3, 4, 5, 6, 7, 8 or 9 of the amino acids at positions 1, 3-8, 13 and 14 of SEQ ID NO: 299 are substituted by other amino acids, preferably alanine.

In yet another embodiment, a polypeptide comprising the amino acid sequence of DAXXXXXXQXXXXYL (SEQ ID NO: 549), preferably a polypeptide comprising an amino acid sequence in which any 1, 2, 3, 4, 5, 6, 7, 8, 9 or 10 of the amino acids at positions 3-8 and 10-13 of SEQ ID NO: 299 are substituted by other amino acids, preferably alanine.

(2β-6) a polypeptide comprising the amino acid sequence of XTXXXACKLXXXXXX (SEQ ID NO: 552), preferably a polypeptide comprising an amino acid sequence in which any 1, 2, 3, 4, 5, 6, 7, 8, 9 or 10 of the amino acids at positions 1, 3-5 and 10-15 of SEQ ID NO: 301 are substituted by other amino acids, preferably alanine.

In one embodiment, the polypeptide (2β) may not comprise a variant or a homolog having 100% or at least 90%, at least 80% or at least 70% identity to the C-terminal portion (SEQ ID NOs: 2 and 13) of vitellogenin-3 or the full-length amino acid sequence thereof.

### (3β) Epitope of apolipoprotein B

In the present invention, the polypeptide (3β) can be any polypeptide selected from the group consisting of (3β-1) to (3β-8) as defined below:
(3β-1) a polypeptide comprising at least one amino acid sequence selected from the group consisting of SEQ ID NOs: 304-311, 553, 555, 556, 557, 561, 562, 565, 569, 571, 573, 576, 577, 579, 581, 583, 584, 585, 587, 589, 592, 594, 595, 598, 600, 602, 304, 605, 608, 609, 613, 616, 617, 619, 622, 623 and 624.

(3β-2) a polypeptide comprising the amino acid sequence of AEXXXXXXXXXXLNX (SEQ ID NO: 558), preferably a polypeptide comprising an amino acid sequence in which any 1, 2, 3, 4, 5, 6, 7, 8, 9, 10 or 11 of the amino acids at positions 3-12 and 15 of SEQ ID NO: 304 are substituted by other amino acids, preferably alanine.

In another embodiment, a polypeptide comprising the amino acid sequence of AEXXXXXX (SEQ ID NO: 554), preferably a polypeptide comprising an amino acid sequence in which any 1, 2, 3, 4, 5 or 6 of the amino acids at positions 3-8 of SEQ ID NO: 555 are substituted by other amino acids, preferably alanine.

(3β-3) a polypeptide comprising the amino acid sequence of XXXXXXXXXXXXXNV (SEQ ID NO: 563), preferably a polypeptide comprising the amino acid sequence of XXXXXXXXXDEPLNV (SEQ ID NO: 564), more preferably a polypeptide comprising an amino acid sequence in which any 1, 2, 3, 4, 5, 6, 7, 8, 9, 10, 11, 12 or 13 of the amino acids at positions 1-13 of SEQ ID NO: 305 are substituted by other amino acids, preferably alanine, further preferably a polypeptide comprising an amino acid sequence in which any 1, 2, 3, 4, 5, 6, 7, 8 or 9 of the amino acids at positions 1-9 of SEQ ID NO: 305 are substituted by other amino acids, preferably alanine.

In another embodiment, a polypeptide comprising the amino acid sequence of XXXXXXXXKXEXXNX (SEQ ID NO: 574), preferably a polypeptide comprising the amino acid sequence of XXXKLYPXKXEXXNX (SEQ ID NO: 575), more preferably a polypeptide comprising an amino acid sequence in which any 1, 2, 3, 4, 5, 6, 7, 8, 9, 10, 11 or 12 of the amino acids at positions 1-8, 10, 12, 13 and 15 of SEQ ID NO: 305 are substituted by other amino acids, preferably alanine, further preferably a polypeptide comprising an amino acid sequence in which any 1, 2, 3, 4, 5, 6, 7 or 8 of the amino acids at positions 1-3, 8, 10, 12, 13 and 15 of SEQ ID NO: 305 are substituted by other amino acids, preferably alanine.

In yet another embodiment, a polypeptide comprising the amino acid sequence of XEPLN (SEQ ID NO: 559), preferably a polypeptide comprising an amino acid sequence in which the amino acid at position 1 of SEQ ID NO: 561 is substituted by another amino acid, preferably alanine.

In yet another embodiment, a polypeptide comprising the amino acid sequence of XEPLNV (SEQ ID NO: 560), preferably a polypeptide comprising an amino acid sequence in which the amino acid at position 1 of SEQ ID NO: 562 is substituted by another amino acid, preferably alanine.

In yet another embodiment, a polypeptide comprising the amino acid sequence of XXXXXXKXEX (SEQ ID NO: 567), preferably a polypeptide comprising the amino acid sequence of XKLYPXKXXX (SEQ ID NO: 568), more preferably a polypeptide comprising an amino acid sequence in which any 1, 2, 3, 4, 5, 6, 7 or 8 of the amino acids at positions 1-6, 8 and 10 of SEQ ID NO: 569 are substituted by other amino acids, preferably alanine, further preferably a polypeptide comprising an amino acid sequence in which any 1, 2, 3, 4 or 5 of the amino acids at positions 1, 6 and 8-10 of SEQ ID NO: 569 are substituted by other amino acids, preferably alanine.

In yet another embodiment, a polypeptide comprising the amino acid sequence of KXEXXNX (SEQ ID NO: 570), preferably a polypeptide comprising an amino acid sequence in which any 1, 2, 3 or 4 of the amino acids at positions 2, 4, 5 and 7 of SEQ ID NO: 571 are substituted by other amino acids, preferably alanine.

In yet another embodiment, a polypeptide comprising the amino acid sequence of KXEX (SEQ ID NO: 572), preferably a polypeptide comprising an amino acid sequence in which any 1 or 2 of the amino acids at positions 2 and 4 of SEQ ID NO: 573 are substituted by other amino acids, preferably alanine.

(3β-4) a polypeptide comprising the amino acid sequence of VSKRXXVXXXXXXXX (SEQ ID NO: 582), preferably a polypeptide comprising an amino acid sequence in which any 1, 2, 3, 4, 5, 6, 7, 8, 9 or 10 of the amino acids at positions 5, 6 and 8-15 of SEQ ID NO: 306 are substituted by other amino acids, preferably alanine.

In another embodiment, a polypeptide comprising the amino acid sequence of VSKRXX (SEQ ID NO: 578), preferably a polypeptide comprising an amino acid sequence in which any 1 or 2 of the amino acids at positions 5 and 6 of SEQ ID NO: 579 are substituted by other amino acids, preferably alanine.

In yet another embodiment, a polypeptide comprising the amino acid sequence of SVPXXXX (SEQ ID NO: 580), preferably a polypeptide comprising an amino acid sequence in which any 1, 2, 3 or 4 of the amino acids at positions 4-7 of SEQ ID NO: 581 are substituted by other amino acids, preferably alanine.

(3β-5) a polypeptide comprising the amino acid sequence of XXXGXXXPXAXXAXX (SEQ ID NO: 590), preferably a polypeptide comprising an amino acid sequence in which any 1, 2, 3, 4, 5, 6, 7, 8 or 9 of the amino acids at positions 1-3, 5-7, 9, 11 and 12 of SEQ ID NO: 308 are substituted by other amino acids, preferably alanine.

In another embodiment, a polypeptide comprising the amino acid sequence of XXXGXXXXGAKVAVX (SEQ ID NO: 596), preferably a polypeptide comprising an amino acid sequence in which any 1, 2, 3, 4, 5, 6, 7 or 8 of the amino acids at positions 1-3, 5-8 and 15 of SEQ ID NO: 308 are substituted by other amino acids, preferably alanine.

In another embodiment, a polypeptide comprising the amino acid sequence of XXXXIXXPXAXXAXK (SEQ ID NO: 603), preferably a polypeptide comprising an amino acid sequence in which any 1, 2, 3, 4, 5, 6, 7, 8, 9 or 10 of the amino acids at positions 1-4, 6, 7, 9, 11, 12 and 14 of SEQ ID NO: 308 are substituted by other amino acids, preferably alanine.

In yet another embodiment, a polypeptide comprising the amino acid sequence of SGXX (SEQ ID NO: 586), preferably a polypeptide comprising an amino acid sequence in which any 1 or 2 of the amino acids at positions 3 and 4 of SEQ ID NO: 587 are substituted by other amino acids, preferably alanine.

In yet another embodiment, a polypeptide comprising the amino acid sequence of XXXPXAXXAX (SEQ ID NO: 588), preferably a polypeptide comprising an amino acid sequence in which any 1, 2, 3, 4, 5, 6 or 7 of the amino acids at positions 1-3, 5, 7, 8 and 10 of SEQ ID NO: 589 are substituted by other amino acids, preferably alanine.

In yet another embodiment, a polypeptide comprising the amino acid sequence of XGXXXXGAK (SEQ ID NO: 591), preferably a polypeptide comprising an amino acid sequence in which any 1, 2, 3, 4 or 5 of the amino acids at positions 1 and 3-6 of SEQ ID NO: 592 are substituted by other amino acids, preferably alanine.

In yet another embodiment, a polypeptide comprising the amino acid sequence of XXXXGAKVAV (SEQ ID NO: 593), preferably a polypeptide comprising an amino acid sequence in which any 1, 2, 3 or 4 of the amino acids at positions 1-4 of SEQ ID NO: 594 are substituted by other amino acids, preferably alanine.

In yet another embodiment, a polypeptide comprising the amino acid sequence of XXIXXPXA (SEQ ID NO: 597), preferably a polypeptide comprising an amino acid sequence in which any 1, 2, 3, 4 or 5 of the amino acids at positions 1, 2, 4, 5 and 7 of SEQ ID NO: 598 are substituted by other amino acids, preferably alanine.

In yet another embodiment, a polypeptide comprising the amino acid sequence of IXXPXAXXAX (SEQ ID NO: 599), preferably a polypeptide comprising an amino acid sequence in which any 1, 2, 3, 4, 5 or 6 of the amino acids at positions 2, 3, 5, 7, 8 and 10 of SEQ ID NO: 600 are substituted by other amino acids, preferably alanine.

In yet another embodiment, a polypeptide comprising the amino acid sequence of XPXAXXAX (SEQ ID NO: 601), preferably a polypeptide comprising an amino acid sequence in which any 1, 2, 3, 4 or 5 of the amino acids at positions 1, 3, 5, 6 and 8 of SEQ ID NO: 602 are substituted by other amino acids, preferably alanine.

(3β-6) a polypeptide comprising the amino acid sequence of XXXXPXXXYXXMSSS (SEQ ID NO: 610), preferably a polypeptide comprising the amino acid sequence of XXXXPXXXYLXMSSS (SEQ ID NO: 611), more preferably a polypeptide comprising an amino acid sequence in which any 1, 2, 3, 4, 5, 6, 7, 8 or 9 of the amino acids at positions 1-4, 6-8, 10 and 11 of SEQ ID NO: 309 are substituted by other amino acids, preferably alanine, further preferably a polypeptide comprising an amino acid sequence in which any 1, 2, 3, 4, 5, 6, 7 or 8 of the amino acids at positions 1-4, 6-8 and 11 of SEQ ID NO: 309 are substituted by other amino acids, preferably alanine.

In another embodiment, a polypeptide comprising the amino acid sequence of XXXXXXKXYXQMSSX (SEQ ID NO: 612), preferably a polypeptide comprising an amino acid sequence in which any 1, 2, 3, 4, 5, 6, 7, 8 or 9 of the amino acids at positions 1-6, 8, 10 and 15 of SEQ ID NO: 309 are substituted by other amino acids, preferably alanine.

In yet another embodiment, a polypeptide comprising the amino acid sequence of PXXXYXXMSS (SEQ ID NO: 606), preferably a polypeptide comprising the amino acid sequence of PXXXYLXMSS (SEQ ID NO: 607), more preferably a polypeptide comprising an amino acid sequence in which any 1, 2, 3, 4 or 5 of the amino acids at positions 2-4, 6 and 7 of SEQ ID NO: 608 are substituted by other amino acids, preferably alanine, further preferably a polypeptide comprising an amino acid sequence in which any 1, 2, 3 or 4 of the amino acids at positions 2-4 and 7 of SEQ ID NO: 608 are substituted by other amino acids, preferably alanine.

(3β-7) a polypeptide comprising the amino acid sequence of XXLXDXXXXXXXXXX (SEQ ID NO: 620), preferably a polypeptide comprising the amino acid sequence of XXLXDXXXXLXXMXX (SEQ ID NO: 621), more preferably a polypeptide comprising an amino acid sequence in which any 1, 2, 3, 4, 5, 6, 7, 8, 9, 10, 11, 12 or 13 of the amino acids at positions 1, 2, 4 and 6-15 of SEQ ID NO: 310 are substituted by other amino acids, preferably alanine, further preferably a polypeptide comprising an amino acid sequence in which any 1, 2, 3, 4, 5, 6, 7, 8, 9, 10 or 11 of the amino acids at positions 1, 2, 4, 6-9, 11, 12, 14 and 15 of SEQ ID NO: 310 are substituted by other amino acids, preferably alanine.

In another embodiment, a polypeptide comprising the amino acid sequence of LXDXXXXXX (SEQ ID NO: 614), preferably a polypeptide comprising the amino acid sequence of LXDXXXXLX (SEQ ID NO: 615), more preferably a polypeptide comprising an amino acid sequence in which any 1, 2, 3, 4, 5, 6 or 7 of the amino acids at positions 2 and 4-9 of SEQ ID NO: 616 are substituted by other amino acids, preferably alanine, further preferably a polypeptide comprising an amino acid sequence in which any 1, 2, 3, 4, 5 or 6 of the amino acids at positions 2, 4-7 and 9 of SEQ ID NO: 616 are substituted by other amino acids, preferably alanine.

In another embodiment, a polypeptide comprising the amino acid sequence of DXXXXLXXMX (SEQ ID NO: 618), preferably a polypeptide comprising an amino acid sequence in which any 1, 2, 3, 4, 5, 6 or 7 of the amino acids at positions 2-5, 7, 8 and 10 of SEQ ID NO: 619 are substituted by other amino acids, preferably alanine.

(3β-8) a polypeptide comprising the amino acid sequence of LHLXXXXXXXXXXXX (SEQ ID NO: 625), preferably a polypeptide comprising an amino acid sequence in which any 1, 2, 3, 4, 5, 6, 7, 8, 9, 10, 11 or 12 of the amino acids at positions 4-15 of SEQ ID NO: 311 are substituted by other amino acids, preferably alanine.

### (4β) Epitope of vitellogenin-1/lipovitellin-1

In the present invention, the polypeptide (4β) can be any polypeptide selected from the group consisting of (4β-1) to (4β-7) as defined below:
(4β-1) a polypeptide comprising at least one amino acid sequence selected from the group consisting of SEQ ID NOs: 312-318, 627, 630, 631, 632, 636, 637 (amino acid sequence: ELL), 641, 642, 644, 647, 649, 657, 658, 659, 660, 663, 664, 665, 668, 669, 672, 674, 676, 680 and 681.

(4β-2) a polypeptide comprising the amino acid sequence of XXXXXMFXQELLFGX (SEQ ID NO: 633), preferably a polypeptide comprising the amino acid sequence of XXXXXMFGQELLFGX (SEQ ID NO: 634), more preferably a polypeptide comprising an amino acid sequence in which any 1, 2, 3, 4, 5, 6 or 7 of the amino acids at positions 1-5, 8 and 15 of SEQ ID NO: 312 are substituted by other amino acids, preferably alanine, further preferably a polypeptide comprising an amino acid sequence in which any 1, 2, 3, 4, 5 or 6 of the amino acids at positions 1-5 and 15 of SEQ ID NO: 312 are substituted by other amino acids, preferably alanine.

In another embodiment, a polypeptide comprising the amino acid sequence of XXXXXMXXXXXLXXX (SEQ ID NO: 639), preferably a polypeptide comprising the amino acid sequence of SXXXXMFXXXXLXXX (SEQ ID NO: 638), more preferably a polypeptide comprising an amino acid sequence in which any 1, 2, 3, 4, 5, 6, 7, 8, 9, 10, 11 or 12 of the amino acids at positions 1-5, 8-11 and 13-15 of SEQ ID NO: 312 are substituted by other amino acids, preferably alanine, further preferably a polypeptide comprising an amino acid sequence in which any 1, 2, 3, 4, 5, 6, 7, 8, 9, 10 or 11 of the amino acids at positions 2-5, 7-11 and 13-15 of SEQ ID NO: 312 are substituted by other amino acids, preferably alanine.

In yet another embodiment, a polypeptide comprising the amino acid sequence of XXYXXMFXXXXXFXX (SEQ ID NO: 643), preferably a polypeptide comprising an amino acid sequence in which any 1, 2, 3, 4, 5, 6, 7, 8, 9, 10, 11 or 12 of the amino acids at positions 1, 2, 4, 5, 8-12, 14 and 15 of SEQ ID NO: 312 are substituted by other amino acids, preferably alanine.

In yet another embodiment, a polypeptide comprising the amino acid sequence of XXXXXMFG (SEQ ID NO: 626), preferably a polypeptide comprising an amino acid sequence in which any 1, 2, 3, 4 or 5 of the amino acids at positions 1-5 of SEQ ID NO: 627 are substituted by other amino acids, preferably alanine.

In yet another embodiment, a polypeptide comprising the amino acid sequence of XXXMFXQELL (SEQ ID NO: 628), preferably a polypeptide comprising the amino acid sequence of XXXMFGQELL (SEQ ID NO: 629), more preferably a polypeptide comprising an amino acid sequence in which any 1, 2, 3 or 4 of the amino acids at positions 1-3 and 6 of SEQ ID NO: 630 are substituted by other amino acids, preferably alanine, further preferably a polypeptide comprising an amino acid sequence in which any 1, 2 or 3 of the amino acids at positions 1-3 of SEQ ID NO: 630 are substituted by other amino acids, preferably alanine.

In yet another embodiment, a polypeptide comprising the amino acid sequence of SXXXXMFX (SEQ ID NO: 635), preferably a polypeptide comprising an amino acid sequence in which any 1, 2, 3, 4 or 5 of the amino acids at positions 2-5 and 8 of SEQ ID NO: 636 are substituted by other amino acids, preferably alanine.

In yet another embodiment, a polypeptide comprising the amino acid sequence of XXYXXMF (SEQ ID NO: 640), preferably a polypeptide comprising an amino acid sequence in which any 1, 2, 3 or 4 of the amino acids at positions 1, 2, 4 and 5 of SEQ ID NO: 641 are substituted by other amino acids, preferably alanine.

(4β-3) a polypeptide comprising the amino acid sequence of XXXXXXXXDTLXXXX (SEQ ID NO: 650), preferably a polypeptide comprising the amino acid sequence of XXXXRXXXDTLXXXX (SEQ ID NO: 651), more preferably a polypeptide comprising an amino acid sequence in which any 1, 2, 3, 4, 5, 6, 7, 8, 9, 10, 11 or 12 of the amino acids at positions 1-8 and 12-15 of SEQ ID NO: 313 are substituted by other amino acids, preferably alanine, further preferably a polypeptide comprising an amino acid sequence in which any 1, 2, 3, 4, 5, 6, 7, 8, 9, 10 or 11 of the amino acids at positions 1-4, 6-8 and 12-15 of SEQ ID NO: 313 are substituted by other amino acids, preferably alanine.

In another embodiment, a polypeptide comprising the amino acid sequence of XXXXDTLX (SEQ ID NO: 645), preferably a polypeptide comprising the amino acid sequence of RXXXDTLX (SEQ ID NO: 646), more preferably a polypeptide comprising an amino acid sequence in which any 1, 2, 3, 4 or 5 of the amino acids at positions 1-4 and 8 of SEQ ID NO: 647 are substituted by other amino acids, preferably alanine, further preferably a polypeptide comprising an amino acid sequence in which any 1, 2, 3 or 4 of the amino acids at positions 2-4 and 8 of SEQ ID NO: 647 are substituted by other amino acids, preferably alanine.

In another embodiment, a polypeptide comprising the amino acid sequence of DTLXXXXX (SEQ ID NO: 648), preferably a polypeptide comprising an amino acid sequence in which any 1, 2, 3, 4 or 5 of the amino acids at positions 4-8 of SEQ ID NO: 649 are substituted by other amino acids, preferably alanine.

(4β-4) a polypeptide comprising the amino acid sequence of XXXSKTAXVXXXXXX (SEQ ID NO: 652), preferably a polypeptide comprising an amino acid sequence in which any 1, 2, 3, 4, 5, 6, 7, 8, 9 or 10 of the amino acids at positions 1-3, 8 and 10-15 of SEQ ID NO: 315 are substituted by other amino acids, preferably alanine.

In another embodiment, a polypeptide comprising the amino acid sequence of XXXSXXFAXXRXXXX (SEQ ID NO: 653), preferably a polypeptide comprising an amino acid sequence in which any 1, 2, 3, 4, 5, 6, 7, 8, 9, 10 or 11 of the amino acids at positions 1-3, 5, 6, 9, 10 and 12-15 of SEQ ID NO: 315 are substituted by other amino acids, preferably alanine.

In another embodiment, a polypeptide comprising the amino acid sequence of XXXSXXFAXXXXIXD (SEQ ID NO: 654), preferably a polypeptide comprising an amino acid sequence in which any 1, 2, 3, 4, 5, 6, 7, 8, 9 or 10 of the amino acids at positions 1-3, 5, 6, 9-12 and 14 of SEQ ID NO: 315 are substituted by other amino acids, preferably alanine.

(4β-5) a polypeptide comprising the amino acid sequence of XNIXXXAAXXXXPXX (SEQ ID NO: 655), preferably a polypeptide comprising an amino acid sequence in which any 1, 2, 3, 4, 5, 6, 7, 8, 9 or 10 of the amino acids at positions 1, 4-6, 9-12, 14 and 15 of SEQ ID NO: 316 are substituted by other amino acids, preferably alanine.

In another embodiment, a polypeptide comprising the amino acid sequence of XXXXXXAASXMXPXX (SEQ ID NO: 656), preferably a polypeptide comprising an amino acid sequence in which any 1, 2, 3, 4, 5, 6, 7, 8, 9 or 10 of the amino acids at positions 1-6, 10, 12, 14 and 15 of SEQ ID NO: 316 are substituted by other amino acids, preferably alanine.

(4β-6) a polypeptide comprising the amino acid sequence of PXXXXMXXDXXXASG (SEQ ID NO: 661), preferably a polypeptide comprising the amino acid sequence of PXIMXMXFDSXSASG (SEQ ID NO: 662), more preferably a polypeptide comprising an amino acid sequence in which any 1, 2, 3, 4, 5, 6, 7, 8 or 9 of the amino acids at positions 2-5, 7, 8 and 10-12 of SEQ ID NO: 317 are substituted by other amino acids, preferably alanine, further preferably a polypeptide comprising an amino acid sequence in which any 1, 2, 3 or 4 of the amino acids at positions 2, 5, 7 and 11 of SEQ ID NO: 317 are substituted by other amino acids, preferably alanine.

In another embodiment, a polypeptide comprising the amino acid sequence of XXXXXXXXXXXSASG (SEQ ID NO: 666), preferably a polypeptide comprising an amino acid sequence in which any 1, 2, 3, 4, 5, 6, 7, 8, 9, 10 or 11 of the amino acids at positions 1-11 of SEQ ID NO: 317 are substituted by other amino acids, preferably alanine.

(4β-7) a polypeptide comprising the amino acid sequence of XXXXXXXDVPIEKIQ (SEQ ID NO: 670), preferably a polypeptide comprising the amino acid sequence of XXXXXYSDVPIEKIQ (SEQ ID NO: 671), more preferably a polypeptide comprising an amino acid sequence in which any 1, 2, 3, 4, 5, 6 or 7 of the amino acids at positions 1-7 of SEQ ID NO: 318 are substituted by other amino acids, preferably alanine, further preferably a polypeptide comprising an amino acid sequence in which any 1, 2, 3, 4 or 5 of the amino acids at positions 1-5 of SEQ ID NO: 318 are substituted by other amino acids, preferably alanine.

In another embodiment, a polypeptide comprising the amino acid sequence of XFXXVXSXXXXXXXQ (SEQ ID NO: 675), preferably a polypeptide comprising an amino acid sequence in which any 1, 2, 3, 4, 5, 6, 7, 8, 9, 10 or 11 of the amino acids at positions 1, 3, 4, 6 and 8-14 of SEQ ID NO: 318 are substituted by other amino acids, preferably alanine.

In yet another embodiment, a polypeptide comprising the amino acid sequence of XXXXXXSXVXIXKXQ (SEQ ID NO: 677), preferably a polypeptide comprising the amino acid sequence of XXXXXXSDVXIXKIQ (SEQ ID NO: 678), more preferably a polypeptide comprising an amino acid sequence in which any 1, 2, 3, 4, 5, 6, 7, 8 or 9 of the amino acids at positions 1-6, 8, 10 and 12 of SEQ ID NO: 318 are substituted by other amino acids, preferably alanine, further preferably a polypeptide comprising an amino acid sequence in which any 1, 2, 3, 4, 5 or 6 of the amino acids at positions 1-6, 10 and 12 of SEQ ID NO: 318 are substituted by other amino acids, preferably alanine.

In another embodiment, a polypeptide comprising the amino acid sequence of SFKPVXXXXXXXXXX (SEQ ID NO: 682), preferably a polypeptide comprising an amino acid sequence in which any 1, 2, 3, 4, 5, 6, 7, 8, 9 or 10 of the amino acids at positions 6-15 of SEQ ID NO: 318 are substituted by other amino acids, preferably alanine.

In yet another embodiment, a polypeptide comprising the amino acid sequence of XXDVPIEK (SEQ ID NO: 667), preferably a polypeptide comprising an amino acid sequence in which any 1 or 2 of the amino acids at positions 1 and 2 of SEQ ID NO: 668 are substituted by other amino acids, preferably alanine.

In yet another embodiment, a polypeptide comprising the amino acid sequence of XSXVXXEX (SEQ ID NO: 673), preferably a polypeptide comprising an amino acid sequence in which any 1, 2, 3, 4 or 5 of the amino acids at positions 1, 3, 5, 6 and 8 of SEQ ID NO: 674 are substituted by other amino acids, preferably alanine.

In yet another embodiment, a polypeptide comprising the amino acid sequence of SFKPVX (SEQ ID NO: 679), preferably a polypeptide comprising an amino acid sequence in which the amino acid at position 6 of SEQ ID NO: 680 is substituted by another amino acid, preferably alanine.

In one embodiment, the polypeptide (4β) may not comprise a variant or a homolog having 100% or at least 90%, at least 80% or at least 70% identity to the central sequence of vitellogenin-1 or the C-terminal moiety (SEQ ID NO: 18) of lipovitellin-1 or the full-length amino acid sequence thereof.

The lengths of the aforementioned polypeptides ((1β) to (4β) are not particularly limited. In a preferred embodiment, the lengths of the aforementioned polypeptides ((1β) to (4β) can be 500 amino acids or less, 300 amino acids or less, 200 amino acids or less,
100 amino acids or less, 50 amino acids or less, 30 amino acids or less, 20 amino acids or less, 15 amino acids or less, 10 amino acids or less, or 5 amino acids or less.

The aforementioned polypeptides (1β) to (4β) may be prepared by a technique of chemical synthesis such as solid-phase peptide synthesis. Alternatively, polypeptides comprising an epitope may be obtained by expressing them as recombinant proteins using a genetic recombination technique well known to those skilled in the art and by separating and producing them using protein producing methods well known to those skilled in the art.

### Diagnosis kit and method (2)

The present invention provides a method for providing an indicator for diagnosing an allergy in a subject, the method comprising the steps of:
(i) contacting a sample obtained from the subject with an antigen, wherein the sample is a solution comprising an IgE antibody;
(ii) detecting binding between the IgE antibody present in the sample obtained from the subject and the antigen; and
(iii) when the binding between the IgE antibody in the subject and the antigen is detected, an indicator of the fact that the subject is allergic is provided;
wherein the antigen is a polypeptide that is at least one of the aforementioned polypeptides (1β) to (4β), or a polypeptide in which two or more of the aforementioned polypeptides (1β) to (4β) are joined together via or without a spacer.

Hereinafter, the polypeptide that is at least one of the aforementioned polypeptides (1β) to (4β), or the polypeptide in which two or more of the aforementioned polypeptides (1β) to (4β) are joined together via or without a spacer is referred to as the "antigen including (1β) to (4β)" in the present specification. The type of the spacer is not particularly limited, and an ordinary spacer that is used by those skilled in the art for joining together two or more peptides can be used. The spacer may be, for example, a hydrocarbon chain such as Acp(6)-OH.

The sample obtained from a subject is as described above in the subsection titled "Diagnosis kit and method (1)".

Detection of contact and binding between the sample obtained from a subject and the antigen can be carried out by using a known method described above in the subsection titled "Diagnosis kit and method (1)", such as ELISA (Enzyme-Linked Immunosorbent Assay), sandwich immunoassay, immunoblotting, immunoprecipitation, or immunochromatography.

The antigen including (1β) to (4β) may be provided in a state immobilized on a carrier. In this case, the steps (i) and (ii) mentioned above can be carried out using ELISA, sandwich immunoassay, immunochromatography, surface plasmon resonance, or the like. The step (i) mentioned above can be carried out by contacting the sample obtained from a subject with a surface on which the antigen including (1β) to (4β) is immobilized. The IgE antibody from the subject may be used in a state immobilized on a carrier, and binding to the antigen including (1β) to (4β) may be detected by the aforementioned technique.

The antigen including (1β) to (4β) may be in a state unimmobilized on a carrier. In this case, flow cytometry or the like can be used in the aforementioned steps (i) and (ii), and the presence of IgE antibody-bound antigen including (1β) to (4β) can be confirmed with laser beam. Examples of this method include a basophil activation test (BAT) which is a method in which a surface antigen CD203c that appears when basophils are activated by the contact of the antigen including (1β) to (4β) is detected. Another example includes a histamine release test (HRT) which examines whether histamine is released by further contacting the antigen including (1β) to (4β) with blood cells in a sample.

The antigen including (1β) to (4β) is an antigen specifically binding to IgE antibodies from allergic patients. Therefore, when binding between an IgE antibody from a subject and the antigen is detected, an indicator of the fact that the subject is allergic to egg is provided.

The present invention further provides a kit for diagnosing an allergy, comprising at least one antigen including (1β) to (4β). The diagnosis kit of this invention may be used in the aforementioned method for providing an indicator for diagnosing an allergy or in a diagnosis method as described later. The diagnosis kit of this invention may comprise not only the at least one antigen including (1β) to (4β), but also an anti-IgE antibody labeled with an enzyme and a chromogenic or luminescent substrate serving as a substrate for the enzyme. Also, a fluorescent-labeled anti-IgE antibody may be used. In the diagnosis kit of this invention, the antigen including (1β) to (4β) may be provided in a state immobilized on a carrier. The diagnosis kit of this invention may also be provided together with instructions on the procedure for diagnosis or a package containing said instructions.

In another embodiment, the aforementioned diagnosis kit comprises a companion diagnostic agent for an allergy. The companion diagnostic agent is used for identifying patients expected to respond to pharmaceutical products or identifying patients having the risk of severe adverse reactions to pharmaceutical products, or for studying the reactivity of pharmaceutical products in order to optimize treatment using the pharmaceutical products. Here, the optimization of treatment includes, for example, determination of dosage and administration, judgment regarding discontinuation of administration, and confirmation of an allergen component that is used to cause immunological tolerance.

The present invention further provides a composition for diagnosing an allergy, comprising at least one antigen including (1β) to (4β). The diagnosis composition of this invention can be used in a diagnosis method as described below. The diagnosis composition of this invention may further comprise a pharmaceutically acceptable carrier and/or additives commonly used with the antigen of this invention depending on the need.

In one embodiment, the present invention provides a method for diagnosing an allergy in a subject, the method comprising:
(i) contacting a sample obtained from the subject with an antigen;
(ii) detecting binding between an IgE antibody present in the sample obtained from the subject and the antigen; and
(iii) when the binding between the IgE antibody in the subject and the antigen is detected, diagnosing the subject as being allergic;
wherein the antigen is at least one of the proteins defined as the antigen including (1β) to (4β). In this method, the steps (i) and (ii) are performed as described above regarding the corresponding steps of the method for providing an indicator for diagnosing an allergy.

In another embodiment, the present invention provides a method for diagnosing an allergy in a subject, the method comprising administering to the subject at least one antigen including (1β) to (4β). This method may be performed in the form of a skin test characterized by applying the antigen including (1β) to (4β) onto the skin. Examples of the skin test include various forms of tests, such as: a prick test in which a diagnosis composition is applied onto the skin and then a tiny prick to such an extent as not to provoke bleeding is made in the skin to allow the antigen including (1β) to (4β) to penetrate the skin, thereby observing a skin reaction; a scratch test in which a diagnosis composition is applied onto the skin and then the skin is lightly scratched to observe a reaction; a patch test in which a diagnosis composition in the form of cream, ointment, etc. is applied onto the skin to observe a reaction; and an intracutaneous test in which the antigen including (1β) to (4β) is administered intracutaneously to observe a reaction. If a skin reaction such as swelling occurs in a skin portion to which the antigen including (1β) to (4β) has been applied, the subject of interest is diagnosed as having an allergy. The amount of the antigen including (1β) to (4β) to be applied to the skin in such tests can be, for example, not more than 100 µg per dose.

In the process of allergy diagnosis, a load test aiming to identify an antigen is often adopted. At least one antigen including (1β) to (4β) can be used as an active ingredient for a load test to diagnose an allergy. Here, the allergen component to be used in the load test may be a polypeptide that has been expressed and purified and may be a protein that has been expressed in food or cooking ingredients, such as rice-based vaccine expressing pollen allergens which are obtained by transforming rice with a gene of a cedar pollen antigen and expressing the antigen protein in the rice.

In still another embodiment, the present invention provides at least one antigen including (1β) to (4β), intended for use in the diagnosis of an allergy.

In still another embodiment, the present invention provides use of at least one antigen including (1β) to (4β) for the production of a diagnostic agent for an allergy.

In this subsection, the allergy to be diagnosed or detected can be allergies to the antigen including (1β) to (4β). Thus, detection of the allergy can be detection of not only an allergy to a single antigen including (1β) to (4β), but also allergies including cross-reactivity.

### Pharmaceutical composition and treatment method (2)

The present invention provides a pharmaceutical composition comprising at least one antigen including (1β) to (4β). In one embodiment, the aforementioned pharmaceutical composition is used for the treatment of an allergy. The treatment of an allergy increases the limit amount of an antigen in which the allergy does not develop even if the antigen is incorporated into the body, and finally aims for the state where the allergy does not develop by the common amount of the antigen to be consumed (remission).

The present invention also provides a method for treating an allergy, the method comprising administering at least one antigen including (1β) to (4β) to a patient in need of a treatment for an allergy.

In another embodiment, the present invention provides at least one antigen including (1β) to (4β), intended for use in the treatment for an allergy. In yet another embodiment, the present invention provides use of at least one antigen including (1β) to (4β) for the production of a therapeutic agent for an allergy.

In the process of allergy treatment, a hyposensitization therapy aiming to induce immunological tolerance by administering an antigen to a patient is often adopted. The at least one antigen including (1β) to (4β) can be used as an active ingredient for hyposensitization therapy for an allergy. Here, the allergen component to be used in the hyposensitization therapy may be a polypeptide that has been expressed and purified and may be a polypeptide that has been expressed in food or a cooking ingredient, such as rice-based vaccine expressing pollen allergens.

The administration route, administration dose, frequency and/or period of the pharmaceutical composition of this invention, and other ingredients to be contained in the pharmaceutical composition, and the dosage form can be as described above in the subsection titled "Pharmaceutical composition and treatment method (1)". In the case of using the antigen including (1β) to (4β), for example, the dose to an adult patient may be a dose of not more than 100 µg per dose.

In this subsection, the allergy to be treated can be allergies to the antigen including (1β) to (4β). Thus, treatment of the allergy can be treatment of not only an allergy to a single antigen including (1β) to (4β), but also allergies including cross-reactivity.

### Tester (2)

The present invention provides a tester comprising an antibody for at least one antigen including (1β) to (4β).

The antibody can be prepared by a conventional method. For example, the antibody may be prepared by immunizing a mammal such as rabbit with the aforementioned antigen including (1β) to (4β). The antibody may be an Ig antibody, a polyclonal antibody, a monoclonal antibody, or an antigen-binding fragment thereof (e.g., Fab, F(ab')₂, Fab').

Further, in the aforementioned tester, the antibody may be provided in a form bound to a carrier. The type of the carrier is not particularly limited as long as it is usable for detection of binding between an antibody and the antigen including (1β) to (4β). Any given carrier known to those skilled in the art can be used. Also, the antibody for the antigen including (1β) to (4β) is preferably an antibody for the epitopes described above in the subsection titled "Epitope of antigen". This can attain a tester that can also detect cross-reactivity.

Examples of a method for determining the presence or absence of the antigen including (1β) to (4β) include the following methods:
a method in which a prepared tester comprising an antibody is contacted with a sample obtained from a raw material, a processed product, etc., ELISA or the like is used to detect whether there is a binding between the antibody and the antigen including (1β) to (4β) in the sample, and if the binding between the antibody and the antigen including (1β) to (4β) is detected, it is determined that the antigen remains in the raw material, the processed product, etc. of interest; and
a method in which filter paper is impregnated with a raw material, a processed product, etc. and reacted with an antibody solution so as to detect the antigen including (1β) to (4β) contained therein.

Another embodiment of the present invention includes a tester for determining the presence or absence of the antigen including (1β) to (4β) of an allergy in an object of interest, the tester comprising a primer appropriate for an epitope. The primer is not limited and may be designed so as to comprise, for example, a portion of the nucleotide sequence of a nucleic acid encoding any of the amino acid sequences defined above in (1β) to (4β), or a complementary strand thereof. Alternatively, the primer may be designed so as to be the nucleotide sequence of a region upstream of a portion encoding an epitope that is any of the amino acid sequences defined above in (1β) to (4β), in a nucleic acid encoding a protein comprising the epitope, or the nucleotide sequence of a complementary strand of a region downstream of the portion encoding the epitope. Examples of such a primer include a primer which is a portion of at least one of the nucleotide sequences of SEQ ID NOs: 46, 58, 105, 151, 236, 250 and 261 and/or a primer which is a portion of a sequence complementary to at least one of the nucleotide sequences of SEQ ID NOs: 46, 58, 105, 151, 236, 250 and 261. Here, the position of the epitope in the full-length sequence of an antigen is as defined in Table 2 of Example 6 given below. Particularly, when mRNA is of interest, the tester has a complementary primer of a poly-A tail.

For example, DNA is amplified by PCR (Polymerase Chain Reaction) including RT-PCR using templated DNA or mRNA obtained from a sample and the aforementioned primer, and the presence or absence of a nucleic acid encoding the amino acid sequences defined above in (1β) to (4β) in the sequence of the amplified DNA is determined to determine the presence or absence of the antigen including (1β) to (4β). Amplification methods by PCR for mRNA of interest can be exemplified by RACE.

In one embodiment, the aforementioned tester is used to determine the presence or absence of the antigen including (1β) to (4β) in raw materials or processed products of interest in a food production line. The raw material may be a cooking ingredient or may be a cosmetic raw material, a pharmaceutical raw material or the like. The processed product may be an edible processed product or may be a cosmetic, a pharmaceutical product or the like. The tester may also be used for search for organism species contained in raw materials, may be used for quality inspection of production lines and pre-shipment products by manufacturers, or may be used for self-checking of the presence or absence of an antigen in raw materials or processed products of interest by consumers or users.

### Allergen-free raw material and the like (2)

The present invention provides a raw material or a processed product in which at least one antigen including (1β) to (4β) is eliminated or reduced.

The method for eliminating or reducing the antigen of the present invention in a raw material or a processed product is not limited. The elimination or reduction of the antigen may be conducted by any method, as long as the method permits the elimination or reduction of the antigen including (1β) to (4β). For example, the techniques described above in the subsection titled "Allergen-free food and the like (1)" may be used.

Elimination or reduction of at least one antigen including (1β) to (4β) may be achieved by eliminating or reducing the whole antigen or may be achieved by cleaving or removing the sequence moiety defined in (1β) to (4β) from the antigen protein. The "removal" includes deletion and modification of the whole or a portion of a sequence moiety defined above in (1) to (6).

For example, the raw material in which the antigen including (1β) to (4β) is eliminated or reduced may be obtained by preparing a raw material in which the expression of the antigen including (1β) to (4β) is knocked out, using a gene knock-out technique. Any technique known to those skilled in the art such as genetic modification can be used as the gene knock-out technique.

The processed product in which the antigen including (1β) to (4β) is eliminated or reduced may be a processed product of the raw material in which the antigen including (1β) to (4β) is eliminated or reduced, such as powdered milk obtained with purified peptide as a raw material. In the case of using an ordinary raw material, a treatment for eliminating or reducing the antigen including (1β) to (4β) is performed before or after preparation of a processed product. The techniques described in the subsection titled "Allergen-free food and the like (1)" may be used as methods for eliminating or reducing the antigen including (1β) to (4β) in a processed product obtained with an ordinary raw material. Examples of the method for cleaving the antigen including (1β) to (4β) include a method in which the antigen is treated by cleavage with a particular digestive enzyme.

### Method for producing allergen-free processed product (2)

The present invention provides a method for producing a processed product in which an antigen is eliminated or reduced, the method comprising the step of confirming that the antigen is eliminated or reduced, in a production process of the processed product, wherein the antigen is at least one of the aforementioned antigens including (1β) to (4β).

In the production method, elimination or reduction of an antigen means that at least one antigen including (1β) to (4β) is eliminated or reduced, or the sequence moiety defined in (1β) to (4β) are cleaved or removed from the antigen.

A technique of confirming that the antigen is eliminated or reduced in the production process of the processed product is not particularly limited, and any technique capable of detecting at least one of the aforementioned antigen including (1β) to (4β) may be used. For example, the presence or absence of the polypeptide or the antigen in the processed product may be confirmed from the binding activity of an antibody for at least one of the aforementioned antigen including (1β) to (4β) against a sample containing a material resulting from the production process of the processed product. Details of such a method are as described above in the subsection titled "Diagnosis kit and method (2)". Thus, in the production method, the "IgE antibody from a subject" described above in the subsection titled "Diagnosis kit and method (2)" is replaced with the "antibody for at least one of the aforementioned antigen including (1β) to (4β)", and the "antigen" described above in the subsection titled "Diagnosis kit and method (2)" is replaced with the "sample containing a material resulting from the production process of the processed product". The techniques described above in the subsection titled "Diagnosis kit and method (2)" can be used to confirm that the antigen is eliminated or reduced in the production process of the processed product. The testers described above in the subsection titled "Tester (2)" can also be used.

### EXAMPLES

The following describes examples of the present invention. The technical scope of this invention is not limited by these examples.

### Example 1: Confirmation of a protein pattern

Proteins contained in eggs were investigated using a two-dimensional electrophoresis method described below.

### Protein extraction

Raw chicken eggs were separated into albumen and yolk.

Extraction and purification of proteins contained in albumen and yolk were carried out as follows. The proteins were extracted by adding a solubilizer to albumen. Then, water or a urea buffer was added thereto to obtain a liquid protein extract. The constituents of the urea buffer are as mentioned below.
30 mM Tris
2 M thiourea
7 M urea
4% (w/v) CHAPS:
3-[(3-cholamidopropyl)dimethylammonio]propanesulfonate
Moderate amount of dilute hydrochloric acid

The total amount was adjusted to 100 mL by the addition of distilled water. The pH was 8.5.

Then, 25 µg (by weight) each of the proteins was mixed to obtain a liquid extract. The proteins were extracted by adding a surfactant buffer (Mammalian Lysis Buffer (MCLI), Sigma-Aldrich Co. LLC) as a solubilizer to yolk.

Thereafter, the precipitation procedure was repeated twice using a 2D-CleanUP Kit (produced by GE). In the first round of precipitation, the collected liquid protein extract was precipitated by adding TCA (trichloroacetic acid) thereto and the precipitated product produced by this procedure (TCA-precipitated product) was collected. In the second round of precipitation, the TCA-precipitated product collected above was further precipitated by adding acetone thereto and the precipitated product (sample) produced by this procedure was collected.

### Preparation of a sample solution

Part of the collected sample (50 µg on a protein weight basis) was dissolved in 150 µL of a DeStreak Rehydration Solution (produced by GE), which is a swelling buffer for first-dimensional isoelectric focusing gels, thereby obtaining a sample solution for first-dimensional isoelectric focusing (sample solution for swelling). The constituents of the DeStreak Rehydration Solution are as mentioned below.
7M thiourea
2M urea
4% (w/v) of CHAPS
0.5% (v/v) IPG buffer; produced by GE
Moderate amount of BPB (bromophenol blue)

### Penetration of the sample into first-dimensional isoelectric focusing gels

First-dimensional isoelectric focusing gel strips (Immobiline Drystrip IPG gels (pH3-10NL); produced by GE) were immersed in 140 µL of the foregoing sample solution for first-dimensional isoelectric focusing (sample solution for swelling) and impregnated with the solution at room temperature overnight.

In this example, an IPGphor electrophoresis system produced by GE was used.

An electrophoresis tray was filled with silicone oil. Filter paper moisten with water was positioned at both ends of the gel strips impregnated with the sample, and the gel strips were set in the electrophoresis tray such that the gel strips were covered with silicone oil. Electrodes were placed on the gel strips with the filter paper intervening therebetween.

The maximum current of the isoelectric focusing system was set to 75 µA per gel strip, and the first-dimensional isoelectric focusing was carried out according to the following voltage program: (1) a constant voltage step was performed at a constant voltage of 300 V until the volt-hours reached 750 Vhr (the current variation width during electrophoresis for 30 minutes before the end of this step was 5 µA); (2) the voltage was increased gradually to 1000 V for 300 Vhr; (3) the voltage was further increased gradually to 5000 V for 4500 Vhr; and then (4) the voltage was held at a constant voltage of 5000 V until the total Vhr reached 12000.

### SDS equilibration of isoelectric focusing gels

After the aforementioned first-dimensional isoelectric focusing was done, the gel strips were taken out of the isoelectric focusing system, immersed in an equilibration buffer containing a reducing agent, and shaken at room temperature for 15 minutes. The constituents of the equilibration buffer containing the reducing agent are as mentioned below.
100 mM Tris-HCl (pH 8.0)
6M urea
30% (v/v) glycerol
2% (w/v) SDS
1% (w/v) DTT

Next, the equilibration buffer containing the reducing agent was removed, and then the gel strips were immersed in an equilibration buffer containing an alkylating agent and shaken at room temperature for 15 minutes to obtain SDS-equilibrated gels. The constituents of the equilibration buffer containing the alkylating agent are as mentioned below.
100 mM Tris-HCl (pH 8.0)
6M urea
30% (v/v) glycerol
2% (w/v) SDS
2.5% (w/v) iodoacetamide

### Second-dimensional SDS-PAGE

In this example, the XCell SureLock Mini-Cell electrophoresis system produced by Life Technologies was used. The second-dimensional electrophoresis gels used were NuPAGE 4-12% Bis-Tris Gels produced by Life Technologies. Also, an electrophoresis buffer composed of the following constituents was prepared and used.
50 mM MOPS
50 mM Tris base
0.1% (w/v) SDS
1 mM EDTA

Further, an agarose solution for gel adhesion was used in this example, which was prepared by dissolving 0.5% (w/v) Agarose S (produced by Nippon Gene Co., Ltd.) and a moderate amount of BPB (bromophenol blue) in the electrophoresis buffer.

SDS-PAGE wells were washed well with the electrophoresis buffer, and then the buffer used for the washing was removed. Next, the washed wells were charged with the fully dissolved agarose solution for gel adhesion. Next, the SDS-equilibrated gel strips were immersed in agarose and closely adhered to second-dimensional electrophoresis gels using tweezers. After it was confirmed that agarose was fully fixed with the gels being closely adhered to each other, electrophoresis was performed at a constant voltage of 200 V for about 45 minutes.

### Fluorescent staining of gels

The gels were fluorescently stained with SYPRO Ruby (produced by Life Technologies).

First, an airtight container to be used was washed well in advance with 98% (v/v) ethanol. The electrophoresed second-dimensional electrophoresis gel strips were taken out of the SDS-PAGE system, placed onto the washed airtight container, and treated twice by immersion in 50% (v/v) methanol and 7% (v/v) aqueous solution containing acetic acid for 30 minutes. Then, a further immersion treatment was done for 10 minutes, with the solution being replaced by water. Next, the second-dimensional electrophoresis gel strips were immersed in 40 mL of SYPRO Ruby and shaken at room temperature overnight. Thereafter, the SYPRO Ruby was removed, and then the second-dimensional electrophoresis gel strips were washed with water and shaken in 10% (v/v) methanol and 7% (v/v) aqueous solution containing acetic acid for 30 minutes. Further shaking was done for at least 30 minutes, with the solution being replaced by water.

### Analysis

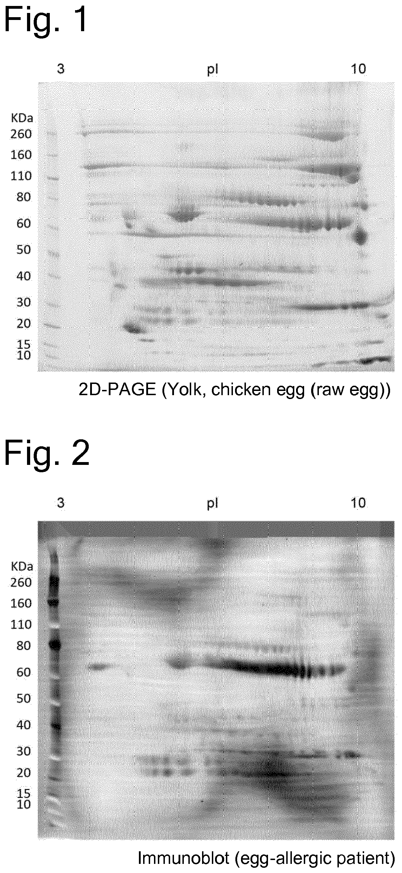

The second-dimensional electrophoresis gels obtained through the foregoing series of treatments were subjected to fluorescent image scanning on Typhoon9400 (produced by GE). The results of the two-dimensional electrophoresis as to the proteins contained in the yolk are shown in Fig. 1 (results about albumen are not shown). Molecular weight marker bands are found at the left of the photograph of the gel. The positions of the bands denote particular molecular weights (in KDa).

### Example 2: Identification of antigens by immunoblotting (1)

Identification of antigens by immunoblotting was carried out by taking all the steps up to the step of "Second-dimensional SDS-PAGE" as described above in Example 1, followed by the steps of "Transfer to membrane", "Immunoblotting" and "Analysis" as described below.

### Transfer to membrane

Transfer to membrane was done using the following transfer system and transfer buffer.
Transfer system: XCell SureLock Mini-Cell and XCell II Blot Module (produced by Life Technologies)
Transfer buffer: NuPAGE Transfer Buffer (X20) (produced by Life Technologies), used in a form diluted 20-fold with milliQ water.

To be specific, proteins in the two-dimensional electrophoresis gels were transferred to a membrane (PVDF membrane) according to the following procedure.
(1) The PVDF membrane was immersed in 100% methanol followed by milliQ water, and then moved into the transfer buffer to hydrophilize the PVDF membrane.
(2) After sponge, filter paper, gels treated by second-dimensional SDS-PAGE, the hydrophilized PVDF membrane, filter paper, and sponge were put in place in this order, the transfer system was energized at a constant voltage of 30 V for one hour.

### Immunoblotting

Immunoblotting of the membrane was carried out using, as a primary antibody, a serum sample from patient (patient 1) with an egg allergy or a serum sample from a non-egg-allergic subject. This egg-allergic patient was a patient diagnosed with food-dependent exercise-induced anaphylaxis (FDEIA) caused by chicken eggs. This patient exhibited negativity to all of raw eggs, boiled eggs, and hot spring eggs in the prick test, and also exhibited negativity to albumen, yolk, and ovomucoid in the specific IgE antibody test.

Immunoblotting of the membrane was carried out according to the following procedure.
(1) The transferred membrane was shaken in a 5% skim milk/PBST solution (a PBS buffer containing 0.1% Tween 20 nonionic surfactant) at room temperature for one hour.
(2) The membrane was left to stand in a solution of 5% primary antibody serum in 5% skim milk/PBST at room temperature for one hour.
(3) The membrane was washed with a PBST solution (5 min. × 3 times).
(4) The membrane was left to stand in a 1:5000 dilution of the secondary antibody, anti-human IgE-HRP (horseradish peroxidase), with a 5% skim milk/PBST solution at room temperature for one hour.
(5) The membrane was washed with a PBST solution (5 min. × 3 times).
(6) The membrane was left to stand in Pierce Western Blotting Substrate Plus (produced by Thermo) for 5 minutes.

### Analysis

The membrane obtained through the foregoing series of treatments was subjected to fluorescent image scanning on Typhoon9500 (produced by GE).

The immunoblots obtained with the serum from the egg-allergic patient were compared with those obtained with the control serum from the non-egg-allergic subject. By immunoblotting of the proteins contained in the raw yolk using the serum from the egg-allergic patient (Fig. 2), three spots were detected, which are different from the spots detected when the serum of the non-egg-allergic subject was used and different from those of the known chicken egg allergen proteins.

The molecular weights and isoelectric points of the 3 spots are as follows (Fig. 3).
Spot 1: Molecular weight 20 to 40 kDa, pI 4.0 to 10.0
Spot 2: Molecular weight 30 to 60 kDa, pI 5.0 to 10.0
Spot 3: Molecular weight 35 to 60 kDa, pI 5.0 to 10.0

### Example 3: Mass spectrometry and identification of antigens (1)

The amino acid sequences of the antigens that form the 3 protein spots of Example 2 were identified by mass spectroscopy.

To be specific, protein extraction and mass spectroscopy were done by the following procedure.
(1) Raw yolk in a chicken egg was subjected to protein extraction, two-dimensional electrophoresis and transfer to membrane by following the procedures described in Example 1 and 2, and the resulting membrane was stained by shaking in a solution of 0.008% Direct blue in 40% ethanol and 10% acetic acid.
(2) Then, the membrane was decolorized by repeating a 5-minute treatment with 40% ethanol and 10% acetic acid three times, washed with water for 5 minutes, and then dried by air.
(3) A protein spot of interest was cut out with a clean cutter blade and put into a centrifugal tube. The cut membrane was subjected to hydrophilization with 50 µL of methanol, followed by washing with 100 µL of water twice and then centrifugal cleaning. Thereafter, 20 µL of 20 mM NH₄HCO₃ and 50% acetonitrile were added.
(4) 1 µL of 1 pmol/µL lysyl endopeptidase (produced by WAKO) was added, and the solution was left to stand at 37°C for 60 minutes and then collected in a new centrifugal tube. After 20 µL of 20 mM NH₄HCO₃ and 70% acetonitrile were added to the membrane, the membrane was immersed therein at room temperature for 10 minutes, and the resulting solution was further collected. The solution was dissolved with 0.1% formic acid and 10 µL of 4% acetonitrile and transferred to a tube.
(5) The collected solution was dried under reduced pressure, dissolved with 15 µL of solution A (a 0.1% formic acid/4% acetonitrile solution), and analyzed by mass spectroscopy (ESI-TOF6600, produced by AB Sciex).
(6) Identification of proteins based on the MS data obtained with the mass spectrometer was done by searching the NCBI or UniProt database.

### Results

The mass spectrometry of the spots resulted in the detection of the following amino acid sequences.
Spot 1: Amino acid sequence of SEQ ID NOs: 3-11
Spot 2: Amino acid sequences of SEQ ID NOs: 9-11, 14 and 16
Spot 3: Amino acid sequences of SEQ ID NOs: 19-45

Furthermore, the spots were identified as the following proteins by the analysis of the mass data obtained from the mass spectrometer for the spots at NCBI for spots 1 and 2, and at UniProt for spot 3.
Spot 1: C-terminal portion (amino acid sequence: SEQ ID NO: 2, encoding nucleotide sequence: SEQ ID NO: 1) of vitellogenin-3 (amino acid sequence: NCBI accession number: XP_015146355, encoding nucleotide sequence: GenBank accession number: XM_015290869.1)
Spot 2: C-terminal portion (amino acid sequence: SEQ ID NO: 13, encoding nucleotide sequence: SEQ ID NO: 12) of vitellogenin-3 (amino acid sequence: NCBI accession number: XP_015146355, encoding nucleotide sequence: GenBank accession number: XM_015290869.1)
Spot 3: Central sequence of vitellogenin-1 (amino acid sequence: UniProt accession number: P87498, encoding nucleotide sequence: ENA (EMBL) accession number: D89547.1), or C-terminal portion (amino acid sequence: SEQ ID NO: 18, encoding nucleotide sequence: SEQ ID NO: 17) of lipovitellin-1

### Example 4: Identification of antigens by immunoblotting (2)

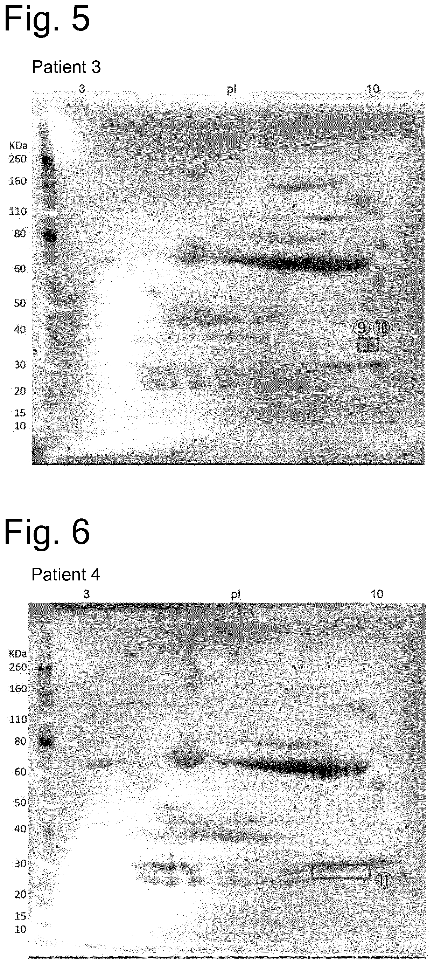

Identification of antigens by immunoblotting was carried out using the sera of three other egg-allergic patients (patient 2, patient 3 and patient 4) in the same manner as in Example 2. By immunoblotting of the proteins contained in the raw yolk using the serum from the egg-allergic patients (Fig. 4 for patient 2, Fig. 5 for patient 3, and Fig. 6 for patient 4), spots were detected, which are different from the spots detected when the serum of the non-egg-allergic subject was used and different from those of the known chicken egg allergen proteins. To be specific, spots 4 to 8 for patient 2, spots 9 and 10 for patient 3, and spot 11 for patient 4 were detected.

The molecular weights and isoelectric points of the spots 4 to 11 are as follows (Figs. 4 to 6).
Spot 4: Molecular weight 20 to 50 kDa, pI 3.0 to 8.0
Spot 5, 6: Molecular weight 80 to 260 kDa, pI 1.0 to 8.0
Spot 7: Molecular weight 110 to 300 kDa, pI 3.0 to 8.0
Spot 8: Molecular weight 160 to 550 kDa, pI 3.0 to 8.0
Spot 9: Molecular weight 20 to 50 kDa, pI 7.0 to 11.0
Spot 10: Molecular weight 20 to 50 kDa, pI 7.0 to 11.0
Spot 11: Molecular weight 15 to 40 kDa, pI 6.0 to 11.0

### Example 5: Mass spectrometry and identification of antigens (2)

The amino acid sequences of the antigens that form spots 4 to 11 of Example 4 were identified by mass spectroscopy in the same manner as in Example 3.

The mass spectrometry of the spots resulted in the detection of the following amino acid sequences.
Spot 4: Amino acid sequences of SEQ ID NOs: 48-57
Spots 5 and 6: Amino acid sequences of SEQ ID NOs: 60-104
Spot 7: Amino acid sequences of SEQ ID NOs: 107-150
Spot 8: Amino acid sequences of SEQ ID NOs: 153-235
Spot 9: Amino acid sequences of SEQ ID NOs: 238-249
Spot 10: Amino acid sequences of SEQ ID NOs: 252-260
Spot 11: Amino acid sequences of SEQ ID NOs: 263-271

Furthermore, the spots were identified as the following proteins by the analysis of the mass data obtained from the mass spectrometer for the spots at NCBI.
Spot 4: Vitellogenin-3 (amino acid sequence: NCBI accession number: XP_015146355, encoding nucleotide sequence: GenBank accession number: XM_015290869.1) (amino acid sequence: SEQ ID NO: 47, encoding nucleotide sequence: SEQ ID NO: 46)
Spots 5 and 6: Vitellogenin-2 precursor (amino acid sequence: NCBI accession number: NP_001026447.1, encoding nucleotide sequence: GenBank accession number: NM_001031276.1) (amino acid sequence: SEQ ID NO: 59, encoding nucleotide sequence: SEQ ID NO: 58)
Spot 7: Vitellogenin-2 precursor (amino acid sequence: NCBI accession number: NP_001026447.1, encoding nucleotide sequence: GenBank accession number: NM_001031276.1) (amino acid sequence: SEQ ID NO: 106, encoding nucleotide sequence: SEQ ID NO: 105)
Spot 8: Apolipoprotein B precursor (amino acid sequence: NCBI accession number: NP_001038098.1, encoding nucleotide sequence: GenBank accession number: NM_001044633.1) (amino acid sequence: SEQ ID NO: 152, encoding nucleotide sequence: SEQ ID NO: 151)
Spot 9: Apolipoprotein B precursor (amino acid sequence: NCBI accession number: NP_001038098.1, encoding nucleotide sequence: GenBank accession number: NM_001044633.1) (amino acid sequence: SEQ ID NO: 237, encoding nucleotide sequence: SEQ ID NO: 236)
Spot 10: Apolipoprotein B precursor (amino acid sequence: NCBI accession number: NP_001038098.1, encoding nucleotide sequence: GenBank accession number: NM 001044633.1) (amino acid sequence: SEQ ID NO: 251, encoding nucleotide sequence: SEQ ID NO: 250)
Spot 11: Vitellogenin-2 precursor (amino acid sequence: NCBI accession number: NP_001026447.1, encoding nucleotide sequence: GenBank accession number: NM_001031276.1) (amino acid sequence: SEQ ID NO: 262, encoding nucleotide sequence: SEQ ID NO: 261)

### Example 6: Identification of epitopes

### Epitopes of chicken-egg allergen components

Identification of epitopes was carried out by the following procedure as to the chicken-egg allergen components.

### (A) Epitope mapping(1)

Epitope mapping was carried out using a library of overlapping peptides (length: 15 amino acids) corresponding to the amino acid sequences identified as allergy components of chicken egg. Specifically, the library of overlapping peptides was prepared on the basis of the amino acid sequences of SEQ ID NOs: 59, 106 and 262 for vitellogenin-2 precursor, SEQ ID NOs: 2, 13 and 47 for vitellogenin-3, SEQ ID NOs: 152, 237 and 251 for apolipoprotein B precursor, and SEQ ID NO: 18 for vitellogenin-1 or lipovitellin-1.

The peptides to be synthesized were shifted by 10 amino acids. Thus, each peptide had an overlap of 5 amino acids with each of the peptides previous and subsequent thereto.

For preparation of peptide arrays, the Intavis CelluSpots(TM) technique was used. Specifically, the peptide arrays were prepared by the following procedure: (1) synthesizing peptides of interest on amino-modified cellulose disks using an automated chemical synthesis apparatus (Intavis MultiPep RS), (2) dissolving the amino-modified cellulose disks to obtain a cellulose-bound peptide solution, and (3) spotting the cellulose-bound peptides onto coated glass slides. The details of each procedure are as described below.

### (1) Synthesis of peptide

Peptide synthesis was carried out in incremental steps through 9-fluorenylmethoxycarbonyl (Fmoc) chemical reaction on amino-modified cellulose disks in 384-well synthesis plates. Specifically, amino acids in which a Fmoc group is bonded to an amino group were activated in a solution of N,N'-diisopropylcarbodiimide (DIC) and 1-hydroxybenzotriazole (HOBt) in dimethylformamide (DMF) and added dropwise to the cellulose disks so that the Fmoc group-bound amino acids were bound to the amino groups on the cellulose disks (coupling). Unreacted amino groups were capped with acetic anhydride and washed with DMF. Furthermore, the Fmoc groups were eliminated from the amino groups of the amino acids bound to the amino groups on the cellulose disks by treatment with piperidine and washing with DMF. The amino acids bound to the amino groups on the cellulose disks were repetitively subjected to the coupling, the capping, and the Fmoc group elimination described above to elongate the amino terminus for peptide synthesis.

### (2) Dissolution of amino-modified cellulose disk

The peptides-bound cellulose disks of interest obtained above in the subsection titled "(1) Synthesis of peptide" were transferred to 96-well plates and treated with a side chain deprotection mixed solution of trifluoroacetic acid (TFA), dichloromethane, triisopropylsilane (TIPS), and water for deprotection of amino acid side chains. Then, the deprotected cellulose-bound peptides were dissolved in a mixed solution of TFA, perfluoromethanesulfonic acid (TFMSA), TIPS, and water and precipitated in tetrabutyl methyl ether (TBME), and the precipitate was resuspended in dimethyl sulfoxide (DMSO) and mixed with a mixed solution of NaCl, sodium citrate, and water to obtain a peptide solution for slide spotting.

### (3) Spotting of cellulose-bound peptide solution

The peptide solution for slide spotting obtained above in the subsection titled "(2) Dissolution of amino-modified cellulose disk" was spotted onto Intavis CelluSpots(TM) slides using Intavis Slide Spotting Robot, and the slides were dried to prepare peptide arrays.

The presence or absence of binding, to each peptide fragment, of an IgE antibody present in the serum of a WDEIA patient was measured through antigen-antibody reaction using the peptide arrays. The measurement was carried out according to the following procedure.
(1) The peptides were shaken at room temperature for 1 hour in Pierce Protein-Free(PBS)Blocking Buffer (produced by Thermo).
(2) The peptide arrays were shaken at overnight at room temperature in 2% serum/Pierce Protein-Free(PBS)Blocking Buffer (produced by Thermo).
(3) The peptide arrays were washed with a PBST solution (a PBS buffer containing 0.1% Tween 20 nonionic surfactant) for 5 minutes (× 3).
(4) Anti-human IgE antibody-HRP(1:10,000, Pierce Protein-Free(PBS)Blocking Buffer (produced by Thermo)) was added thereto, and the peptide arrays were shaken at room temperature for 1 hour.
(5) The peptide arrays were washed with a PBST solution for 5 minutes (× 3).
(6) Pierce ECL Plus Western Blotting Substrate (produced by Thermo) was added thereto, and the peptide arrays were shaken at room temperature for 5 minutes.
(7) The chemiluminescence of the peptides treated as described above in (1) to (6) was measured using Amersham Imager 600.

Chemiluminescence intensity in images obtained by the measurement described above in (7) was converted into a numeric value using ImageQuant TL (GE Healthcare). The maximum value of numeric values determined from images obtained from results about the serum of 5 non-egg-allergic subjects was defined as a Nmax value as a value from the non-egg-allergic subjects. Numeric values determined from images obtained from results about the serum of 6 patients were divided by the Nmax value of each peptide. It was determined that a peptide having the resulting value larger than 0 and having a value of at least 30,000 in terms of the numeric values determined from images obtained from results about the serum of the patients was a peptide bound to an IgE antibody in a patient-specific manner.

### (B) Epitope mapping (2): overlapping

On the basis of the sequences (SEQ ID NOs: 272-295 for vitellogenin-2 precursor, SEQ ID NOs: 296-303 for vitellogenin-3, SEQ ID NOs: 304-311 for apolipoprotein B precursor, and SEQ ID NOs: 312-318 for vitellogenin-1 or lipovitellin-1) of the peptides bound to an IgE antibody in serum in a patient-specific manner as described above in (A), a library of overlapping peptide fragments (length: 10 amino acids) was prepared using the sequences of the peptides and sequences in which sequences were added so as to flank each peptide in the amino acid sequences of allergen components (amino acid sequences of SEQ ID NO: 59, 106 or 262 for vitellogenin-2 precursor, SEQ ID NO: 2, 13 or 47 for vitellogenin-3, SEQ ID NO: 152, 237 or 251 for apolipoprotein B, and SEQ ID NO: 18 for vitellogenin-1 or lipovitellin-1) comprising the sequences of the peptide to carry out epitope mapping.

The peptides to be synthesized were shifted by one amino acid. Thus, each peptide had an overlap of 9 amino acids with each of the peptides previous and subsequent thereto.

The library was prepared by the same procedure as described above in (A), and the presence or absence of binding of an IgE antibody present in the serum of a patient and a non-egg-allergic subject to each peptide fragment was measured by the same technique as described above. Amino acids at positions where the binding activity against IgE antibodies from patients were lost or decreased in the peptides shifted by one amino acid while binding activity against IgE antibodies from non-egg-allergic subjects arose were confirmed as amino acids adversely affecting the specificity of binding to IgE antibodies.

Chemiluminescence intensity in images obtained by measurement was converted into a numeric value in the same manner as in (A) using ImageQuant TL (GE Healthcare). Numeric values determined from images obtained as a result of using only anti-human IgE antibody-HRP antibody without carrying out the procedures (2) and (3) described above in the subsection titled (3) Spotting of cellulose-bound peptide solution were divided by numeric values determined from images obtained from results about the serum of 6 patients. It was determined that a peptide having the resulting value larger than 0 had no binding activity against IgE antibodies when having less than 20% of values obtained using the sequences (amino acid sequences of SEQ ID NO: 59, 106 or 262 for vitellogenin-2, SEQ ID NO: 2, 13 or 47 for vitellogenin-3, SEQ ID NO: 152, 237 or 251 for apolipoprotein B, and SEQ ID NO: 18 for vitellogenin-1 or lipovitellin-1) serving as the basis of overlapping, had binding activity, albeit poor, against IgE antibodies when having 20% or more and less than 50% thereof, had binding activity, albeit somewhat poor, against IgE antibodies when having 50% or more and less than 70% thereof, had almost equivalent binding activity against IgE antibodies when having 70% or more and less than 90% thereof, and had equivalent or good binding activity against IgE antibodies and thus had remaining binding activity against IgE antibodies when having 90% or more thereof.

### (C) Epitope mapping(3): alanine scanning

From the amino acid sequences identified above in (A), a library of peptide fragments in which N-terminal amino acids were substituted one by one by alanine according to a technique called alanine scanning (Non Patent Literature 1) was prepared by the same technique as described above. The presence or absence of binding of an IgE antibody present in the serum of a patient and a non-egg-allergic subject to each peptide fragment was measured by the same technique as described above. Amino acids at positions where the binding activity against IgE antibodies from patients were lost or decreased by the substitution by alanine while binding activity against IgE antibodies from non-egg-allergic subjects arose were confirmed as amino acids important for exertion of original antigenicity, or amino acids influencing exertion of original antigenicity. Also, amino acids at positions where the binding activity against IgE antibodies from patients was maintained even after the substitution by alanine while binding activity against IgE antibodies from non-egg-allergic subjects did not arise were confirmed as substitutable positions that were not important for exertion of original antigenicity,

Chemiluminescence intensity in images obtained by measurement was converted into a numeric value in the same manner as in (A) using ImageQuant TL (GE Healthcare). Numeric values determined from images obtained as a result of using only anti-human IgE antibody-HRP antibody without carrying out the procedures (2) and (3) described above in the subsection titled (3) Spotting of cellulose-bound peptide solution were divided by numeric values determined from images obtained from results about the serum of 6 patients. It was determined that a peptide having the resulting value larger than 0 had no binding activity against IgE antibodies when having less than 20% of values obtained using the sequences (amino acid sequences of SEQ ID NO: 59, 106 or 262 for vitellogenin-2, SEQ ID NO: 2, 13 or 47 for vitellogenin-3, SEQ ID NO: 152, 237 or 251 for apolipoprotein B, and SEQ ID NO: 18 for vitellogenin-1 or lipovitellin-1) serving as the basis of overlapping, had binding activity, albeit poor, against IgE antibodies when having 20% or more and less than 50% thereof, had binding activity, albeit somewhat poor, against IgE antibodies when having 50% or more and less than 70% thereof, had almost equivalent binding activity against IgE antibodies when having 70% or more and less than 90% thereof, and had equivalent or good binding activity against IgE antibodies and thus had remaining binding activity against IgE antibodies when having 90% or more thereof.

By this analysis, common sequences important for exertion of original antigenicity were found.

### (D) Results

As a result of the epitope mapping described above in (A), an IgE antibody was confirmed to bind in a patient-specific manner to each of epitope Nos. 1-24 (peptides having the amino acid sequences of SEQ ID NOs: 272-295, respectively) for vitellogenin-2 precursor, epitope Nos. 25-32 (peptides having the amino acid sequences of SEQ ID NOs: 296-303, respectively) for vitellogenin-3, epitope Nos. 33-40 (peptides having the amino acid sequences of SEQ ID NOs: 304-311, respectively) for apolipoprotein B precursor, and epitope Nos. 41-47 (peptides having the amino acid sequences of SEQ ID NOs: 312-318, respectively) for vitellogenin-1 or lipovitellin-1. Portions to which the peptide sequences of these epitopes correspond in the sequences of the allergen components identified in Examples 2 to 5 are shown in Table 2.

**[Table 2-1]**

| Epitope No. | Protein name | Sequence of epitope | Position in sequence of allergen component |
|---|---|---|---|
| 1 | Vitellogenin-2 | SEQ ID NO: 272 | Amino acids 71-85 of SEQ ID NO: 59 or 106 |
| 2 | Vitellogenin-2 | SEQ ID NO: 273 | Amino acids 141-155 of SEQ ID NO: 59 or 106 |
| 3 | Vitellogenin-2 | SEQ ID NO: 274 | Amino acids 471-485 of SEQ ID NO: 59 or 106 |
| 4 | Vitellogenin-2 | SEQ ID NO: 275 | Amino acids 521-535 of SEQ ID NO: 59 or 106 |
| 5 | Vitellogenin-2 | SEQ ID NO: 276 | Amino acids 611-625 of SEQ ID NO: 59 or 106 |
| 6 | Vitellogenin-2 | SEQ ID NO: 277 | Amino acids 651-665 of SEQ ID NO: 59 or 106 |
| 7 | Vitellogenin-2 | SEQ ID NO: 278 | Amino acids 661-675 of SEQ ID NO: 59 or 106 |
| 8 | Vitellogenin-2 | SEQ ID NO: 279 | Amino acids 711-725 of SEQ ID NO: 59 or 106 |
| 9 | Vitellogenin-2 | SEQ ID NO: 280 | Amino acids 741-755 of SEQ ID NO: 59 or 106 |
| 10 | Vitellogenin-2 | SEQ ID NO: 281 | Amino acids 751-765 of SEQ ID NO: 59 or 106 |
| 11 | Vitellogenin-2 | SEQ ID NO: 282 | Amino acids 881-895 of SEQ ID NO: 59 or 106 |
| 12 | Vitellogenin-2 | SEQ ID NO: 283 | Amino acids 1011-1025 of SEQ ID NO: 59 or 106 |
| 13 | Vitellogenin-2 | SEQ ID NO: 284 | Amino acids 1071-1085 of SEQ ID NO: 59 or 106 |
| 14 | Vitellogenin-2 | SEQ ID NO: 285 | Amino acids 1101-1115 of SEQ ID NO: 59 or 106 |
| 15 | Vitellogenin-2 | SEQ ID NO: 286 | Amino acids 1207-1221 of SEQ ID NO: 106 |
| 16 | Vitellogenin-2 | SEQ ID NO: 287 | Amino acids 1337-1351 of SEQ ID NO: 106 |
| 17 | Vitellogenin-2 | SEQ ID NO: 288 | Amino acids 1417-1431 of SEQ ID NO: 106 |
| | | | Amino acids 41-55 of SEQ ID NO: 262 |
| 18 | Vitellogenin-2 | SEQ ID NO: 289 | Amino acids 1537-1551 of SEQ ID NO: 106 |
| | | | Amino acids 161-175 of SEQ ID NO: 262 |
| 19 | Vitellogenin-2 | SEQ ID NO: 290 | Amino acids 1587-1601 of SEQ ID NO: 106 |
| | | | Amino acids 211-225 of SEQ ID NO: 262 |
| 20 | Vitellogenin-2 | SEQ ID NO: 291 | Amino acids 1627-1641 of SEQ ID NO: 106 |
| 21 | Vitellogenin-2 | SEQ ID NO: 292 | Amino acids 1687-1701 of SEQ ID NO: 106 |
| 22 | Vitellogenin-2 | SEQ ID NO: 293 | Amino acids 1727-1741 of SEQ ID NO: 106 |
| 23 | Vitellogenin-2 | SEQ ID NO: 294 | Amino acids 1757-1771 of SEQ ID NO: 106 |

**[Table 2-2]**

| | | | |
|---|---|---|---|
| 24 | Vitellogenin-2 | SEQ ID NO: 295 | Amino acids 1797-1811 of SEQ ID NO: 106 |
| 25 | Vitellogenin-3 | SEQ ID NO: 296 | Amino acids 111-125 of SEQ ID NO: 13 |
| | | | Amino acids 14-28 of SEQ ID NO: 47 |
| 26 | Vitellogenin-3 | SEQ ID NO: 297 | Amino acids 100-114 of SEQ ID NO: 2 |
| | | | Amino acids 261-275 of SEQ ID NO: 13 |
| | | | Amino acids 164-178 of SEQ ID NO: 47 |
| 27 | Vitellogenin-3 | SEQ ID NO: 298 | Amino acids 140-154 of SEQ ID NO: 2 |
| | | | Amino acids 301-315 of SEQ ID NO: 13 |
| | | | Amino acids 204-218 of SEQ ID NO: 47 |
| 28 | Vitellogenin-3 | SEQ ID NO: 299 | Amino acids 150-164 of SEQ ID NO: 2 |
| | | | Amino acids 311-325 of SEQ ID NO: 13 |
| | | | Amino acids 214-228 of SEQ ID NO: 47 |
| 29 | Vitellogenin-3 | SEQ ID NO: 300 | Amino acids 160-174 of SEQ ID NO: 2 |
| | | | Amino acids 321-335 of SEQ ID NO: 13 |
| | | | Amino acids 224-238 of SEQ ID NO: 47 |
| 30 | Vitellogenin-3 | SEQ ID NO: 301 | Amino acids 180-194 of SEQ ID NO: 2 |
| | | | Amino acids 341-355 of SEQ ID NO: 13 |
| | | | Amino acids 244-258 of SEQ ID NO: 47 |
| 31 | Vitellogenin-3 | SEQ ID NO: 302 | Amino acids 190-204 of SEQ ID NO: 2 |
| | | | Amino acids 351-365 of SEQ ID NO: 13 |
| | | | Amino acids 254-268 of SEQ ID NO: 47 |
| 32 | Vitellogenin-3 | SEQ ID NO: 303 | Amino acids 220-234 of SEQ ID NO: 2 |
| | | | Amino acids 381-395 of SEQ ID NO: 13 |
| | | | Amino acids 284-298 of SEQ ID NO: 47 |
| 33 | Apolipoprotein B | SEQ ID NO: 304 | Amino acids 2556-2570 of SEQ ID NO: 152 |
| 34 | Apolipoprotein B | SEQ ID NO: 305 | Amino acids 131-145 of SEQ ID NO: 152 or 237 |
| 35 | Apolipoprotein B | SEQ ID NO: 306 | Amino acids 631-645 of SEQ ID NO: 152 |
| 36 | Apolipoprotein B | SEQ ID NO: 307 | Amino acids 681-695 of SEQ ID NO: 152 |

**[Table 2-3]**

| | | | |
|---|---|---|---|
| 37 | Apolipoprotein B | SEQ ID NO: 308 | Amino acids 841-855 of SEQ ID NO: 152 |
| 38 | Apolipoprotein B | SEQ ID NO: 309 | Amino acids 1131-1145 of SEQ ID NO: 152 |
| 39 | Apolipoprotein B | SEQ ID NO: 310 | Amino acids 1201-1215 of SEQ ID NO: 152 |
| 40 | Apolipoprotein B | SEQ ID NO: 311 | Amino acids 1571-1585 of SEQ ID NO: 152 |
| 41 | Vitellogenin-1/lipovitellin-1 | SEQ ID NO: 312 | Amino acids 6-20 of SEQ ID NO: 18 |
| 42 | Vitellogenin-1/lipovitellin-1 | SEQ ID NO: 313 | Amino acids 16-30 of SEQ ID NO: 18 |
| 43 | Vitellogenin-1/lipovitellin-1 | SEQ ID NO: 314 | Amino acids 36-50 of SEQ ID NO: 18 |
| 44 | Vitellogenin-1/lipovitellin-1 | SEQ ID NO: 315 | Amino acids 186-200 of SEQ ID NO: 18 |
| 45 | Vitellogenin-1/lipovitellin-1 | SEQ ID NO: 316 | Amino acids 196-210 of SEQ ID NO: 18 |
| 46 | Vitellogenin-1/lipovitellin-1 | SEQ ID NO: 317 | Amino acids 216-230 of SEQ ID NO: 18 |
| 47 | Vitellogenin-1/lipovitellin-1 | SEQ ID NO: 318 | Amino acids 306-320 of SEQ ID NO: 18 |

Amino acids important for binding of the epitope sequences to IgE antibodies from allergic patients were identified by the procedures of (B) and (C) mentioned above as to each of the epitopes.

### (1) Epitope 1

In epitope 1 (SEQ ID NO: 272), SEQ ID NO: 323 was identified as a region important for binding to IgE antibodies from patients. Further, SEQ ID NO: 319 was identified as a sequence comprising a region amino-terminally adjacent to epitope 1 and binding to IgE antibodies from patients.

In SEQ ID NO: 272, the amino acids at positions 6, 8-12, 14 and 15 were identified as amino acids particularly important for binding to IgE antibodies from allergic patients, and the amino acids at positions 4, 5, 7 and 13 were identified as amino acids influencing binding to IgE antibodies from allergic patients. The amino acids at positions 1-3 of SEQ ID NO: 272 had no influence on the binding activity against IgE antibodies from allergic patients when substituted by alanine.

In SEQ ID NO: 323, the amino acids at positions 2-6 were identified as amino acids particularly important for binding to IgE antibodies from allergic patients, and the amino acid at position 1 was identified as an amino acid influencing binding to IgE antibodies from allergic patients.

### (2) Epitope 2

In epitope 2 (SEQ ID NO: 273), SEQ ID NO: 327 was identified as a region important for binding to IgE antibodies from patients. Further, SEQ ID NO: 324 was identified as a sequence comprising a region amino-terminally adjacent to epitope 2 and binding to IgE antibodies from patients.

In SEQ ID NO: 273, the amino acids at positions 9-12 were identified as amino acids particularly important for binding to IgE antibodies from allergic patients. The amino acids at positions 1-8 and 13-15 of SEQ ID NO: 273 had no influence on the binding activity against IgE antibodies from allergic patients when substituted by alanine.

In SEQ ID NO: 327, the amino acids at positions 9 and 10 were identified as amino acids particularly important for binding to IgE antibodies from allergic patients, and the amino acids at positions 4 and 5 were identified as amino acids influencing binding to IgE antibodies from allergic patients. The amino acids at positions 1-3 and 6-8 of SEQ ID NO: 327 had no influence on the binding activity against IgE antibodies from allergic patients when substituted by alanine.

### (3) Epitope 3

In epitope 3 (SEQ ID NO: 274), SEQ ID NOs: 330, 331, 333 and 335 were identified as regions important for binding to IgE antibodies from patients. Further, SEQ ID NO: 336 was identified as a sequence comprising a region carboxy-terminally adjacent to epitope 3 and binding to IgE antibodies from patients.

In SEQ ID NO: 274, the amino acids at positions 1, 3 and 6 were identified as amino acids particularly important for binding to IgE antibodies from allergic patients. The amino acids at positions 2, 4, 5 and 7-15 of SEQ ID NO: 274 had no influence on the binding activity against IgE antibodies from allergic patients when substituted by alanine.

In SEQ ID NO: 274, the amino acids at positions 1, 6, 10 and 11 were identified as possible amino acids particularly important for binding to IgE antibodies from allergic patients. In this case, the amino acids at positions 2-5, 7-9 and 12-15 of SEQ ID NO: 274 had no influence on the binding activity against IgE antibodies from allergic patients when substituted by alanine.

In SEQ ID NO: 330, the amino acids at positions 1, 3 and 6 were identified as amino acids particularly important for binding to IgE antibodies from allergic patients. The amino acids at positions 2, 4, 5, 7 and 8 of SEQ ID NO: 330 had no influence on the binding activity against IgE antibodies from allergic patients when substituted by alanine.

In SEQ ID NO: 335, the amino acids at positions 1, 5 and 6 were identified as amino acids particularly important for binding to IgE antibodies from allergic patients. The amino acids at positions 2-4 and 7-10 of SEQ ID NO: 335 had no influence on the binding activity against IgE antibodies from allergic patients when substituted by alanine.

### (4) Epitope 4

In epitope 4 (SEQ ID NO: 275), SEQ ID NOs: 339, 343, 344, 346, 347 and
350 were identified as regions important for binding to IgE antibodies from patients. Further, SEQ ID NO: 342 was identified as a sequence comprising a region amino-terminally adjacent to epitope 4 and binding to IgE antibodies from patients, and SEQ ID NO: 351 was identified as a sequence comprising a region carboxy-terminally adjacent to epitope 4 and binding to IgE antibodies from patients.

In SEQ ID NO: 275, the amino acids at positions 1, 4-6 and 10-13 were identified as amino acids particularly important for binding to IgE antibodies from allergic patients. The amino acids at positions 2, 3, 7-9, 14 and 15 of SEQ ID NO: 275 had no influence on the binding activity against IgE antibodies from allergic patients when substituted by alanine.

In SEQ ID NO: 275, the amino acids at positions 1, 6, 12-14 were identified as possible amino acids particularly important for binding to IgE antibodies from allergic patients. In this case, the amino acids at positions 2-5, 7-11 and 15 of SEQ ID NO: 275 had no influence on the binding activity against IgE antibodies from allergic patients when substituted by alanine.

In SEQ ID NO: 275, the amino acids at positions 1, 6, 8, 14 and 15 were identified as possible amino acids particularly important for binding to IgE antibodies from allergic patients. In this case, the amino acids at positions 2-5, 7, 9-13 of SEQ ID NO: 275 had no influence on the binding activity against IgE antibodies from allergic patients when substituted by alanine.

In SEQ ID NO: 339, the amino acids at positions 1, 4-6 and 10 were identified as amino acids particularly important for binding to IgE antibodies from allergic patients. The amino acids at positions 2, 3 and 7-9 of SEQ ID NO: 339 had no influence on the binding activity against IgE antibodies from allergic patients when substituted by alanine.

In SEQ ID NO: 346, the amino acids at positions 1-6 and 10 were identified as amino acids particularly important for binding to IgE antibodies from allergic patients. The amino acids at positions 7-9 of SEQ ID NO: 346 had no influence on the binding activity against IgE antibodies from allergic patients when substituted by alanine.

In SEQ ID NO: 350, the amino acids at positions 1 and 3 were identified as amino acids particularly important for binding to IgE antibodies from allergic patients. The amino acids at positions 2 and 4-8 of SEQ ID NO: 350 had no influence on the binding activity against IgE antibodies from allergic patients when substituted by alanine.

### (5) Epitope 5

In epitope 5 (SEQ ID NO: 276), SEQ ID NOs: 355 and 356 were identified as regions important for binding to IgE antibodies from patients. Further, SEQ ID NO: 353 was identified as a sequence comprising a region amino-terminally adjacent to epitope 5 and binding to IgE antibodies from patients, and SEQ ID NO: 357 was identified as a sequence comprising a region carboxy-terminally adjacent to epitope 5 and binding to IgE antibodies from patients.

In SEQ ID NO: 276, the amino acids at positions 2, 3 and 6-15 were identified as amino acids particularly important for binding to IgE antibodies from allergic patients. The amino acids at positions 1, 4 and 5 of SEQ ID NO: 276 had no influence on the binding activity against IgE antibodies from allergic patients when substituted by alanine.

In SEQ ID NO: 355, the amino acids at positions 2-8 were identified as amino acids particularly important for binding to IgE antibodies from allergic patients. The amino acid at position 1 of SEQ ID NO: 355 had no influence on the binding activity against IgE antibodies from allergic patients when substituted by alanine.

### (6) Epitope 6

In epitope 6 (SEQ ID NO: 277), SEQ ID NOs: 366 and 369 were identified as regions important for binding to IgE antibodies from patients. Further, SEQ ID NOs: 359 and 363 were identified as sequences comprising a region amino-terminally adjacent to epitope 6 and binding to IgE antibodies from patients, and SEQ ID NO: 360 was identified as a sequence comprising a region carboxy-terminally adjacent to epitope 6 and binding to IgE antibodies from patients.

In SEQ ID NO: 277, the amino acids at positions 1, 5-9, 11 and 13-15 were identified as amino acids particularly important for binding to IgE antibodies from allergic patients, and the amino acids at positions 2 and 10 were identified as amino acids influencing binding to IgE antibodies from allergic patients. The amino acids at positions 3, 4 and 12 of SEQ ID NO: 277 had no influence on the binding activity against IgE antibodies from allergic patients when substituted by alanine.

In SEQ ID NO: 277, the amino acids at positions 3, 7, 9, 11 and 15 were identified as amino acids particularly important for binding to IgE antibodies from allergic patients, and the amino acids at positions 1, 2 and 4-6 were identified as possible amino acids influencing binding to IgE antibodies from allergic patients. In this case, the amino acids at positions 8, 10 and 12-14 of SEQ ID NO: 277 had no influence on the binding activity against IgE antibodies from allergic patients when substituted by alanine.

In SEQ ID NO: 366, the amino acids at positions 1, 5, 7 and 9 were identified as amino acids particularly important for binding to IgE antibodies from allergic patients, and the amino acids at positions 2-4 were identified as amino acids influencing binding to IgE antibodies from allergic patients. The amino acids at positions 6, 8 and 10 of SEQ ID NO: 366 had no influence on the binding activity against IgE antibodies from allergic patients when substituted by alanine.

In SEQ ID NO: 369, the amino acids at positions 2, 4, 6 and 10 were identified as amino acids particularly important for binding to IgE antibodies from allergic patients, and the amino acid at position 1 was identified as an amino acid influencing binding to IgE antibodies from allergic patients. The amino acids at positions 3, 5 and 7-9 of SEQ ID NO: 369 had no influence on the binding activity against IgE antibodies from allergic patients when substituted by alanine.

### (7) Epitope 7

In epitope 7 (SEQ ID NO: 278), SEQ ID NO: 374 was identified as a region important for binding to IgE antibodies from patients. Further, SEQ ID NO: 375 was identified as a sequence comprising a region carboxy-terminally adjacent to epitope 7 and binding to IgE antibodies from patients.

In SEQ ID NO: 278, the amino acids at positions 1-6, 8, 11, 13 and 15 were identified as amino acids particularly important for binding to IgE antibodies from allergic patients. The amino acids at positions 7, 9, 10, 12 and 14 of SEQ ID NO: 278 had no influence on the binding activity against IgE antibodies from allergic patients when substituted by alanine.

In SEQ ID NO: 374, the amino acids at positions 1, 3, 4, 6 and 9 were identified as amino acids particularly important for binding to IgE antibodies from allergic patients, and the amino acid at position 2 was identified as an amino acid influencing binding to IgE antibodies from allergic patients. The amino acids at positions 5, 7 and 8 of SEQ ID NO: 374 had no influence on the binding activity against IgE antibodies from allergic patients when substituted by alanine.

### (8) Epitope 8

In epitope 8 (SEQ ID NO: 279), SEQ ID NOs: 379, 388, 391, 392, 397 and 400 were identified as regions important for binding to IgE antibodies from patients. Further, SEQ ID NOs: 377, 384 and 385 were identified as sequences comprising a region amino-terminally adjacent to epitope 8 and binding to IgE antibodies from patients, and SEQ ID NOs: 380, 381, 393, 401 and 402 were identified as sequences comprising a region carboxy-terminally adjacent to epitope 8 and binding to IgE antibodies from patients.

In SEQ ID NO: 279, the amino acids at positions 1-5, 8-12, 14 and 15 were identified as amino acids particularly important for binding to IgE antibodies from allergic patients, and the amino acid at position 13 was identified as an amino acid influencing binding to IgE antibodies from allergic patients. The amino acids at positions 6 and 7 of SEQ ID NO: 279 had no influence on the binding activity against IgE antibodies from allergic patients when substituted by alanine.

In SEQ ID NO: 279, the amino acids at positions 3, 9 and 15 were identified as amino acids particularly important for binding to IgE antibodies from allergic patients, and the amino acid at position 4 was identified as a possible amino acid influencing binding to IgE antibodies from allergic patients. In this case, the amino acids at positions 1, 2, 5-8 and 10-14 of SEQ ID NO: 279 had no influence on the binding activity against IgE antibodies from allergic patients when substituted by alanine.

In SEQ ID NO: 279, the amino acids at positions 1-3, 8 and 9 were identified as possible amino acids particularly important for binding to IgE antibodies from allergic patients. In this case, the amino acids at positions 4-7 and 10-15 of SEQ ID NO: 279 had no influence on the binding activity against IgE antibodies from allergic patients when substituted by alanine.

In SEQ ID NO: 379, the amino acids at positions 1-4 and 7-10 were identified as amino acids particularly important for binding to IgE antibodies from allergic patients. The amino acids at positions 5 and 6 of SEQ ID NO: 379 had no influence on the binding activity against IgE antibodies from allergic patients when substituted by alanine.

In SEQ ID NO: 388, the amino acids at positions 2 and 8 were identified as amino acids particularly important for binding to IgE antibodies from allergic patients, and the amino acid at position 3 was identified as an amino acid influencing binding to IgE antibodies from allergic patients. The amino acids at positions 1, 4-7 and 9 of SEQ ID NO: 388 had no influence on the binding activity against IgE antibodies from allergic patients when substituted by alanine.

In SEQ ID NO: 392, the amino acids at positions 4 and 10 were identified as amino acids particularly important for binding to IgE antibodies from allergic patients, and the amino acid at position 8 was identified as an amino acid influencing binding to IgE antibodies from allergic patients. The amino acids at positions 1-3, 5-7 and 9 of SEQ ID NO: 392 had no influence on the binding activity against IgE antibodies from allergic patients when substituted by alanine.

In SEQ ID NO: 397, the amino acids at positions 1, 2, 7 and 8 were identified as amino acids particularly important for binding to IgE antibodies from allergic patients. The amino acids at positions 3-6, 9 and 10 of SEQ ID NO: 397 had no influence on the binding activity against IgE antibodies from allergic patients when substituted by alanine.

In SEQ ID NO: 400, the amino acids at positions 3 and 4 were identified as amino acids particularly important for binding to IgE antibodies from allergic patients, and the amino acids at positions 1, 6 and 9 were identified as amino acids influencing binding to IgE antibodies from allergic patients. The amino acids at positions 2, 5, 7, 8 and 10 of SEQ ID NO: 400 had no influence on the binding activity against IgE antibodies from allergic patients when substituted by alanine.

### (9) Epitope 9

In epitope 9 (SEQ ID NO: 280), SEQ ID NO: 407 was identified as a region important for binding to IgE antibodies from patients. Further, SEQ ID NO: 404 was identified as a sequence comprising a region amino-terminally adjacent to epitope 9 and binding to IgE antibodies from patients.

In SEQ ID NO: 280, the amino acids at positions 4-7, 9-12 and 15 were identified as amino acids particularly important for binding to IgE antibodies from allergic patients. The amino acids at positions 1-3, 8, 13 and 14 of SEQ ID NO: 280 had no influence on the binding activity against IgE antibodies from allergic patients when substituted by alanine.

In SEQ ID NO: 407, the amino acids at positions 1, 2, 4 and 5 were identified as amino acids particularly important for binding to IgE antibodies from allergic patients. The amino acid at position 3 of SEQ ID NO: 407 had no influence on the binding activity against IgE antibodies from allergic patients when substituted by alanine.

### (10) Epitope 10

In epitope 10 (SEQ ID NO: 281), SEQ ID NOs: 412 and 414 were identified as regions important for binding to IgE antibodies from patients. Further, SEQ ID NO: 409 was identified as a sequence comprising a region amino-terminally adjacent to epitope 10 and binding to IgE antibodies from patients.

In SEQ ID NO: 281, the amino acids at positions 5, 6, 8-10 and 12-15 were identified as amino acids particularly important for binding to IgE antibodies from allergic patients, and the amino acids at positions 4 and 7 were identified as amino acids influencing binding to IgE antibodies from allergic patients. The amino acids at positions 1-3 and 11 of SEQ ID NO: 281 had no influence on the binding activity against IgE antibodies from allergic patients when substituted by alanine.

In SEQ ID NO: 412, the amino acids at positions 4-8 were identified as amino acids particularly important for binding to IgE antibodies from allergic patients, and the amino acid at position 3 was identified as an amino acid influencing binding to IgE antibodies from allergic patients. The amino acids at positions 1 and 2 of SEQ ID NO: 412 had no influence on the binding activity against IgE antibodies from allergic patients when substituted by alanine.

In SEQ ID NO: 414, the amino acids at positions 1, 2, 4 and 5 were identified as amino acids particularly important for binding to IgE antibodies from allergic patients. The amino acid at position 3 of SEQ ID NO: 414 had no influence on the binding activity against IgE antibodies from allergic patients when substituted by alanine.

### (11) Epitope 11

In epitope 11 (SEQ ID NO: 282), SEQ ID NOs: 419 and 425 were identified as regions important for binding to IgE antibodies from patients. Further, SEQ ID NO: 422 was identified as a sequence comprising a region amino-terminally adjacent to epitope 11 and binding to IgE antibodies from patients, and SEQ ID NO: 426 was identified as a sequence comprising a region carboxy-terminally adjacent to epitope 11 and binding to IgE antibodies from patients.

In SEQ ID NO: 282, the amino acids at positions 1, 4, 5, 11 and 12 were identified as amino acids particularly important for binding to IgE antibodies from allergic patients, and the amino acids at positions 7, 8 and 13 were identified as amino acids influencing binding to IgE antibodies from allergic patients. The amino acids at positions 2, 3, 6, 9, 10, 14 and 15 of SEQ ID NO: 282 had no influence on the binding activity against IgE antibodies from allergic patients when substituted by alanine.

In SEQ ID NO: 282, the amino acids at positions 1, 2, 4, 5, 7, 9, 11, 12 and 14 were identified as amino acids particularly important for binding to IgE antibodies from allergic patients, and the amino acids at positions 3 and 8 were identified as possible amino acids influencing binding to IgE antibodies from allergic patients. In this case, the amino acids at positions 6, 10, 13 and 15 of SEQ ID NO: 282 had no influence on the binding activity against IgE antibodies from allergic patients when substituted by alanine.

In SEQ ID NO: 419, the amino acids at positions 5-7 were identified as amino acids particularly important for binding to IgE antibodies from allergic patients, and the amino acids at positions 1 and 2 were identified as amino acids influencing binding to IgE antibodies from allergic patients. The amino acids at positions 3, 4 and 8 of SEQ ID NO: 419 had no influence on the binding activity against IgE antibodies from allergic patients when substituted by alanine.

In SEQ ID NO: 425, the amino acids at positions 1, 3, 5, 6 and 8 were identified as amino acids particularly important for binding to IgE antibodies from allergic patients, and the amino acid at position 2 was identified as an amino acid influencing binding to IgE antibodies from allergic patients. The amino acids at positions 4, 7 and 9 of SEQ ID NO: 425 had no influence on the binding activity against IgE antibodies from allergic patients when substituted by alanine.

### (14) Epitope 14

In epitope 14 (SEQ ID NO: 285), SEQ ID NO: 430 was identified as a region important for binding to IgE antibodies from patients.

In SEQ ID NO: 285, the amino acids at positions 1, 5, 6, 8, 10 and 12 were identified as amino acids particularly important for binding to IgE antibodies from allergic patients, and the amino acids at positions 2, 9 and 11 were identified as amino acids influencing binding to IgE antibodies from allergic patients. The amino acids at positions 3, 4, 7 and 13-15 of SEQ ID NO: 285 had no influence on the binding activity against IgE antibodies from allergic patients when substituted by alanine.

In SEQ ID NO: 430, the amino acids at positions 3, 4 and 6-9 were identified as amino acids particularly important for binding to IgE antibodies from allergic patients. The amino acids at positions 1, 2 and 5 of SEQ ID NO: 430 had no influence on the binding activity against IgE antibodies from allergic patients when substituted by alanine.

### (15) Epitope 15

In epitope 15 (SEQ ID NO: 286), SEQ ID NOs: 434, 436 and 440 were identified as regions important for binding to IgE antibodies from patients.

In SEQ ID NO: 286, the amino acids at positions 1-7 and 9-15 were identified as amino acids particularly important for binding to IgE antibodies from allergic patients. The amino acid at position 8 of SEQ ID NO: 286 had no influence on the binding activity against IgE antibodies from allergic patients when substituted by alanine.

In SEQ ID NO: 286, the amino acids at positions 3, 5-7 and 11-15 were identified as amino acids particularly important for binding to IgE antibodies from allergic patients, and the amino acids at positions 4 and 10 were identified as possible amino acids influencing binding to IgE antibodies from allergic patients. In this case, the amino acids at positions 1, 2, 8 and 9 of SEQ ID NO: 286 had no influence on the binding activity against IgE antibodies from allergic patients when substituted by alanine.

In SEQ ID NO: 434, the amino acids at positions 1-3 and 5-9 were identified as amino acids particularly important for binding to IgE antibodies from allergic patients. The amino acid at position 4 of SEQ ID NO: 434 had no influence on the binding activity against IgE antibodies from allergic patients when substituted by alanine.

In SEQ ID NO: 436, the amino acids at positions 1 and 3-10 were identified as amino acids particularly important for binding to IgE antibodies from allergic patients. The amino acid at position 2 of SEQ ID NO: 436 had no influence on the binding activity against IgE antibodies from allergic patients when substituted by alanine.

In SEQ ID NO: 440, the amino acids at positions 4-7 were identified as amino acids particularly important for binding to IgE antibodies from allergic patients, and the amino acid at position 3 was identified as an amino acid influencing binding to IgE antibodies from allergic patients. The amino acids at positions 1 and 2 of SEQ ID NO: 440 had no influence on the binding activity against IgE antibodies from allergic patients when substituted by alanine.

### (16) Epitope 16

In epitope 16 (SEQ ID NO: 287), SEQ ID NOs: 444, 446 and 449 were identified as regions important for binding to IgE antibodies from patients.

In SEQ ID NO: 287, the amino acids at positions 2, 7, 9 and 13 were identified as amino acids particularly important for binding to IgE antibodies from allergic patients. The amino acids at positions 1, 3-6, 8, 10-12, 14 and 15 of SEQ ID NO: 287 had no influence on the binding activity against IgE antibodies from allergic patients when substituted by alanine.

In SEQ ID NO: 444, the amino acids at positions 2, 7 and 9 were identified as amino acids particularly important for binding to IgE antibodies from allergic patients. The amino acids at positions 1, 3-6, 8 and 10 of SEQ ID NO: 444 had no influence on the binding activity against IgE antibodies from allergic patients when substituted by alanine.

In SEQ ID NO: 446, the amino acids at positions 4, 6 and 10 were identified as amino acids particularly important for binding to IgE antibodies from allergic patients. The amino acids at positions 1-3, 5 and 7-9 of SEQ ID NO: 446 had no influence on the binding activity against IgE antibodies from allergic patients when substituted by alanine.

In SEQ ID NO: 449, the amino acids at positions 2, 4 and 8 were identified as amino acids particularly important for binding to IgE antibodies from allergic patients, and the amino acid at position 10 was identified as an amino acid influencing binding to IgE antibodies from allergic patients. The amino acids at positions 1, 3, 5-7 and 9 of SEQ ID NO: 449 had no influence on the binding activity against IgE antibodies from allergic patients when substituted by alanine.

### (17) Epitope 17

In epitope 17 (SEQ ID NO: 288), SEQ ID NOs: 455 and 458 were identified as regions important for binding to IgE antibodies from patients. Further, SEQ ID NOs: 451 and 452 were identified as sequences comprising a region amino-terminally adjacent to epitope 17 and binding to IgE antibodies from patients.

In SEQ ID NO: 288, the amino acids at positions 3, 7, 10 and 12-14 were identified as amino acids particularly important for binding to IgE antibodies from allergic patients. The amino acids at positions 1, 2, 4-6, 8, 9, 11 and 15 of SEQ ID NO: 288 had no influence on the binding activity against IgE antibodies from allergic patients when substituted by alanine.

In SEQ ID NO: 455, the amino acids at positions 1, 5, 8 and 10 were identified as amino acids particularly important for binding to IgE antibodies from allergic patients, and the amino acids at positions 7 and 9 were identified as amino acids influencing binding to IgE antibodies from allergic patients. The amino acids at positions 2-4 and 6 of SEQ ID NO: 455 had no influence on the binding activity against IgE antibodies from allergic patients when substituted by alanine.

In SEQ ID NO: 458, the amino acids at positions 1, 4 and 6-8 were identified as amino acids particularly important for binding to IgE antibodies from allergic patients, and the amino acids at positions 3 and 5 were identified as amino acids influencing binding to IgE antibodies from allergic patients. The amino acids at positions 2 and 9 of SEQ ID NO: 458 had no influence on the binding activity against IgE antibodies from allergic patients when substituted by alanine.

### (19) Epitope 19

In epitope 19 (SEQ ID NO: 290), SEQ ID NOs: 461 and 464 were identified as regions important for binding to IgE antibodies from patients. Further, SEQ ID NO: 462 was identified as a sequence comprising a region amino-terminally adjacent to epitope 19 and binding to IgE antibodies from patients, and SEQ ID NOs: 465 and 466 were identified as sequences comprising a region carboxy-terminally adjacent to epitope 19 and binding to IgE antibodies from patients.

In SEQ ID NO: 290, the amino acids at positions 1-4, 6, 7, 10 and 13-15 were identified as amino acids particularly important for binding to IgE antibodies from allergic patients. The amino acids at positions 5, 8, 9, 11 and 12 of SEQ ID NO: 290 had no influence on the binding activity against IgE antibodies from allergic patients when substituted by alanine.

In SEQ ID NO: 461, the amino acids at positions 1-4 were identified as amino acids particularly important for binding to IgE antibodies from allergic patients. The amino acid at position 5 of SEQ ID NO: 461 had no influence on the binding activity against IgE antibodies from allergic patients when substituted by alanine.

In SEQ ID NO: 464, the amino acids at positions 2, 2 and 6-10 were identified as amino acids particularly important for binding to IgE antibodies from allergic patients. The amino acids at positions 1, 4 and 5 of SEQ ID NO: 464 had no influence on the binding activity against IgE antibodies from allergic patients when substituted by alanine.

### (20) Epitope 20

In epitope 20 (SEQ ID NO: 291), SEQ ID NOs: 470, 473, 482, 483 and 486 were identified as regions important for binding to IgE antibodies from patients. Further, SEQ ID NOs: 471, 479 and 480 were identified as sequences comprising a region amino-terminally adjacent to epitope 20 and binding to IgE antibodies from patients, and SEQ ID NOs: 476 and 487 were identified as sequences comprising a region carboxy-terminally adjacent to epitope 20 and binding to IgE antibodies from patients.

In SEQ ID NO: 291, the amino acids at positions 2, 7, 10, 12, 13 and 15 were identified as amino acids particularly important for binding to IgE antibodies from allergic patients, and the amino acid at position 1 was identified as an amino acid influencing binding to IgE antibodies from allergic patients. The amino acids at positions 3-6, 8, 9, 11 and 14 of SEQ ID NO: 291 had no influence on the binding activity against IgE antibodies from allergic patients when substituted by alanine.

In SEQ ID NO: 291, the amino acids at positions 2, 4, 7, 11-13 and 15 were identified as amino acids particularly important for binding to IgE antibodies from allergic patients, and the amino acid at position 6 was identified as a possible amino acid influencing binding to IgE antibodies from allergic patients. In this case, the amino acids at positions 1, 3, 5, 8-10 and 14 of SEQ ID NO: 291 had no influence on the binding activity against IgE antibodies from allergic patients when substituted by alanine.

In SEQ ID NO: 291, the amino acids at positions 2, 5, 7, 9, 10, 13 and 15 were identified as amino acids particularly important for binding to IgE antibodies from allergic patients, and the amino acids at positions 1 and 6 were identified as possible amino acids influencing binding to IgE antibodies from allergic patients. In this case, the amino acids at positions 3, 4, 8, 11, 12 and 14 of SEQ ID NO: 291 had no influence on the binding activity against IgE antibodies from allergic patients when substituted by alanine.

In SEQ ID NO: 470, the amino acids at positions 2, 4 and 7 were identified as amino acids particularly important for binding to IgE antibodies from allergic patients, and the amino acids at positions 1 and 5 were identified as amino acids influencing binding to IgE antibodies from allergic patients. The amino acids at positions 3, 6, 8 and 9 of SEQ ID NO: 470 had no influence on the binding activity against IgE antibodies from allergic patients when substituted by alanine.

In SEQ ID NO: 473, the amino acids at positions 3, 5, 6 and 8 were identified as amino acids particularly important for binding to IgE antibodies from allergic patients. The amino acids at positions 1, 2, 4 and 7 of SEQ ID NO: 473 had no influence on the binding activity against IgE antibodies from allergic patients when substituted by alanine.

In SEQ ID NO: 482, the amino acids at positions 1, 4-6, 8 and 9 were identified as amino acids particularly important for binding to IgE antibodies from allergic patients. The amino acids at positions 2, 3 and 7 of SEQ ID NO: 483 had no influence on the binding activity against IgE antibodies from allergic patients when substituted by alanine.

In SEQ ID NO: 486, the amino acids at positions 2, 4, 5 and 8 were identified as amino acids particularly important for binding to IgE antibodies from allergic patients, and the amino acid at position 1 was identified as an amino acid influencing binding to IgE antibodies from allergic patients. The amino acids at positions 3, 6, 7 and 9 of SEQ ID NO: 486 had no influence on the binding activity against IgE antibodies from allergic patients when substituted by alanine.

### (21) Epitope 21

In epitope 21 (SEQ ID NO: 292), SEQ ID NOs: 491, 494, 500, 505, 507 and 509 were identified as regions important for binding to IgE antibodies from patients. Further, SEQ ID NOs: 492, 497, 498 and 503 were identified as sequences comprising a region amino-terminally adjacent to epitope 21 and binding to IgE antibodies from patients, and SEQ ID NO: 495 was identified as a sequence comprising a region carboxy-terminally adjacent to epitope 21 and binding to IgE antibodies from patients.

In SEQ ID NO: 292, the amino acids at positions 1-3, 6, 8, 9, 14 and 15 were identified as amino acids particularly important for binding to IgE antibodies from allergic patients. The amino acids at positions 4, 5, 7 and 10-13 of SEQ ID NO: 292 had no influence on the binding activity against IgE antibodies from allergic patients when substituted by alanine.

In SEQ ID NO: 292, the amino acids at positions 1-3, 6-10 and 12-15 were identified as amino acids particularly important for binding to IgE antibodies from allergic patients, and the amino acid at position 11 was identified as a possible amino acid influencing binding to IgE antibodies from allergic patients. In this case, the amino acids at positions 4 and 5 of SEQ ID NO: 292 had no influence on the binding activity against IgE antibodies from allergic patients when substituted by alanine.

In SEQ ID NO: 292, the amino acids at positions 1, 2, 7-9 and 15 were identified as possible amino acids particularly important for binding to IgE antibodies from allergic patients. In this case, the amino acids at positions 3-6 and 10-14 of SEQ ID NO: 292 had no influence on the binding activity against IgE antibodies from allergic patients when substituted by alanine.

In SEQ ID NO: 491, the amino acids at positions 1-3 and 6 were identified as amino acids particularly important for binding to IgE antibodies from allergic patients. The amino acids at positions 4 and 5 of SEQ ID NO: 491 had no influence on the binding activity against IgE antibodies from allergic patients when substituted by alanine.

In SEQ ID NO: 494, the amino acids at positions 1 and 2 were identified as amino acids particularly important for binding to IgE antibodies from allergic patients. The amino acids at positions 3-6 of SEQ ID NO: 494 had no influence on the binding activity against IgE antibodies from allergic patients when substituted by alanine.

In SEQ ID NO: 500, the amino acids at positions 1-3 and 5-7 were identified as amino acids particularly important for binding to IgE antibodies from allergic patients. The amino acid at position 4 of SEQ ID NO: 500 had no influence on the binding activity against IgE antibodies from allergic patients when substituted by alanine.

In SEQ ID NO: 505, the amino acids at positions 1, 2, 7 and 8 were identified as amino acids particularly important for binding to IgE antibodies from allergic patients. The amino acids at positions 3-6 of SEQ ID NO: 505 had no influence on the binding activity against IgE antibodies from allergic patients when substituted by alanine.

In SEQ ID NO: 507, the amino acids at positions 5-7 were identified as amino acids particularly important for binding to IgE antibodies from allergic patients. The amino acids at positions 1-4 and 8-10 of SEQ ID NO: 507 had no influence on the binding activity against IgE antibodies from allergic patients when substituted by alanine.

In SEQ ID NO: 509, the amino acids at positions 1, 2, 8 and 9 were identified as amino acids particularly important for binding to IgE antibodies from allergic patients. The amino acids at positions 3-7 of SEQ ID NO: 509 had no influence on the binding activity against IgE antibodies from allergic patients when substituted by alanine.

### (22) Epitope 22

In epitope 22 (SEQ ID NO: 293), SEQ ID NO: 512 was identified as a region important for binding to IgE antibodies from patients. Further, SEQ ID NO: 515 was identified as a sequence comprising a region carboxy-terminally adjacent to epitope 22 and binding to IgE antibodies from patients.

In SEQ ID NO: 293, the amino acids at positions 3, 6, 8-10 and 12-15 were identified as amino acids particularly important for binding to IgE antibodies from allergic patients, and the amino acids at positions 2 and 11 were identified as amino acids influencing binding to IgE antibodies from allergic patients. The amino acids at positions 1, 4, 5 and 7 of SEQ ID NO: 293 had no influence on the binding activity against IgE antibodies from allergic patients when substituted by alanine.

In SEQ ID NO: 512, the amino acids at positions 1 and 3-10 were identified as amino acids particularly important for binding to IgE antibodies from allergic patients. The amino acid at position 2 of SEQ ID NO: 512 had no influence on the binding activity against IgE antibodies from allergic patients when substituted by alanine.

### (23) Epitope 23

In epitope 23 (SEQ ID NO: 294), SEQ ID NOs: 520 and 522 were identified as regions important for binding to IgE antibodies from patients. Further, SEQ ID NOs: 516 and 517 were identified as sequences comprising a region amino-terminally adjacent to epitope 23 and binding to IgE antibodies from patients, and SEQ ID NO: 523 was identified as a sequence comprising a region carboxy-terminally adjacent to epitope 23 and binding to IgE antibodies from patients.

In SEQ ID NO: 294, the amino acids at positions 2, 5 and 9-12 were identified as amino acids particularly important for binding to IgE antibodies from allergic patients, and the amino acid at position 8 was identified as an amino acid influencing binding to IgE antibodies from allergic patients. The amino acids at positions 1, 3, 4, 6, 7 and 13-15 of SEQ ID NO: 294 had no influence on the binding activity against IgE antibodies from allergic patients when substituted by alanine.

In SEQ ID NO: 520, the amino acids at positions 1, 4 and 8-10 were identified as amino acids particularly important for binding to IgE antibodies from allergic patients, and the amino acid at position 7 was identified as an amino acid influencing binding to IgE antibodies from allergic patients. The amino acids at positions 2, 3, 5 and 6 of SEQ ID NO: 520 had no influence on the binding activity against IgE antibodies from allergic patients when substituted by alanine.

In SEQ ID NO: 522, the amino acids at positions 2-6 were identified as amino acids particularly important for binding to IgE antibodies from allergic patients. The amino acids at positions 1 and 7 of SEQ ID NO: 522 had no influence on the binding activity against IgE antibodies from allergic patients when substituted by alanine.

### (25) Epitope 25

In SEQ ID NO: 296 (epitope 25), the amino acids at positions 2-6 and 10 were identified as amino acids particularly important for binding to IgE antibodies from allergic patients. The amino acids at positions 1, 7-9 and 11-15 of SEQ ID NO: 296 had no influence on the binding activity against IgE antibodies from allergic patients when substituted by alanine.

In SEQ ID NO: 296, the amino acids at positions 1-3, 9, 11 and 12 were identified as possible amino acids particularly important for binding to IgE antibodies from allergic patients. In this case, the amino acids at positions 4-8, 10 and 13-15 of SEQ ID NO: 296 had no influence on the binding activity against IgE antibodies from allergic patients when substituted by alanine.

### (26) Epitope 26

In epitope 26 (SEQ ID NO: 297), SEQ ID NOs: 528 and 531 were identified as regions important for binding to IgE antibodies from patients.

In SEQ ID NO: 297, the amino acids at positions 1 and 8 were identified as amino acids particularly important for binding to IgE antibodies from allergic patients, and the amino acid at position 6 was identified as an amino acid influencing binding to IgE antibodies from allergic patients. The amino acids at positions 2-5, 7 and 9-15 of SEQ ID NO: 297 had no influence on the binding activity against IgE antibodies from allergic patients when substituted by alanine.

In SEQ ID NO: 297, the amino acids at positions 4 and 6 were identified as possible amino acids particularly important for binding to IgE antibodies from allergic patients. In this case, the amino acids at positions 1-3, 5 and 7-15 of SEQ ID NO: 297 had no influence on the binding activity against IgE antibodies from allergic patients when substituted by alanine.

### (27) Epitope 27

In epitope 27 (SEQ ID NO: 298), SEQ ID NOs: 533, 534, 539, 541 and 544 were identified as regions important for binding to IgE antibodies from patients. Further, SEQ ID NOs: 535 and 542 were identified as sequences comprising a region carboxy-terminally adjacent to epitope 27 and binding to IgE antibodies from patients.

In SEQ ID NO: 298, the amino acids at positions 1, 2, 14 and 15 were identified as amino acids particularly important for binding to IgE antibodies from allergic patients, and the amino acid at position 13 was identified as an amino acid influencing binding to IgE antibodies from allergic patients. The amino acids at positions 3-12 of SEQ ID NO: 298 had no influence on the binding activity against IgE antibodies from allergic patients when substituted by alanine.

In SEQ ID NO: 298, the amino acids at positions 3, 4, and 13-15 were identified as amino acids particularly important for binding to IgE antibodies from allergic patients, and the amino acids at positions 2 and 6 were identified as possible amino acids influencing binding to IgE antibodies from allergic patients. In this case, the amino acids at positions 1, 5 and 7-12 of SEQ ID NO: 298 had no influence on the binding activity against IgE antibodies from allergic patients when substituted by alanine.

In SEQ ID NO: 539, the amino acids at positions 1 and 3 were identified as amino acids particularly important for binding to IgE antibodies from allergic patients. The amino acids at positions 2 and 4-9 of SEQ ID NO: 539 had no influence on the binding activity against IgE antibodies from allergic patients when substituted by alanine.

In SEQ ID NO: 541, the amino acids at positions 1 and 8-10 were identified as amino acids particularly important for binding to IgE antibodies from allergic patients. The amino acids at positions 2-7 of SEQ ID NO: 541 had no influence on the binding activity against IgE antibodies from allergic patients when substituted by alanine.

In SEQ ID NO: 544, the amino acids at positions 4-6 were identified as amino acids particularly important for binding to IgE antibodies from allergic patients. The amino acids at positions 1-3 of SEQ ID NO: 544 had no influence on the binding activity against IgE antibodies from allergic patients when substituted by alanine.

### (28) Epitope 28

In SEQ ID NO: 299 (epitope 28), the amino acids at positions 2 and 6-15 were identified as amino acids particularly important for binding to IgE antibodies from allergic patients. The amino acids at positions 1 and 3-5 of SEQ ID NO: 299 had no influence on the binding activity against IgE antibodies from allergic patients when substituted by alanine.

In SEQ ID NO: 299, the amino acids at positions 2, 9-12 and 15 were identified as possible amino acids particularly important for binding to IgE antibodies from allergic patients. In this case, the amino acids at positions 1, 3-8, 13 and 14 of SEQ ID NO: 299 had no influence on the binding activity against IgE antibodies from allergic patients when substituted by alanine.

In SEQ ID NO: 299, the amino acids at positions 1, 2, 9, 14 and 15 were identified as possible amino acids particularly important for binding to IgE antibodies from allergic patients. In this case, the amino acids at positions 3-8 and 10-13 of SEQ ID NO: 299 had no influence on the binding activity against IgE antibodies from allergic patients when substituted by alanine.

### (30) Epitope 30

SEQ ID NO: 550 was identified as a sequence comprising a region amino-terminally adjacent to epitope 30 (SEQ ID NO: 301) and binding to IgE antibodies from patients, and SEQ ID NO: 551 was identified as a sequence comprising a region carboxy-terminally adjacent to epitope 30 and binding to IgE antibodies from patients.

In SEQ ID NO: 301, the amino acids at positions 2 and 6-9 were identified as amino acids particularly important for binding to IgE antibodies from allergic patients. The amino acids at positions 1, 3-5 and 10-15 of SEQ ID NO: 301 had no influence on the binding activity against IgE antibodies from allergic patients when substituted by alanine.

### (33) Epitope 33

In epitope 33 (SEQ ID NO: 304), SEQ ID NO: 555 was identified as a region important for binding to IgE antibodies from patients. Further, SEQ ID NO: 553 was identified as a sequence comprising a region amino-terminally adjacent to epitope 33 and binding to IgE antibodies from patients, and SEQ ID NOs: 556 and 557 were identified as sequences comprising a region carboxy-terminally adjacent to epitope 33 and binding to IgE antibodies from patients.

In SEQ ID NO: 304, the amino acids at positions 1, 2, 13 and 14 were identified as amino acids particularly important for binding to IgE antibodies from allergic patients. The amino acids at positions 3-12 and 15 of SEQ ID NO: 304 had no influence on the binding activity against IgE antibodies from allergic patients when substituted by alanine.

In SEQ ID NO: 555, the amino acids at positions 1 and 2 were identified as amino acids particularly important for binding to IgE antibodies from allergic patients. The amino acids at positions 3-8 of SEQ ID NO: 555 had no influence on the binding activity against IgE antibodies from allergic patients when substituted by alanine.

### (34) Epitope 34

In epitope 34 (SEQ ID NO: 305), SEQ ID NOs: 561, 562, 569, 571 and 573 were identified as regions important for binding to IgE antibodies from patients. Further, SEQ ID NO: 565 was identified as a sequence comprising a region amino-terminally adjacent to epitope 34 and binding to IgE antibodies from patients.

In SEQ ID NO: 305, the amino acids at positions 14 and 15 were identified as amino acids particularly important for binding to IgE antibodies from allergic patients, and the amino acids at positions 10-13 were identified as amino acids influencing binding to IgE antibodies from allergic patients. The amino acids at positions 1-9 of SEQ ID NO: 305 had no influence on the binding activity against IgE antibodies from allergic patients when substituted by alanine.

In SEQ ID NO: 305, the amino acids at positions 9, 11 and 14 were identified as amino acids particularly important for binding to IgE antibodies from allergic patients, and the amino acids at positions 4-7 were identified as possible amino acids influencing binding to IgE antibodies from allergic patients. The amino acids at positions 1-3, 8, 10, 12, 13 and 15 of SEQ ID NO: 305 had no influence on the binding activity against IgE antibodies from allergic patients when substituted by alanine.

In SEQ ID NO: 561, the amino acids at positions 2-5 were identified as amino acids particularly important for binding to IgE antibodies from allergic patients. The amino acid at position 1 of SEQ ID NO: 561 had no influence on the binding activity against IgE antibodies from allergic patients when substituted by alanine.

In SEQ ID NO: 562, the amino acids at positions 2-6 were identified as amino acids particularly important for binding to IgE antibodies from allergic patients. The amino acid at position 1 of SEQ ID NO: 562 had no influence on the binding activity against IgE antibodies from allergic patients when substituted by alanine.

In SEQ ID NO: 569, the amino acids at positions 7 and 9 were identified as amino acids particularly important for binding to IgE antibodies from allergic patients, and the amino acids at positions 2-5 were identified as amino acids influencing binding to IgE antibodies from allergic patients. The amino acids at positions 1, 6, 8 and 10 of SEQ ID NO: 569 had no influence on the binding activity against IgE antibodies from allergic patients when substituted by alanine.

In SEQ ID NO: 571, the amino acids at positions 1, 3 and 6 were identified as amino acids particularly important for binding to IgE antibodies from allergic patients. The amino acids at positions 2, 4, 5 and 7 of SEQ ID NO: 571 had no influence on the binding activity against IgE antibodies from allergic patients when substituted by alanine.

In SEQ ID NO: 573, the amino acids at positions 1 and 3 were identified as amino acids particularly important for binding to IgE antibodies from allergic patients. The amino acids at positions 2 and 4 of SEQ ID NO: 573 had no influence on the binding activity against IgE antibodies from allergic patients when substituted by alanine.

### (35) Epitope 35

In epitope 35 (SEQ ID NO: 306), SEQ ID NOs: 579 and 581 were identified as regions important for binding to IgE antibodies from patients. Further, SEQ ID NO: 576 was identified as a sequence comprising a region amino-terminally adjacent to epitope 35 and binding to IgE antibodies from patients, and SEQ ID NO: 577 was identified as a sequence comprising a region carboxy-terminally adjacent to epitope 35 and binding to IgE antibodies from patients.

In SEQ ID NO: 306, the amino acids at positions 1-4 and 7 were identified as amino acids particularly important for binding to IgE antibodies from allergic patients. The amino acids at positions 5, 6 and 8-15 of SEQ ID NO: 306 had no influence on the binding activity against IgE antibodies from allergic patients when substituted by alanine.

In SEQ ID NO: 579, the amino acids at positions 1-4 were identified as amino acids particularly important for binding to IgE antibodies from allergic patients. The amino acids at positions 5 and 6 of SEQ ID NO: 579 had no influence on the binding activity against IgE antibodies from allergic patients when substituted by alanine.

In SEQ ID NO: 581, the amino acids at positions 1-3 were identified as amino acids particularly important for binding to IgE antibodies from allergic patients. The amino acids at positions 4-7 of SEQ ID NO: 581 had no influence on the binding activity against IgE antibodies from allergic patients when substituted by alanine.

### (36) Epitope 36

In epitope 36 (SEQ ID NO: 307), SEQ ID NO: 585 was identified as a region important for binding to IgE antibodies from patients. Further, SEQ ID NO: 583 was identified as a sequence comprising a region amino-terminally adjacent to epitope 36 and binding to IgE antibodies from patients, and further, SEQ ID NO: 584 was identified as a sequence comprising a region carboxy-terminally adjacent to epitope 36 and binding to IgE antibodies from patients.

### (37) Epitope 37

In epitope 37 (SEQ ID NO: 308), SEQ ID NOs: 587, 589, 592, 594, 598, 600 and 602 were identified as regions important for binding to IgE antibodies from patients. Further, SEQ ID NO: 595 was identified as a sequence comprising a region carboxy-terminally adjacent to epitope 37 and binding to IgE antibodies from patients.

In SEQ ID NO: 308, the amino acids at positions 4, 8, 10 and 13 were identified as amino acids particularly important for binding to IgE antibodies from allergic patients. The amino acids at positions 1-3, 5-7, 9, 11, 12, 14 and 15 of SEQ ID NO: 308 had no influence on the binding activity against IgE antibodies from allergic patients when substituted by alanine.

In SEQ ID NO: 308, the amino acids at positions 4 and 9-14 were identified as possible amino acids particularly important for binding to IgE antibodies from allergic patients. In this case, the amino acids at positions 1-3, 5-8 and 15 of SEQ ID NO: 308 had no influence on the binding activity against IgE antibodies from allergic patients when substituted by alanine.

In SEQ ID NO: 308, the amino acids at positions 5, 8, 10, 13 and 15 were identified as possible amino acids particularly important for binding to IgE antibodies from allergic patients. In this case, the amino acids at positions 1-4, 6, 7, 9, 11, 12 and 14 of SEQ ID NO: 308 had no influence on the binding activity against IgE antibodies from allergic patients when substituted by alanine.

In SEQ ID NO: 587, the amino acids at positions 1 and 2 were identified as amino acids particularly important for binding to IgE antibodies from allergic patients. The amino acids at positions 3 and 4 of SEQ ID NO: 587 had no influence on the binding activity against IgE antibodies from allergic patients when substituted by alanine.

In SEQ ID NO: 589, the amino acids at positions 4, 6 and 9 were identified as amino acids particularly important for binding to IgE antibodies from allergic patients. The amino acids at positions 1-3, 5, 7, 8 and 10 of SEQ ID NO: 589 had no influence on the binding activity against IgE antibodies from allergic patients when substituted by alanine.

In SEQ ID NO: 592, the amino acids at positions 2 and 7-9 were identified as amino acids particularly important for binding to IgE antibodies from allergic patients. The amino acids at positions 1 and 3-6 of SEQ ID NO: 592 had no influence on the binding activity against IgE antibodies from allergic patients when substituted by alanine.

In SEQ ID NO: 594, the amino acids at positions 5-10 were identified as amino acids particularly important for binding to IgE antibodies from allergic patients. The amino acids at positions 1-4 of SEQ ID NO: 594 had no influence on the binding activity against IgE antibodies from allergic patients when substituted by alanine.

In SEQ ID NO: 598, the amino acids at positions 3, 6 and 8 were identified as amino acids particularly important for binding to IgE antibodies from allergic patients. The amino acids at positions 1, 2, 4, 5 and 7 of SEQ ID NO: 598 had no influence on the binding activity against IgE antibodies from allergic patients when substituted by alanine.

In SEQ ID NO: 600, the amino acids at positions 1, 4, 6 and 9 were identified as amino acids particularly important for binding to IgE antibodies from allergic patients. The amino acids at positions 2, 3, 5, 7, 8 and 10 of SEQ ID NO: 600 had no influence on the binding activity against IgE antibodies from allergic patients when substituted by alanine.

In SEQ ID NO: 602, the amino acids at positions 2, 4 and 7 were identified as amino acids particularly important for binding to IgE antibodies from allergic patients. The amino acids at positions 1, 3, 5, 6 and 8 of SEQ ID NO: 602 had no influence on the binding activity against IgE antibodies from allergic patients when substituted by alanine.

### (38) Epitope 38

In epitope 38 (SEQ ID NO: 309), SEQ ID NO: 608 was identified as a region important for binding to IgE antibodies from patients. Further, SEQ ID NOs: 604 and 605 were identified as sequences comprising a region amino-terminally adjacent to epitope 38 and binding to IgE antibodies from patients, and further, SEQ ID NO: 609 was identified as a sequence comprising a region carboxy-terminally adjacent to epitope 38 and binding to IgE antibodies from patients.

In SEQ ID NO: 309, the amino acids at positions 5, 9 and 12-15 were identified as amino acids particularly important for binding to IgE antibodies from allergic patients, and the amino acid at position 10 was identified as an amino acid influencing binding to IgE antibodies from allergic patients. The amino acids at positions 1-4, 6-8 and 11 of SEQ ID NO: 309 had no influence on the binding activity against IgE antibodies from allergic patients when substituted by alanine.

In SEQ ID NO: 309, the amino acids at positions 7, 9 and 11-14 were identified as possible amino acids particularly important for binding to IgE antibodies from allergic patients. In this case, the amino acids at positions 1-6, 8, 10 and 15 of SEQ ID NO: 309 had no influence on the binding activity against IgE antibodies from allergic patients when substituted by alanine.

In SEQ ID NO: 608, the amino acids at positions 1, 5 and 8-10 were identified as amino acids particularly important for binding to IgE antibodies from allergic patients, and the amino acid at position 6 was identified as an amino acid influencing binding to IgE antibodies from allergic patients. The amino acids at positions 2-4 and 7 of SEQ ID NO: 608 had no influence on the binding activity against IgE antibodies from allergic patients when substituted by alanine.

### (39) Epitope 39

In epitope 39 (SEQ ID NO: 310), SEQ ID NOs: 616, 617 and 619 were identified as regions important for binding to IgE antibodies from patients. Further, SEQ ID NO: 613 was identified as a sequence comprising a region amino-terminally adjacent to epitope 39 and binding to IgE antibodies from patients.

In SEQ ID NO: 310, the amino acids at positions 3 and 5 were identified as amino acids particularly important for binding to IgE antibodies from allergic patients, and the amino acids at positions 10 and 13 were identified as amino acids influencing binding to IgE antibodies from allergic patients. The amino acids at positions 1, 2, 4, 6-9, 11, 12, 14 and 15 of SEQ ID NO: 310 had no influence on the binding activity against IgE antibodies from allergic patients when substituted by alanine.

In SEQ ID NO: 616, the amino acids at positions 1 and 3 were identified as amino acids particularly important for binding to IgE antibodies from allergic patients, and the amino acid at position 8 was identified as an amino acid influencing binding to IgE antibodies from allergic patients. The amino acids at positions 2, 4-7 and 9 of SEQ ID NO: 616 had no influence on the binding activity against IgE antibodies from allergic patients when substituted by alanine.

In SEQ ID NO: 619, the amino acids at positions 1, 6 and 9 were identified as amino acids particularly important for binding to IgE antibodies from allergic patients. The amino acids at positions 2-5, 7, 8 and 10 of SEQ ID NO: 619 had no influence on the binding activity against IgE antibodies from allergic patients when substituted by alanine.

### (40) Epitope 40

SEQ ID NOs: 622 and 623 were identified as sequences comprising a region amino-terminally adjacent to epitope 40 (SEQ ID NO: 311) and binding to IgE antibodies from patients, and SEQ ID NO: 624 was identified as a sequence comprising a region carboxy-terminally adjacent to epitope 40 and binding to IgE antibodies from patients.

In SEQ ID NO: 311, the amino acids at positions 1-3 were identified as amino acids particularly important for binding to IgE antibodies from allergic patients. The amino acids at positions 4-15 of SEQ ID NO: 311 had no influence on the binding activity against IgE antibodies from allergic patients when substituted by alanine.

### (41) Epitope 41

In epitope 41 (SEQ ID NO: 312), SEQ ID NOs: 627, 630, 636, 637 (amino acid sequence: ELL), 641 and 642 were identified as regions important for binding to IgE antibodies from patients. Further, SEQ ID NOs: 631 and 632 were identified as sequences comprising a region carboxy-terminally adjacent to epitope 41 and binding to IgE antibodies from patients.

In SEQ ID NO: 312, the amino acids at positions 6, 7 and 9-14 were identified as amino acids particularly important for binding to IgE antibodies from allergic patients, and the amino acid at position 8 was identified as an amino acid influencing binding to IgE antibodies from allergic patients. The amino acids at positions 1-5 and 15 of SEQ ID NO: 312 had no influence on the binding activity against IgE antibodies from allergic patients when substituted by alanine.

In SEQ ID NO: 312, the amino acids at positions 6 and 12 were identified as amino acids particularly important for binding to IgE antibodies from allergic patients, and the amino acids at positions 1 and 7 were identified as possible amino acids influencing binding to IgE antibodies from allergic patients. In this case, the amino acids at positions 2-5, 8-11 and 13-15 of SEQ ID NO: 312 had no influence on the binding activity against IgE antibodies from allergic patients when substituted by alanine.

In SEQ ID NO: 312, the amino acids at positions 3, 6, 7 and 13 were identified as possible amino acids particularly important for binding to IgE antibodies from allergic patients. In this case, the amino acids at positions 1, 2, 4, 5, 8-12, 14 and 15 of SEQ ID NO: 312 had no influence on the binding activity against IgE antibodies from allergic patients when substituted by alanine.

In SEQ ID NO: 627, the amino acids at positions 6-8 were identified as amino acids particularly important for binding to IgE antibodies from allergic patients. The amino acids at positions 1-5 of SEQ ID NO: 627 had no influence on the binding activity against IgE antibodies from allergic patients when substituted by alanine.

In SEQ ID NO: 630, the amino acids at positions 4, 5 and 7-10 were identified as amino acids particularly important for binding to IgE antibodies from allergic patients, and the amino acid at position 6 was identified as an amino acid influencing binding to IgE antibodies from allergic patients. The amino acids at positions 1-3 of SEQ ID NO: 630 had no influence on the binding activity against IgE antibodies from allergic patients when substituted by alanine.

In SEQ ID NO: 636, the amino acids at positions 1, 6 and 7 were identified as amino acids particularly important for binding to IgE antibodies from allergic patients. The amino acids at positions 2-5 and 8 of SEQ ID NO: 636 had no influence on the binding activity against IgE antibodies from allergic patients when substituted by alanine.

In SEQ ID NO: 641, the amino acids at positions 3, 6 and 7 were identified as amino acids particularly important for binding to IgE antibodies from allergic patients. The amino acids at positions 1, 2, 4 and 5 of SEQ ID NO: 641 had no influence on the binding activity against IgE antibodies from allergic patients when substituted by alanine.

### (42) Epitope 42

In epitope 42 (SEQ ID NO: 313), SEQ ID NOs: 647 and 649 were identified as regions important for binding to IgE antibodies from patients. Further, SEQ ID NO: 644 was identified as a sequence comprising a region amino-terminally adjacent to epitope 42 and binding to IgE antibodies from patients.

In SEQ ID NO: 313, the amino acids at positions 9-11 were identified as amino acids particularly important for binding to IgE antibodies from allergic patients, and the amino acid at position 4 was identified as an amino acid influencing binding to IgE antibodies from allergic patients. The amino acids at positions 1-4, 6-8 and 12-15 of SEQ ID NO: 313 had no influence on the binding activity against IgE antibodies from allergic patients when substituted by alanine.

In SEQ ID NO: 647, the amino acids at positions 5-7 were identified as amino acids particularly important for binding to IgE antibodies from allergic patients, and the amino acid at position 1 was identified as an amino acid influencing binding to IgE antibodies from allergic patients. The amino acids at positions 2-4 and 8 of SEQ ID NO: 647 had no influence on the binding activity against IgE antibodies from allergic patients when substituted by alanine.

In SEQ ID NO: 649, the amino acids at positions 1-3 were identified as amino acids particularly important for binding to IgE antibodies from allergic patients. The amino acids at positions 4-8 of SEQ ID NO: 649 had no influence on the binding activity against IgE antibodies from allergic patients when substituted by alanine.

### (44) Epitope 44

In SEQ ID NO: 315 (epitope 44), the amino acids at positions 4-7 and 9 were identified as amino acids particularly important for binding to IgE antibodies from allergic patients. The amino acids at positions 1-3, 8 and 10-15 of SEQ ID NO: 315 had no influence on the binding activity against IgE antibodies from allergic patients when substituted by alanine.

In SEQ ID NO: 315, the amino acids at positions 4, 7, 8 and 11 were identified as possible amino acids particularly important for binding to IgE antibodies from allergic patients. In this case, the amino acids at positions 1-3, 5, 6, 9, 10 and 12-15 of SEQ ID NO: 315 had no influence on the binding activity against IgE antibodies from allergic patients when substituted by alanine.

In SEQ ID NO: 315, the amino acids at positions 4, 7, 8, 13 and 15 were identified as possible amino acids particularly important for binding to IgE antibodies from allergic patients. In this case, the amino acids at positions 1-3, 5, 6, 9-12 and 14 of SEQ ID NO: 315 had no influence on the binding activity against IgE antibodies from allergic patients when substituted by alanine.

### (45) Epitope 45

In SEQ ID NO: 316 (epitope 45), the amino acids at positions 2, 3, 7, 8 and 13 were identified as amino acids particularly important for binding to IgE antibodies from allergic patients. The amino acids at positions 1, 4-6, 9-12, 14 and 15 of SEQ ID NO: 316 had no influence on the binding activity against IgE antibodies from allergic patients when substituted by alanine.

In SEQ ID NO: 316, the amino acids at positions 7-9, 11 and 13 were identified as possible amino acids particularly important for binding to IgE antibodies from allergic patients. In this case, the amino acids at positions 1-6, 10, 12, 14 and 15 of SEQ ID NO: 316 had no influence on the binding activity against IgE antibodies from allergic patients when substituted by alanine.

### (46) Epitope 46

In epitope 46 (SEQ ID NO: 317), SEQ ID NOs: 658, 659, 663 and 664 were identified as regions important for binding to IgE antibodies from patients. Further, SEQ ID NO: 657 was identified as a sequence comprising a region amino-terminally adjacent to epitope 46 and binding to IgE antibodies from patients, and further, SEQ ID NOs: 660 and 665 were identified as sequences comprising a region carboxy-terminally adjacent to epitope 46 and binding to IgE antibodies from patients.

In SEQ ID NO: 317, the amino acids at positions 1, 6, 9 and 13-15 were identified as amino acids particularly important for binding to IgE antibodies from allergic patients, and the amino acids at positions 3, 4, 8, 10 and 12 were identified as amino acids influencing binding to IgE antibodies from allergic patients. The amino acids at positions 2, 5, 7 and 11 of SEQ ID NO: 317 had no influence on the binding activity against IgE antibodies from allergic patients when substituted by alanine.

In SEQ ID NO: 317, the amino acids at positions 12-15 were identified as possible amino acids particularly important for binding to IgE antibodies from allergic patients. In this case, the amino acids at positions 1-11 of SEQ ID NO: 317 had no influence on the binding activity against IgE antibodies from allergic patients when substituted by alanine.

### (47) Epitope 47

In epitope 47 (SEQ ID NO: 318), SEQ ID NOs: 669, 674, 680 and 681 were identified as regions important for binding to IgE antibodies from patients. Further, SEQ ID NO: 672 was identified as a sequence comprising a region amino-terminally adjacent to epitope 47 and binding to IgE antibodies from patients, and further, SEQ ID NO: 676 was identified as a sequence comprising a region carboxy-terminally adjacent to epitope 47 and binding to IgE antibodies from patients.

In SEQ ID NO: 318, the amino acids at positions 8-15 were identified as amino acids particularly important for binding to IgE antibodies from allergic patients, and the amino acids at positions 6 and 7 were identified as amino acids influencing binding to IgE antibodies from allergic patients. The amino acids at positions 1-5 of SEQ ID NO: 318 had no influence on the binding activity against IgE antibodies from allergic patients when substituted by alanine.

In SEQ ID NO: 318, the amino acids at positions 2, 5, 7 and 15 were identified as possible amino acids particularly important for binding to IgE antibodies from allergic patients. In this case, the amino acids at positions 1, 3, 4, 6 and 8-14 of SEQ ID NO: 318 had no influence on the binding activity against IgE antibodies from allergic patients when substituted by alanine.

In SEQ ID NO: 318, the amino acids at positions 7, 9, 11, 13 and 15 were identified as amino acids particularly important for binding to IgE antibodies from allergic patients, and the amino acids at positions 8 and 14 were identified as possible amino acids influencing binding to IgE antibodies from allergic patients. In this case, the amino acids at positions 1-6, 10 and 12 of SEQ ID NO: 318 had no influence on the binding activity against IgE antibodies from allergic patients when substituted by alanine.

In SEQ ID NO: 318, the amino acids at positions 1-5 were identified as possible amino acids particularly important for binding to IgE antibodies from allergic patients. In this case, the amino acids at positions 6-15 of SEQ ID NO: 318 had no influence on the binding activity against IgE antibodies from allergic patients when substituted by alanine.

### Example 7: Study on cross-reactivity based on amino acid sequence

Cross-reactivity based on amino acid sequences was studied by PHI-BLAST analysis with initial set parameters of PHI-BLAST using the query sequences and the PHI pattern sequences shown below in Table 3.

As a result, proteins derived from the species shown in Table 3 were identified. This indicates that SEQ ID NO: 1 can be used for detecting antigen cross-reactivity irrespective of species, genera, or plant kingdom.

**[Table 3]**

| Query sequence | PHI pattern sequence | Origin of identified protein |
|---|---|---|
| KVRSPQVEEYNGVWP (SEQ ID NO: 272) | XEEYNG (SEQ ID NO: 322) | Mallard of family *Anatidae*, Japanese quail of family *Phasianidae* etc. |
| QMTIKKSQNVYELQE (SEQ ID NO: 273) | XXXXXXXXNV (SEQ ID NO: 325) | Mallard of family *Anatidae*, Japanese quail of family *Phasianidae* etc., Olive of family *Oleaceae*, Artichoke of family *Compositae*, Sunflower of family *Compositae*, Tausch's goatgrass of family *Poaceae*, Pineapple of family *Bromeliaceae*, Shinano walnut of family *Juglandaceae,* Cattle of family *Bovidae*, Amberjack of family *Carangidae*, Seabream of family *Sparidae,* Rainbow trout of family *Salmonidae*, Squash of family *Cucurbitaceae* etc. |
| | XXXIKXXXNV (SEQ ID NO: 326) | |
| RGREDKMKLALKCIG (SEQ ID NO: 274) | RXRXXKXX (SEQ ID NO: 329) | Mallard of family *Anatidae*, Japanese quail of family *Phasianidae* etc., Whiteleg shrimp of family *Penaeidae*, Bread wheat of family *Poaceae*, Soybean of family *Leguminosae* etc. |
| SFKPVYSDVPIEKIQ (SEQ ID NO: 318) | SFKPVX (SEQ ID NO: 679) | Japanese quail of family *Phasianidae*, Turkey of family *Phasianidae,* Soybean of family *Leguminosae*, Shinano walnut of family *Juglandaceae* etc. |

The peptides of the aforementioned SEQ ID NOs were prepared by the Fmoc method and studied for their binding activity against IgE antibodies in serum by ELISA. On the basis of RXRXXKXX (SEQ ID NO: 329), the peptides of chicken egg RGREDKMK and soybean RFREDKEE were prepared, and their binding activity against IgE antibodies were confirmed by ELISA using the serum of cases having food allergies to chicken egg and soybean in combination. As a result, patient-specific binding activity was able to be confirmed.

## Claims

1. A polypeptide specifically binding to an IgE antibody from an allergic patient, the polypeptide being any one of the following:
(1β) a polypeptide comprising at least one amino acid sequence selected from the group consisting of SEQ ID NOs: 272-295 and 319-525;
(2β) a polypeptide comprising at least one amino acid sequence selected from the group consisting of SEQ ID NOs: 296-303 and 526-552;
(3β) a polypeptide comprising at least one amino acid sequence selected from the group consisting of SEQ ID NOs: 304-311 and 553-625; and
(4β) a polypeptide comprising at least one amino acid sequence selected from the group consisting of SEQ ID NOs: 312-318 and 626-682.

2. The polypeptide according to claim 1, wherein the number of amino acid residues is 500 or less.

3. A kit for diagnosing an allergy, comprising at least one of polypeptides according to claim 1 or 2.

4. A composition for diagnosing an allergy, the composition comprising at least one of polypeptides according to claim 1 or 2 as an antigen.

5. A method for providing an indicator for diagnosing an allergy in a subject, the method comprising the steps of:
(i) contacting a sample obtained from the subject with an antigen, wherein the sample is a solution comprising an IgE antibody;
(ii) detecting binding between the IgE antibody present in the sample obtained from the subject and the antigen; and
(iii) when the binding between the IgE antibody in the subject and the antigen is detected, an indicator of the fact that the subject is allergic is provided;
wherein the antigen is at least one of polypeptides according to claim 1 or 2.

6. A pharmaceutical composition comprising at least one of polypeptides according to claim 1 or 2.

7. The pharmaceutical composition according to claim 6, wherein the pharmaceutical composition is intended for the treatment of an allergy.

8. A tester for determining the presence or absence of an antigen in an object of interest, the tester comprising an antibody that binds to at least one of polypeptides according to claim 1 or 2.

9. A tester for determining the presence or absence of an antigen in an object of interest, the tester comprising any of the following primers:
(a) a primer comprising a portion of the nucleotide sequence of a nucleic acid encoding a polypeptide according to claim 1 or 2, and/or a complementary strand thereof; and
(b) a primer which is a portion of at least one of the nucleotide sequences of SEQ ID NOs: 46, 58, 105, 151, 236, 250 and 261 and/or a primer which is a portion of a sequence complementary to at least one of the nucleotide sequences of SEQ ID NOs: 46, 58, 105, 151, 236, 250 and 261.

10. A raw material or a processed product in which an antigen is eliminated or reduced, wherein the antigen is at least one of polypeptides according to claim 1 or 2.

11. A method for producing a processed product in which an antigen is eliminated or reduced, the method comprising the step of confirming that the antigen is eliminated or reduced in a production process of the processed product, wherein the antigen is at least one of polypeptides according to claim 1 or 2.


SEQUENCE LISTING

<110>  FUJITA ACADEMY
       Hoyu Co., Ltd.

<120>  Antigens that causes allergy, and epitopes thereof

<130>  FA1621-17248

<150>  PCT/JP2017/020654
<151>  2017-06-02

<160>  682

<170>  PatentIn version 3.5

<210>  1
<211>  831
<212>  DNA
<213>  Gallus gallus

<400>  1
tcaatcattg aaaaccttaa agctcgttgt tcagtttcac aaaatatgat aacaaccttc      60

aatggagttg agtttaacta ttcaatgcct gcaaattgct accacatctt ggcacaggat     120

tgtagtccag aactgaagtt cctggtaatg atgaaaagac ttggagaatc tgctgatctc     180

acagcaataa gtgtcagact tgccagccat gaggttgaca tgtatgtttc caatggacta     240

atccagctga agattaatgg tgttcaaacc ccaacagacg ttccatacac atctaagtct     300

ggtctgctga taagcagtga gaaggaaggt ttgtcattaa aagcccctga atatggtgta     360

gaaaaactat attacgatag acgtaaactt gagattcggg ttgctttctg gatggttgga     420

aaaacatgtg gtatttgtgg aaaatatgat gctgagaaga aaagggagta tcaaatgccc     480

agtggatatt tagctaaaga tgcagtgagt tttgctcagt cctgggtcat ctcagaagac     540

acgtgtactg gagcttgcaa gctgcagagg aaatttgtca aaattgagaa gccggttgca     600

tttaacaaaa aggcctcaaa atgcttttcc attgagccag ttttacgctg tgcagaaggc     660

tgttcagcaa ccaggactgt tcctgtctct gtgggtttcc actgtgtccc atctgactcc     720

acactcgagc tggaggaaga gcaggtgagg ttagaccaga agtctgagga cttggtgagc     780

agggtcgatg cccatacggc gtgttcctgc gcccagcagc tgtgctcagc g              831


<210>  2
<211>  277
<212>  PRT
<213>  Gallus gallus

<400>  2

Ser Ile Ile Glu Asn Leu Lys Ala Arg Cys Ser Val Ser Gln Asn Met
1               5                   10                  15


Ile Thr Thr Phe Asn Gly Val Glu Phe Asn Tyr Ser Met Pro Ala Asn

20                  25                  30

Cys Tyr His Ile Leu Ala Gln Asp Cys Ser Pro Glu Leu Lys Phe Leu
        35                  40                  45

Val Met Met Lys Arg Leu Gly Glu Ser Ala Asp Leu Thr Ala Ile Ser
    50                  55                  60

Val Arg Leu Ala Ser His Glu Val Asp Met Tyr Val Ser Asn Gly Leu
65                  70                  75                  80

Ile Gln Leu Lys Ile Asn Gly Val Gln Thr Pro Thr Asp Val Pro Tyr
                85                  90                  95

Thr Ser Lys Ser Gly Leu Leu Ile Ser Ser Glu Lys Glu Gly Leu Ser
            100                 105                 110

Leu Lys Ala Pro Glu Tyr Gly Val Glu Lys Leu Tyr Tyr Asp Arg Arg
        115                 120                 125

Lys Leu Glu Ile Arg Val Ala Phe Trp Met Val Gly Lys Thr Cys Gly
    130                 135                 140

Ile Cys Gly Lys Tyr Asp Ala Glu Lys Lys Arg Glu Tyr Gln Met Pro
145                 150                 155                 160

Ser Gly Tyr Leu Ala Lys Asp Ala Val Ser Phe Ala Gln Ser Trp Val
                165                 170                 175

Ile Ser Glu Asp Thr Cys Thr Gly Ala Cys Lys Leu Gln Arg Lys Phe
            180                 185                 190

Val Lys Ile Glu Lys Pro Val Ala Phe Asn Lys Lys Ala Ser Lys Cys
        195                 200                 205

Phe Ser Ile Glu Pro Val Leu Arg Cys Ala Glu Gly Cys Ser Ala Thr
    210                 215                 220

Arg Thr Val Pro Val Ser Val Gly Phe His Cys Val Pro Ser Asp Ser
225                 230                 235                 240

Thr Leu Glu Leu Glu Glu Glu Gln Val Arg Leu Asp Gln Lys Ser Glu
                245                 250                 255

Asp Leu Val Ser Arg Val Asp Ala His Thr Ala Cys Ser Cys Ala Gln
            260                 265                 270

Gln Leu Cys Ser Ala
        275

<210>  3
<211>  8
<212>  PRT
<213>  Gallus gallus

<400>  3

Ala Pro Glu Tyr Gly Val Glu Lys
1               5

<210>  4
<211>  13
<212>  PRT
<213>  Gallus gallus

<400>  4

Gly Val Gln Thr Pro Thr Asp Val Pro Tyr Thr Ser Lys
1               5                   10

<210>  5
<211>  8
<212>  PRT
<213>  Gallus gallus

<400>  5

Ile Glu Lys Pro Val Ala Phe Asn
1               5

<210>  6
<211>  5
<212>  PRT
<213>  Gallus gallus

<400>  6

Pro Val Ala Phe Asn
1               5

<210>  7
<211>  6
<212>  PRT
<213>  Gallus gallus

<400>  7

Ser Ile Ile Glu Asn Leu
1               5

<210>  8
<211>  9
<212>  PRT
<213>  Gallus gallus

<400>  8

Ile Glu Lys Pro Val Ala Phe Asn Lys
1               5

<210>  9
<211>  15
<212>  PRT
<213>  Gallus gallus

<400>  9

Ile Asn Gly Val Gln Thr Pro Thr Asp Val Pro Tyr Thr Ser Lys
1               5                   10                  15

<210>  10
<211>  13
<212>  PRT
<213>  Gallus gallus

<400>  10

Leu Gly Glu Ser Ala Asp Leu Thr Ala Ile Ser Val Arg
1               5                   10

<210>  11
<211>  14
<212>  PRT
<213>  Gallus gallus

<400>  11

Arg Leu Gly Glu Ser Ala Asp Leu Thr Ala Ile Ser Val Arg
1               5                   10

<210>  12
<211>  1314
<212>  DNA
<213>  Gallus gallus

<400>  12
gtgcaggtgt ttgtttcaag catcacagaa tcagataggt ggaaactctg tgctgatgct      60

tcagtagtaa attcccataa agcatcgggt acccttaaat ggggtaaaga ttgccaggac     120

tatcaggttg ctactcagat tgcaacaggg caatttgctg cacacccggc tatacaggtg     180

aagctggagt ggtcagaagt gccttcaagt gtccgaaaaa ctgccagatg gttctacaca     240

tatcttccag gagctgcgta tatgcttggc tactcccaga agcagcagcg tggtccttct     300

caccaggcag ccatggtgat ggctctgacg tctccaagaa cctgcgatgt ggtcctgaag     360

ctacctgagc tcacagttta caacagagcc atcaggcttc ccctgccact cccttcaagt     420

tcagatacac caacctcaac actaccatcc tccaaatgga acgtctttta ccaagcagcc     480

ttttcaatca ttgaaaacct taaagctcgt tgttcagttt cacaaaatat gataacaacc     540

ttcaatggag ttgagtttaa ctattcaatg cctgcaaatt gctaccacat cttggcacag 600

gattgtagtc cagaactgaa gttcctggta atgatgaaaa gacttggaga atctgctgat 660

ctcacagcaa taagtgtcag acttgccagc catgaggttg acatgtatgt ttccaatgga 720

ctaatccagc tgaagattaa tggtgttcaa accccaacag acgttccata cacatctaag 780

tctggtctgc tgataagcag tgagaaggaa ggtttgtcat taaaagcccc tgaatatggt 840

gtagaaaaac tatattacga tagacgtaaa cttgagattc gggttgcttt ctggatggtt 900

ggaaaaacat gtggtatttg tggaaaatat gatgctgaga agaaaaggga gtatcaaatg 960

cccagtggat atttagctaa agatgcagtg agttttgctc agtcctgggt catctcagaa 1020

gacacgtgta ctggagcttg caagctgcag aggaaatttg tcaaaattga gaagccggtt 1080

gcatttaaca aaaaggcctc aaaatgcttt tccattgagc cagttttacg ctgtgcagaa 1140

ggctgttcag caaccaggac tgttcctgtc tctgtgggtt tccactgtgt cccatctgac 1200

tccacactcg agctggagga agagcaggtg aggttagacc agaagtctga ggacttggtg 1260

agcagggtcg atgcccatac ggcgtgttcc tgcgcccagc agctgtgctc agcg 1314

<210> 13
<211> 438
<212> PRT
<213> Gallus gallus

<400> 13

Val Gln Val Phe Val Ser Ser Ile Thr Glu Ser Asp Arg Trp Lys Leu
1               5                   10                  15

Cys Ala Asp Ala Ser Val Val Asn Ser His Lys Ala Ser Gly Thr Leu
            20                  25                  30

Lys Trp Gly Lys Asp Cys Gln Asp Tyr Gln Val Ala Thr Gln Ile Ala
        35                  40                  45

Thr Gly Gln Phe Ala Ala His Pro Ala Ile Gln Val Lys Leu Glu Trp
    50                  55                  60

Ser Glu Val Pro Ser Ser Val Arg Lys Thr Ala Arg Trp Phe Tyr Thr
65                  70                  75                  80

Tyr Leu Pro Gly Ala Ala Tyr Met Leu Gly Tyr Ser Gln Lys Gln Gln
                85                  90                  95

Arg Gly Pro Ser His Gln Ala Ala Met Val Met Ala Leu Thr Ser Pro
            100                 105                 110

Arg Thr Cys Asp Val Val Leu Lys Leu Pro Glu Leu Thr Val Tyr Asn
        115                 120                 125

Arg Ala Ile Arg Leu Pro Leu Pro Leu Pro Ser Ser Ser Asp Thr Pro
    130                 135                 140

Thr Ser Thr Leu Pro Ser Ser Lys Trp Asn Val Phe Tyr Gln Ala Ala
145                 150                 155                 160

Phe Ser Ile Ile Glu Asn Leu Lys Ala Arg Cys Ser Val Ser Gln Asn
                165                 170                 175

Met Ile Thr Thr Phe Asn Gly Val Glu Phe Asn Tyr Ser Met Pro Ala
            180                 185                 190

Asn Cys Tyr His Ile Leu Ala Gln Asp Cys Ser Pro Glu Leu Lys Phe
        195                 200                 205

Leu Val Met Met Lys Arg Leu Gly Glu Ser Ala Asp Leu Thr Ala Ile
    210                 215                 220

Ser Val Arg Leu Ala Ser His Glu Val Asp Met Tyr Val Ser Asn Gly
225                 230                 235                 240

Leu Ile Gln Leu Lys Ile Asn Gly Val Gln Thr Pro Thr Asp Val Pro
                245                 250                 255

Tyr Thr Ser Lys Ser Gly Leu Leu Ile Ser Ser Glu Lys Glu Gly Leu
            260                 265                 270

Ser Leu Lys Ala Pro Glu Tyr Gly Val Glu Lys Leu Tyr Tyr Asp Arg
        275                 280                 285

Arg Lys Leu Glu Ile Arg Val Ala Phe Trp Met Val Gly Lys Thr Cys
    290                 295                 300

Gly Ile Cys Gly Lys Tyr Asp Ala Glu Lys Lys Arg Glu Tyr Gln Met
305                 310                 315                 320

Pro Ser Gly Tyr Leu Ala Lys Asp Ala Val Ser Phe Ala Gln Ser Trp
                325                 330                 335

Val Ile Ser Glu Asp Thr Cys Thr Gly Ala Cys Lys Leu Gln Arg Lys
            340                 345                 350

Phe Val Lys Ile Glu Lys Pro Val Ala Phe Asn Lys Lys Ala Ser Lys
        355                 360                 365

Cys Phe Ser Ile Glu Pro Val Leu Arg Cys Ala Glu Gly Cys Ser Ala
    370                 375                 380

Thr Arg Thr Val Pro Val Ser Val Gly Phe His Cys Val Pro Ser Asp
385                 390                 395                 400

Ser Thr Leu Glu Leu Glu Glu Glu Gln Val Arg Leu Asp Gln Lys Ser
                405                 410                 415

Glu Asp Leu Val Ser Arg Val Asp Ala His Thr Ala Cys Ser Cys Ala
            420                 425                 430

Gln Gln Leu Cys Ser Ala
        435

<210>  14
<211>  9
<212>  PRT
<213>  Gallus gallus

<400>  14

Leu Pro Glu Leu Thr Val Tyr Asn Arg
1               5

<210>  15
<211>  13
<212>  PRT
<213>  Gallus gallus

<400>  15

Leu Gly Glu Ser Ala Asp Leu Thr Ala Ile Ser Val Arg
1               5                   10

<210>  16
<211>  13
<212>  PRT
<213>  Gallus gallus

<400>  16

Val Gln Val Phe Val Ser Ser Ile Thr Glu Ser Asp Arg
1               5                   10

<210>  17
<211>  996
<212>  DNA
<213>  Gallus gallus

<400>  17
gacaaacctt ttgcatcagg ttacttgaag atgtttggcc aggagttgct ctttggtaga       60

ctcgataaag acaccctcca aaatgtattg caggtatggt atggacctga tgaaaaaatc      120

ccttcaataa ggagattaat cagtagcctt caaactggca taggaagaca atggactaag 180

gctttactat tgtctgagat tcgttgtatt gtgcctacct gtgttgggtt cccgatggag 240

accagcttct attactcttc tgtcacaaaa gtggcaggaa acgttcaagc gcaaattaca 300

ccttcaccga ggtctgattt cagattgact gagttactaa attccaacgt taggctgcga 360

tccaaaatga gtctaagcat ggctaaacat atgacctttg taattgggat caacacaaac 420

atgatccagg cagggctgga agcacacacc aaagtaaatg ctcatgtacc tgtgaatgtt 480

gttgccacta ttcaaatgaa ggaaaaaagt atcaaagctg aaattccacc atgcaaagaa 540

gagactaact taattattgt aagctctaag acatttgctg ttacacgaaa tattgaagat 600

ttggctgcta gtaagatgac tccagttctt ctacctgaag cagtgcctga cataatgaag 660

atgtccttcg actcagattc tgcatcaggc gagactgata acatcaggga cagacagtct 720

gtagaagatg tttcgtctgg aaattccttc tcctttggac atccttcttc cgggaaggag 780

ccatttattc agtccatgtg ctccaacgca agtacatttg gggttcaagt gtgcattgag 840

aagaaaagtg tacatgcagc attcatcaga aatgtgcctc tttataacgc tattggagaa 900

catgccctta gaatgagctt caagccagtc tactcagatg tacctattga aaaaatacaa 960

gtcacaattc aagcaggaga tcaagctcct acaaaa 996

<210> 18
<211> 332
<212> PRT
<213> Gallus gallus

<400> 18

Asp Lys Pro Phe Ala Ser Gly Tyr Leu Lys Met Phe Gly Gln Glu Leu
1               5                   10                  15

Leu Phe Gly Arg Leu Asp Lys Asp Thr Leu Gln Asn Val Leu Gln Val
            20                  25                  30

Trp Tyr Gly Pro Asp Glu Lys Ile Pro Ser Ile Arg Arg Leu Ile Ser
        35                  40                  45

Ser Leu Gln Thr Gly Ile Gly Arg Gln Trp Thr Lys Ala Leu Leu Leu
    50                  55                  60

Ser Glu Ile Arg Cys Ile Val Pro Thr Cys Val Gly Phe Pro Met Glu
65                  70                  75                  80

Thr Ser Phe Tyr Tyr Ser Ser Val Thr Lys Val Ala Gly Asn Val Gln
                85                  90                  95

Ala Gln Ile Thr Pro Ser Pro Arg Ser Asp Phe Arg Leu Thr Glu Leu
            100                 105                 110

Leu Asn Ser Asn Val Arg Leu Arg Ser Lys Met Ser Leu Ser Met Ala
        115                 120                 125

Lys His Met Thr Phe Val Ile Gly Ile Asn Thr Asn Met Ile Gln Ala
    130                 135                 140

Gly Leu Glu Ala His Thr Lys Val Asn Ala His Val Pro Val Asn Val
145                 150                 155                 160

Val Ala Thr Ile Gln Met Lys Glu Lys Ser Ile Lys Ala Glu Ile Pro
                165                 170                 175

Pro Cys Lys Glu Glu Thr Asn Leu Ile Ile Val Ser Ser Lys Thr Phe
            180                 185                 190

Ala Val Thr Arg Asn Ile Glu Asp Leu Ala Ala Ser Lys Met Thr Pro
        195                 200                 205

Val Leu Leu Pro Glu Ala Val Pro Asp Ile Met Lys Met Ser Phe Asp
    210                 215                 220

Ser Asp Ser Ala Ser Gly Glu Thr Asp Asn Ile Arg Asp Arg Gln Ser
225                 230                 235                 240

Val Glu Asp Val Ser Ser Gly Asn Ser Phe Ser Phe Gly His Pro Ser
                245                 250                 255

Ser Gly Lys Glu Pro Phe Ile Gln Ser Met Cys Ser Asn Ala Ser Thr
            260                 265                 270

Phe Gly Val Gln Val Cys Ile Glu Lys Lys Ser Val His Ala Ala Phe
        275                 280                 285

Ile Arg Asn Val Pro Leu Tyr Asn Ala Ile Gly Glu His Ala Leu Arg
    290                 295                 300

Met Ser Phe Lys Pro Val Tyr Ser Asp Val Pro Ile Glu Lys Ile Gln
305                 310                 315                 320

Val Thr Ile Gln Ala Gly Asp Gln Ala Pro Thr Lys
                325                 330

<210> 19
<211> 13
<212> PRT

<213>  Gallus gallus

<400>  19

Ala Gly Asn Val Gln Ala Gln Ile Thr Pro Ser Pro Arg
1               5                   10


<210>  20
<211>  8
<212>  PRT
<213>  Gallus gallus

<400>  20

Ala Leu Leu Leu Ser Glu Ile Arg
1               5


<210>  21
<211>  8
<212>  PRT
<213>  Gallus gallus

<400>  21

Asp Lys Pro Phe Ala Ser Gly Tyr
1               5


<210>  22
<211>  10
<212>  PRT
<213>  Gallus gallus

<400>  22

Asp Lys Pro Phe Ala Ser Gly Tyr Leu Lys
1               5                   10


<210>  23
<211>  16
<212>  PRT
<213>  Gallus gallus

<400>  23

Asp Thr Leu Gln Asn Val Leu Gln Val Trp Tyr Gly Pro Asp Glu Lys
1               5                   10                  15


<210>  24
<211>  11
<212>  PRT
<213>  Gallus gallus

<400>  24

Glu Glu Thr Asn Leu Ile Ile Val Ser Ser Lys
1               5                   10

<210>  25
<211>  12
<212>  PRT
<213>  Gallus gallus

<400>  25

Gly Asn Val Gln Ala Gln Ile Thr Pro Ser Pro Arg
1               5                   10


<210>  26
<211>  10
<212>  PRT
<213>  Gallus gallus

<400>  26

Ile Gln Ala Gly Asp Gln Ala Pro Thr Lys
1               5                   10


<210>  27
<211>  13
<212>  PRT
<213>  Gallus gallus

<400>  27

Ile Gln Val Thr Ile Gln Ala Gly Asp Gln Ala Pro Thr
1               5                   10


<210>  28
<211>  14
<212>  PRT
<213>  Gallus gallus

<400>  28

Ile Gln Val Thr Ile Gln Ala Gly Asp Gln Ala Pro Thr Lys
1               5                   10


<210>  29
<211>  20
<212>  PRT
<213>  Gallus gallus

<400>  29

Ile Thr Pro Ser Pro Arg Ser Asp Phe Arg Leu Thr Glu Leu Leu Asn
1               5                   10                  15


Ser Asn Val Arg
            20


<210>  30
<211>  12
<212>  PRT
<213>  Gallus gallus

<400>  30

Lys Glu Glu Thr Asn Leu Ile Ile Val Ser Ser Lys
1               5                   10


<210>  31
<211>  11
<212>  PRT
<213>  Gallus gallus

<400>  31

Lys Pro Val Tyr Ser Asp Val Pro Ile Glu Lys
1               5                   10


<210>  32
<211>  8
<212>  PRT
<213>  Gallus gallus

<400>  32

Leu Asp Lys Asp Thr Leu Gln Asn
1               5


<210>  33
<211>  11
<212>  PRT
<213>  Gallus gallus

<400>  33

Leu Asp Lys Asp Thr Leu Gln Asn Val Leu Gln
1               5                   10


<210>  34
<211>  19
<212>  PRT
<213>  Gallus gallus

<400>  34

Leu Asp Lys Asp Thr Leu Gln Asn Val Leu Gln Val Trp Tyr Gly Pro
1               5                   10                  15


Asp Glu Lys



<210>  35
<211>  11
<212>  PRT
<213>  Gallus gallus

<400>  35

Leu Ile Ser Ser Leu Gln Thr Gly Ile Gly Arg

1               5                   10


<210>  36
<211>  8
<212>  PRT
<213>  Gallus gallus

<400>  36

Leu Thr Glu Leu Leu Asn Ser Asn
1               5


<210>  37
<211>  10
<212>  PRT
<213>  Gallus gallus

<400>  37

Leu Thr Glu Leu Leu Asn Ser Asn Val Arg
1               5                   10


<210>  38
<211>  9
<212>  PRT
<213>  Gallus gallus

<400>  38

Asn Ile Glu Asp Leu Ala Ala Ser Lys
1               5


<210>  39
<211>  14
<212>  PRT
<213>  Gallus gallus

<400>  39

Asn Val Pro Leu Tyr Asn Ala Ile Gly Glu His Ala Leu Arg
1               5                   10


<210>  40
<211>  11
<212>  PRT
<213>  Gallus gallus

<400>  40

Asn Val Gln Ala Gln Ile Thr Pro Ser Pro Arg
1               5                   10


<210>  41
<211>  8
<212>  PRT
<213>  Gallus gallus

<400>  41

Pro Ser Ser Gly Lys Glu Pro Phe
1               5


<210>  42
<211>  10
<212>  PRT
<213>  Gallus gallus

<400>  42

Pro Val Tyr Ser Asp Val Pro Ile Glu Lys
1               5                   10


<210>  43
<211>  12
<212>  PRT
<213>  Gallus gallus

<400>  43

Arg Leu Ile Ser Ser Leu Gln Thr Gly Ile Gly Arg
1               5                   10


<210>  44
<211>  8
<212>  PRT
<213>  Gallus gallus

<400>  44

Ser Val His Ala Ala Phe Ile Arg
1               5


<210>  45
<211>  10
<212>  PRT
<213>  Gallus gallus

<400>  45

Val Gln Ala Gln Ile Thr Pro Ser Pro Arg
1               5                   10


<210>  46
<211>  1023
<212>  DNA
<213>  Gallus gallus

<400>  46
ggtccttctc accaggcagc catggtgatg gctctgacgt ctccaagaac ctgcgatgtg      60

gtcctgaagc tacctgagct cacagtttac aacagagcca tcaggcttcc cctgccactc     120

ccttcaagtt cagatacacc aacctcaaca ctaccatcct ccaaatggaa cgtcttttac     180

caagcagcct tttcaatcat tgaaaacctt aaagctcgtt gttcagtttc acaaaatatg     240

ataacaacct tcaatggagt tgagtttaac tattcaatgc ctgcaaattg ctaccacatc 300

ttggcacagg attgtagtcc agaactgaag ttcctggtaa tgatgaaaag acttggagaa 360

tctgctgatc tcacagcaat aagtgtcaga cttgccagcc atgaggttga catgtatgtt 420

tccaatggac taatccagct gaagattaat ggtgttcaaa ccccaacaga cgttccatac 480

acatctaagt ctggtctgct gataagcagt gagaaggaag gtttgtcatt aaaagcccct 540

gaatatggtg tagaaaaact atattacgat agacgtaaac ttgagattcg ggttgctttc 600

tggatggttg gaaaaacatg tggtatttgt ggaaaatatg atgctgagaa gaaaagggag 660

tatcaaatgc ccagtggata tttagctaaa gatgcagtga gttttgctca gtcctgggtc 720

atctcagaag acacgtgtac tggagcttgc aagctgcaga ggaaatttgt caaaattgag 780

aagccggttg catttaacaa aaaggcctca aaatgctttt ccattgagcc agttttacgc 840

tgtgcagaag gctgttcagc aaccaggact gttcctgtct ctgtgggttt ccactgtgtc 900

ccatctgact ccacactcga gctggaggaa gagcaggtga ggttagacca gaagtctgag 960

gacttggtga gcagggtcga tgcccatacg gcgtgttcct gcgcccagca gctgtgctca 1020

gcg 1023

<210> 47
<211> 341
<212> PRT
<213> Gallus gallus

<400> 47

Gly Pro Ser His Gln Ala Ala Met Val Met Ala Leu Thr Ser Pro Arg
1               5                   10                  15

Thr Cys Asp Val Val Leu Lys Leu Pro Glu Leu Thr Val Tyr Asn Arg
            20                  25                  30

Ala Ile Arg Leu Pro Leu Pro Leu Pro Ser Ser Ser Asp Thr Pro Thr
        35                  40                  45

Ser Thr Leu Pro Ser Ser Lys Trp Asn Val Phe Tyr Gln Ala Ala Phe
    50                  55                  60

Ser Ile Ile Glu Asn Leu Lys Ala Arg Cys Ser Val Ser Gln Asn Met
65                  70                  75                  80

Ile Thr Thr Phe Asn Gly Val Glu Phe Asn Tyr Ser Met Pro Ala Asn
                85                  90                  95

Cys Tyr His Ile Leu Ala Gln Asp Cys Ser Pro Glu Leu Lys Phe Leu
            100                 105                 110

Val Met Met Lys Arg Leu Gly Glu Ser Ala Asp Leu Thr Ala Ile Ser
        115                 120                 125

Val Arg Leu Ala Ser His Glu Val Asp Met Tyr Val Ser Asn Gly Leu
    130                 135                 140

Ile Gln Leu Lys Ile Asn Gly Val Gln Thr Pro Thr Asp Val Pro Tyr
145                 150                 155                 160

Thr Ser Lys Ser Gly Leu Leu Ile Ser Ser Glu Lys Glu Gly Leu Ser
                165                 170                 175

Leu Lys Ala Pro Glu Tyr Gly Val Glu Lys Leu Tyr Tyr Asp Arg Arg
            180                 185                 190

Lys Leu Glu Ile Arg Val Ala Phe Trp Met Val Gly Lys Thr Cys Gly
        195                 200                 205

Ile Cys Gly Lys Tyr Asp Ala Glu Lys Lys Arg Glu Tyr Gln Met Pro
    210                 215                 220

Ser Gly Tyr Leu Ala Lys Asp Ala Val Ser Phe Ala Gln Ser Trp Val
225                 230                 235                 240

Ile Ser Glu Asp Thr Cys Thr Gly Ala Cys Lys Leu Gln Arg Lys Phe
                245                 250                 255

Val Lys Ile Glu Lys Pro Val Ala Phe Asn Lys Lys Ala Ser Lys Cys
            260                 265                 270

Phe Ser Ile Glu Pro Val Leu Arg Cys Ala Glu Gly Cys Ser Ala Thr
        275                 280                 285

Arg Thr Val Pro Val Ser Val Gly Phe His Cys Val Pro Ser Asp Ser
    290                 295                 300

Thr Leu Glu Leu Glu Glu Glu Gln Val Arg Leu Asp Gln Lys Ser Glu
305                 310                 315                 320

Asp Leu Val Ser Arg Val Asp Ala His Thr Ala Cys Ser Cys Ala Gln
                325                 330                 335

Gln Leu Cys Ser Ala
            340

<210>  48

<211>  8
<212>  PRT
<213>  Gallus gallus

<400>  48

Ala Pro Glu Tyr Gly Val Glu Lys
1               5


<210>  49
<211>  7
<212>  PRT
<213>  Gallus gallus

<400>  49

Ile Glu Lys Pro Val Ala Phe
1               5


<210>  50
<211>  15
<212>  PRT
<213>  Gallus gallus

<400>  50

Ile Asn Gly Val Gln Thr Pro Thr Asp Val Pro Tyr Thr Ser Lys
1               5                   10                  15


<210>  51
<211>  13
<212>  PRT
<213>  Gallus gallus

<400>  51

Leu Gly Glu Ser Ala Asp Leu Thr Ala Ile Ser Val Arg
1               5                   10


<210>  52
<211>  9
<212>  PRT
<213>  Gallus gallus

<400>  52

Leu Pro Glu Leu Thr Val Tyr Asn Arg
1               5


<210>  53
<211>  16
<212>  PRT
<213>  Gallus gallus

<400>  53

Leu Pro Leu Pro Leu Pro Ser Ser Ser Asp Thr Pro Thr Ser Thr Leu
1               5                   10                  15

<210>  54
<211>  12
<212>  PRT
<213>  Gallus gallus

<400>  54

Pro Ser Asp Ser Thr Leu Glu Leu Glu Glu Glu Gln
1               5                   10


<210>  55
<211>  9
<212>  PRT
<213>  Gallus gallus

<400>  55

Ser Gly Leu Leu Ile Ser Ser Glu Lys
1               5


<210>  56
<211>  7
<212>  PRT
<213>  Gallus gallus

<400>  56

Ser Ile Glu Pro Val Leu Arg
1               5


<210>  57
<211>  9
<212>  PRT
<213>  Gallus gallus

<400>  57

Thr Val Pro Val Ser Val Gly Phe His
1               5


<210>  58
<211>  3363
<212>  DNA
<213>  Gallus gallus

<400>  58
   atgaggggga tcatactggc attagtgctc acccttgtag gcagccagaa gtttgacatt      60
   gacccaggat tcaatagcag aaggagttac ctgtacaact atgaaggttc tatgttgaat     120
   gggcttcaag acagaagttt gggcaaagct ggtgtgcgct tgagcagcaa gctagagatc     180
   agtgggctac cagagaatgc ttacctcctc aaggtccgct ctccacaagt ggaggagtac     240
   aatggggtct ggcccaggga tcccttcact cgatcttcca aaatcaccca agtgatctca     300
   tcgtgtttca cccggctctt caaatttgaa tacagcagtg gacggatcgg aaacatttat     360
   gccccagaag actgcccaga tctgtgtgtt aacatagtga gaggaatatt gaacatgttc 420
   cagatgacca ttaaaaaatc acagaacgtc tacgaattac aagaggctgg aattggaggt 480
   atttgtcatg caaggtatgt cattcaggaa gacaggaaga atagccgaat ctatgttacc 540
   agaactgtgg acttgaataa ttgccaggaa aaggtgcaaa aaagcattgg aatggcttac 600
   atctatccct gccctgtgga cgtgatgaaa gaaaggctca ccaaagggac caccgctttc 660
   tcctacaagc tgaagcagtc agacagcggc acgctgatca cagatgtctc gtcgcggcag 720
   gtgtatcaga tctccccatt caatgagccc actggggtgg ctgtcatgga agcaagacag 780
   cagctcactt tggtcgaggt gagaagtgag cggggcagtg ccccagatgt ccccatgcag 840
   aactatggca gccttcgcta ccgcttccca gccgtactgc cacagatgcc acttcagctg 900
   atcaagacaa aaaaccctga gcaacggata gtagaaacgc tgcagcacat agtcctgaat 960
   aaccaacaag atttccatga cgatgtttca tacagattct tagaggtggt ccagctttgc 1020
   cggatagcaa atgctgacaa tcttgagtct atctggagac aagtttcaga taaacctcgt 1080
   tacaggcgat ggctcctgag cgcagtttct gcgagtggca ccacagaaac actaaaattc 1140
   cttaagaaca gaattcgcaa tgatgacctc aactacattc agacccttct aactgtttct 1200
   ttgactcttc atttattgca agctgatgaa cacacacttc caatagcagc agatttaatg 1260
   accagctctc gaattcagaa aaatcctgtg cttcagcaag tggcctgctt gggatatagc 1320
   tctgtagtca acagatactg ctctcagacc tcagcatgtc ctaaggaagc tcttcagccc 1380
   atccatgacc tggcagatga agcaatcagc aggggccgtg aagacaaaat gaaattagct 1440
   ctaaagtgca ttggtaacat gggagaacca gccagcttaa agcgcatcct gaagttcctt 1500
   ccaatatctt catccagtgc tgctgatatc ccagtccaca ttcagataga tgccataacg 1560
   gccttgaaaa agatagcttg gaaggacccc aaaacagtgc agggctatct catccagatc 1620
   cttgcagacc aatcacttcc ccctgaggtg cgaatgatgg cttgtgctgt tatctttgag 1680
   acaaggcctg cccttgcttt gataacgact atagctaacg tggcaatgaa ggagagcaat 1740
   atgcaagtgg ccagttttgt atattcccac atgaagtctt tgtcaaagag cagattgcca 1800
   tttatgtaca acatatcttc cgcttgtaac attgccctta agctcctgtc ccccaaactg 1860
   gacagtatga gctatcggta cagcaaggtc attcgagcag acacttactt tgataactat 1920
   agagttggtg ctactggaga aatctttgtt gtgaacagcc caagaactat gttcccatca 1980
   gcaataattt ccaaattgat ggcaaattct gcaggttcag tggctgatct ggtagaggtt 2040
   ggcatccgag tggaaggcct cgcagatgtc ataatgaaaa gaaacatccc atttgctgaa 2100
   tatcccacat acaagcagat aaaggagctt ggaaaagctc tgcagggatg gaaagagctg 2160
   ccgacagaaa cccctttggt atcagcctac ttgaaaatac ttggccaaga agtggccttc 2220
   atcaacatca acaaggaact cctgcaacag gtcatgaaga ctgtagtgga acctgctgat 2280
   cgaaacgcag caataaagag aatcgccaac cagatccgca acagcattgc agggcagtgg 2340
   acgcagccgg tgtggatggg agagctgcga tacgtggttc ccagctgtct cggcctgccg 2400
   ctggagtacg ggtcctacac caccgccctg gcacgagctg cagtcagcgt tgagggaaag 2460
   atgacgccgc ctttaaccgg agatttcaga ctttctcagt tgcttgaatc caccatgcag 2520
   attcggtctg acttaaagcc cagtttatat gtgcatacag ttgcaacgat gggtgtcaac 2580
   acagaatact ttcaacatgc tgttgaaatt caaggcgagg tccagacaag aatgccaatg 2640
   aagtttgatg ccaagataga tgtgaaattg aaaaacctta agattgaaac gaacccatgc 2700
   cgtgaggaaa ctgagatagt ggttggaaga cataaggctt ttgctgtatc aaggaacata 2760
   ggagaactag gtgttgaaaa gaggacctca attctgccgg aagatgctcc attagatgtt 2820
   acagaagaac ctttccaaac atcagagaga gcttccaggg aacacttcgc aatgcaaggg 2880
   cctgacagca tgccaaggaa acagtcccat agttctcgag aagatcttcg ccgtagcaca 2940
   ggaaaaagag cacataaacg agacatttgc ctcaaaatgc atcatattgg ttgccagctt 3000
   tgcttttcca gaaggtcaag agatgccagt ttcatacaga atacgtattt gcacaaatta 3060
   attggagaac atgaagctaa aatagttttg atgccagttc atacagatgc tgatattgac 3120
   aaaattcagc tggagattca ggcaggatct agagcagctg ccagaataat tactgaggta 3180
   aacccagagt ctgaggaaga ggatgaatca tctccatatg aggacattca agctaaactg 3240
   aagaggattc taggcattga cagtatgttc aaggttgcaa acaaaacacg gcacccgaaa 3300
   aatcgaccat ctaagaaagg aaacactgtg ctagcagagt ttgggacaga gcctgatgca 3360
   aaa 3363

<210> 59
<211> 1121
<212> PRT
<213> Gallus gallus

<400> 59

Met Arg Gly Ile Ile Leu Ala Leu Val Leu Thr Leu Val Gly Ser Gln
1               5                   10                  15

Lys Phe Asp Ile Asp Pro Gly Phe Asn Ser Arg Arg Ser Tyr Leu Tyr
            20                  25                  30

Asn Tyr Glu Gly Ser Met Leu Asn Gly Leu Gln Asp Arg Ser Leu Gly
        35                  40                  45

Lys Ala Gly Val Arg Leu Ser Ser Lys Leu Glu Ile Ser Gly Leu Pro
    50                  55                  60

Glu Asn Ala Tyr Leu Leu Lys Val Arg Ser Pro Gln Val Glu Glu Tyr
65                  70                  75                  80

Asn Gly Val Trp Pro Arg Asp Pro Phe Thr Arg Ser Ser Lys Ile Thr
                85                  90                  95

Gln Val Ile Ser Ser Cys Phe Thr Arg Leu Phe Lys Phe Glu Tyr Ser
            100                 105                 110

Ser Gly Arg Ile Gly Asn Ile Tyr Ala Pro Glu Asp Cys Pro Asp Leu
        115                 120                 125

Cys Val Asn Ile Val Arg Gly Ile Leu Asn Met Phe Gln Met Thr Ile
    130                 135                 140

Lys Lys Ser Gln Asn Val Tyr Glu Leu Gln Glu Ala Gly Ile Gly Gly
145                 150                 155                 160

Ile Cys His Ala Arg Tyr Val Ile Gln Glu Asp Arg Lys Asn Ser Arg
                165                 170                 175

Ile Tyr Val Thr Arg Thr Val Asp Leu Asn Asn Cys Gln Glu Lys Val
            180                 185                 190

Gln Lys Ser Ile Gly Met Ala Tyr Ile Tyr Pro Cys Pro Val Asp Val
        195                 200                 205

Met Lys Glu Arg Leu Thr Lys Gly Thr Thr Ala Phe Ser Tyr Lys Leu
    210                 215                 220

Lys Gln Ser Asp Ser Gly Thr Leu Ile Thr Asp Val Ser Ser Arg Gln
225                 230                 235                 240

Val Tyr Gln Ile Ser Pro Phe Asn Glu Pro Thr Gly Val Ala Val Met
                245                 250                 255

Glu Ala Arg Gln Gln Leu Thr Leu Val Glu Val Arg Ser Glu Arg Gly
            260                 265                 270

Ser Ala Pro Asp Val Pro Met Gln Asn Tyr Gly Ser Leu Arg Tyr Arg
        275                 280                 285

Phe Pro Ala Val Leu Pro Gln Met Pro Leu Gln Leu Ile Lys Thr Lys
    290                 295                 300

Asn Pro Glu Gln Arg Ile Ile Glu Thr Leu Gln His Ile Val Leu Asn
305                 310                 315                 320

Asn Gln Gln Asp Phe His Asp Asp Val Ser Tyr Arg Phe Leu Glu Val
                325                 330                 335

Val Gln Leu Cys Arg Ile Ala Asn Ala Asp Asn Leu Glu Ser Ile Trp
            340                 345                 350

Arg Gln Val Ser Asp Lys Pro Arg Tyr Arg Arg Trp Leu Leu Ser Ala
        355                 360                 365

Val Ser Ala Ser Gly Thr Thr Glu Thr Leu Lys Phe Leu Lys Asn Arg
    370                 375                 380

Ile Arg Asn Asp Asp Leu Asn Tyr Ile Gln Thr Leu Leu Thr Val Ser
385                 390                 395                 400

Leu Thr Leu His Leu Leu Gln Ala Asp Glu His Thr Leu Pro Ile Ala
                405                 410                 415

Ala Asp Leu Met Thr Ser Ser Arg Ile Gln Lys Asn Pro Val Leu Gln
            420                 425                 430

Gln Val Ala Cys Leu Gly Tyr Ser Ser Val Val Asn Arg Tyr Cys Ser
        435                 440                 445

Gln Thr Ser Ala Cys Pro Lys Glu Ala Leu Gln Pro Ile His Asp Leu
    450                 455                 460

Ala Asp Glu Ala Ile Ser Arg Gly Arg Glu Asp Lys Met Lys Leu Ala
465                 470                 475                 480

Leu Lys Cys Ile Gly Asn Met Gly Glu Pro Ala Ser Leu Lys Arg Ile
                485                 490                 495

Leu Lys Phe Leu Pro Ile Ser Ser Ser Ser Ala Ala Asp Ile Pro Val
            500                 505                 510

His Ile Gln Ile Asp Ala Ile Thr Ala Leu Lys Lys Ile Ala Trp Lys
        515                 520                 525

Asp Pro Lys Thr Val Gln Gly Tyr Leu Ile Gln Ile Leu Ala Asp Gln
    530                 535                 540

Ser Leu Pro Pro Glu Val Arg Met Met Ala Cys Ala Val Ile Phe Glu
545                 550                 555                 560

Thr Arg Pro Ala Leu Ala Leu Ile Thr Thr Ile Ala Asn Val Ala Met

565                 570                 575

Lys Glu Ser Asn Met Gln Val Ala Ser Phe Val Tyr Ser His Met Lys
            580                 585                 590

Ser Leu Ser Lys Ser Arg Leu Pro Phe Met Tyr Asn Ile Ser Ser Ala
        595                 600                 605

Cys Asn Ile Ala Leu Lys Leu Leu Ser Pro Lys Leu Asp Ser Met Ser
    610                 615                 620

Tyr Arg Tyr Ser Lys Val Ile Arg Ala Asp Thr Tyr Phe Asp Asn Tyr
625                 630                 635                 640

Arg Val Gly Ala Thr Gly Glu Ile Phe Val Val Asn Ser Pro Arg Thr
                645                 650                 655

Met Phe Pro Ser Ala Ile Ile Ser Lys Leu Met Ala Asn Ser Ala Gly
            660                 665                 670

Ser Val Ala Asp Leu Val Glu Val Gly Ile Arg Val Glu Gly Leu Ala
        675                 680                 685

Asp Val Ile Met Lys Arg Asn Ile Pro Phe Ala Glu Tyr Pro Thr Tyr
    690                 695                 700

Lys Gln Ile Lys Glu Leu Gly Lys Ala Leu Gln Gly Trp Lys Glu Leu
705                 710                 715                 720

Pro Thr Glu Thr Pro Leu Val Ser Ala Tyr Leu Lys Ile Leu Gly Gln
                725                 730                 735

Glu Val Ala Phe Ile Asn Ile Asn Lys Glu Leu Leu Gln Gln Val Met
            740                 745                 750

Lys Thr Val Val Glu Pro Ala Asp Arg Asn Ala Ala Ile Lys Arg Ile
        755                 760                 765

Ala Asn Gln Ile Arg Asn Ser Ile Ala Gly Gln Trp Thr Gln Pro Val
    770                 775                 780

Trp Met Gly Glu Leu Arg Tyr Val Val Pro Ser Cys Leu Gly Leu Pro
785                 790                 795                 800

Leu Glu Tyr Gly Ser Tyr Thr Thr Ala Leu Ala Arg Ala Ala Val Ser
                805                 810                 815

Val Glu Gly Lys Met Thr Pro Pro Leu Thr Gly Asp Phe Arg Leu Ser
            820                 825                 830

Gln Leu Leu Glu Ser Thr Met Gln Ile Arg Ser Asp Leu Lys Pro Ser
        835                 840                 845

Leu Tyr Val His Thr Val Ala Thr Met Gly Val Asn Thr Glu Tyr Phe
    850                 855                 860

Gln His Ala Val Glu Ile Gln Gly Glu Val Gln Thr Arg Met Pro Met
865                 870                 875                 880

Lys Phe Asp Ala Lys Ile Asp Val Lys Leu Lys Asn Leu Lys Ile Glu
                885                 890                 895

Thr Asn Pro Cys Arg Glu Glu Thr Glu Ile Val Val Gly Arg His Lys
            900                 905                 910

Ala Phe Ala Val Ser Arg Asn Ile Gly Glu Leu Gly Val Glu Lys Arg
        915                 920                 925

Thr Ser Ile Leu Pro Glu Asp Ala Pro Leu Asp Val Thr Glu Glu Pro
    930                 935                 940

Phe Gln Thr Ser Glu Arg Ala Ser Arg Glu His Phe Ala Met Gln Gly
945                 950                 955                 960

Pro Asp Ser Met Pro Arg Lys Gln Ser His Ser Ser Arg Glu Asp Leu
                965                 970                 975

Arg Arg Ser Thr Gly Lys Arg Ala His Lys Arg Asp Ile Cys Leu Lys
            980                 985                 990

Met His His Ile Gly Cys Gln Leu  Cys Phe Ser Arg Arg  Ser Arg Asp
        995                 1000                 1005

Ala Ser  Phe Ile Gln Asn Thr  Tyr Leu His Lys Leu  Ile Gly Glu
    1010                 1015                 1020

His Glu  Ala Lys Ile Val Leu  Met Pro Val His Thr  Asp Ala Asp
    1025                 1030                 1035

Ile Asp  Lys Ile Gln Leu Glu  Ile Gln Ala Gly Ser  Arg Ala Ala
    1040                 1045                 1050

Ala Arg  Ile Ile Thr Glu Val  Asn Pro Glu Ser Glu  Glu Glu Asp
    1055                 1060                 1065

Glu Ser Ser Pro Tyr Glu Asp Ile Gln Ala Lys Leu Lys Arg Ile
    1070                1075                1080

Leu Gly Ile Asp Ser Met Phe Lys Val Ala Asn Lys Thr Arg His
    1085                1090                1095

Pro Lys Asn Arg Pro Ser Lys Lys Gly Asn Thr Val Leu Ala Glu
    1100                1105                1110

Phe Gly Thr Glu Pro Asp Ala Lys
    1115                1120

<210> 60
<211> 10
<212> PRT
<213> Gallus gallus

<400> 60

Ala Asp Gln Ser Leu Pro Pro Glu Val Arg
1               5                   10

<210> 61
<211> 9
<212> PRT
<213> Gallus gallus

<400> 61

Ala Asp Thr Tyr Phe Asp Asn Tyr Arg
1               5

<210> 62
<211> 7
<212> PRT
<213> Gallus gallus

<400> 62

Ala Val Ile Phe Glu Thr Arg
1               5

<210> 63
<211> 12
<212> PRT
<213> Gallus gallus

<400> 63

Asp Ala Ser Phe Ile Gln Asn Thr Tyr Leu His Lys
1               5                   10

<210> 64
<211> 9

<212>  PRT
<213>  Gallus gallus

<400>  64

Asp Leu Ala Asp Glu Ala Ile Ser Arg
1               5


<210>  65
<211>  16
<212>  PRT
<213>  Gallus gallus

<400>  65

Glu Ala Leu Gln Pro Ile His Asp Leu Ala Asp Glu Ala Ile Ser Arg
1               5                   10                  15


<210>  66
<211>  9
<212>  PRT
<213>  Gallus gallus

<400>  66

Glu Glu Thr Glu Ile Val Val Gly Arg
1               5


<210>  67
<211>  9
<212>  PRT
<213>  Gallus gallus

<400>  67

Glu Ile Phe Val Val Asn Ser Pro Arg
1               5


<210>  68
<211>  14
<212>  PRT
<213>  Gallus gallus

<400>  68

Glu Leu Pro Thr Glu Thr Pro Leu Val Ser Ala Tyr Leu Lys
1               5                   10


<210>  69
<211>  10
<212>  PRT
<213>  Gallus gallus

<400>  69

Phe Asp Ile Asp Pro Gly Phe Asn Ser Arg
1               5                   10

<210>  70
<211>  7
<212>  PRT
<213>  Gallus gallus

<400>  70

Phe Glu Tyr Ser Ser Gly Arg
1               5


<210>  71
<211>  25
<212>  PRT
<213>  Gallus gallus

<400>  71

Phe Leu Pro Ile Ser Ser Ser Ser Ala Ala Asp Ile Pro Val His Ile
1               5                   10                  15


Gln Ile Asp Ala Ile Thr Ala Leu Lys
            20                  25


<210>  72
<211>  8
<212>  PRT
<213>  Gallus gallus

<400>  72

Gly Tyr Ser Ser Val Val Asn Arg
1               5


<210>  73
<211>  12
<212>  PRT
<213>  Gallus gallus

<400>  73

Ile Ala Asn Ala Asp Asn Leu Glu Ser Ile Trp Arg
1               5                   10


<210>  74
<211>  8
<212>  PRT
<213>  Gallus gallus

<400>  74

Ile Asp Ala Ile Thr Ala Leu Lys
1               5


<210>  75
<211>  24
<212>  PRT

<213>  Gallus gallus

<400>  75

Ile Ile Thr Glu Val Asn Pro Glu Ser Glu Glu Glu Asp Glu Ser Ser
1               5                   10                  15

Pro Tyr Glu Asp Ile Gln Ala Lys
            20

<210>  76
<211>  12
<212>  PRT
<213>  Gallus gallus

<400>  76

Ile Leu Ala Asp Gln Ser Leu Pro Pro Glu Val Arg
1               5                   10

<210>  77
<211>  13
<212>  PRT
<213>  Gallus gallus

<400>  77

Ile Leu Gly Gln Glu Val Ala Phe Ile Asn Ile Asn Lys
1               5                   10

<210>  78
<211>  10
<212>  PRT
<213>  Gallus gallus

<400>  78

Ile Gln Leu Glu Ile Gln Ala Gly Ser Arg
1               5                   10

<210>  79
<211>  13
<212>  PRT
<213>  Gallus gallus

<400>  79

Ile Arg Asn Asp Asp Leu Asn Tyr Ile Gln Thr Leu Leu
1               5                   10

<210>  80
<211>  8
<212>  PRT
<213>  Gallus gallus

<400>  80

Leu Ala Asp Glu Ala Ile Ser Arg
1               5

<210>  81
<211>  14
<212>  PRT
<213>  Gallus gallus

<400>  81

Leu Glu Ile Ser Gly Leu Pro Glu Asn Ala Tyr Leu Leu Lys
1               5                   10

<210>  82
<211>  9
<212>  PRT
<213>  Gallus gallus

<400>  82

Leu Gly Tyr Ser Ser Val Val Asn Arg
1               5

<210>  83
<211>  15
<212>  PRT
<213>  Gallus gallus

<400>  83

Leu Ile Gln Ile Leu Ala Asp Gln Ser Leu Pro Pro Glu Val Arg
1               5                   10                  15

<210>  84
<211>  16
<212>  PRT
<213>  Gallus gallus

<400>  84

Leu Lys Gln Ser Asp Ser Gly Thr Leu Ile Thr Asp Val Ser Ser Arg
1               5                   10                  15

<210>  85
<211>  15
<212>  PRT
<213>  Gallus gallus

<400>  85

Leu Leu Ser Ala Val Ser Ala Ser Gly Thr Thr Glu Thr Leu Lys
1               5                   10                  15

<210>  86
<211>  14
<212>  PRT
<213>  Gallus gallus

<400>  86

Leu Ser Ala Val Ser Ala Ser Gly Thr Thr Glu Thr Leu Lys
1               5                   10


<210>  87
<211>  11
<212>  PRT
<213>  Gallus gallus

<400>  87

Asn Ile Pro Phe Ala Glu Tyr Pro Thr Tyr Lys
1               5                   10


<210>  88
<211>  11
<212>  PRT
<213>  Gallus gallus

<400>  88

Pro Ala Leu Ala Leu Ile Thr Thr Ile Ala Asn
1               5                   10


<210>  89
<211>  8
<212>  PRT
<213>  Gallus gallus

<400>  89

Pro Pro Leu Thr Gly Asp Phe Arg
1               5


<210>  90
<211>  9
<212>  PRT
<213>  Gallus gallus

<400>  90

Gln Gln Leu Thr Leu Val Glu Val Arg
1               5


<210>  91
<211>  14
<212>  PRT
<213>  Gallus gallus

<400>  91

Gln Ser Asp Ser Gly Thr Leu Ile Thr Asp Val Ser Ser Arg
1               5                   10


<210>  92

<211>  12
<212>  PRT
<213>  Gallus gallus

<400>  92

Arg Asn Ile Pro Phe Ala Glu Tyr Pro Thr Tyr Lys
1               5                   10


<210>  93
<211>  14
<212>  PRT
<213>  Gallus gallus

<400>  93

Ser Ala Gly Ser Val Ala Asp Leu Val Glu Val Gly Ile Arg
1               5                   10


<210>  94
<211>  13
<212>  PRT
<213>  Gallus gallus

<400>  94

Ser Ala Val Ser Ala Ser Gly Thr Thr Glu Thr Leu Lys
1               5                   10


<210>  95
<211>  8
<212>  PRT
<213>  Gallus gallus

<400>  95

Ser Asp Leu Lys Pro Ser Leu Tyr
1               5


<210>  96
<211>  11
<212>  PRT
<213>  Gallus gallus

<400>  96

Ser Gly Thr Leu Ile Thr Asp Val Ser Ser Arg
1               5                   10


<210>  97
<211>  13
<212>  PRT
<213>  Gallus gallus

<400>  97

Ser Pro Gln Val Glu Glu Tyr Asn Gly Val Trp Pro Arg
1               5                   10

<210>  98
<211>  22
<212>  PRT
<213>  Gallus gallus

<400>  98

Thr Ser Ile Leu Pro Glu Asp Ala Pro Leu Asp Val Thr Glu Glu Pro
1               5                   10                  15

Phe Gln Thr Ser Glu Arg
            20

<210>  99
<211>  20
<212>  PRT
<213>  Gallus gallus

<400>  99

Thr Val Gln Gly Tyr Leu Ile Gln Ile Leu Ala Asp Gln Ser Leu Pro
1               5                   10                  15

Pro Glu Val Arg
            20

<210>  100
<211>  8
<212>  PRT
<213>  Gallus gallus

<400>  100

Thr Val Val Glu Pro Ala Asp Arg
1               5

<210>  101
<211>  14
<212>  PRT
<213>  Gallus gallus

<400>  101

Val Gly Ala Thr Gly Glu Ile Phe Val Val Asn Ser Pro Arg
1               5                   10

<210>  102
<211>  11
<212>  PRT
<213>  Gallus gallus

<400>  102

Val Ser Ala Ser Gly Thr Thr Glu Thr Leu Lys
1               5                   10

<210>  103
<211>  16
<212>  PRT
<213>  Gallus gallus

<400>  103

Trp Leu Leu Ser Ala Val Ser Ala Ser Gly Thr Thr Glu Thr Leu Lys
1               5                   10                  15


<210>  104
<211>  8
<212>  PRT
<213>  Gallus gallus

<400>  104

Tyr Val Ile Gln Glu Asp Arg Lys
1               5


<210>  105
<211>  5550
<212>  DNA
<213>  Gallus gallus

<400>  105
atgaggggga tcatactggc attagtgctc acccttgtag gcagccagaa gtttgacatt      60

gacccaggat tcaatagcag aaggagttac ctgtacaact atgaaggttc tatgttgaat     120

gggcttcaag acagaagttt gggcaaagct ggtgtgcgct tgagcagcaa gctagagatc     180

agtgggctac cagagaatgc ttacctcctc aaggtccgct ctccacaagt ggaggagtac     240

aatggggtct ggcccaggga tcccttcact cgatcttcca aaatcaccca agtgatctca     300

tcgtgtttca cccggctctt caaatttgaa tacagcagtg gacggatcgg aaacatttat     360

gccccagaag actgcccaga tctgtgtgtt aacatagtga gaggaatatt gaacatgttc     420

cagatgacca ttaaaaaatc acagaacgtc tacgaattac aagaggctgg aattggaggt     480

atttgtcatg caaggtatgt cattcaggaa gacaggaaga atagccgaat ctatgttacc     540

agaactgtgg acttgaataa ttgccaggaa aaggtgcaaa aaagcattgg aatggcttac     600

atctatccct gccctgtgga cgtgatgaaa gaaaggctca ccaaagggac caccgctttc     660

tcctacaagc tgaagcagtc agacagcggc acgctgatca cagatgtctc gtcgcggcag     720

gtgtatcaga tctccccatt caatgagccc actggggtgg ctgtcatgga agcaagacag     780

cagctcactt tggtcgaggt gagaagtgag cggggcagtg ccccagatgt ccccatgcag     840

aactatggca gccttcgcta ccgcttccca gccgtactgc cacagatgcc acttcagctg     900

atcaagacaa aaaaccctga gcaacggata gtagaaacgc tgcagcacat agtcctgaat     960

aaccaacaag atttccatga cgatgtttca tacagattct tagaggtggt ccagctttgc    1020

cggatagcaa atgctgacaa tcttgagtct atctggagac aagtttcaga taaacctcgt 1080

tacaggcgat ggctcctgag cgcagtttct gcgagtggca ccacagaaac actaaaattc 1140

cttaagaaca gaattcgcaa tgatgacctc aactacattc agacccttct aactgtttct 1200

ttgactcttc atttattgca agctgatgaa cacacacttc caatagcagc agatttaatg 1260

accagctctc gaattcagaa aaatcctgtg cttcagcaag tggcctgctt gggatatagc 1320

tctgtagtca acagatactg ctctcagacc tcagcatgtc ctaaggaagc tcttcagccc 1380

atccatgacc tggcagatga agcaatcagc aggggccgtg aagacaaaat gaaattagct 1440

ctaaagtgca ttggtaacat gggagaacca gccagcttaa agcgcatcct gaagttcctt 1500

ccaatatctt catccagtgc tgctgatatc ccagtccaca ttcagataga tgccataacg 1560

gccttgaaaa agatagcttg gaaggacccc aaaacagtgc agggctatct catccagatc 1620

cttgcagacc aatcacttcc ccctgaggtg cgaatgatgg cttgtgctgt tatctttgag 1680

acaaggcctg cccttgcttt gataacgact atagctaacg tggcaatgaa ggagagcaat 1740

atgcaagtgg ccagttttgt atattcccac atgaagtctt tgtcaaagag cagattgcca 1800

tttatgtaca acatatcttc cgcttgtaac attgccctta agctcctgtc ccccaaactg 1860

gacagtatga gctatcggta cagcaaggtc attcgagcag acacttactt tgataactat 1920

agagttggtg ctactggaga aatctttgtt gtgaacagcc caagaactat gttcccatca 1980

gcaataattt ccaaattgat ggcaaattct gcaggttcag tggctgatct ggtagaggtt 2040

ggcatccgag tggaaggcct cgcagatgtc ataatgaaaa gaaacatccc atttgctgaa 2100

tatcccacat acaagcagat aaaggagctt ggaaaagctc tgcagggatg gaaagagctg 2160

ccgacagaaa cccctttggt atcagcctac ttgaaaatac ttggccaaga agtggccttc 2220

atcaacatca acaaggaact cctgcaacag gtcatgaaga ctgtagtgga acctgctgat 2280

cgaaacgcag caataaagag aatcgccaac cagatccgca acagcattgc agggcagtgg 2340

acgcagccgg tgtggatggg agagctgcga tacgtggttc ccagctgtct cggcctgccg 2400

ctggagtacg ggtcctacac caccgccctg gcacgagctg cagtcagcgt tgagggaaag 2460

atgacgccgc ctttaaccgg agatttcaga ctttctcagt tgcttgaatc caccatgcag 2520

attcggtctg acttaaagcc cagtttatat gtgcatacag ttgcaacgat gggtgtcaac 2580

acagaatact ttcaacatgc tgttgaaatt caaggcgagg tccagacaag aatgccaatg 2640

aagtttgatg ccaagataga tgtgaaattg aaaaacctta agattgaaac gaacccatgc 2700

cgtgaggaaa ctgagatagt ggttggaaga cataaggctt ttgctgtatc aaggaacata 2760

ggagaactag gtgttgaaaa gaggacctca attctgccgg aagatgctcc attagatgtt 2820

acagaagaac ctttccaaac atcagagaga gcttccaggg aacacttcgc aatgcaaggg 2880

cctgacagca tgccaaggaa acagtcccat agttctcgag aagatcttcg ccgtagcaca 2940

ggaaaaagag cacataaacg agacatttgc ctcaaaatgc atcatattgg ttgccagctt 3000

tgcttttcca gaaggtcaag agatgccagt ttcatacaga atacgtattt gcacaaatta 3060

attggagaac atgaagctaa aatagttttg atgccagttc atacagatgc tgatattgac 3120

aaaattcagc tggagattca ggcaggatct agagcagctg ccagaataat tactgaggta 3180

aacccagagt ctgaggaaga ggatgaatca tctccatatg aggacattca agctaaactg 3240

aagaggattc taggcattga cagtatgttc aaggttgcaa acaaaacacg gcacccgaaa 3300

aatcgaccat ctaagaaagg aaacactgtg ctagcagagt ttgggacaga gcctgatgca 3360

aaaacttcct ccagctcatc ttctgcctcc tcaactgcca cctcttcttc ctcatcatct 3420

gcctcctctc ctaatcgtaa aaagcctatg gatgaagagg agaatgatca agtaaagcaa 3480

gcaagaaaca aagatgcaag cagcagcagc aggagcagca agagcagtaa cagcagcaag 3540

agaagcagca gcaagagcag taacagcagc aagagaagca gcagcagcag tagcagtagc 3600

agtagcagca gcaggagcag cagcagtagc agcagtagta gcagtaacag caagagcagc 3660

agtagcagca gcaagagcag cagtagcagc agcaggagca gaagcagcag caagagcagc 3720

agtagcagca gcagcagtag cagcagcagc agcagcaaaa gtagcagtag taggagcagt 3780

agcagcagca gcaagtcaag cagtcaccat agccatagcc atcattcagg gcatctaaat 3840

ggcagcagca gcagcagcag cagcagcagg tcagtgagtc accacagcca tgagcatcac 3900

tcaggacatc tggaagatga cagcagtagc agcagcagca gcagcgtgct ttccaaaata 3960

tgggggcgtc atgagattta tcagtatcgc tttagatcag cacacagaca agagttcccc 4020

aaaagaaaac tcccaggtga ccgcgctacc agcagatact cctctaccag atccagccat 4080

gacacatccc gagctgcttc ctggcctaag tttctgggtg acatcaaaac cccagtgtta 4140

gctgcttttc tccatggcat tagtaacaat aagaagacag gaggcctcca gcttgtggta 4200

tatgctgata ccgactcggt caggccgcgg gtgcaggtat ttgtgacaaa cctcacagat 4260

tcgagcaagt ggaagctctg cgcagatgct tcggtccgca atgctcacaa ggcagtggcc 4320

tacgtgaaat ggggctggga ctgccgggac tacaaggttt ctactgagct ggtaactggg 4380

cggtttgctg ggcaccctgc tgcacaagtg aagctggagt ggcccaaggt tccttcgaat 4440

gtcagatcag tggttgaatg gttttacgag tttgtccctg gggctgcatt tatgctgggt 4500

ttctctgaga gaatggacaa gaatccttct cgacaagcca ggatggttgt ggctctaact 4560

tctccgagga catgtgatgt tgttgtcaag ctgcctgata taatcctcta tcaaaaagcc 4620

gtgaggcttc ctctatcact ccctgtgggt ccaaggatcc cagcttcaga gctgcagcct 4680

ccaatctgga acgtctttgc tgaagccccc tctgccgtgc tcgagaattt gaaagctcgc 4740

tgctcagttt cgtacaacaa gatcaaaacc tttaatgaag tcaagttcaa ctactcgatg 4800

cccgcaaact gctatcacat cttggttcag gattgcagct ctgaacttaa gttcctggtg 4860

atgatgaaaa gtgctggaga agctacaaac cttaaagcca tcaacatcaa gattggcagt 4920

catgaaattg atatgcatcc tgtgaatgga caggtgaaac tgctggtaga tggggctgag 4980

agccccacag ccaacatttc cctcatatct gctggtgctt ctctgtggat tcacaatgaa 5040

aaccaagggt ttgcacttgc tgccccaggc catggtatcg ataaattgta cttcgatgga 5100

aaaacaatca cgattcaagt tcctttatgg atggcaggga aaacatgtgg aatctgtgga 5160

aaatatgatg cagaatgcga acaggagtat cggatgccca atggatatct agctaaaaat 5220

gccgtgagct ttggtcattc ttggatcttg gaagaagcgc cctgtagagg agcttgtaaa 5280

ctgcatcgtt cattcgtgaa gcttgagaag acggttcagc tggcgggtgt tgattccaag 5340

tgctactcta cagagcctgt gctgcgctgt gcaaagggat gctctgctac caagacaact 5400

ccagtaactg ttggcttcca ctgcctccca gctgattcag ctaacagcct aactgacaaa 5460

cagatgaagt acgaccagaa gtcagaagac atgcaggata ctgttgatgc acacacaacg 5520

tgttcatgtg agaatgagga atgcagtaca 5550

<210> 106
<211> 1850
<212> PRT
<213> Gallus gallus

<400> 106

Met Arg Gly Ile Ile Leu Ala Leu Val Leu Thr Leu Val Gly Ser Gln
1               5                   10                  15

Lys Phe Asp Ile Asp Pro Gly Phe Asn Ser Arg Arg Ser Tyr Leu Tyr
            20                  25                  30

Asn Tyr Glu Gly Ser Met Leu Asn Gly Leu Gln Asp Arg Ser Leu Gly
        35                  40                  45

Lys Ala Gly Val Arg Leu Ser Ser Lys Leu Glu Ile Ser Gly Leu Pro
    50                  55                  60

Glu Asn Ala Tyr Leu Leu Lys Val Arg Ser Pro Gln Val Glu Glu Tyr
65                  70                  75                  80

Asn Gly Val Trp Pro Arg Asp Pro Phe Thr Arg Ser Ser Lys Ile Thr
                85                  90                  95

Gln Val Ile Ser Ser Cys Phe Thr Arg Leu Phe Lys Phe Glu Tyr Ser
            100                 105                 110

Ser Gly Arg Ile Gly Asn Ile Tyr Ala Pro Glu Asp Cys Pro Asp Leu
        115                 120                 125

Cys Val Asn Ile Val Arg Gly Ile Leu Asn Met Phe Gln Met Thr Ile
    130                 135                 140

Lys Lys Ser Gln Asn Val Tyr Glu Leu Gln Glu Ala Gly Ile Gly Gly
145                 150                 155                 160

Ile Cys His Ala Arg Tyr Val Ile Gln Glu Asp Arg Lys Asn Ser Arg
                165                 170                 175

Ile Tyr Val Thr Arg Thr Val Asp Leu Asn Asn Cys Gln Glu Lys Val
            180                 185                 190

Gln Lys Ser Ile Gly Met Ala Tyr Ile Tyr Pro Cys Pro Val Asp Val
        195                 200                 205

Met Lys Glu Arg Leu Thr Lys Gly Thr Thr Ala Phe Ser Tyr Lys Leu
    210                 215                 220

Lys Gln Ser Asp Ser Gly Thr Leu Ile Thr Asp Val Ser Ser Arg Gln
225                 230                 235                 240

Val Tyr Gln Ile Ser Pro Phe Asn Glu Pro Thr Gly Val Ala Val Met
                245                 250                 255

Glu Ala Arg Gln Gln Leu Thr Leu Val Glu Val Arg Ser Glu Arg Gly
            260                 265                 270

Ser Ala Pro Asp Val Pro Met Gln Asn Tyr Gly Ser Leu Arg Tyr Arg
        275                 280                 285

Phe Pro Ala Val Leu Pro Gln Met Pro Leu Gln Leu Ile Lys Thr Lys
    290                 295                 300

Asn Pro Glu Gln Arg Ile Val Glu Thr Leu Gln His Ile Val Leu Asn
305                 310                 315                 320

Asn Gln Gln Asp Phe His Asp Asp Val Ser Tyr Arg Phe Leu Glu Val
                325                 330                 335

Val Gln Leu Cys Arg Ile Ala Asn Ala Asp Asn Leu Glu Ser Ile Trp
            340                 345                 350

Arg Gln Val Ser Asp Lys Pro Arg Tyr Arg Arg Trp Leu Leu Ser Ala
        355                 360                 365

Val Ser Ala Ser Gly Thr Thr Glu Thr Leu Lys Phe Leu Lys Asn Arg
    370                 375                 380

Ile Arg Asn Asp Asp Leu Asn Tyr Ile Gln Thr Leu Leu Thr Val Ser
385                 390                 395                 400

Leu Thr Leu His Leu Leu Gln Ala Asp Glu His Thr Leu Pro Ile Ala
                405                 410                 415

Ala Asp Leu Met Thr Ser Ser Arg Ile Gln Lys Asn Pro Val Leu Gln
            420                 425                 430

Gln Val Ala Cys Leu Gly Tyr Ser Ser Val Val Asn Arg Tyr Cys Ser
        435                 440                 445

Gln Thr Ser Ala Cys Pro Lys Glu Ala Leu Gln Pro Ile His Asp Leu
    450                 455                 460

Ala Asp Glu Ala Ile Ser Arg Gly Arg Glu Asp Lys Met Lys Leu Ala
465                 470                 475                 480

Leu Lys Cys Ile Gly Asn Met Gly Glu Pro Ala Ser Leu Lys Arg Ile
                485                 490                 495

Leu Lys Phe Leu Pro Ile Ser Ser Ser Ser Ala Ala Asp Ile Pro Val
            500                 505                 510

His Ile Gln Ile Asp Ala Ile Thr Ala Leu Lys Lys Ile Ala Trp Lys
        515                 520                 525

Asp Pro Lys Thr Val Gln Gly Tyr Leu Ile Gln Ile Leu Ala Asp Gln
    530                 535                 540

Ser Leu Pro Pro Glu Val Arg Met Met Ala Cys Ala Val Ile Phe Glu
545                 550                 555                 560

Thr Arg Pro Ala Leu Ala Leu Ile Thr Thr Ile Ala Asn Val Ala Met
                565                 570                 575

Lys Glu Ser Asn Met Gln Val Ala Ser Phe Val Tyr Ser His Met Lys
            580                 585                 590

Ser Leu Ser Lys Ser Arg Leu Pro Phe Met Tyr Asn Ile Ser Ser Ala
        595                 600                 605

Cys Asn Ile Ala Leu Lys Leu Leu Ser Pro Lys Leu Asp Ser Met Ser
    610                 615                 620

Tyr Arg Tyr Ser Lys Val Ile Arg Ala Asp Thr Tyr Phe Asp Asn Tyr
625                 630                 635                 640

Arg Val Gly Ala Thr Gly Glu Ile Phe Val Val Asn Ser Pro Arg Thr
                645                 650                 655

Met Phe Pro Ser Ala Ile Ile Ser Lys Leu Met Ala Asn Ser Ala Gly
            660                 665                 670

Ser Val Ala Asp Leu Val Glu Val Gly Ile Arg Val Glu Gly Leu Ala
        675                 680                 685

Asp Val Ile Met Lys Arg Asn Ile Pro Phe Ala Glu Tyr Pro Thr Tyr
    690                 695                 700

Lys Gln Ile Lys Glu Leu Gly Lys Ala Leu Gln Gly Trp Lys Glu Leu
705                 710                 715                 720

Pro Thr Glu Thr Pro Leu Val Ser Ala Tyr Leu Lys Ile Leu Gly Gln
                725                 730                 735

Glu Val Ala Phe Ile Asn Ile Asn Lys Glu Leu Leu Gln Gln Val Met
            740                 745                 750

Lys Thr Val Val Glu Pro Ala Asp Arg Asn Ala Ala Ile Lys Arg Ile
        755                 760                 765

Ala Asn Gln Ile Arg Asn Ser Ile Ala Gly Gln Trp Thr Gln Pro Val
    770                 775                 780

Trp Met Gly Glu Leu Arg Tyr Val Val Pro Ser Cys Leu Gly Leu Pro
785                 790                 795                 800

Leu Glu Tyr Gly Ser Tyr Thr Thr Ala Leu Ala Arg Ala Ala Val Ser
                805                 810                 815

Val Glu Gly Lys Met Thr Pro Pro Leu Thr Gly Asp Phe Arg Leu Ser
            820                 825                 830

Gln Leu Leu Glu Ser Thr Met Gln Ile Arg Ser Asp Leu Lys Pro Ser
        835                 840                 845

Leu Tyr Val His Thr Val Ala Thr Met Gly Val Asn Thr Glu Tyr Phe
    850                 855                 860

Gln His Ala Val Glu Ile Gln Gly Glu Val Gln Thr Arg Met Pro Met

865                 870                 875                 880

Lys Phe Asp Ala Lys Ile Asp Val Lys Leu Lys Asn Leu Lys Ile Glu
                885                 890                 895

Thr Asn Pro Cys Arg Glu Glu Thr Glu Ile Val Val Gly Arg His Lys
            900                 905                 910

Ala Phe Ala Val Ser Arg Asn Ile Gly Glu Leu Gly Val Glu Lys Arg
        915                 920                 925

Thr Ser Ile Leu Pro Glu Asp Ala Pro Leu Asp Val Thr Glu Glu Pro
    930                 935                 940

Phe Gln Thr Ser Glu Arg Ala Ser Arg Glu His Phe Ala Met Gln Gly
945                 950                 955                 960

Pro Asp Ser Met Pro Arg Lys Gln Ser His Ser Ser Arg Glu Asp Leu
                965                 970                 975

Arg Arg Ser Thr Gly Lys Arg Ala His Lys Arg Asp Ile Cys Leu Lys
            980                 985                 990

Met His His Ile Gly Cys Gln Leu  Cys Phe Ser Arg Arg  Ser Arg Asp
        995                 1000                1005

Ala Ser  Phe Ile Gln Asn Thr  Tyr Leu His Lys Leu  Ile Gly Glu
    1010                1015                1020

His Glu  Ala Lys Ile Val Leu  Met Pro Val His Thr  Asp Ala Asp
    1025                1030                1035

Ile Asp  Lys Ile Gln Leu Glu  Ile Gln Ala Gly Ser  Arg Ala Ala
    1040                1045                1050

Ala Arg  Ile Ile Thr Glu Val  Asn Pro Glu Ser Glu  Glu Glu Asp
    1055                1060                1065

Glu Ser  Ser Pro Tyr Glu Asp  Ile Gln Ala Lys Leu  Lys Arg Ile
    1070                1075                1080

Leu Gly  Ile Asp Ser Met Phe  Lys Val Ala Asn Lys  Thr Arg His
    1085                1090                1095

Pro Lys  Asn Arg Pro Ser Lys  Lys Gly Asn Thr Val  Leu Ala Glu
    1100                1105                1110

Phe Gly Thr Glu Pro Asp Ala Lys Thr Ser Ser Ser Ser Ser Ser
    1115                1120                1125

Ala Ser Ser Thr Ala Thr Ser Ser Ser Ser Ser Ser Ala Ser Ser
    1130                1135                1140

Pro Asn Arg Lys Lys Pro Met Asp Glu Glu Glu Asn Asp Gln Val
    1145                1150                1155

Lys Gln Ala Arg Asn Lys Asp Ala Ser Ser Ser Ser Arg Ser Ser
    1160                1165                1170

Lys Ser Ser Asn Ser Ser Lys Arg Ser Ser Ser Lys Ser Ser Asn
    1175                1180                1185

Ser Ser Lys Arg Ser Ser Ser Ser Ser Ser Ser Ser Ser Ser Ser
    1190                1195                1200

Ser Arg Ser Ser Ser Ser Ser Ser Ser Ser Ser Ser Asn Ser Lys
    1205                1210                1215

Ser Ser Ser Ser Ser Ser Lys Ser Ser Ser Ser Ser Ser Arg Ser
    1220                1225                1230

Arg Ser Ser Ser Lys Ser Ser Ser Ser Ser Ser Ser Ser Ser Ser
    1235                1240                1245

Ser Ser Ser Ser Lys Ser Ser Ser Ser Arg Ser Ser Ser Ser Ser
    1250                1255                1260

Ser Lys Ser Ser Ser His His Ser His Ser His His Ser Gly His
    1265                1270                1275

Leu Asn Gly Ser Ser Ser Ser Ser Ser Ser Ser Arg Ser Val Ser
    1280                1285                1290

His His Ser His Glu His His Ser Gly His Leu Glu Asp Asp Ser
    1295                1300                1305

Ser Ser Ser Ser Ser Ser Ser Val Leu Ser Lys Ile Trp Gly Arg
    1310                1315                1320

His Glu Ile Tyr Gln Tyr Arg Phe Arg Ser Ala His Arg Gln Glu
    1325                1330                1335

Phe Pro Lys Arg Lys Leu Pro Gly Asp Arg Ala Thr Ser Arg Tyr
    1340                1345                1350

Ser Ser Thr Arg Ser Ser His Asp Thr Ser Arg Ala Ala Ser Trp
    1355                1360                1365

Pro Lys Phe Leu Gly Asp Ile Lys Thr Pro Val Leu Ala Ala Phe
    1370                1375                1380

Leu His Gly Ile Ser Asn Asn Lys Lys Thr Gly Gly Leu Gln Leu
    1385                1390                1395

Val Val Tyr Ala Asp Thr Asp Ser Val Arg Pro Arg Val Gln Val
    1400                1405                1410

Phe Val Thr Asn Leu Thr Asp Ser Ser Lys Trp Lys Leu Cys Ala
    1415                1420                1425

Asp Ala Ser Val Arg Asn Ala His Lys Ala Val Ala Tyr Val Lys
    1430                1435                1440

Trp Gly Trp Asp Cys Arg Asp Tyr Lys Val Ser Thr Glu Leu Val
    1445                1450                1455

Thr Gly Arg Phe Ala Gly His Pro Ala Ala Gln Val Lys Leu Glu
    1460                1465                1470

Trp Pro Lys Val Pro Ser Asn Val Arg Ser Val Val Glu Trp Phe
    1475                1480                1485

Tyr Glu Phe Val Pro Gly Ala Ala Phe Met Leu Gly Phe Ser Glu
    1490                1495                1500

Arg Met Asp Lys Asn Pro Ser Arg Gln Ala Arg Met Val Val Ala
    1505                1510                1515

Leu Thr Ser Pro Arg Thr Cys Asp Val Val Val Lys Leu Pro Asp
    1520                1525                1530

Ile Ile Leu Tyr Gln Lys Ala Val Arg Leu Pro Leu Ser Leu Pro
    1535                1540                1545

Val Gly Pro Arg Ile Pro Ala Ser Glu Leu Gln Pro Pro Ile Trp
    1550                1555                1560

Asn Val Phe Ala Glu Ala Pro Ser Ala Val Leu Glu Asn Leu Lys
    1565                1570                1575

Ala Arg Cys Ser Val Ser Tyr Asn Lys Ile Lys Thr Phe Asn Glu
    1580                1585                1590

Val Lys Phe Asn Tyr Ser Met Pro Ala Asn Cys Tyr His Ile Leu
    1595                1600                1605

Val Gln Asp Cys Ser Ser Glu Leu Lys Phe Leu Val Met Met Lys
    1610                1615                1620

Ser Ala Gly Glu Ala Thr Asn Leu Lys Ala Ile Asn Ile Lys Ile
    1625                1630                1635

Gly Ser His Glu Ile Asp Met His Pro Val Asn Gly Gln Val Lys
    1640                1645                1650

Leu Leu Val Asp Gly Ala Glu Ser Pro Thr Ala Asn Ile Ser Leu
    1655                1660                1665

Ile Ser Ala Gly Ala Ser Leu Trp Ile His Asn Glu Asn Gln Gly
    1670                1675                1680

Phe Ala Leu Ala Ala Pro Gly His Gly Ile Asp Lys Leu Tyr Phe
    1685                1690                1695

Asp Gly Lys Thr Ile Thr Ile Gln Val Pro Leu Trp Met Ala Gly
    1700                1705                1710

Lys Thr Cys Gly Ile Cys Gly Lys Tyr Asp Ala Glu Cys Glu Gln
    1715                1720                1725

Glu Tyr Arg Met Pro Asn Gly Tyr Leu Ala Lys Asn Ala Val Ser
    1730                1735                1740

Phe Gly His Ser Trp Ile Leu Glu Glu Ala Pro Cys Arg Gly Ala
    1745                1750                1755

Cys Lys Leu His Arg Ser Phe Val Lys Leu Glu Lys Thr Val Gln
    1760                1765                1770

Leu Ala Gly Val Asp Ser Lys Cys Tyr Ser Thr Glu Pro Val Leu
    1775                1780                1785

Arg Cys Ala Lys Gly Cys Ser Ala Thr Lys Thr Thr Pro Val Thr
    1790                1795                1800

Val Gly Phe His Cys Leu Pro Ala Asp Ser Ala Asn Ser Leu Thr
    1805                1810                1815

Asp Lys Gln Met Lys Tyr Asp Gln Lys Ser Glu Asp Met Gln Asp

1820                    1825                    1830

Thr Val  Asp Ala His Thr Thr  Cys Ser Cys Glu Asn  Glu Glu Cys
    1835                    1840                    1845

Ser Thr
    1850

<210>  107
<211>  9
<212>  PRT
<213>  Gallus gallus

<400>  107

Ala Asp Thr Tyr Phe Asp Asn Tyr Arg
1               5

<210>  108
<211>  10
<212>  PRT
<213>  Gallus gallus

<400>  108

Ala Glu Phe Gly Thr Glu Pro Asp Ala Lys
1               5                   10

<210>  109
<211>  7
<212>  PRT
<213>  Gallus gallus

<400>  109

Ala Glu Tyr Pro Thr Tyr Lys
1               5

<210>  110
<211>  12
<212>  PRT
<213>  Gallus gallus

<400>  110

Ala Thr Gly Glu Ile Phe Val Val Asn Ser Pro Arg
1               5                   10

<210>  111
<211>  11
<212>  PRT
<213>  Gallus gallus

<400>  111

Ala Val Glu Ile Gln Gly Glu Val Gln Thr Arg

1               5                   10


<210>  112
<211>  7
<212>  PRT
<213>  Gallus gallus

<400>  112

Ala Val Ile Phe Glu Thr Arg
1               5


<210>  113
<211>  12
<212>  PRT
<213>  Gallus gallus

<400>  113

Ala Val Ser Ala Ser Gly Thr Thr Glu Thr Leu Lys
1               5                   10


<210>  114
<211>  16
<212>  PRT
<213>  Gallus gallus

<400>  114

Glu Ala Leu Gln Pro Ile His Asp Leu Ala Asp Glu Ala Ile Ser Arg
1               5                   10                  15


<210>  115
<211>  9
<212>  PRT
<213>  Gallus gallus

<400>  115

Glu Glu Thr Glu Ile Val Val Gly Arg
1               5


<210>  116
<211>  14
<212>  PRT
<213>  Gallus gallus

<400>  116

Glu Leu Pro Thr Glu Thr Pro Leu Val Ser Ala Tyr Leu Lys
1               5                   10


<210>  117
<211>  10
<212>  PRT
<213>  Gallus gallus

<400>  117

Phe Asp Ile Asp Pro Gly Phe Asn Ser Arg
1               5                   10


<210>  118
<211>  7
<212>  PRT
<213>  Gallus gallus

<400>  118

Phe Glu Tyr Ser Ser Gly Arg
1               5


<210>  119
<211>  8
<212>  PRT
<213>  Gallus gallus

<400>  119

Gly Thr Thr Ala Phe Ser Tyr Lys
1               5


<210>  120
<211>  10
<212>  PRT
<213>  Gallus gallus

<400>  120

Ile Ala Asn Ala Asp Asn Leu Glu Ser Ile
1               5                   10


<210>  121
<211>  12
<212>  PRT
<213>  Gallus gallus

<400>  121

Ile Leu Ala Asp Gln Ser Leu Pro Pro Glu Val Arg
1               5                   10


<210>  122
<211>  13
<212>  PRT
<213>  Gallus gallus

<400>  122

Ile Leu Gly Gln Glu Val Ala Phe Ile Asn Ile Asn Lys
1               5                   10


<210>  123
<211>  10

<212>  PRT
<213>  Gallus gallus

<400>  123

Ile Gln Leu Glu Ile Gln Ala Gly Ser Arg
1               5                   10


<210>  124
<211>  14
<212>  PRT
<213>  Gallus gallus

<400>  124

Leu Glu Ile Ser Gly Leu Pro Glu Asn Ala Tyr Leu Leu Lys
1               5                   10


<210>  125
<211>  9
<212>  PRT
<213>  Gallus gallus

<400>  125

Leu Gly Tyr Ser Ser Val Val Asn Arg
1               5


<210>  126
<211>  15
<212>  PRT
<213>  Gallus gallus

<400>  126

Leu Ile Gln Ile Leu Ala Asp Gln Ser Leu Pro Pro Glu Val Arg
1               5                   10                  15


<210>  127
<211>  16
<212>  PRT
<213>  Gallus gallus

<400>  127

Leu Lys Gln Ser Asp Ser Gly Thr Leu Ile Thr Asp Val Ser Ser Arg
1               5                   10                  15


<210>  128
<211>  15
<212>  PRT
<213>  Gallus gallus

<400>  128

Leu Leu Ser Ala Val Ser Ala Ser Gly Thr Thr Glu Thr Leu Lys
1               5                   10                  15

<210>  129
<211>  10
<212>  PRT
<213>  Gallus gallus

<400>  129

Leu Pro Leu Ser Leu Pro Val Gly Pro Arg
1               5                   10


<210>  130
<211>  14
<212>  PRT
<213>  Gallus gallus

<400>  130

Leu Ser Ala Val Ser Ala Ser Gly Thr Thr Glu Thr Leu Lys
1               5                   10


<210>  131
<211>  11
<212>  PRT
<213>  Gallus gallus

<400>  131

Asn Asp Asp Leu Asn Tyr Ile Gln Thr Leu Leu
1               5                   10


<210>  132
<211>  9
<212>  PRT
<213>  Gallus gallus

<400>  132

Asn Ile Gly Glu Leu Gly Val Glu Lys
1               5


<210>  133
<211>  11
<212>  PRT
<213>  Gallus gallus

<400>  133

Asn Ile Pro Phe Ala Glu Tyr Pro Thr Tyr Lys
1               5                   10


<210>  134
<211>  11
<212>  PRT
<213>  Gallus gallus

<400>  134

Pro Ala Leu Ala Leu Ile Thr Thr Ile Ala Asn
1               5                   10

<210>  135
<211>  9
<212>  PRT
<213>  Gallus gallus

<400>  135

Gln Gln Leu Thr Leu Val Glu Val Arg
1               5

<210>  136
<211>  14
<212>  PRT
<213>  Gallus gallus

<400>  136

Gln Ser Asp Ser Gly Thr Leu Ile Thr Asp Val Ser Ser Arg
1               5                   10

<210>  137
<211>  14
<212>  PRT
<213>  Gallus gallus

<400>  137

Ser Ala Gly Ser Val Ala Asp Leu Val Glu Val Gly Ile Arg
1               5                   10

<210>  138
<211>  13
<212>  PRT
<213>  Gallus gallus

<400>  138

Ser Ala Val Ser Ala Ser Gly Thr Thr Glu Thr Leu Lys
1               5                   10

<210>  139
<211>  8
<212>  PRT
<213>  Gallus gallus

<400>  139

Ser Asp Leu Lys Pro Ser Leu Tyr
1               5

<210>  140
<211>  18
<212>  PRT
<213>  Gallus gallus

<400>  140

Thr Gly Gly Leu Gln Leu Val Val Tyr Ala Asp Thr Asp Ser Val Arg
1               5                   10                  15

Pro Arg

<210>  141
<211>  15
<212>  PRT
<213>  Gallus gallus

<400>  141

Thr Pro Val Leu Ala Ala Phe Leu His Gly Ile Ser Asn Asn Lys
1               5                   10                  15

<210>  142
<211>  20
<212>  PRT
<213>  Gallus gallus

<400>  142

Thr Val Gln Gly Tyr Leu Ile Gln Ile Leu Ala Asp Gln Ser Leu Pro
1               5                   10                  15

Pro Glu Val Arg
            20

<210>  143
<211>  10
<212>  PRT
<213>  Gallus gallus

<400>  143

Thr Val Gln Leu Ala Gly Val Asp Ser Lys
1               5                   10

<210>  144
<211>  8
<212>  PRT
<213>  Gallus gallus

<400>  144

Thr Val Val Glu Pro Ala Asp Arg
1               5

<210>  145
<211>  10
<212>  PRT
<213>  Gallus gallus

<400>  145

Val Glu Ile Gln Gly Glu Val Gln Thr Arg
1               5                   10


<210>  146
<211>  14
<212>  PRT
<213>  Gallus gallus

<400>  146

Val Gly Ala Thr Gly Glu Ile Phe Val Val Asn Ser Pro Arg
1               5                   10


<210>  147
<211>  11
<212>  PRT
<213>  Gallus gallus

<400>  147

Val Ser Ala Ser Gly Thr Thr Glu Thr Leu Lys
1               5                   10


<210>  148
<211>  9
<212>  PRT
<213>  Gallus gallus

<400>  148

Val Ser Thr Glu Leu Val Thr Gly Arg
1               5


<210>  149
<211>  16
<212>  PRT
<213>  Gallus gallus

<400>  149

Trp Leu Leu Ser Ala Val Ser Ala Ser Gly Thr Thr Glu Thr Leu Lys
1               5                   10                  15


<210>  150
<211>  8
<212>  PRT
<213>  Gallus gallus

<400>  150

Tyr Val Ile Gln Glu Asp Arg Lys
1               5


<210>  151

<211>  7875
<212>  PRT
<213>  Gallus gallus

<400>  151

Ala Thr Gly Gly Gly Cys Cys Cys Thr Gly Thr Gly Cys Gly Gly Cys
1               5                   10                  15

Thr Cys Thr Thr Gly Cys Thr Gly Cys Thr Cys Thr Thr Gly Cys Thr
            20                  25                  30

Gly Cys Thr Gly Cys Thr Cys Ala Gly Cys Ala Gly Thr Gly Gly Thr
        35                  40                  45

Gly Thr Thr Cys Thr Cys Ala Cys Ala Cys Ala Gly Gly Ala Ala Gly
    50                  55                  60

Gly Ala Ala Cys Ala Cys Cys Thr Gly Ala Ala Ala Ala Thr Gly Gly
65                  70                  75                  80

Ala Ala Ala Cys Cys Cys Ala Gly Gly Ala Thr Gly Thr Thr Cys Ala
                85                  90                  95

Ala Ala Ala Gly Ala Thr Gly Cys Gly Gly Cys Cys Cys Gly Ala Thr
            100                 105                 110

Thr Thr Ala Ala Ala Ala Gly Cys Cys Thr Thr Ala Gly Ala Ala Ala
        115                 120                 125

Ala Thr Ala Thr Gly Thr Thr Thr Ala Cys Thr Thr Ala Thr Ala Thr
    130                 135                 140

Gly Ala Ala Gly Cys Ala Gly Ala Ala Ala Cys Ala Thr Cys Ala Ala
145                 150                 155                 160

Gly Thr Gly Gly Gly Ala Thr Cys Ala Cys Gly Gly Gly Ala Ala Cys
                165                 170                 175

Ala Gly Cys Ala Gly Ala Thr Thr Cys Thr Cys Gly Ala Ala Gly Cys
            180                 185                 190

Gly Gly Thr Thr Cys Ala Ala Ala Gly Ala Thr Cys Ala Cys Cys Thr
        195                 200                 205

Gly Thr Ala Ala Ala Gly Thr Thr Gly Ala Gly Thr Thr Gly Gly Ala
    210                 215                 220

Gly Gly Thr Ala Cys Cys Ala Cys Ala Gly Cys Thr Gly Thr Gly Cys

225                 230                 235                 240

Cys Ala Gly Thr Thr Cys Ala Thr Cys Cys Thr Gly Ala Gly Gly Ala
                245                 250                 255

Cys Ala Ala Thr Gly Cys Ala Thr Thr Gly Cys Thr Cys Cys Cys Thr
            260                 265                 270

Ala Ala Gly Gly Gly Ala Gly Ala Cys Ala Thr Thr Thr Gly Gly Thr
        275                 280                 285

Gly Thr Thr Gly Ala Cys Ala Gly Thr Gly Ala Gly Ala Gly Ala Ala
    290                 295                 300

Gly Ala Gly Cys Cys Ala Thr Gly Cys Thr Gly Ala Gly Gly Ala Ala
305                 310                 315                 320

Gly Thr Cys Ala Ala Ala Gly Ala Ala Cys Thr Cys Thr Gly Ala Thr
                325                 330                 335

Gly Ala Cys Thr Thr Thr Gly Cys Ala Ala Ala Thr Gly Cys Cys Ala
            340                 345                 350

Thr Gly Thr Cys Cys Ala Ala Ala Cys Ala Thr Gly Ala Gly Cys Thr
        355                 360                 365

Ala Ala Gly Ala Thr Thr Cys Ala Gly Cys Ala Cys Thr Cys Ala Gly
    370                 375                 380

Gly Ala Thr Gly Gly Ala Ala Cys Ala Ala Ala Ala Gly Thr Thr Ala
385                 390                 395                 400

Ala Ala Cys Thr Ala Thr Ala Thr Cys Cys Ala Gly Ala Gly Ala Ala
                405                 410                 415

Gly Gly Ala Thr Gly Ala Ala Cys Cys Thr Cys Thr Gly Ala Ala Thr
            420                 425                 430

Gly Thr Cys Cys Thr Cys Ala Ala Thr Cys Thr Cys Ala Ala Gly Ala
        435                 440                 445

Gly Ala Gly Gly Ala Ala Thr Thr Ala Thr Cys Thr Cys Ala Gly Cys
    450                 455                 460

Thr Cys Thr Cys Cys Thr Thr Gly Cys Ala Cys Cys Ala Ala Cys Ala
465                 470                 475                 480

Gly Ala Ala Ala Cys Ala Gly Ala Gly Gly Ala Ala Ala Ala Cys Ala
                485                 490                 495

Thr Ala Ala Ala Ala Ala Cys Ala Ala Thr Thr Thr Cys Cys Ala Thr
            500                 505                 510

Gly Gly Ala Thr Ala Cys Thr Gly Thr Ala Thr Ala Thr Gly Gly Ala
        515                 520                 525

Ala Ala Gly Thr Gly Thr Gly Ala Cys Ala Gly Thr Gly Ala Gly Gly
    530                 535                 540

Thr Thr Gly Ala Gly Thr Thr Cys Ala Ala Ala Thr Cys Cys Ala Gly
545                 550                 555                 560

Ala Ala Gly Ala Gly Gly Ala Ala Gly Thr Gly Thr Thr Gly Cys Ala
                565                 570                 575

Gly Ala Ala Gly Ala Thr Ala Thr Thr Thr Cys Ala Ala Thr Thr Ala
            580                 585                 590

Ala Cys Ala Gly Gly Ala Ala Cys Cys Thr Gly Ala Ala Ala Gly Cys
        595                 600                 605

Cys Thr Gly Thr Gly Ala Cys Ala Ala Cys Thr Thr Cys Ala Gly Thr
    610                 615                 620

Cys Cys Ala Ala Thr Cys Ala Gly Ala Gly Ala Thr Thr Ala Thr Gly
625                 630                 635                 640

Thr Cys Ala Gly Cys Cys Cys Thr Gly Thr Thr Gly Cys Cys Ala Thr
                645                 650                 655

Thr Gly Thr Ala Ala Ala Ala Gly Gly Ala Cys Thr Ala Ala Ala Cys
            660                 665                 670

Ala Thr Cys Cys Cys Ala Cys Thr Ala Thr Cys Thr Ala Cys Cys Cys
        675                 680                 685

Thr Thr Cys Thr Gly Ala Gly Thr Ala Gly Cys Ala Cys Thr Cys Ala
    690                 695                 700

Ala Thr Cys Cys Thr Gly Thr Cys Ala Cys Thr Ala Cys Ala Gly Thr
705                 710                 715                 720

Ala Thr Thr Gly Ala Thr Gly Cys Ala Ala Ala Gly Ala Ala Ala Ala
                725                 730                 735

Ala Gly Cys Ala Thr Ala Thr Cys Ala Gly Ala Gly Ala Thr Gly Thr
        740                 745                 750

Thr Gly Thr Cys Thr Gly Cys Ala Gly Thr Gly Ala Ala Ala Ala Ala
    755                 760                 765

Cys Ala Cys Cys Thr Gly Thr Thr Thr Cys Thr Gly Cys Cys Thr Thr
    770                 775                 780

Cys Cys Thr Cys Ala Thr Ala Cys Ala Ala Ala Ala Ala Thr Cys Ala
785                 790                 795                 800

Gly Thr Ala Thr Gly Gly Thr Ala Thr Gly Ala Thr Gly Ala Cys Ala
                805                 810                 815

Gly Ala Ala Gly Thr Cys Ala Ala Cys Cys Ala Gly Ala Cys Ala Cys
            820                 825                 830

Thr Gly Ala Ala Gly Cys Thr Thr Gly Ala Ala Gly Ala Thr Ala Ala
        835                 840                 845

Thr Cys Ala Gly Ala Gly Gly Ala Thr Gly Ala Ala Thr Ala Ala Cys
    850                 855                 860

Ala Gly Ala Ala Ala Thr Cys Cys Thr Gly Ala Thr Gly Gly Ala Gly
865                 870                 875                 880

Ala Thr Gly Ala Ala Cys Thr Gly Gly Ala Ala Gly Ala Gly Ala Ala
                885                 890                 895

Ala Gly Gly Ala Cys Thr Thr Gly Cys Ala Cys Thr Gly Gly Ala Ala
            900                 905                 910

Ala Gly Cys Ala Cys Ala Gly Ala Thr Gly Cys Thr Ala Ala Ala Thr
        915                 920                 925

Thr Thr Thr Cys Cys Ala Gly Gly Cys Ala Gly Gly Gly Thr Gly Ala
    930                 935                 940

Thr Gly Cys Thr Gly Thr Thr Thr Thr Gly Ala Ala Ala Ala Thr Thr
945                 950                 955                 960

Cys Thr Thr Cys Ala Gly Gly Ala Gly Cys Thr Gly Cys Ala Gly Ala
                965                 970                 975

Ala Ala Cys Thr Thr Ala Cys Thr Gly Cys Cys Thr Cys Cys Cys Ala
            980                 985                 990

Gly Cys Ala Gly Ala Ala Cys Cys  Ala Gly Cys Ala Gly  Ala Gly Ala
        995                 1000                1005

Gly Cys  Ala Ala Ala Ala Cys  Thr Cys Thr Thr Thr  Thr Ala Cys
    1010                1015                1020

Ala Ala  Ala Thr Thr Thr Gly  Thr Thr Thr Cys Thr  Gly Gly Ala
    1025                1030                1035

Cys Thr  Thr Ala Gly Ala Ala  Gly Thr Thr Thr Gly  Cys Ala Cys
    1040                1045                1050

Ala Ala  Cys Ala Gly Cys Ala  Cys Thr Cys Thr Thr  Gly Gly Cys
    1055                1060                1065

Thr Cys  Thr Cys Thr Thr Gly  Thr Ala Cys Cys Ala  Ala Ala Gly
    1070                1075                1080

Ala Thr  Gly Ala Thr Gly Gly  Ala Ala Ala Cys Thr  Thr Cys Ala
    1085                1090                1095

Ala Gly  Thr Thr Cys Cys Ala  Thr Cys Ala Cys Gly  Ala Thr Thr
    1100                1105                1110

Cys Ala  Gly Gly Cys Cys Cys  Thr Gly Ala Thr Thr  Cys Ala Gly
    1115                1120                1125

Thr Gly  Thr Gly Gly Gly Ala  Cys Thr Cys Cys Ala  Gly Ala Gly
    1130                1135                1140

Thr Gly  Thr Thr Ala Cys Ala  Gly Thr Gly Cys Ala  Gly Thr Cys
    1145                1150                1155

Cys Thr  Thr Cys Ala Gly Ala  Thr Ala Cys Thr Gly  Ala Gly Ala
    1160                1165                1170

Ala Cys  Thr Gly Gly Ala Ala  Ala Cys Gly Thr Ala  Ala Ala Thr
    1175                1180                1185

Cys Cys  Ala Cys Thr Thr Gly  Thr Gly Gly Thr Ala  Gly Ala Cys
    1190                1195                1200

Cys Thr  Gly Gly Thr Cys Ala  Cys Gly Thr Ala Thr  Ala Cys Cys
    1205                1210                1215

Cys Thr  Gly Gly Gly Ala Cys  Thr Cys Thr Thr Gly  Cys Cys Thr

1220                    1225                    1230

Thr Cys  Thr Cys Cys Thr Ala  Cys Thr Cys Cys Ala  Ala Ala Gly
    1235                    1240                    1245

Ala Gly  Ala Ala Thr Cys Cys  Gly Gly Gly Ala Ala  Ala Thr Thr
    1250                    1255                    1260

Cys Thr  Thr Ala Ala Cys Ala  Thr Gly Gly Cys Cys  Cys Ala Gly
    1265                    1270                    1275

Thr Ala  Thr Cys Ala Gly Cys  Cys Ala Ala Gly Cys  Ala Gly Ala
    1280                    1285                    1290

Gly Cys  Thr Thr Cys Cys Thr  Thr Thr Thr Ala Thr  Gly Gly Cys
    1295                    1300                    1305

Thr Thr  Gly Ala Gly Thr Cys  Ala Thr Gly Cys Thr  Gly Thr Thr
    1310                    1315                    1320

Ala Cys  Cys Ala Ala Gly Thr  Thr Cys Thr Ala Thr  Ala Ala Thr
    1325                    1330                    1335

Gly Ala  Gly Ala Ala Gly Ala  Thr Gly Ala Thr Thr  Gly Thr Ala
    1340                    1345                    1350

Ala Cys  Ala Gly Ala Gly Gly  Ala Ala Ala Thr Ala  Ala Cys Ala
    1355                    1360                    1365

Gly Ala  Thr Gly Thr Thr Gly  Cys Thr Gly Ala Cys  Thr Thr Cys
    1370                    1375                    1380

Ala Thr  Gly Gly Thr Ala Thr  Cys Ala Cys Thr Gly  Cys Thr Cys
    1385                    1390                    1395

Gly Gly  Cys Ala Cys Thr Gly  Ala Cys Thr Gly Thr  Thr Cys Thr
    1400                    1405                    1410

Gly Gly  Ala Gly Ala Thr Gly  Cys Thr Gly Ala Ala  Cys Thr Thr
    1415                    1420                    1425

Ala Cys  Ala Thr Ala Thr Cys  Thr Cys Ala Cys Ala  Cys Thr Thr
    1430                    1435                    1440

Cys Gly  Gly Gly Cys Thr Ala  Thr Thr Gly Gly Ala  Ala Ala Cys
    1445                    1450                    1455

Ala Thr Gly Gly Gly Thr Gly Cys Ala Gly Thr Ala Ala Thr Gly
    1460                1465                1470

Gly Ala Gly Ala Ala Ala Gly Cys Thr Ala Ala Ala Cys Cys Cys
    1475                1480                1485

Ala Gly Cys Cys Thr Gly Ala Ala Ala Gly Cys Thr Thr Cys Thr
    1490                1495                1500

Cys Thr Thr Ala Ala Ala Ala Cys Ala Thr Gly Thr Ala Thr Cys
    1505                1510                1515

Ala Gly Ala Ala Ala Thr Cys Ala Ala Gly Cys Thr Gly Cys Ala
    1520                1525                1530

Thr Cys Ala Cys Thr Thr Thr Cys Ala Gly Thr Thr Cys Ala Ala
    1535                1540                1545

Ala Ala Ala Gly Cys Ala Gly Cys Cys Ala Thr Ala Cys Ala Gly
    1550                1555                1560

Gly Cys Ala Thr Thr Cys Cys Gly Gly Ala Ala Ala Ala Thr Gly
    1565                1570                1575

Ala Cys Cys Ala Thr Thr Ala Cys Ala Gly Ala Ala Gly Ala Gly
    1580                1585                1590

Gly Ala Thr Cys Gly Thr Thr Cys Ala Gly Cys Ala Cys Thr Thr
    1595                1600                1605

Cys Thr Gly Ala Ala Ala Gly Ala Ala Thr Thr Cys Cys Ala Gly
    1610                1615                1620

Gly Ala Ala Gly Gly Gly Gly Ala Thr Gly Cys Ala Cys Cys Thr
    1625                1630                1635

Ala Cys Ala Gly Ala Thr Ala Ala Ala Cys Gly Thr Cys Thr Ala
    1640                1645                1650

Gly Cys Ala Ala Cys Cys Thr Ala Thr Cys Thr Cys Ala Thr Ala
    1655                1660                1665

Cys Thr Gly Ala Thr Gly Ala Ala Gly Ala Ala Thr Cys Cys Thr
    1670                1675                1680

Thr Cys Cys Cys Cys Ala Gly Cys Thr Gly Ala Thr Cys Thr Cys
    1685                1690                1695

Gly Cys Ala Ala Ala Gly Ala Thr Thr Ala Thr Gly Ala Gly Ala
    1700                1705                1710

Ala Thr Cys Cys Thr Cys Ala Cys Ala Ala Gly Gly Gly Ala Gly
    1715                1720                1725

Ala Ala Gly Ala Ala Cys Gly Ala Gly Cys Ala Ala Gly Thr Gly
    1730                1735                1740

Ala Ala Ala Ala Gly Cys Thr Thr Thr Gly Thr Cys Gly Cys Thr
    1745                1750                1755

Thr Cys Ala Cys Ala Cys Ala Thr Thr Gly Cys Cys Ala Ala Cys
    1760                1765                1770

Ala Thr Cys Cys Thr Ala Gly Ala Cys Thr Cys Thr Gly Ala Thr
    1775                1780                1785

Gly Ala Ala Gly Thr Ala Gly Gly Cys Ala Thr Thr Gly Ala Ala
    1790                1795                1800

Gly Ala Thr Cys Thr Ala Ala Ala Ala Ala Gly Cys Cys Ala Thr
    1805                1810                1815

Gly Thr Thr Gly Ala Ala Gly Ala Ala Gly Cys Ala Cys Thr Gly
    1820                1825                1830

Ala Ala Ala Gly Gly Ala Ala Ala Thr Gly Ala Gly Gly Thr Thr
    1835                1840                1845

Cys Cys Ala Ala Cys Ala Gly Cys Cys Ala Ala Ala Gly Ala Thr
    1850                1855                1860

Thr Thr Cys Ala Gly Ala Ala Ala Ala Thr Thr Cys Thr Cys Ala
    1865                1870                1875

Cys Ala Ala Ala Ala Thr Thr Ala Thr Cys Ala Ala Gly Thr Thr
    1880                1885                1890

Thr Cys Cys Ala Ala Ala Ala Gly Ala Gly Thr Thr Thr Cys Thr
    1895                1900                1905

Gly Thr Ala Cys Cys Thr Gly Gly Cys Cys Thr Cys Ala Ala Thr
    1910                1915                1920

Cys Cys Cys Ala Thr Cys Thr Cys Thr Gly Cys Cys Ala Ala Ala
    1925                1930                1935

Gly Thr Ala Gly Ala Ala Gly Gly Ala Ala Ala Thr Gly Thr Gly
    1940                1945                1950

Ala Thr Ala Thr Thr Thr Gly Ala Thr Cys Cys Ala Ala Gly Cys
    1955                1960                1965

Ala Gly Cys Thr Ala Thr Gly Thr Thr Cys Cys Thr Ala Ala Ala
    1970                1975                1980

Gly Ala Ala Ala Cys Thr Ala Thr Gly Cys Thr Gly Ala Ala Ala
    1985                1990                1995

Ala Cys Cys Ala Cys Cys Cys Thr Gly Ala Ala Cys Thr Thr Gly
    2000                2005                2010

Thr Ala Thr Gly Gly Thr Thr Thr Thr Gly Gly Cys Cys Cys Ala
    2015                2020                2025

Ala Gly Thr Gly Ala Thr Ala Thr Cys Thr Thr Thr Gly Ala Gly
    2030                2035                2040

Cys Thr Thr Gly Gly Cys Thr Thr Gly Gly Ala Thr Gly Gly Ala
    2045                2050                2055

Ala Ala Gly Gly Gly Ala Thr Thr Thr Gly Ala Ala Cys Cys Ala
    2060                2065                2070

Ala Cys Ala Cys Thr Gly Gly Ala Ala Gly Cys Thr Thr Thr Gly
    2075                2080                2085

Thr Thr Thr Gly Gly Ala Gly Ala Gly Ala Ala Gly Gly Gly Ala
    2090                2095                2100

Thr Thr Thr Thr Thr Cys Cys Cys Ala Gly Ala Thr Ala Cys Thr
    2105                2110                2115

Gly Cys Ala Ala Gly Cys Ala Ala Gly Gly Cys Cys Cys Thr Gly
    2120                2125                2130

Thr Ala Thr Thr Gly Gly Gly Thr Thr Gly Ala Thr Gly Gly Cys
    2135                2140                2145

Ala Ala Ala Gly Thr Gly Cys Cys Ala Gly Ala Gly Cys Ala Gly
    2150                2155                2160

Gly Thr Thr Thr Cys Cys Ala Ala Gly Gly Cys Thr Cys Thr Cys

2165                2170                2175

Thr Thr Thr Gly Ala Cys Thr Ala Cys Thr Thr Thr Gly Gly Thr
    2180                2185                2190

Thr Ala Cys Thr Cys Cys Cys Ala Cys Gly Ala Thr Gly Gly Cys
    2195                2200                2205

Ala Ala Gly Cys Ala Gly Gly Ala Thr Cys Ala Gly Gly Ala Gly
    2210                2215                2220

Ala Thr Cys Ala Cys Thr Ala Ala Ala Gly Cys Ala Gly Thr Ala
    2225                2230                2235

Ala Thr Thr Cys Thr Thr Ala Ala Cys Cys Thr Thr Gly Ala Ala
    2240                2245                2250

Ala Ala Ala Cys Thr Gly Ala Thr Ala Ala Ala Ala Gly Ala Ala
    2255                2260                2265

Thr Thr Gly Ala Gly Cys Ala Ala Gly Ala Ala Gly Gly Ala Ala
    2270                2275                2280

Gly Cys Thr Cys Cys Thr Gly Ala Ala Gly Gly Ala Ala Gly Gly
    2285                2290                2295

Gly Cys Ala Thr Thr Thr Cys Thr Gly Cys Gly Ala Ala Thr Cys
    2300                2305                2310

Cys Thr Gly Gly Gly Ala Gly Ala Ala Gly Ala Gly Cys Thr Thr
    2315                2320                2325

Gly Gly Gly Thr Ala Cys Ala Thr Gly Ala Ala Ala Cys Thr Cys
    2330                2335                2340

Ala Gly Thr Gly Ala Thr Thr Thr Cys Ala Ala Ala Thr Thr Gly
    2345                2350                2355

Cys Thr Gly Gly Gly Ala Ala Gly Cys Gly Thr Gly Gly Cys Thr
    2360                2365                2370

Cys Thr Ala Gly Ala Gly Thr Gly Cys Ala Thr Thr Ala Ala Ala
    2375                2380                2385

Ala Cys Thr Cys Thr Thr Cys Ala Gly Ala Gly Ala Ala Thr Thr
    2390                2395                2400

Cys Cys Thr Gly Ala Ala Ala Thr Ala Ala Thr Thr Gly Cys Ala
    2405                2410                2415

Cys Ala Ala Gly Cys Cys Ala Thr Cys Thr Cys Cys Ala Ala Ala
    2420                2425                2430

Gly Gly Thr Gly Thr Thr Gly Ala Cys Ala Ala Gly Gly Ala Cys
    2435                2440                2445

Thr Thr Ala Thr Thr Thr Gly Thr Cys Cys Ala Cys Thr Ala Thr
    2450                2455                2460

Ala Thr Gly Thr Thr Thr Ala Thr Gly Gly Ala Cys Ala Ala Thr
    2465                2470                2475

Gly Ala Gly Thr Thr Thr Gly Ala Ala Cys Thr Cys Cys Cys Ala
    2480                2485                2490

Ala Cys Thr Gly Gly Ala Gly Cys Ala Gly Gly Thr Thr Thr Gly
    2495                2500                2505

Cys Ala Ala Cys Thr Gly Ala Ala Gly Thr Thr Thr Gly Cys Cys
    2510                2515                2520

Thr Thr Gly Thr Cys Thr Gly Gly Ala Ala Thr Ala Gly Thr Gly
    2525                2530                2535

Ala Cys Ala Cys Cys Thr Gly Gly Gly Gly Cys Cys Ala Ala Ala
    2540                2545                2550

Gly Thr Ala Gly Cys Thr Gly Thr Gly Ala Ala Ala Cys Thr Thr
    2555                2560                2565

Cys Ala Thr Cys Ala Ala Ala Ala Ala Ala Gly Thr Ala Thr Gly
    2570                2575                2580

Cys Ala Ala Gly Cys Ala Gly Ala Ala Cys Thr Cys Ala Thr Thr
    2585                2590                2595

Gly Cys Thr Ala Ala Ala Cys Cys Thr Thr Cys Ala Gly Thr Ala
    2600                2605                2610

Gly Cys Ala Gly Thr Thr Gly Ala Gly Thr Thr Thr Gly Thr Ala
    2615                2620                2625

Ala Cys Ala Cys Ala Thr Cys Thr Ala Gly Gly Ala Ala Thr Ala
    2630                2635                2640

Ala Ala Cys Ala Thr Gly Cys Cys Thr Gly Ala Ala Thr Thr Thr
    2645                2650                2655

Gly Cys Thr Ala Gly Ala Ala Gly Thr Gly Gly Thr Gly Thr Gly
    2660                2665                2670

Gly Ala Gly Ala Thr Gly Ala Ala Thr Thr Cys Cys Ala Ala Thr
    2675                2680                2685

Ala Thr Ala Thr Thr Cys Cys Ala Thr Gly Ala Gly Thr Cys Thr
    2690                2695                2700

Gly Gly Ala Ala Thr Thr Gly Ala Ala Gly Cys Ala Cys Ala Thr
    2705                2710                2715

Gly Thr Thr Ala Gly Thr Gly Thr Gly Ala Ala Ala Gly Cys Thr
    2720                2725                2730

Gly Gly Ala Cys Ala Gly Cys Thr Gly Ala Ala Ala Thr Thr Cys
    2735                2740                2745

Ala Gly Cys Ala Thr Thr Cys Cys Thr Gly Cys Cys Cys Cys Gly
    2750                2755                2760

Ala Ala Gly Ala Cys Cys Cys Cys Ala Ala Cys Ala Ala Ala Ala
    2765                2770                2775

Cys Thr Cys Cys Thr Cys Ala Gly Thr Ala Thr Cys Ala Gly Cys
    2780                2785                2790

Ala Ala Cys Ala Cys Ala Cys Thr Thr Cys Ala Thr Thr Thr Gly
    2795                2800                2805

Gly Thr Gly Thr Cys Thr Cys Cys Ala Gly Cys Cys Ala Ala Ala
    2810                2815                2820

Ala Cys Thr Gly Ala Ala Gly Ala Gly Ala Thr Thr Cys Cys Ala
    2825                2830                2835

Cys Cys Thr Cys Thr Gly Ala Thr Thr Gly Ala Gly Ala Ala Cys
    2840                2845                2850

Cys Gly Ala Gly Ala Ala Thr Thr Cys Thr Cys Ala Ala Cys Thr
    2855                2860                2865

Thr Cys Gly Thr Gly Cys Ala Ala Gly Cys Cys Thr Thr Thr Cys
    2870                2875                2880

Ala Thr Thr Thr Cys Thr Gly Gly Thr Cys Thr Ala Ala Ala Thr
    2885                2890                2895

Thr Thr Cys Thr Gly Cys Ala Cys Thr Ala Ala Ala Thr Thr Ala
    2900                2905                2910

Thr Thr Gly Thr Ala Cys Thr Cys Thr Ala Ala Thr Gly Cys Cys
    2915                2920                2925

Ala Gly Thr Thr Cys Cys Ala Thr Gly Gly Ala Gly Gly Cys Ala
    2930                2935                2940

Gly Cys Thr Cys Cys Ala Thr Ala Thr Thr Ala Thr Cys Cys Cys
    2945                2950                2955

Thr Thr Gly Ala Cys Thr Gly Gly Ala Gly Ala Ala Ala Cys Cys
    2960                2965                2970

Ala Gly Ala Thr Thr Thr Gly Ala Ala Gly Thr Thr Gly Ala Ala
    2975                2980                2985

Ala Thr Ala Gly Thr Gly Thr Cys Cys Ala Cys Ala Gly Gly Gly
    2990                2995                3000

Gly Ala Ala Gly Thr Gly Ala Ala Ala Gly Ala Ala Thr Ala Cys
    3005                3010                3015

Thr Cys Thr Gly Cys Ala Ala Gly Thr Gly Cys Ala Ala Ala Cys
    3020                3025                3030

Thr Ala Thr Gly Ala Thr Cys Thr Gly Cys Ala Gly Ala Gly Ala
    3035                3040                3045

Gly Ala Gly Gly Gly Ala Ala Cys Thr Gly Ala Cys Cys Thr Gly
    3050                3055                3060

Gly Thr Ala Gly Ala Cys Ala Cys Ala Thr Thr Ala Ala Ala Gly
    3065                3070                3075

Thr Thr Thr Gly Cys Gly Gly Thr Ala Cys Ala Ala Gly Cys Ala
    3080                3085                3090

Gly Ala Ala Gly Gly Thr Gly Thr Ala Ala Ala Gly Cys Ala Gly
    3095                3100                3105

Cys Ala Thr Gly Ala Ala Gly Cys Cys Ala Cys Ala Cys Thr Ala

3110                3115                3120

Ala Cys Cys Thr Thr Thr Ala Ala Gly Thr Ala Cys Ala Ala Thr
    3125                3130                3135

Ala Gly Ala Gly Ala Cys Ala Gly Gly Ala Ala Gly Ala Thr Thr
    3140                3145                3150

Cys Thr Gly Ala Cys Cys Ala Gly Cys Gly Ala Cys Gly Thr Cys
    3155                3160                3165

Thr Cys Cys Ala Thr Thr Cys Cys Ala Gly Ala Thr Gly Thr Thr
    3170                3175                3180

Gly Ala Thr Gly Thr Thr Gly Ala Cys Thr Thr Thr Gly Gly Thr
    3185                3190                3195

Ala Cys Thr Ala Ala Cys Thr Thr Cys Ala Gly Ala Ala Thr Cys
    3200                3205                3210

Ala Cys Thr Gly Ala Thr Gly Ala Ala Thr Cys Thr Gly Thr Thr
    3215                3220                3225

Thr Cys Thr Gly Gly Gly Ala Ala Gly Ala Ala Ala Gly Cys Ala
    3230                3235                3240

Thr Ala Thr Ala Thr Cys Thr Thr Cys Gly Thr Thr Ala Thr Ala
    3245                3250                3255

Gly Ala Cys Thr Thr Thr Ala Ala Thr Ala Ala Cys Ala Ala Gly
    3260                3265                3270

Ala Ala Gly Ala Thr Thr Thr Cys Thr Gly Ala Ala Gly Thr Cys
    3275                3280                3285

Ala Cys Thr Cys Thr Cys Ala Cys Thr Gly Gly Ala Cys Ala Ala
    3290                3295                3300

Ala Thr Ala Ala Gly Ala Thr Ala Thr Gly Cys Thr Gly Gly Ala
    3305                3310                3315

Ala Thr Ala Gly Ala Ala Gly Ala Ala Gly Cys Ala Ala Thr Gly
    3320                3325                3330

Cys Thr Gly Ala Gly Ala Gly Gly Cys Ala Cys Cys Gly Thr Thr
    3335                3340                3345

Thr Cys Thr Ala Thr Thr Cys Cys Thr Cys Gly Thr Cys Thr Gly
    3350                3355                3360

Cys Ala Ala Ala Cys Thr Gly Ala Ala Cys Thr Thr Ala Ala Ala
    3365                3370                3375

Ala Cys Thr Gly Ala Ala Gly Cys Ala Cys Thr Gly Gly Thr Thr
    3380                3385                3390

Ala Ala Thr Thr Ala Thr Thr Cys Ala Cys Cys Cys Ala Cys Cys
    3395                3400                3405

Ala Ala Ala Gly Gly Ala Thr Ala Cys Cys Thr Thr Cys Ala Gly
    3410                3415                3420

Ala Thr Gly Ala Gly Cys Thr Cys Ala Thr Cys Thr Gly Thr Ala
    3425                3430                3435

Gly Gly Ala Ala Cys Cys Cys Ala Thr Gly Gly Gly Ala Ala Cys
    3440                3445                3450

Ala Cys Ala Gly Thr Thr Thr Cys Ala Ala Ala Ala Ala Gly Ala
    3455                3460                3465

Gly Thr Thr Cys Thr Thr Cys Thr Cys Ala Gly Ala Thr Ala Thr
    3470                3475                3480

Gly Ala Thr Thr Cys Thr Gly Ala Ala Ala Ala Ala Gly Cys Thr
    3485                3490                3495

Gly Ala Gly Cys Thr Ala Gly Ala Gly Thr Gly Gly Ala Ala Thr
    3500                3505                3510

Thr Cys Ala Gly Gly Thr Gly Cys Cys Ala Cys Thr Gly Cys Thr
    3515                3520                3525

Gly Cys Thr Gly Thr Gly Gly Gly Ala Ala Gly Ala Ala Thr Gly
    3530                3535                3540

Thr Cys Thr Thr Cys Thr Gly Cys Cys Thr Thr Cys Cys Ala Ala
    3545                3550                3555

Gly Thr Thr Gly Ala Cys Thr Thr Thr Thr Cys Ala Gly Ala Cys
    3560                3565                3570

Thr Ala Cys Thr Cys Ala Ala Ala Gly Ala Cys Thr Thr Thr Ala
    3575                3580                3585

Gly Ala Gly Ala Ala Gly Thr Ala Thr Gly Cys Cys Ala Ala Cys
    3590                3595                3600

Gly Ala Ala Cys Thr Thr Cys Thr Gly Gly Ala Thr Cys Gly Gly
    3605                3610                3615

Ala Ala Ala Gly Thr Cys Gly Cys Thr Cys Thr Thr Ala Cys Ala
    3620                3625                3630

Gly Ala Thr Ala Thr Gly Ala Cys Ala Ala Thr Gly Cys Gly Ala
    3635                3640                3645

Cys Ala Thr Ala Thr Thr Gly Thr Ala Thr Cys Ala Cys Ala Ala
    3650                3655                3660

Thr Thr Thr Ala Thr Thr Gly Thr Gly Gly Cys Ala Ala Cys Cys
    3665                3670                3675

Ala Ala Cys Ala Cys Cys Thr Gly Gly Cys Thr Ala Cys Ala Ala
    3680                3685                3690

Ala Ala Gly Gly Cys Ala Thr Cys Ala Ala Ala Ala Gly Ala Thr
    3695                3700                3705

Gly Thr Cys Cys Cys Cys Thr Ala Thr Gly Cys Cys Cys Ala Gly
    3710                3715                3720

Ala Cys Thr Cys Thr Ala Cys Ala Ala Gly Cys Cys Ala Ala Ala
    3725                3730                3735

Cys Thr Ala Ala Gly Thr Gly Gly Ala Cys Thr Gly Cys Ala Gly
    3740                3745                3750

Gly Ala Ala Cys Thr Gly Ala Ala Cys Ala Thr Thr Cys Ala Gly
    3755                3760                3765

Ala Ala Gly Ala Thr Thr Ala Ala Ala Cys Thr Gly Cys Cys Thr
    3770                3775                3780

Gly Thr Thr Ala Thr Cys Ala Cys Cys Ala Thr Thr Cys Cys Thr
    3785                3790                3795

Gly Ala Ala Gly Ala Ala Cys Thr Thr Thr Thr Cys Cys Thr Gly
    3800                3805                3810

Ala Ala Ala Ala Gly Thr Gly Ala Ala Gly Gly Cys Cys Gly Gly
    3815                3820                3825

Ala Thr Cys Ala Ala Ala Thr Ala Thr Ala Gly Cys Thr Thr Cys
    3830                3835                3840

Ala Ala Thr Ala Ala Gly Ala Ala Cys Ala Gly Thr Thr Thr Thr
    3845                3850                3855

Cys Thr Ala Ala Thr Thr Ala Ala Thr Ala Thr Thr Cys Cys Ala
    3860                3865                3870

Thr Thr Ala Cys Cys Ala Thr Thr Thr Gly Gly Thr Gly Gly Ala
    3875                3880                3885

Ala Gly Ala Thr Cys Gly Thr Cys Cys Cys Ala Thr Gly Ala Cys
    3890                3895                3900

Ala Thr Ala Ala Gly Ala Gly Thr Gly Cys Cys Ala Cys Ala Gly
    3905                3910                3915

Ala Cys Thr Gly Thr Thr Ala Ala Ala Ala Cys Ala Cys Cys Thr
    3920                3925                3930

Cys Gly Thr Cys Thr Ala Gly Thr Ala Ala Thr Ala Gly Ala Gly
    3935                3940                3945

Thr Cys Thr Ala Thr Gly Gly Gly Ala Ala Thr Ala Ala Ala Cys
    3950                3955                3960

Ala Thr Ala Cys Cys Ala Thr Cys Ala Cys Ala Gly Gly Ala Ala
    3965                3970                3975

Thr Ala Cys Ala Gly Ala Ala Thr Gly Cys Cys Ala Ala Cys Ala
    3980                3985                3990

Thr Thr Thr Ala Cys Thr Gly Thr Thr Cys Cys Ala Gly Ala Ala
    3995                4000                4005

Thr Cys Cys Thr Ala Thr Cys Cys Thr Cys Thr Thr Cys Thr Thr
    4010                4015                4020

Gly Thr Gly Cys Cys Thr Thr Thr Ala Thr Thr Thr Gly Gly Thr
    4025                4030                4035

Gly Cys Thr Thr Thr Ala Gly Ala Gly Gly Cys Cys Thr Cys Cys
    4040                4045                4050

Gly Cys Cys Ala Gly Thr Gly Thr Gly Cys Ala Cys Ala Gly Cys

4055                4060                4065

Ala Ala Cys Thr Ala Thr Thr Ala Cys Ala Ala Cys Thr Gly Gly
4070                4075                4080

Ala Cys Ala Gly Cys Ala Gly Cys Ala Thr Ala Cys Ala Cys Thr
4085                4090                4095

Thr Thr Gly Ala Cys Ala Ala Ala Thr Ala Gly Cys Ala Gly Cys
4100                4105                4110

Ala Cys Ala Gly Ala Gly Ala Ala Ala Ala Cys Ala Gly Cys Cys
4115                4120                4125

Ala Gly Ala Ala Thr Thr Gly Gly Ala Ala Cys Thr Ala Cys Thr
4130                4135                4140

Thr Ala Thr Gly Cys Thr Gly Thr Gly Ala Ala Thr Gly Cys Thr
4145                4150                4155

Gly Ala Thr Thr Cys Thr Gly Thr Thr Thr Thr Thr Gly Ala Gly
4160                4165                4170

Cys Thr Ala Cys Thr Ala Thr Cys Cys Thr Ala Cys Ala Ala Cys
4175                4180                4185

Ala Thr Gly Ala Ala Ala Gly Gly Thr Thr Cys Thr Gly Gly Ala
4190                4195                4200

Gly Ala Gly Gly Cys Ala Thr Cys Thr Thr Cys Thr Ala Gly Thr
4205                4210                4215

Ala Gly Ala Ala Ala Thr Gly Gly Ala Thr Thr Cys Ala Cys Thr
4220                4225                4230

Thr Gly Thr Gly Cys Ala Thr Ala Thr Gly Ala Ala Ala Ala Thr
4235                4240                4245

Cys Ala Thr Cys Thr Gly Ala Ala Gly Cys Ala Cys Ala Gly Ala
4250                4255                4260

Cys Thr Cys Thr Thr Ala Ala Cys Thr Thr Cys Ala Gly Ala Thr
4265                4270                4275

Thr Thr Thr Ala Ala Ala Ala Thr Gly Thr Cys Thr Ala Gly Gly
4280                4285                4290

Ala Cys Ala Ala Ala Gly Ala Gly Thr Thr Ala Thr Gly Ala Ala
    4295                4300                4305

Cys Cys Thr Ala Cys Ala Thr Cys Ala Gly Thr Thr Thr Cys Ala
    4310                4315                4320

Ala Ala Cys Thr Gly Thr Ala Cys Cys Ala Thr Ala Thr Thr Thr
    4325                4330                4335

Cys Thr Ala Ala Thr Gly Gly Cys Ala Thr Cys Cys Ala Gly Thr
    4340                4345                4350

Gly Cys Thr Cys Thr Gly Gly Gly Thr Cys Cys Thr Cys Ala Gly
    4355                4360                4365

Cys Thr Thr Thr Cys Ala Thr Thr Thr Thr Cys Cys Ala Gly Thr
    4370                4375                4380

Gly Ala Thr Gly Thr Thr Gly Thr Thr Thr Cys Thr Gly Ala Ala
    4385                4390                4395

Ala Ala Gly Ala Cys Ala Ala Ala Thr Ala Ala Cys Ala Thr Gly
    4400                4405                4410

Ala Ala Thr Ala Thr Thr Ala Ala Thr Ala Ala Thr Gly Thr Cys
    4415                4420                4425

Ala Gly Gly Ala Thr Ala Gly Ala Ala Gly Gly Gly Cys Ala Ala
    4430                4435                4440

Cys Thr Gly Gly Ala Ala Gly Thr Gly Gly Cys Thr Thr Cys Thr
    4445                4450                4455

Gly Thr Gly Thr Thr Thr Gly Cys Ala Ala Gly Ala Ala Gly Thr
    4460                4465                4470

Gly Thr Thr Thr Ala Cys Ala Cys Thr Ala Thr Gly Thr Cys Ala
    4475                4480                4485

Ala Gly Cys Thr Cys Ala Thr Ala Cys Ala Ala Thr Gly Ala Ala
    4490                4495                4500

Ala Ala Gly Ala Gly Ala Cys Gly Ala Gly Thr Thr Thr Thr Ala
    4505                4510                4515

Gly Ala Ala Gly Gly Gly Ala Ala Gly Thr Cys Ala Ala Ala Thr
    4520                4525                4530

Cys Thr Gly Ala Gly Gly Thr Thr Gly Gly Ala Thr Thr Cys Thr
    4535                4540                4545

Thr Cys Thr Thr Ala Cys Cys Thr Thr Cys Ala Ala Gly Cys Thr
    4550                4555                4560

Ala Cys Cys Ala Ala Thr Cys Ala Thr Cys Thr Ala Thr Cys Cys
    4565                4570                4575

Gly Gly Thr Ala Gly Ala Thr Ala Cys Ala Cys Thr Gly Ala Thr
    4580                4585                4590

Gly Gly Thr Gly Thr Ala Thr Thr Thr Thr Cys Cys Ala Thr Thr
    4595                4600                4605

Ala Cys Thr Thr Cys Ala Gly Cys Thr Thr Cys Thr Gly Ala Thr
    4610                4615                4620

Gly Thr Ala Cys Ala Ala Ala Ala Thr Gly Gly Ala Cys Thr Ala
    4625                4630                4635

Thr Thr Ala Ala Ala Gly Ala Ala Cys Ala Cys Ala Gly Cys Thr
    4640                4645                4650

Thr Cys Ala Cys Thr Gly Ala Ala Gly Thr Ala Thr Gly Ala Ala
    4655                4660                4665

Ala Ala Thr Ala Gly Cys Cys Ala Gly Cys Thr Gly Ala Ala Ala
    4670                4675                4680

Ala Thr Ala Ala Cys Ala Thr Cys Thr Gly Ala Ala Ala Cys Ala
    4685                4690                4695

Ala Ala Thr Gly Gly Ala Ala Gly Gly Thr Ala Thr Cys Thr Ala
    4700                4705                4710

Cys Ala Thr Cys Thr Cys Gly Cys Ala Gly Cys Thr Gly Thr Cys
    4715                4720                4725

Ala Ala Thr Ala Ala Ala Cys Thr Thr Gly Ala Ala Thr Thr Cys
    4730                4735                4740

Cys Thr Thr Thr Thr Gly Thr Cys Ala Ala Ala Gly Ala Ala Gly
    4745                4750                4755

Ala Thr Gly Gly Cys Ala Gly Cys Ala Cys Thr Thr Cys Gly Thr
    4760                4765                4770

Thr Cys Thr Gly Ala Gly Thr Ala Cys Cys Ala Ala Gly Cys Cys
    4775                4780                4785

Ala Cys Thr Thr Ala Cys Ala Ala Gly Cys Ala Ala Ala Cys Cys
    4790                4795                4800

Cys Ala Ala Thr Gly Thr Thr Ala Thr Gly Cys Cys Thr Thr Gly
    4805                4810                4815

Thr Thr Cys Gly Cys Ala Gly Gly Thr Thr Cys Thr Cys Thr Cys
    4820                4825                4830

Ala Ala Thr Thr Cys Cys Cys Ala Gly Gly Ala Thr Cys Thr Thr
    4835                4840                4845

Gly Thr Thr Thr Thr Cys Ala Ala Cys Ala Cys Thr Gly Ala Thr
    4850                4855                4860

Thr Thr Cys Thr Cys Thr Cys Thr Gly Ala Cys Thr Gly Ala Thr
    4865                4870                4875

Cys Ala Ala Ala Gly Gly Ala Ala Thr Cys Gly Ala Gly Cys Thr
    4880                4885                4890

Gly Cys Ala Cys Ala Thr Ala Ala Gly Thr Cys Ala Thr Cys Ala
    4895                4900                4905

Cys Thr Cys Ala Ala Thr Gly Thr Thr Ala Ala Thr Cys Ala Gly
    4910                4915                4920

Thr Ala Thr Gly Gly Thr Cys Thr Ala Gly Cys Cys Ala Gly Cys
    4925                4930                4935

Ala Gly Thr Gly Cys Ala Ala Cys Thr Ala Cys Ala Ala Ala Thr
    4940                4945                4950

Gly Thr Ala Cys Ala Gly Thr Thr Thr Ala Gly Thr Cys Cys Ala
    4955                4960                4965

Cys Thr Gly Ala Cys Ala Ala Thr Gly Cys Ala Gly Ala Gly Thr
    4970                4975                4980

Gly Ala Ala Ala Thr Gly Ala Ala Thr Gly Cys Thr Ala Ala Ala
    4985                4990                4995

Cys Thr Thr Gly Ala Thr Ala Cys Cys Thr Cys Thr Gly Gly Ala

5000                5005                5010

Gly Gly  Cys Thr Cys Thr Gly  Thr Gly Thr Cys Ala  Cys Thr Gly
    5015                5020                5025

Thr Cys  Ala Thr Cys Ala Thr  Cys Thr Gly Gly Gly  Cys Gly Cys
    5030                5035                5040

Thr Ala  Thr Gly Gly Ala Ala  Ala Ala Ala Ala Cys  Ala Ala Thr
    5045                5050                5055

Gly Cys  Ala Ala Ala Ala Thr  Thr Cys Ala Ala Thr  Gly Thr Ala
    5060                5065                5070

Gly Gly  Thr Gly Gly Ala Ala  Gly Ala Gly Thr Gly  Ala Gly Cys
    5075                5080                5085

Thr Thr  Ala Ala Cys Gly Gly  Ala Ala Ala Thr Ala  Ala Cys Gly
    5090                5095                5100

Cys Thr  Gly Gly Gly Ala Ala  Gly Cys Gly Ala Ala  Thr Ala Thr
    5105                5110                5115

Cys Ala  Gly Ala Gly Thr Ala  Cys Ala Ala Thr Thr  Cys Thr Gly
    5120                5125                5130

Gly Gly  Thr Ala Thr Gly Gly  Ala Cys Ala Ala Thr  Ala Ala Ala
    5135                5140                5145

Cys Ala  Thr Gly Thr Cys Thr  Thr Ala Ala Ala Cys  Thr Thr Cys
    5150                5155                5160

Ala Gly  Ala Ala Thr Thr Ala  Ala Thr Ala Ala Ala  Gly Ala Ala
    5165                5170                5175

Gly Gly  Ala Cys Thr Cys Ala  Ala Gly Thr Thr Thr  Thr Cys Ala
    5180                5185                5190

Ala Ala  Thr Ala Ala Thr Thr  Thr Gly Cys Ala Ala  Gly Gly Gly
    5195                5200                5205

Thr Cys  Ala Thr Thr Ala Ala  Ala Ala Gly Ala Gly  Ala Thr Thr
    5210                5215                5220

Ala Ala  Gly Cys Thr Gly Gly  Ala Gly Thr Ala Cys  Ala Cys Ala
    5225                5230                5235

Ala Ala Thr Gly Ala Cys Thr Thr Gly Ala Ala Thr Ala Thr Thr
    5240                5245                5250

Cys Cys Thr Gly Gly Cys Thr Thr Ala Thr Cys Cys Cys Thr Ala
    5255                5260                5265

Ala Cys Ala Thr Thr Thr Gly Thr Thr Thr Cys Ala Ala Ala Gly
    5270                5275                5280

Thr Thr Ala Gly Ala Cys Ala Ala Cys Ala Gly Cys Thr Thr Cys
    5285                5290                5295

Ala Gly Cys Thr Thr Thr Gly Ala Cys Ala Ala Gly Thr Thr Cys
    5300                5305                5310

Cys Ala Cys Ala Ala Gly Cys Ala Thr Gly Thr Thr Thr Thr Thr
    5315                5320                5325

Gly Ala Cys Cys Thr Thr Cys Ala Gly Cys Thr Gly Cys Ala Gly
    5330                5335                5340

Cys Cys Cys Ala Gly Ala Thr Cys Ala Cys Thr Thr Ala Cys Ala
    5345                5350                5355

Gly Cys Thr Ala Ala Gly Cys Thr Gly Ala Ala Cys Ala Ala Thr
    5360                5365                5370

Ala Ala Thr Ala Thr Thr Ala Ala Ala Thr Ala Cys Ala Cys Cys
    5375                5380                5385

Ala Ala Ala Ala Cys Thr Gly Ala Ala Gly Thr Thr Thr Cys Cys
    5390                5395                5400

Ala Ala Cys Ala Ala Ala Gly Cys Ala Gly Ala Ala Cys Thr Gly
    5405                5410                5415

Cys Thr Cys Cys Thr Thr Gly Ala Gly Cys Cys Thr Cys Thr Gly
    5420                5425                5430

Ala Ala Gly Cys Thr Gly Ala Ala Cys Thr Thr Gly Gly Gly Thr
    5435                5440                5445

Gly Gly Cys Ala Ala Thr Gly Thr Gly Ala Gly Ala Gly Cys Ala
    5450                5455                5460

Gly Cys Cys Thr Ala Thr Gly Gly Ala Ala Cys Ala Gly Ala Thr
    5465                5470                5475

Gly Ala Ala Gly Thr Ala Ala Gly Ala Cys Ala Thr Ala Cys Cys
    5480                5485                5490

Thr Ala Cys Gly Cys Cys Ala Thr Thr Ala Cys Ala Thr Ala Thr
    5495                5500                5505

Gly Cys Thr Gly Ala Thr Cys Thr Ala Ala Cys Thr Gly Cys Ala
    5510                5515                5520

Ala Ala Cys Thr Thr Thr Ala Ala Ala Ala Cys Ala Gly Ala Cys
    5525                5530                5535

Ala Cys Ala Gly Thr Thr Gly Cys Ala Ala Ala Thr Gly Thr Cys
    5540                5545                5550

Cys Ala Ala Gly Gly Thr Gly Cys Ala Gly Cys Ala Gly Thr Gly
    5555                5560                5565

Ala Gly Thr Cys Ala Cys Ala Gly Ala Gly Thr Thr Ala Ala Thr
    5570                5575                5580

Cys Thr Gly Ala Ala Thr Gly Thr Gly Gly Cys Ala Gly Gly Ala
    5585                5590                5595

Cys Thr Gly Gly Cys Thr Thr Cys Cys Thr Cys Cys Ala Thr Thr
    5600                5605                5610

Ala Cys Thr Ala Thr Gly Ala Ala Cys Ala Cys Ala Ala Ala Thr
    5615                5620                5625

Thr Gly Thr Gly Ala Thr Thr Cys Cys Ala Ala Ala Thr Cys Thr
    5630                5635                5640

Cys Thr Cys Cys Gly Thr Thr Thr Cys Ala Gly Cys Ala Ala Thr
    5645                5650                5655

Gly Cys Thr Cys Thr Gly Cys Gly Thr Thr Cys Cys Ala Cys Thr
    5660                5665                5670

Ala Thr Gly Gly Cys Cys Cys Cys Ala Thr Thr Cys Ala Cys Thr
    5675                5680                5685

Ala Thr Cys Ala Cys Gly Gly Cys Thr Gly Ala Thr Gly Thr Thr
    5690                5695                5700

Cys Ala Cys Ala Cys Cys Ala Ala Cys Gly Gly Thr Ala Ala Thr
    5705                5710                5715

Gly Gly Gly Ala Ala Gly Cys Thr Gly Ala Thr Thr Gly Cys Thr
    5720                5725                5730

Cys Thr Gly Gly Gly Thr Gly Ala Ala Cys Ala Thr Ala Cys Ala
    5735                5740                5745

Gly Gly Ala Gly Ala Cys Cys Thr Thr Thr Ala Cys Ala Gly Cys
    5750                5755                5760

Ala Ala Ala Ala Thr Cys Cys Thr Gly Thr Thr Cys Ala Ala Ala
    5765                5770                5775

Gly Cys Ala Gly Ala Ala Cys Cys Thr Cys Thr Thr Gly Cys Thr
    5780                5785                5790

Thr Thr Cys Ala Cys Thr Thr Thr Cys Thr Cys Cys Cys Ala Thr
    5795                5800                5805

Gly Ala Thr Thr Ala Cys Ala Gly Ala Gly Gly Ala Thr Cys Thr
    5810                5815                5820

Ala Cys Cys Ala Gly Thr Cys Ala Thr Ala Gly Cys Thr Thr Cys
    5825                5830                5835

Ala Ala Gly Thr Cys Thr Ala Thr Gly Ala Gly Ala Ala Gly Ala
    5840                5845                5850

Thr Ala Cys Thr Cys Thr Ala Cys Ala Cys Ala Gly Cys Thr Thr
    5855                5860                5865

Gly Ala Thr Ala Ala Cys Ala Ala Ala Thr Thr Thr Cys Ala Thr
    5870                5875                5880

Ala Thr Gly Cys Thr Thr Thr Thr Thr Ala Cys Thr Cys Cys Ala
    5885                5890                5895

Thr Cys Ala Gly Ala Gly Cys Ala Gly Thr Cys Gly Ala Gly Thr
    5900                5905                5910

Gly Cys Thr Thr Gly Gly Ala Ala Ala Thr Thr Gly Ala Ala Ala
    5915                5920                5925

Ala Gly Thr Cys Ala Gly Cys Thr Ala Ala Ala Cys Ala Ala Cys
    5930                5935                5940

Ala Ala Cys Ala Thr Ala Thr Ala Thr Thr Cys Ala Cys Ala Ala

5945                5950                5955

Gly Ala Thr Ala Thr Cys Ala Ala Thr Gly Cys Thr Thr Ala Cys
    5960                5965                5970

Ala Ala Thr Gly Ala Thr Gly Cys Ala Gly Ala Ala Ala Ala Gly
    5975                5980                5985

Ala Thr Thr Gly Gly Thr Gly Thr Gly Gly Ala Ala Cys Thr Thr
    5990                5995                6000

Ala Gly Thr Gly Gly Ala Ala Gly Ala Gly Cys Thr Thr Thr Ala
    6005                6010                6015

Gly Cys Ala Gly Ala Thr Cys Thr Cys Thr Cys Thr Gly Thr Gly
    6020                6025                6030

Gly Thr Gly Gly Ala Thr Ala Cys Ala Gly Cys Ala Ala Thr Cys
    6035                6040                6045

Ala Gly Ala Cys Thr Ala Cys Cys Ala Thr Thr Cys Ala Thr Gly
    6050                6055                6060

Thr Cys Thr Gly Ala Ala Gly Ala Ala Gly Thr Thr Ala Ala Thr
    6065                6070                6075

Gly Thr Ala Ala Thr Thr Gly Ala Thr Gly Thr Ala Thr Thr Ala
    6080                6085                6090

Gly Gly Cys Cys Thr Cys Ala Gly Gly Gly Ala Cys Ala Gly Thr
    6095                6100                6105

Gly Thr Gly Thr Cys Ala Gly Ala Gly Cys Cys Thr Cys Ala Ala
    6110                6115                6120

Gly Ala Ala Thr Thr Cys Ala Gly Cys Ala Thr Cys Thr Cys Thr
    6125                6130                6135

Gly Gly Cys Thr Cys Thr Gly Thr Gly Ala Ala Gly Thr Ala Thr
    6140                6145                6150

Gly Ala Cys Ala Ala Ala Ala Ala Thr Ala Ala Ala Gly Ala Thr
    6155                6160                6165

Ala Thr Gly Cys Ala Thr Gly Thr Thr Ala Thr Thr Ala Ala Cys
    6170                6175                6180

Cys Thr Ala Cys Cys Ala Thr Thr Cys Thr Thr Gly Gly Ala Ala
    6185                6190                6195

Cys Ala Cys Thr Thr Thr Cys Cys Ala Gly Thr Cys Thr Ala Cys
    6200                6205                6210

Thr Thr Thr Gly Ala Ala Cys Ala Ala Ala Thr Cys Ala Gly Ala
    6215                6220                6225

Gly Gly Thr Gly Cys Cys Ala Thr Thr Cys Thr Ala Thr Cys Cys
    6230                6235                6240

Ala Cys Ala Cys Thr Gly Cys Ala Gly Gly Cys Thr Gly Thr Ala
    6245                6250                6255

Cys Ala Gly Ala Ala Thr Thr Ala Cys Cys Thr Gly Ala Ala Ala
    6260                6265                6270

Ala Ala Cys Ala Thr Thr Gly Ala Cys Gly Thr Ala Gly Ala Thr
    6275                6280                6285

Cys Ala Ala Thr Ala Thr Ala Thr Gly Ala Ala Ala Ala Ala Ala
    6290                6295                6300

Thr Ala Cys Ala Ala Gly Gly Cys Ala Ala Cys Thr Thr Thr Ala
    6305                6310                6315

Gly Ala Thr Gly Ala Ala Thr Thr Cys Cys Cys Ala Cys Ala Gly
    6320                6325                6330

Cys Ala Cys Cys Thr Thr Ala Ala Thr Gly Ala Thr Thr Ala Cys
    6335                6340                6345

Ala Thr Gly Gly Ala Cys Ala Ala Ala Thr Thr Ala Gly Ala Thr
    6350                6355                6360

Cys Thr Gly Ala Ala Ala Gly Gly Cys Ala Gly Ala Gly Cys Thr
    6365                6370                6375

Ala Gly Cys Ala Cys Cys Ala Thr Ala Ala Ala Ala Ala Cys Thr
    6380                6385                6390

Ala Ala Thr Thr Thr Gly Ala Thr Thr Gly Cys Thr Thr Thr Cys
    6395                6400                6405

Ala Cys Gly Ala Ala Gly Gly Ala Cys Thr Ala Thr Ala Gly Ala
    6410                6415                6420

Ala Thr Ala Ala Cys Ala Thr Cys Thr Gly Ala Thr Gly Ala Thr
    6425                6430                6435

Cys Thr Cys Gly Ala Ala Ala Thr Thr Ala Thr Thr Cys Thr Gly
    6440                6445                6450

Gly Ala Ala Ala Ala Ala Gly Cys Cys Cys Thr Gly Gly Ala Thr
    6455                6460                6465

Ala Ala Thr Cys Thr Thr Cys Ala Ala Gly Ala Ala Ala Thr Ala
    6470                6475                6480

Cys Thr Gly Cys Thr Cys Cys Ala Gly Cys Thr Thr Cys Ala Ala
    6485                6490                6495

Gly Thr Thr Thr Ala Thr Cys Thr Thr Gly Thr Gly Cys Ala Ala
    6500                6505                6510

Ala Thr Ala Gly Ala Gly Cys Ala Gly Thr Ala Cys Ala Thr Cys
    6515                6520                6525

Ala Ala Ala Gly Ala Gly Ala Ala Thr Thr Ala Cys Gly Ala Thr
    6530                6535                6540

Cys Ala Gly Thr Thr Thr Gly Ala Cys Ala Thr Thr Ala Ala Thr
    6545                6550                6555

Gly Cys Ala Cys Thr Thr Ala Thr Thr Gly Cys Ala Cys Ala Ala
    6560                6565                6570

Cys Thr Thr Cys Thr Thr Gly Ala Cys Ala Ala Ala Ala Thr Ala
    6575                6580                6585

Gly Thr Thr Gly Ala Ala Ala Ala Ala Ala Thr Gly Ala Cys Ala
    6590                6595                6600

Gly Cys Thr Cys Thr Ala Gly Ala Thr Gly Ala Ala Ala Ala Ala
    6605                6610                6615

Thr Ala Thr Ala Ala Gly Ala Thr Ala Ala Gly Ala Gly Thr Gly
    6620                6625                6630

Ala Cys Thr Gly Thr Ala Gly Thr Thr Gly Ala Cys Ala Cys Thr
    6635                6640                6645

Ala Thr Thr Cys Ala Gly Ala Ala Gly Thr Thr Ala Cys Ala Ala
    6650                6655                6660

Thr Thr Thr Thr Thr Thr Thr Thr Ala Ala Ala Thr Cys Ala Ala
    6665                6670                6675

Thr Ala Thr Thr Ala Cys Cys Cys Cys Ala Gly Cys Ala Ala Cys
    6680                6685                6690

Ala Thr Thr Gly Gly Ala Ala Gly Cys Ala Ala Cys Ala Cys Thr
    6695                6700                6705

Ala Thr Gly Ala Cys Thr Thr Gly Gly Ala Thr Thr Ala Ala Ala
    6710                6715                6720

Ala Ala Thr Ala Thr Ala Gly Ala Thr Gly Ala Thr Gly Ala Gly
    6725                6730                6735

Thr Ala Thr Ala Gly Gly Ala Thr Cys Ala Cys Ala Gly Gly Thr
    6740                6745                6750

Ala Gly Ala Ala Thr Ala Ala Ala Gly Gly Ala Gly Ala Ala Cys
    6755                6760                6765

Cys Thr Ala Gly Ala Ala Cys Ala Gly Cys Thr Cys Ala Ala Gly
    6770                6775                6780

Ala Thr Thr Cys Ala Gly Ala Thr Thr Cys Ala Gly Ala Ala Cys
    6785                6790                6795

Ala Thr Thr Gly Ala Thr Ala Thr Cys Ala Gly Ala Ala Gly Cys
    6800                6805                6810

Thr Thr Thr Gly Cys Ala Gly Ala Ala Ala Ala Thr Thr Thr Gly
    6815                6820                6825

Ala Ala Ala Ala Gly Gly Ala Ala Gly Ala Thr Thr Ala Ala Ala
    6830                6835                6840

Ala Thr Ala Ala Thr Thr Gly Ala Thr Gly Thr Thr Ala Ala Ala
    6845                6850                6855

Cys Ala Ala Cys Thr Ala Thr Thr Ala Gly Ala Ala Ala Ala Ala
    6860                6865                6870

Thr Thr Ala Ala Ala Gly Cys Gly Thr Thr Cys Ala Thr Thr Ala
    6875                6880                6885

Cys Cys Ala Ala Thr Thr Ala Ala Ala Ala Ala Ala Ala Thr Gly

6890                6895                6900

Ala Ala Gly Gly Ala Ala Gly Thr Thr Cys Thr Thr Gly Ala Ala
    6905                6910                6915

Cys Ala Ala Ala Thr Cys Ala Ala Ala Gly Ala Cys Thr Thr Thr
    6920                6925                6930

Ala Thr Thr Cys Thr Gly Ala Gly Thr Thr Gly Gly Ala Thr Gly
    6935                6940                6945

Gly Ala Gly Gly Ala Gly Thr Ala Cys Gly Ala Ala Gly Thr Gly
    6950                6955                6960

Thr Cys Thr Gly Ala Gly Ala Ala Gly Ala Thr Cys Ala Gly Thr
    6965                6970                6975

Gly Cys Thr Thr Thr Cys Cys Gly Ala Gly Gly Thr Cys Ala Thr
    6980                6985                6990

Ala Thr Gly Cys Ala Thr Ala Ala Ala Cys Thr Gly Ala Thr Thr
    6995                7000                7005

Gly Thr Ala Ala Ala Ala Thr Ala Thr Gly Ala Ala Ala Thr Thr
    7010                7015                7020

Gly Ala Thr Ala Ala Gly Cys Ala Thr Gly Thr Gly Thr Ala Thr
    7025                7030                7035

Thr Thr Thr Thr Thr Ala Thr Thr Gly Gly Ala Cys Ala Gly Ala
    7040                7045                7050

Ala Thr Gly Ala Thr Ala Gly Ala Ala Cys Thr Ala Cys Thr Thr
    7055                7060                7065

Ala Ala Cys Cys Ala Gly Thr Ala Cys Ala Gly Ala Ala Thr Ala
    7070                7075                7080

Ala Gly Ala Gly Ala Ala Ala Cys Gly Gly Thr Ala Cys Gly Gly
    7085                7090                7095

Ala Ala Ala Ala Thr Gly Ala Cys Gly Ala Cys Cys Thr Ala Cys
    7100                7105                7110

Cys Thr Ala Ala Gly Ala Ala Ala Ala Ala Thr Thr Gly Ala Cys
    7115                7120                7125

Gly Thr Gly Ala Ala Gly Ala Cys Ala Thr Gly Cys Thr Thr Thr
    7130                7135                7140

Gly Ala Cys Ala Ala Ala Ala Thr Thr Gly Thr Gly Thr Cys Thr
    7145                7150                7155

Thr Thr Ala Ala Thr Thr Gly Ala Thr Gly Ala Thr Gly Cys Thr
    7160                7165                7170

Gly Thr Gly Ala Ala Gly Ala Ala Ala Gly Thr Gly Cys Ala Ala
    7175                7180                7185

Ala Cys Cys Thr Thr Thr Gly Ala Thr Thr Ala Thr Gly Ala Ala
    7190                7195                7200

Ala Thr Gly Ala Thr Gly Ala Thr Ala Ala Ala Gly Ala Ala Ala
    7205                7210                7215

Cys Thr Cys Ala Ala Cys Ala Ala Ala Thr Thr Cys Cys Thr Thr
    7220                7225                7230

Gly Ala Cys Ala Thr Gly Ala Thr Thr Ala Thr Ala Ala Ala Gly
    7235                7240                7245

Ala Ala Ala Cys Thr Cys Ala Ala Ala Thr Cys Thr Thr Thr Thr
    7250                7255                7260

Gly Ala Cys Thr Ala Thr Ala Ala Cys Cys Ala Gly Thr Thr Thr
    7265                7270                7275

Gly Thr Thr Gly Ala Thr Gly Ala Cys Ala Cys Ala Ala Ala Thr
    7280                7285                7290

Ala Ala Cys Ala Ala Ala Ala Thr Cys Cys Ala Ala Gly Ala Ala
    7295                7300                7305

Ala Thr Thr Ala Thr Ala Cys Ala Gly Ala Ala Ala Ala Thr Ala
    7310                7315                7320

Ala Ala Thr Gly Ala Gly Gly Ala Ala Cys Thr Cys Ala Gly Ala
    7325                7330                7335

Ala Ala Thr Cys Thr Ala Gly Ala Ala Cys Thr Gly Cys Cys Ala
    7340                7345                7350

Cys Ala Gly Ala Ala Ala Gly Cys Ala Gly Ala Ala Gly Cys Ala
    7355                7360                7365

Thr Thr Gly Ala Ala Ala Cys Ala Gly Thr Ala Thr Ala Thr Gly
    7370                7375                7380

Ala Gly Ala Gly Ala Thr Thr Thr Thr Ala Ala Thr Gly Cys Thr
    7385                7390                7395

Gly Thr Ala Gly Thr Thr Thr Cys Ala Ala Ala Ala Thr Ala Cys
    7400                7405                7410

Gly Thr Ala Gly Ala Ala Cys Ala Ala Cys Thr Ala Ala Gly Gly
    7415                7420                7425

Gly Ala Cys Ala Cys Cys Ala Ala Gly Cys Thr Thr Gly Thr Cys
    7430                7435                7440

Gly Cys Thr Ala Thr Ala Ala Thr Thr Ala Ala Cys Thr Gly Gly
    7445                7450                7455

Cys Thr Cys Ala Ala Gly Gly Ala Gly Cys Thr Cys Ala Thr Cys
    7460                7465                7470

Gly Ala Cys Thr Cys Ala Ala Cys Ala Ala Cys Ala Thr Thr Thr
    7475                7480                7485

Ala Cys Thr Ala Ala Thr Cys Thr Gly Ala Ala Ala Gly Cys Cys
    7490                7495                7500

Ala Ala Ala Gly Thr Ala Ala Ala Thr Gly Ala Gly Cys Ala Thr
    7505                7510                7515

Cys Thr Gly Gly Ala Ala Gly Gly Thr Cys Thr Gly Cys Gly Ala
    7520                7525                7530

Gly Ala Gly Ala Gly Ala Ala Thr Thr Thr Cys Thr Gly Ala Cys
    7535                7540                7545

Ala Thr Gly Gly Ala Thr Ala Thr Cys Gly Cys Cys Ala Ala Gly
    7550                7555                7560

Gly Ala Ala Thr Thr Thr Gly Ala Ala Thr Gly Gly Thr Ala Thr
    7565                7570                7575

Cys Thr Thr Cys Ala Ala Ala Ala Gly Ala Thr Ala Ala Gly Cys
    7580                7585                7590

Cys Ala Gly Thr Thr Cys Thr Ala Cys Ala Ala Thr Thr Cys Thr
    7595                7600                7605

Gly Thr  Thr Gly Thr Cys Ala  Thr Ala Thr Ala Cys  Ala Thr Thr
    7610                 7615                 7620

Thr Cys  Thr Gly Ala Ala Cys  Ala Gly Thr Gly Gly  Ala Ala Cys
    7625                 7630                 7635

Ala Thr  Ala Gly Cys Thr Thr  Thr Thr Ala Ala Ala  Ala Ala Ala
    7640                 7645                 7650

Ala Thr  Cys Gly Thr Thr Ala  Cys Thr Thr Thr Gly  Gly Cys Thr
    7655                 7660                 7665

Gly Ala  Gly Ala Ala Gly Thr  Ala Thr Gly Ala Cys  Cys Thr Ala
    7670                 7675                 7680

Ala Ala  Ala Ala Ala Thr Thr  Gly Gly Gly Cys Thr  Gly Ala Ala
    7685                 7690                 7695

Ala Ala  Thr Thr Thr Ala Ala  Ala Cys Cys Ala Gly  Thr Thr Cys
    7700                 7705                 7710

Ala Thr  Thr Gly Ala Ala Ala  Cys Ala Gly Gly Ala  Thr Thr Thr
    7715                 7720                 7725

Ala Ala  Ala Gly Thr Cys Cys  Cys Thr Gly Ala Ala  Ala Thr Ala
    7730                 7735                 7740

Ala Gly  Ala Ala Cys Ala Gly  Thr Thr Ala Thr Ala  Gly Thr Cys
    7745                 7750                 7755

Ala Cys  Thr Ala Thr Ala Cys  Cys Thr Gly Cys Cys  Thr Thr Thr
    7760                 7765                 7770

Gly Ala  Ala Thr Thr Cys Ala  Gly Thr Cys Thr Thr  Cys Gly Ala
    7775                 7780                 7785

Ala Gly  Thr Cys Thr Thr Cys  Gly Gly Gly Ala Ala  Gly Cys Ala
    7790                 7795                 7800

Ala Cys  Thr Thr Thr Cys Cys  Gly Ala Ala Cys Ala  Cys Cys Gly
    7805                 7810                 7815

Gly Ala  Cys Thr Thr Cys Ala  Thr Thr Gly Thr Thr  Cys Cys Ala
    7820                 7825                 7830

Cys Thr  Gly Ala Cys Thr Gly  Ala Thr Cys Thr Gly  Ala Ala Ala

7835                7840                7845

Ala Thr  Cys Cys Cys Cys Thr  Cys Cys Thr Ala Thr  Gly Ala Ala
    7850                7855                7860

Ala Thr  Ala Ala Ala Thr Ala  Thr Thr Ala Gly Gly
    7865                7870                7875

<210>  152
<211>  2625
<212>  PRT
<213>  Gallus gallus

<400>  152

Met Gly Pro Val Arg Leu Leu Leu Leu Leu Leu Leu Leu Ser Ser Gly
1               5                   10                  15

Val Leu Thr Gln Glu Gly Thr Pro Glu Asn Gly Asn Pro Gly Cys Ser
            20                  25                  30

Lys Asp Ala Ala Arg Phe Lys Ser Leu Arg Lys Tyr Val Tyr Leu Tyr
        35                  40                  45

Glu Ala Glu Thr Ser Ser Gly Ile Thr Gly Thr Ala Asp Ser Arg Ser
    50                  55                  60

Gly Ser Lys Ile Thr Cys Lys Val Glu Leu Glu Val Pro Gln Leu Cys
65                  70                  75                  80

Gln Phe Ile Leu Arg Thr Met His Cys Ser Leu Arg Glu Thr Phe Gly
                85                  90                  95

Val Asp Ser Glu Arg Arg Ala Met Leu Arg Lys Ser Lys Asn Ser Asp
            100                 105                 110

Asp Phe Ala Asn Ala Met Ser Lys His Glu Leu Arg Phe Ser Thr Gln
        115                 120                 125

Asp Gly Thr Lys Val Lys Leu Tyr Pro Glu Lys Asp Glu Pro Leu Asn
    130                 135                 140

Val Leu Asn Leu Lys Arg Gly Ile Ile Ser Ala Leu Leu Ala Pro Thr
145                 150                 155                 160

Glu Thr Glu Glu Asn Ile Lys Thr Ile Ser Met Asp Thr Val Tyr Gly
                165                 170                 175

Lys Cys Asp Ser Glu Val Glu Phe Lys Ser Arg Arg Gly Ser Val Ala

            180                 185                 190

Glu Asp Ile Ser Ile Asn Arg Asn Leu Lys Ala Cys Asp Asn Phe Ser
        195                 200                 205

Pro Ile Arg Asp Tyr Val Ser Pro Val Ala Ile Val Lys Gly Leu Asn
    210                 215                 220

Ile Pro Leu Ser Thr Leu Leu Ser Ser Thr Gln Ser Cys His Tyr Ser
225                 230                 235                 240

Ile Asp Ala Lys Lys Lys His Ile Arg Asp Val Val Cys Ser Glu Lys
                245                 250                 255

His Leu Phe Leu Pro Ser Ser Tyr Lys Asn Gln Tyr Gly Met Met Thr
            260                 265                 270

Glu Val Asn Gln Thr Leu Lys Leu Glu Asp Asn Gln Arg Met Asn Asn
        275                 280                 285

Arg Asn Pro Asp Gly Asp Glu Leu Glu Glu Lys Gly Leu Ala Leu Glu
    290                 295                 300

Ser Thr Asp Ala Lys Phe Ser Arg Gln Gly Asp Ala Val Leu Lys Ile
305                 310                 315                 320

Leu Gln Glu Leu Gln Lys Leu Thr Ala Ser Gln Gln Asn Gln Gln Arg
                325                 330                 335

Ala Lys Leu Phe Tyr Lys Phe Val Ser Gly Leu Arg Ser Leu His Asn
            340                 345                 350

Ser Thr Leu Gly Ser Leu Val Pro Lys Met Met Glu Thr Ser Ser Ser
        355                 360                 365

Ile Thr Ile Gln Ala Leu Ile Gln Cys Gly Thr Pro Glu Cys Tyr Ser
    370                 375                 380

Ala Val Leu Gln Ile Leu Arg Thr Gly Asn Val Asn Pro Leu Val Val
385                 390                 395                 400

Asp Leu Val Thr Tyr Thr Leu Gly Leu Leu Pro Ser Pro Thr Pro Lys
                405                 410                 415

Arg Ile Arg Glu Ile Leu Asn Met Ala Gln Tyr Gln Pro Ser Arg Ala
            420                 425                 430

Ser Phe Tyr Gly Leu Ser His Ala Val Thr Lys Phe Tyr Asn Glu Lys
        435                 440                 445

Met Ile Val Thr Glu Glu Ile Thr Asp Val Ala Asp Phe Met Val Ser
    450                 455                 460

Leu Leu Gly Thr Asp Cys Ser Gly Asp Ala Glu Leu Thr Tyr Leu Thr
465                 470                 475                 480

Leu Arg Ala Ile Gly Asn Met Gly Ala Val Met Glu Lys Ala Lys Pro
                485                 490                 495

Ser Leu Lys Ala Ser Leu Lys Thr Cys Ile Arg Asn Gln Ala Ala Ser
            500                 505                 510

Leu Ser Val Gln Lys Ala Ala Ile Gln Ala Phe Arg Lys Met Thr Ile
        515                 520                 525

Thr Glu Glu Asp Arg Ser Ala Leu Leu Lys Glu Phe Gln Glu Gly Asp
    530                 535                 540

Ala Pro Thr Asp Lys Arg Leu Ala Thr Tyr Leu Ile Leu Met Lys Asn
545                 550                 555                 560

Pro Ser Pro Ala Asp Leu Ala Lys Ile Met Arg Ile Leu Thr Arg Glu
                565                 570                 575

Lys Asn Glu Gln Val Lys Ser Phe Val Ala Ser His Ile Ala Asn Ile
            580                 585                 590

Leu Asp Ser Asp Glu Val Gly Ile Glu Asp Leu Lys Ser His Val Glu
        595                 600                 605

Glu Ala Leu Lys Gly Asn Glu Val Pro Thr Ala Lys Asp Phe Arg Lys
    610                 615                 620

Phe Ser Gln Asn Tyr Gln Val Ser Lys Arg Val Ser Val Pro Gly Leu
625                 630                 635                 640

Asn Pro Ile Ser Ala Lys Val Glu Gly Asn Val Ile Phe Asp Pro Ser
                645                 650                 655

Ser Tyr Val Pro Lys Glu Thr Met Leu Lys Thr Thr Leu Asn Leu Tyr
            660                 665                 670

Gly Phe Gly Pro Ser Asp Ile Phe Glu Leu Gly Leu Asp Gly Lys Gly
        675                 680                 685

Phe Glu Pro Thr Leu Glu Ala Leu Phe Gly Glu Lys Gly Phe Phe Pro
    690                 695                 700

Asp Thr Ala Ser Lys Ala Leu Tyr Trp Val Asp Gly Lys Val Pro Glu
705                 710                 715                 720

Gln Val Ser Lys Ala Leu Phe Asp Tyr Phe Gly Tyr Ser His Asp Gly
                725                 730                 735

Lys Gln Asp Gln Glu Ile Thr Lys Ala Val Ile Leu Asn Leu Glu Lys
            740                 745                 750

Leu Ile Lys Glu Leu Ser Lys Lys Glu Ala Pro Glu Gly Arg Ala Phe
        755                 760                 765

Leu Arg Ile Leu Gly Glu Glu Leu Gly Tyr Met Lys Leu Ser Asp Phe
    770                 775                 780

Lys Leu Leu Gly Ser Val Ala Leu Glu Cys Ile Lys Thr Leu Gln Arg
785                 790                 795                 800

Ile Pro Glu Ile Ile Ala Gln Ala Ile Ser Lys Gly Val Asp Lys Asp
                805                 810                 815

Leu Phe Val His Tyr Met Phe Met Asp Asn Glu Phe Glu Leu Pro Thr
            820                 825                 830

Gly Ala Gly Leu Gln Leu Lys Phe Ala Leu Ser Gly Ile Val Thr Pro
        835                 840                 845

Gly Ala Lys Val Ala Val Lys Leu His Gln Lys Ser Met Gln Ala Glu
    850                 855                 860

Leu Ile Ala Lys Pro Ser Val Ala Val Glu Phe Val Thr His Leu Gly
865                 870                 875                 880

Ile Asn Met Pro Glu Phe Ala Arg Ser Gly Val Glu Met Asn Ser Asn
                885                 890                 895

Ile Phe His Glu Ser Gly Ile Glu Ala His Val Ser Val Lys Ala Gly
            900                 905                 910

Gln Leu Lys Phe Ser Ile Pro Ala Pro Lys Thr Pro Thr Lys Leu Leu
        915                 920                 925

Ser Ile Ser Asn Thr Leu His Leu Val Ser Pro Ala Lys Thr Glu Glu
    930                 935                 940

Ile Pro Pro Leu Ile Glu Asn Arg Glu Phe Ser Thr Ser Cys Lys Pro
945                 950                 955                 960

Phe Ile Ser Gly Leu Asn Phe Cys Thr Lys Leu Leu Tyr Ser Asn Ala
                965                 970                 975

Ser Ser Met Glu Ala Ala Pro Tyr Tyr Pro Leu Thr Gly Glu Thr Arg
            980                 985                 990

Phe Glu Val Glu Ile Val Ser Thr  Gly Glu Val Lys Glu  Tyr Ser Ala
        995                 1000                 1005

Ser Ala  Asn Tyr Asp Leu Gln  Arg Glu Gly Thr Asp  Leu Val Asp
    1010                 1015                 1020

Thr Leu  Lys Phe Ala Val Gln  Ala Glu Gly Val Lys  Gln His Glu
    1025                 1030                 1035

Ala Thr  Leu Thr Phe Lys Tyr  Asn Arg Asp Arg Lys  Ile Leu Thr
    1040                 1045                 1050

Ser Asp  Val Ser Ile Pro Asp  Val Asp Val Asp Phe  Gly Thr Asn
    1055                 1060                 1065

Phe Arg  Ile Thr Asp Glu Ser  Val Ser Gly Lys Lys  Ala Tyr Ile
    1070                 1075                 1080

Phe Val  Ile Asp Phe Asn Asn  Lys Lys Ile Ser Glu  Val Thr Leu
    1085                 1090                 1095

Thr Gly  Gln Ile Arg Tyr Ala  Gly Ile Glu Glu Ala  Met Leu Arg
    1100                 1105                 1110

Gly Thr  Val Ser Ile Pro Arg  Leu Gln Thr Glu Leu  Lys Thr Glu
    1115                 1120                 1125

Ala Leu  Val Asn Tyr Ser Pro  Thr Lys Gly Tyr Leu  Gln Met Ser
    1130                 1135                 1140

Ser Ser  Val Gly Thr His Gly  Asn Thr Val Ser Lys  Arg Val Leu
    1145                 1150                 1155

Leu Arg  Tyr Asp Ser Glu Lys  Ala Glu Leu Glu Trp  Asn Ser Gly
    1160                 1165                 1170

Ala Thr  Ala Ala Val Gly Arg  Met Ser Ser Ala Phe  Gln Val Asp

1175                1180                1185

Phe Ser Asp Tyr Ser Lys Thr Leu Glu Lys Tyr Ala Asn Glu Leu
    1190                1195                1200

Leu Asp Arg Lys Val Ala Leu Thr Asp Met Thr Met Arg His Ile
    1205                1210                1215

Val Ser Gln Phe Ile Val Ala Thr Asn Thr Trp Leu Gln Lys Ala
    1220                1225                1230

Ser Lys Asp Val Pro Tyr Ala Gln Thr Leu Gln Ala Lys Leu Ser
    1235                1240                1245

Gly Leu Gln Glu Leu Asn Ile Gln Lys Ile Lys Leu Pro Val Ile
    1250                1255                1260

Thr Ile Pro Glu Glu Leu Phe Leu Lys Ser Glu Gly Arg Ile Lys
    1265                1270                1275

Tyr Ser Phe Asn Lys Asn Ser Phe Leu Ile Asn Ile Pro Leu Pro
    1280                1285                1290

Phe Gly Gly Arg Ser Ser His Asp Ile Arg Val Pro Gln Thr Val
    1295                1300                1305

Lys Thr Pro Arg Leu Val Ile Glu Ser Met Gly Ile Asn Ile Pro
    1310                1315                1320

Ser Gln Glu Tyr Arg Met Pro Thr Phe Thr Val Pro Glu Ser Tyr
    1325                1330                1335

Pro Leu Leu Val Pro Leu Phe Gly Ala Leu Glu Ala Ser Ala Ser
    1340                1345                1350

Val His Ser Asn Tyr Tyr Asn Trp Thr Ala Ala Tyr Thr Leu Thr
    1355                1360                1365

Asn Ser Ser Thr Glu Lys Thr Ala Arg Ile Gly Thr Thr Tyr Ala
    1370                1375                1380

Val Asn Ala Asp Ser Val Phe Glu Leu Leu Ser Tyr Asn Met Lys
    1385                1390                1395

Gly Ser Gly Glu Ala Ser Ser Ser Arg Asn Gly Phe Thr Cys Ala
    1400                1405                1410

Tyr Glu Asn His Leu Lys His Arg Leu Leu Thr Ser Asp Phe Lys
    1415                1420                1425

Met Ser Arg Thr Lys Ser Tyr Glu Pro Thr Ser Val Ser Asn Cys
    1430                1435                1440

Thr Ile Phe Leu Met Ala Ser Ser Ala Leu Gly Pro Gln Leu Ser
    1445                1450                1455

Phe Ser Ser Asp Val Val Ser Glu Lys Thr Asn Asn Met Asn Ile
    1460                1465                1470

Asn Asn Val Arg Ile Glu Gly Gln Leu Glu Val Ala Ser Val Phe
    1475                1480                1485

Ala Arg Ser Val Tyr Thr Met Ser Ser Ser Tyr Asn Glu Lys Arg
    1490                1495                1500

Arg Val Leu Glu Gly Lys Ser Asn Leu Arg Leu Asp Ser Ser Tyr
    1505                1510                1515

Leu Gln Ala Thr Asn His Leu Ser Gly Arg Tyr Thr Asp Gly Val
    1520                1525                1530

Phe Ser Ile Thr Ser Ala Ser Asp Val Gln Asn Gly Leu Leu Lys
    1535                1540                1545

Asn Thr Ala Ser Leu Lys Tyr Glu Asn Ser Gln Leu Lys Ile Thr
    1550                1555                1560

Ser Glu Thr Asn Gly Arg Tyr Leu His Leu Ala Ala Val Asn Lys
    1565                1570                1575

Leu Glu Phe Leu Leu Ser Lys Lys Met Ala Ala Leu Arg Ser Glu
    1580                1585                1590

Tyr Gln Ala Thr Tyr Lys Gln Thr Gln Cys Tyr Ala Leu Phe Ala
    1595                1600                1605

Gly Ser Leu Asn Ser Gln Asp Leu Val Phe Asn Thr Asp Phe Ser
    1610                1615                1620

Leu Thr Asp Gln Arg Asn Arg Ala Ala His Lys Ser Ser Leu Asn
    1625                1630                1635

Val Asn Gln Tyr Gly Leu Ala Ser Ser Ala Thr Thr Asn Val Gln
    1640                1645                1650

Phe Ser Pro Leu Thr Met Gln Ser Glu Met Asn Ala Lys Leu Asp
    1655                1660                1665

Thr Ser Gly Gly Ser Val Ser Leu Ser Ser Ser Gly Arg Tyr Gly
    1670                1675                1680

Lys Asn Asn Ala Lys Phe Asn Val Gly Gly Arg Val Ser Leu Thr
    1685                1690                1695

Glu Ile Thr Leu Gly Ser Glu Tyr Gln Ser Thr Ile Leu Gly Met
    1700                1705                1710

Asp Asn Lys His Val Leu Asn Phe Arg Ile Asn Lys Glu Gly Leu
    1715                1720                1725

Lys Phe Ser Asn Asn Leu Gln Gly Ser Leu Lys Glu Ile Lys Leu
    1730                1735                1740

Glu Tyr Thr Asn Asp Leu Asn Ile Pro Gly Leu Ser Leu Thr Phe
    1745                1750                1755

Val Ser Lys Leu Asp Asn Ser Phe Ser Phe Asp Lys Phe His Lys
    1760                1765                1770

His Val Phe Asp Leu Gln Leu Gln Pro Arg Ser Leu Thr Ala Lys
    1775                1780                1785

Leu Asn Asn Asn Ile Lys Tyr Thr Lys Thr Glu Val Ser Asn Lys
    1790                1795                1800

Ala Glu Leu Leu Leu Glu Pro Leu Lys Leu Asn Leu Gly Gly Asn
    1805                1810                1815

Val Arg Ala Ala Tyr Gly Thr Asp Glu Val Arg His Thr Tyr Ala
    1820                1825                1830

Ile Thr Tyr Ala Asp Leu Thr Ala Asn Phe Lys Thr Asp Thr Val
    1835                1840                1845

Ala Asn Val Gln Gly Ala Ala Val Ser His Arg Val Asn Leu Asn
    1850                1855                1860

Val Ala Gly Leu Ala Ser Ser Ile Thr Met Asn Thr Asn Cys Asp
    1865                1870                1875

Ser Lys Ser Leu Arg Phe Ser Asn Ala Leu Arg Ser Thr Met Ala
    1880                1885                1890

Pro Phe Thr Ile Thr Ala Asp Val His Thr Asn Gly Asn Gly Lys
    1895                1900                1905

Leu Ile Ala Leu Gly Glu His Thr Gly Asp Leu Tyr Ser Lys Ile
    1910                1915                1920

Leu Phe Lys Ala Glu Pro Leu Ala Phe Thr Phe Ser His Asp Tyr
    1925                1930                1935

Arg Gly Ser Thr Ser His Ser Phe Lys Ser Met Arg Arg Tyr Ser
    1940                1945                1950

Thr Gln Leu Asp Asn Lys Phe His Met Leu Phe Thr Pro Ser Glu
    1955                1960                1965

Gln Ser Ser Ala Trp Lys Leu Lys Ser Gln Leu Asn Asn Asn Ile
    1970                1975                1980

Tyr Ser Gln Asp Ile Asn Ala Tyr Asn Asp Ala Glu Lys Ile Gly
    1985                1990                1995

Val Glu Leu Ser Gly Arg Ala Leu Ala Asp Leu Ser Val Val Asp
    2000                2005                2010

Thr Ala Ile Arg Leu Pro Phe Met Ser Glu Glu Val Asn Val Ile
    2015                2020                2025

Asp Val Leu Gly Leu Arg Asp Ser Val Ser Glu Pro Gln Glu Phe
    2030                2035                2040

Ser Ile Ser Gly Ser Val Lys Tyr Asp Lys Asn Lys Asp Met His
    2045                2050                2055

Val Ile Asn Leu Pro Phe Leu Glu His Phe Pro Val Tyr Phe Glu
    2060                2065                2070

Gln Ile Arg Gly Ala Ile Leu Ser Thr Leu Gln Ala Val Gln Asn
    2075                2080                2085

Tyr Leu Lys Asn Ile Asp Val Asp Gln Tyr Met Lys Lys Tyr Lys
    2090                2095                2100

Ala Thr Leu Asp Glu Phe Pro Gln His Leu Asn Asp Tyr Met Asp
    2105                2110                2115

Lys Leu Asp Leu Lys Gly Arg Ala Ser Thr Ile Lys Thr Asn Leu

2120                2125                2130

Ile Ala Phe Thr Lys Asp Tyr Arg Ile Thr Ser Asp Asp Leu Glu
    2135                2140                2145

Ile Ile Leu Glu Lys Ala Leu Asp Asn Leu Gln Glu Ile Leu Leu
    2150                2155                2160

Gln Leu Gln Val Tyr Leu Val Gln Ile Glu Gln Tyr Ile Lys Glu
    2165                2170                2175

Asn Tyr Asp Gln Phe Asp Ile Asn Ala Leu Ile Ala Gln Leu Leu
    2180                2185                2190

Asp Lys Ile Val Glu Lys Met Thr Ala Leu Asp Glu Lys Tyr Lys
    2195                2200                2205

Ile Arg Val Thr Val Val Asp Thr Ile Gln Lys Leu Gln Phe Phe
    2210                2215                2220

Leu Asn Gln Tyr Tyr Pro Ser Asn Ile Gly Ser Asn Thr Met Thr
    2225                2230                2235

Trp Ile Lys Asn Ile Asp Asp Glu Tyr Arg Ile Thr Gly Arg Ile
    2240                2245                2250

Lys Glu Asn Leu Glu Gln Leu Lys Ile Gln Ile Gln Asn Ile Asp
    2255                2260                2265

Ile Arg Ser Phe Ala Glu Asn Leu Lys Arg Lys Ile Lys Ile Ile
    2270                2275                2280

Asp Val Lys Gln Leu Leu Glu Lys Leu Lys Arg Ser Leu Pro Ile
    2285                2290                2295

Lys Lys Met Lys Glu Val Leu Glu Gln Ile Lys Asp Phe Ile Leu
    2300                2305                2310

Ser Trp Met Glu Glu Tyr Glu Val Ser Glu Lys Ile Ser Ala Phe
    2315                2320                2325

Arg Gly His Met His Lys Leu Ile Val Lys Tyr Glu Ile Asp Lys
    2330                2335                2340

His Val Tyr Phe Leu Leu Asp Arg Met Ile Glu Leu Leu Asn Gln
    2345                2350                2355

Tyr Arg Ile Arg Glu Thr Val Arg Lys Met Thr Thr Tyr Leu Arg
    2360                2365                2370

Lys Ile Asp Val Lys Thr Cys Phe Asp Lys Ile Val Ser Leu Ile
    2375                2380                2385

Asp Asp Ala Val Lys Lys Val Gln Thr Phe Asp Tyr Glu Met Met
    2390                2395                2400

Ile Lys Lys Leu Asn Lys Phe Leu Asp Met Ile Ile Lys Lys Leu
    2405                2410                2415

Lys Ser Phe Asp Tyr Asn Gln Phe Val Asp Asp Thr Asn Asn Lys
    2420                2425                2430

Ile Gln Glu Ile Ile Gln Lys Ile Asn Glu Glu Leu Arg Asn Leu
    2435                2440                2445

Glu Leu Pro Gln Lys Ala Glu Ala Leu Lys Gln Tyr Met Arg Asp
    2450                2455                2460

Phe Asn Ala Val Val Ser Lys Tyr Val Glu Gln Leu Arg Asp Thr
    2465                2470                2475

Lys Leu Val Ala Ile Ile Asn Trp Leu Lys Glu Leu Ile Asp Ser
    2480                2485                2490

Thr Thr Phe Thr Asn Leu Lys Ala Lys Val Asn Glu His Leu Glu
    2495                2500                2505

Gly Leu Arg Glu Arg Ile Ser Asp Met Asp Ile Ala Lys Glu Phe
    2510                2515                2520

Glu Trp Tyr Leu Gln Lys Ile Ser Gln Phe Tyr Asn Ser Val Val
    2525                2530                2535

Ile Tyr Ile Ser Glu Gln Trp Asn Ile Ala Phe Lys Lys Ile Val
    2540                2545                2550

Thr Leu Ala Glu Lys Tyr Asp Leu Lys Asn Trp Ala Glu Asn Leu
    2555                2560                2565

Asn Gln Phe Ile Glu Thr Gly Phe Lys Val Pro Glu Ile Arg Thr
    2570                2575                2580

Val Ile Val Thr Ile Pro Ala Phe Glu Phe Ser Leu Arg Ser Leu
    2585                2590                2595

Arg Glu Ala Thr Phe Arg Thr Pro Asp Phe Ile Val Pro Leu Thr
    2600                2605                2610

Asp Leu Lys Ile Pro Ser Tyr Glu Ile Asn Ile Arg
    2615                2620                2625

<210> 153
<211> 9
<212> PRT
<213> Gallus gallus

<400> 153

Ala Ala Tyr Gly Thr Asp Glu Val Arg
1               5

<210> 154
<211> 15
<212> PRT
<213> Gallus gallus

<400> 154

Ala Glu Leu Glu Trp Asn Ser Gly Ala Thr Ala Ala Val Gly Arg
1               5                   10                  15

<210> 155
<211> 9
<212> PRT
<213> Gallus gallus

<400> 155

Ala Glu Leu Leu Leu Glu Pro Leu Lys
1               5

<210> 156
<211> 8
<212> PRT
<213> Gallus gallus

<400> 156

Ala Glu Pro Leu Ala Phe Thr Phe
1               5

<210> 157
<211> 12
<212> PRT
<213> Gallus gallus

<400> 157

Ala Ile Thr Tyr Ala Asp Leu Thr Ala Asn Phe Lys
1               5                   10

<210>  158
<211>  13
<212>  PRT
<213>  Gallus gallus

<400>  158

Ala Leu Ala Asp Leu Ser Val Val Asp Thr Ala Ile Arg
1               5                   10


<210>  159
<211>  11
<212>  PRT
<213>  Gallus gallus

<400>  159

Ala Leu Ser Gly Ile Val Thr Pro Gly Ala Lys
1               5                   10


<210>  160
<211>  12
<212>  PRT
<213>  Gallus gallus

<400>  160

Ala Ser Phe Tyr Gly Leu Ser His Ala Val Thr Lys
1               5                   10


<210>  161
<211>  8
<212>  PRT
<213>  Gallus gallus

<400>  161

Ala Thr Leu Asp Glu Phe Pro Gln
1               5


<210>  162
<211>  8
<212>  PRT
<213>  Gallus gallus

<400>  162

Ala Val Ile Leu Asn Leu Glu Lys
1               5


<210>  163
<211>  11
<212>  PRT
<213>  Gallus gallus

<400>  163

Ala Tyr Thr Phe Val Ile Asp Phe Asn Asn Lys
1               5                   10

<210>  164
<211>  10
<212>  PRT
<213>  Gallus gallus

<400>  164

Asp Glu Pro Leu Asn Val Leu Asn Leu Lys
1               5                   10

<210>  165
<211>  8
<212>  PRT
<213>  Gallus gallus

<400>  165

Asp Phe Asn Ala Val Val Ser Lys
1               5

<210>  166
<211>  7
<212>  PRT
<213>  Gallus gallus

<400>  166

Asp Leu Gln Leu Gln Pro Arg
1               5

<210>  167
<211>  16
<212>  PRT
<213>  Gallus gallus

<400>  167

Asp Ser Val Ser Glu Pro Gln Glu Phe Ser Ile Ser Gly Ser Val Lys
1               5                   10                  15

<210>  168
<211>  11
<212>  PRT
<213>  Gallus gallus

<400>  168

Asp Val Pro Tyr Ala Gln Thr Leu Gln Ala Lys
1               5                   10

<210>  169
<211>  10
<212>  PRT
<213>  Gallus gallus

<400>  169

Asp Tyr Val Ser Pro Val Ala Ile Val Lys
1               5                   10


<210>  170
<211>  11
<212>  PRT
<213>  Gallus gallus

<400>  170

Glu Phe Gln Glu Gly Asp Ala Pro Thr Asp Lys
1               5                   10


<210>  171
<211>  10
<212>  PRT
<213>  Gallus gallus

<400>  171

Glu Gly Thr Asp Leu Val Asp Thr Leu Lys
1               5                   10


<210>  172
<211>  14
<212>  PRT
<213>  Gallus gallus

<400>  172

Glu Leu Glu Trp Asn Ser Gly Ala Thr Ala Ala Val Gly Arg
1               5                   10


<210>  173
<211>  9
<212>  PRT
<213>  Gallus gallus

<400>  173

Glu Thr Phe Gly Val Asp Ser Glu Arg
1               5


<210>  174
<211>  12
<212>  PRT
<213>  Gallus gallus

<400>  174

Glu Tyr Ser Ala Ser Ala Asn Tyr Asp Leu Gln Arg
1               5                   10


<210>  175

<211>  12
<212>  PRT
<213>  Gallus gallus

<400>  175

Phe Ala Leu Ser Gly Ile Val Thr Pro Gly Ala Lys
1               5                   10


<210>  176
<211>  9
<212>  PRT
<213>  Gallus gallus

<400>  176

Phe Ala Val Gln Ala Glu Gly Val Lys
1               5


<210>  177
<211>  12
<212>  PRT
<213>  Gallus gallus

<400>  177

Phe Glu Val Glu Ile Val Ser Thr Gly Glu Val Lys
1               5                   10


<210>  178
<211>  10
<212>  PRT
<213>  Gallus gallus

<400>  178

Phe Ser Asn Asn Leu Gln Gly Ser Leu Lys
1               5                   10


<210>  179
<211>  9
<212>  PRT
<213>  Gallus gallus

<400>  179

Phe Ser Gln Asn Tyr Gln Val Ser Lys
1               5


<210>  180
<211>  13
<212>  PRT
<213>  Gallus gallus

<400>  180

Gly Phe Glu Pro Thr Leu Glu Ala Leu Phe Gly Glu Lys
1               5                   10

<210>  181
<211>  9
<212>  PRT
<213>  Gallus gallus

<400>  181

Gly Phe Phe Pro Asp Thr Ala Ser Lys
1               5

<210>  182
<211>  17
<212>  PRT
<213>  Gallus gallus

<400>  182

Gly Ile Ile Ser Ala Leu Leu Ala Pro Thr Glu Thr Glu Glu Asn Ile
1               5                   10                  15

Lys

<210>  183
<211>  10
<212>  PRT
<213>  Gallus gallus

<400>  183

Gly Leu Ala Leu Glu Ser Thr Asp Ala Lys
1               5                   10

<210>  184
<211>  11
<212>  PRT
<213>  Gallus gallus

<400>  184

Gly Ser Val Ala Glu Asp Ile Ser Ile Asn Arg
1               5                   10

<210>  185
<211>  13
<212>  PRT
<213>  Gallus gallus

<400>  185

Ile Glu Gly Gln Leu Glu Val Ala Ser Val Phe Ala Arg
1               5                   10

<210>  186
<211>  8

<212>  PRT
<213>  Gallus gallus

<400>  186

Ile Gly Val Glu Leu Ser Gly Arg
1               5


<210>  187
<211>  15
<212>  PRT
<213>  Gallus gallus

<400>  187

Ile Lys Leu Pro Val Ile Thr Ile Pro Glu Glu Leu Phe Leu Lys
1               5                   10                  15


<210>  188
<211>  7
<212>  PRT
<213>  Gallus gallus

<400>  188

Ile Leu Gln Glu Leu Gln Lys
1               5


<210>  189
<211>  20
<212>  PRT
<213>  Gallus gallus

<400>  189

Ile Leu Thr Ser Asp Val Ser Ile Pro Asp Val Asp Val Asp Phe Gly
1               5                   10                  15


Thr Asn Phe Arg
            20


<210>  190
<211>  11
<212>  PRT
<213>  Gallus gallus

<400>  190

Ile Pro Glu Ile Ile Ala Gln Ala Ile Ser Lys
1               5                   10


<210>  191
<211>  9
<212>  PRT
<213>  Gallus gallus

<400>  191

Ile Pro Ser Tyr Glu Ile Asn Ile Arg
1               5

<210>  192
<211>  11
<212>  PRT
<213>  Gallus gallus

<400>  192

Ile Ser Glu Val Thr Leu Thr Gly Gln Ile Arg
1               5                   10

<210>  193
<211>  9
<212>  PRT
<213>  Gallus gallus

<400>  193

Ile Thr Asp Glu Ser Val Ser Gly Lys
1               5

<210>  194
<211>  10
<212>  PRT
<213>  Gallus gallus

<400>  194

Lys Phe Ser Gln Asn Tyr Gln Val Ser Lys
1               5                   10

<210>  195
<211>  12
<212>  PRT
<213>  Gallus gallus

<400>  195

Lys Ile Ser Glu Val Thr Leu Thr Gly Gln Ile Arg
1               5                   10

<210>  196
<211>  11
<212>  PRT
<213>  Gallus gallus

<400>  196

Leu Ala Pro Thr Glu Thr Glu Glu Asn Ile Lys
1               5                   10

<210>  197
<211>  9
<212>  PRT

<213>  Gallus gallus

<400>  197

Leu Asp Asn Ser Phe Ser Phe Asp Lys
1               5


<210>  198
<211>  15
<212>  PRT
<213>  Gallus gallus

<400>  198

Leu Asp Ser Ser Tyr Leu Gln Ala Thr Asn His Leu Ser Gly Arg
1               5                   10                  15


<210>  199
<211>  15
<212>  PRT
<213>  Gallus gallus

<400>  199

Leu Asp Thr Ser Gly Gly Ser Val Ser Leu Ser Ser Ser Gly Arg
1               5                   10                  15


<210>  200
<211>  19
<212>  PRT
<213>  Gallus gallus

<400>  200

Leu Glu Tyr Thr Asn Asp Leu Asn Ile Pro Gly Leu Ser Leu Thr Phe
1               5                   10                  15


Val Ser Lys


<210>  201
<211>  7
<212>  PRT
<213>  Gallus gallus

<400>  201

Leu Leu Thr Ser Asp Phe Lys
1               5


<210>  202
<211>  8
<212>  PRT
<213>  Gallus gallus

<400>  202

Leu Asn Leu Gly Gly Asn Val Arg
1               5

<210>  203
<211>  13
<212>  PRT
<213>  Gallus gallus

<400>  203

Leu Pro Val Ile Thr Ile Pro Glu Glu Leu Phe Leu Lys
1               5                   10

<210>  204
<211>  11
<212>  PRT
<213>  Gallus gallus

<400>  204

Leu Ser Phe Ser Ser Asp Val Val Ser Glu Lys
1               5                   10

<210>  205
<211>  11
<212>  PRT
<213>  Gallus gallus

<400>  205

Leu Ser Gly Leu Gln Glu Leu Asn Ile Gln Lys
1               5                   10

<210>  206
<211>  17
<212>  PRT
<213>  Gallus gallus

<400>  206

Leu Tyr Gly Phe Gly Pro Ser Asp Ile Phe Glu Leu Gly Leu Asp Gly
1               5                   10                  15

Lys

<210>  207
<211>  15
<212>  PRT
<213>  Gallus gallus

<400>  207

Leu Tyr Pro Glu Lys Asp Glu Pro Leu Asn Val Leu Asn Leu Lys
1               5                   10                  15

<210>  208
<211>  7
<212>  PRT
<213>  Gallus gallus

<400>  208

Asn Ile Asp Asp Glu Tyr Arg
1               5


<210>  209
<211>  10
<212>  PRT
<213>  Gallus gallus

<400>  209

Asn Pro Asp Gly Asp Glu Leu Glu Glu Lys
1               5                   10


<210>  210
<211>  9
<212>  PRT
<213>  Gallus gallus

<400>  210

Asn Pro Ser Pro Ala Asp Leu Ala Lys
1               5


<210>  211
<211>  10
<212>  PRT
<213>  Gallus gallus

<400>  211

Asn Gln Ala Ala Ser Leu Ser Val Gln Lys
1               5                   10


<210>  212
<211>  14
<212>  PRT
<213>  Gallus gallus

<400>  212

Asn Ser Phe Leu Ile Asn Ile Pro Leu Pro Phe Gly Gly Arg
1               5                   10


<210>  213
<211>  9
<212>  PRT
<213>  Gallus gallus

<400>  213

Gln Val Asp Phe Ser Asp Tyr Ser Lys

1               5


<210>  214
<211>  12
<212>  PRT
<213>  Gallus gallus

<400>  214

Arg Gly Ser Val Ala Glu Asp Ile Ser Ile Asn Arg
1               5                   10


<210>  215
<211>  13
<212>  PRT
<213>  Gallus gallus

<400>  215

Arg Val Ser Val Pro Gly Leu Asn Pro Ile Ser Ala Lys
1               5                   10


<210>  216
<211>  10
<212>  PRT
<213>  Gallus gallus

<400>  216

Ser Ala Ser Ala Asn Tyr Asp Leu Gln Arg
1               5                   10


<210>  217
<211>  8
<212>  PRT
<213>  Gallus gallus

<400>  217

Ser Glu Tyr Gln Ala Thr Tyr Lys
1               5


<210>  218
<211>  9
<212>  PRT
<213>  Gallus gallus

<400>  218

Ser Phe Val Ala Ser His Ile Ala Asn
1               5


<210>  219
<211>  9
<212>  PRT
<213>  Gallus gallus

<400>  219

Ser Gly Ile Val Thr Pro Gly Ala Lys
1               5


<210>  220
<211>  12
<212>  PRT
<213>  Gallus gallus

<400>  220

Ser Gln Asp Ile Asn Ala Tyr Asn Asp Ala Glu Lys
1               5                   10


<210>  221
<211>  20
<212>  PRT
<213>  Gallus gallus

<400>  221

Ser Gln Leu Asn Asn Asn Ile Tyr Ser Gln Asp Ile Asn Ala Tyr Asn
1               5                   10                  15


Asp Ala Glu Lys
            20


<210>  222
<211>  15
<212>  PRT
<213>  Gallus gallus

<400>  222

Thr Asp Thr Val Ala Asn Val Gln Gly Ala Ala Val Ser His Arg
1               5                   10                  15


<210>  223
<211>  11
<212>  PRT
<213>  Gallus gallus

<400>  223

Thr Glu Ala Leu Val Asn Tyr Ser Pro Thr Lys
1               5                   10


<210>  224
<211>  11
<212>  PRT
<213>  Gallus gallus

<400>  224

Thr Glu Glu Ile Pro Pro Leu Ile Glu Asn Arg
1               5                   10

<210>  225
<211>  14
<212>  PRT
<213>  Gallus gallus

<400>  225

Thr Gly Asn Val Asn Pro Leu Val Val Asp Leu Val Thr Tyr
1               5                   10


<210>  226
<211>  25
<212>  PRT
<213>  Gallus gallus

<400>  226

Thr Gly Asn Val Asn Pro Leu Val Val Asp Leu Val Thr Tyr Thr Leu
1               5                   10                  15


Gly Leu Leu Pro Ser Pro Thr Pro Lys
            20                  25


<210>  227
<211>  12
<212>  PRT
<213>  Gallus gallus

<400>  227

Thr Pro Asp Phe Ile Val Pro Leu Thr Asp Leu Lys
1               5                   10


<210>  228
<211>  21
<212>  PRT
<213>  Gallus gallus

<400>  228

Thr Thr Leu Asn Leu Tyr Gly Phe Gly Pro Ser Asp Ile Phe Glu Leu
1               5                   10                  15


Gly Leu Asp Gly Lys
            20


<210>  229
<211>  15
<212>  PRT
<213>  Gallus gallus

<400>  229

Val Glu Gly Asn Val Ile Phe Asp Pro Ser Ser Tyr Val Pro Lys
1               5                   10                  15

<210>  230
<211>  9
<212>  PRT
<213>  Gallus gallus

<400>  230

Val Phe Asp Leu Gln Leu Gln Pro Arg
1               5


<210>  231
<211>  10
<212>  PRT
<213>  Gallus gallus

<400>  231

Val Asn Leu Asn Val Ala Gly Leu Ala Ser
1               5                   10


<210>  232
<211>  12
<212>  PRT
<213>  Gallus gallus

<400>  232

Val Ser Val Pro Gly Leu Asn Pro Ile Ser Ala Lys
1               5                   10


<210>  233
<211>  9
<212>  PRT
<213>  Gallus gallus

<400>  233

Val Thr Val Val Asp Thr Ile Gln Lys
1               5


<210>  234
<211>  8
<212>  PRT
<213>  Gallus gallus

<400>  234

Tyr Ala Asn Glu Leu Leu Asp Arg
1               5


<210>  235
<211>  8
<212>  PRT
<213>  Gallus gallus

<400>  235

Tyr Ser Thr Gln Leu Asp Asn Lys
1               5

<210>  236
<211>  927
<212>  DNA
<213>  Gallus gallus

<400>  236
atgggccctg tgcggctctt gctgctcttg ctgctgctca gcagtggtgt tctcacacag      60

gaaggaacac ctgaaaatgg aaacccagga tgttcaaaag atgcggcccg atttaaaagc     120

cttagaaaat atgtttactt atatgaagca gaaacatcaa gtgggatcac gggaacagca     180

gattctcgaa gcggttcaaa gatcacctgt aaagttgagt tggaggtacc acagctgtgc     240

cagttcatcc tgaggacaat gcattgctcc ctaagggaga catttggtgt tgacagtgag     300

agaagagcca tgctgaggaa gtcaaagaac tctgatgact ttgcaaatgc catgtccaaa     360

catgagctaa gattcagcac tcaggatgga acaaaagtta aactatatcc agagaaggat     420

gaacctctga atgtcctcaa tctcaagaga ggaattatct cagctctcct tgcaccaaca     480

gaaacagagg aaaacataaa aacaatttcc atggatactg tatatggaaa gtgtgacagt     540

gaggttgagt tcaaatccag aagaggaagt gttgcagaag atatttcaat taacaggaac     600

ctgaaagcct gtgacaactt cagtccaatc agagattatg tcagccctgt tgccattgta     660

aaaggactaa acatcccact atctaccctt ctgagtagca ctcaatcctg tcactacagt     720

attgatgcaa agaaaaagca tatcagagat gttgtctgca gtgaaaaaca cctgtttctg     780

ccttcctcat acaaaaatca gtatggtatg atgacagaag tcaaccagac actgaagctt     840

gaagataatc agaggatgaa taacagaaat cctgatggag atgaactgga agagaaagga     900

cttgcactgg aaagcacaga tgctaaa                                         927

<210>  237
<211>  309
<212>  PRT
<213>  Gallus gallus

<400>  237

Met Gly Pro Val Arg Leu Leu Leu Leu Leu Leu Leu Leu Ser Ser Gly
1               5                   10                  15

Val Leu Thr Gln Glu Gly Thr Pro Glu Asn Gly Asn Pro Gly Cys Ser
            20                  25                  30

Lys Asp Ala Ala Arg Phe Lys Ser Leu Arg Lys Tyr Val Tyr Leu Tyr
        35                  40                  45

Glu Ala Glu Thr Ser Ser Gly Ile Thr Gly Thr Ala Asp Ser Arg Ser
    50                  55                  60

Gly Ser Lys Ile Thr Cys Lys Val Glu Leu Glu Val Pro Gln Leu Cys
65                  70                  75                  80

Gln Phe Ile Leu Arg Thr Met His Cys Ser Leu Arg Glu Thr Phe Gly
                85                  90                  95

Val Asp Ser Glu Arg Arg Ala Met Leu Arg Lys Ser Lys Asn Ser Asp
            100                 105                 110

Asp Phe Ala Asn Ala Met Ser Lys His Glu Leu Arg Phe Ser Thr Gln
        115                 120                 125

Asp Gly Thr Lys Val Lys Leu Tyr Pro Glu Lys Asp Glu Pro Leu Asn
    130                 135                 140

Val Leu Asn Leu Lys Arg Gly Ile Ile Ser Ala Leu Leu Ala Pro Thr
145                 150                 155                 160

Glu Thr Glu Glu Asn Ile Lys Thr Ile Ser Met Asp Thr Val Tyr Gly
                165                 170                 175

Lys Cys Asp Ser Glu Val Glu Phe Lys Ser Arg Arg Gly Ser Val Ala
            180                 185                 190

Glu Asp Ile Ser Ile Asn Arg Asn Leu Lys Ala Cys Asp Asn Phe Ser
        195                 200                 205

Pro Ile Arg Asp Tyr Val Ser Pro Val Ala Ile Val Lys Gly Leu Asn
    210                 215                 220

Ile Pro Leu Ser Thr Leu Leu Ser Ser Thr Gln Ser Cys His Tyr Ser
225                 230                 235                 240

Ile Asp Ala Lys Lys Lys His Ile Arg Asp Val Val Cys Ser Glu Lys
                245                 250                 255

His Leu Phe Leu Pro Ser Ser Tyr Lys Asn Gln Tyr Gly Met Met Thr
            260                 265                 270

Glu Val Asn Gln Thr Leu Lys Leu Glu Asp Asn Gln Arg Met Asn Asn
        275                 280                 285

Arg Asn Pro Asp Gly Asp Glu Leu Glu Glu Lys Gly Leu Ala Leu Glu
    290                 295                 300

Ser Thr Asp Ala Lys
305

<210> 238
<211> 13
<212> PRT
<213> Gallus gallus

<400> 238

Ala Leu Leu Ala Pro Thr Glu Thr Glu Glu Asn Ile Lys
1               5                   10

<210> 239
<211> 10
<212> PRT
<213> Gallus gallus

<400> 239

Asp Tyr Val Ser Pro Val Ala Ile Val Lys
1               5                   10

<210> 240
<211> 9
<212> PRT
<213> Gallus gallus

<400> 240

Glu Thr Phe Gly Val Asp Ser Glu Arg
1               5

<210> 241
<211> 17
<212> PRT
<213> Gallus gallus

<400> 241

Gly Ile Ile Ser Ala Leu Leu Ala Pro Thr Glu Thr Glu Glu Asn Ile
1               5                   10                  15

Lys

<210> 242
<211> 11
<212> PRT
<213> Gallus gallus

<400> 242

Gly Ser Val Ala Glu Asp Ile Ser Ile Asn Arg
1               5                   10

<210>  243
<211>  9
<212>  PRT
<213>  Gallus gallus

<400>  243

His Leu Phe Leu Pro Ser Ser Tyr Lys
1               5

<210>  244
<211>  11
<212>  PRT
<213>  Gallus gallus

<400>  244

Leu Ala Pro Thr Glu Thr Glu Glu Asn Ile Lys
1               5                   10

<210>  245
<211>  8
<212>  PRT
<213>  Gallus gallus

<400>  245

Leu Phe Leu Pro Ser Ser Tyr Lys
1               5

<210>  246
<211>  12
<212>  PRT
<213>  Gallus gallus

<400>  246

Leu Leu Ala Pro Thr Glu Thr Glu Glu Asn Ile Lys
1               5                   10

<210>  247
<211>  15
<212>  PRT
<213>  Gallus gallus

<400>  247

Leu Tyr Pro Glu Lys Asp Glu Pro Leu Asn Val Leu Asn Leu Lys
1               5                   10                  15

<210>  248
<211>  18
<212>  PRT
<213>  Gallus gallus

<400>  248

Arg Gly Ile Ile Ser Ala Leu Leu Ala Pro Thr Glu Thr Glu Glu Asn
1               5                   10                  15

Ile Lys

<210>  249
<211>  10
<212>  PRT
<213>  Gallus gallus

<400>  249

Ser Val Ala Glu Asp Ile Ser Ile Asn Arg
1               5                   10

<210>  250
<211>  1041
<212>  DNA
<213>  Gallus gallus

<400>  250
ctggatggct caaccagttt gacaagaaaa agaggactga agctggccac agcactgtct      60

ctgaataata acaaatttct aggaggaagt catgacaaca gtattagtct cacaaagaag     120

aacctggaag cttcaatgat aacaaatgca aaaatcaaca caccagtttt taaaatgaat     180

ttcagccaag agctttctgg aaatactaaa tctaaaccca ctatttcttc agggctaaaa     240

gtaacatatg actttactac tcctaagcat ggcatcagtg ctaagggagg agttgctcac     300

aaacttgctt tagagactct tacatcttac ctgtcagtag aaacatcaac aaaggggaat     360

atcgatggag caatttacac tggaaattca ttttctggag ctctagacca tgaagcaaac     420

acctatctgc atgctaatgg agttcggtca tctctcaagc ttgaggccaa ctccaaagta     480

gatggactct ggaacagtga aatgaaagaa atacttgcag ttgaagcatc taccagtcgt     540

gtttatgcag tctgggagca caatgggaaa aactttgcac gatacacacc tctcttcaca     600

acgacaggat ctcagaaatg caaagcaacc ttcgagctgg ctccttggac agtgtcagca     660

gatcttcaga ttcaggtcac tcagccaaat tccttcctgg acacagcatc agttaatcaa     720

gttgtcttaa tgaaagtcag tcctacagac cagaaagttg gctggaaggg tgaaggccaa     780

attcagtcat tatctctgag acatgacatg caattatcaa atgaaaaatc aaatgcaaag     840

tttgatattt ctggatcttt ggaagggtac atggacttcc tgaaaagaat taattgtgct     900

atttctaaga agagcttgtg ggatatcttg aagttggatg tcactactgt tgctgacaga     960

aagcactact taaatgcttc agcatccttc atatacagga agagtgatga tggttacttt    1020

ttccctatgc ctgtgattag g                                              1041

<210>  251

<211>  347
<212>  PRT
<213>  Gallus gallus

<400>  251

Leu Asp Gly Ser Thr Ser Leu Thr Arg Lys Arg Gly Leu Lys Leu Ala
1               5                   10                  15

Thr Ala Leu Ser Leu Asn Asn Asn Lys Phe Leu Gly Gly Ser His Asp
            20                  25                  30

Asn Ser Ile Ser Leu Thr Lys Lys Asn Leu Glu Ala Ser Met Ile Thr
        35                  40                  45

Asn Ala Lys Ile Asn Thr Pro Val Phe Lys Met Asn Phe Ser Gln Glu
    50                  55                  60

Leu Ser Gly Asn Thr Lys Ser Lys Pro Thr Ile Ser Ser Gly Leu Lys
65                  70                  75                  80

Val Thr Tyr Asp Phe Thr Thr Pro Lys His Gly Ile Ser Ala Lys Gly
                85                  90                  95

Gly Val Ala His Lys Leu Ala Leu Glu Thr Leu Thr Ser Tyr Leu Ser
            100                 105                 110

Val Glu Thr Ser Thr Lys Gly Asn Ile Asp Gly Ala Ile Tyr Thr Gly
        115                 120                 125

Asn Ser Phe Ser Gly Ala Leu Asp His Glu Ala Asn Thr Tyr Leu His
    130                 135                 140

Ala Asn Gly Val Arg Ser Ser Leu Lys Leu Glu Ala Asn Ser Lys Val
145                 150                 155                 160

Asp Gly Leu Trp Asn Ser Glu Met Lys Glu Ile Leu Ala Val Glu Ala
                165                 170                 175

Ser Thr Ser Arg Val Tyr Ala Val Trp Glu His Asn Gly Lys Asn Phe
            180                 185                 190

Ala Arg Tyr Thr Pro Leu Phe Thr Thr Thr Gly Ser Gln Lys Cys Lys
        195                 200                 205

Ala Thr Phe Glu Leu Ala Pro Trp Thr Val Ser Ala Asp Leu Gln Ile
    210                 215                 220

Gln Val Thr Gln Pro Asn Ser Phe Leu Asp Thr Ala Ser Val Asn Gln

225                 230                 235                 240

Val Val Leu Met Lys Val Ser Pro Thr Asp Gln Lys Val Gly Trp Lys
                245                 250                 255

Gly Glu Gly Gln Ile Gln Ser Leu Ser Leu Arg His Asp Met Gln Leu
            260                 265                 270

Ser Asn Glu Lys Ser Asn Ala Lys Phe Asp Ile Ser Gly Ser Leu Glu
        275                 280                 285

Gly Tyr Met Asp Phe Leu Lys Arg Ile Asn Cys Ala Ile Ser Lys Lys
    290                 295                 300

Ser Leu Trp Asp Ile Leu Lys Leu Asp Val Thr Thr Val Ala Asp Arg
305                 310                 315                 320

Lys His Tyr Leu Asn Ala Ser Ala Ser Phe Ile Tyr Arg Lys Ser Asp
                325                 330                 335

Asp Gly Tyr Phe Phe Pro Met Pro Val Ile Arg
            340                 345

<210>  252
<211>  11
<212>  PRT
<213>  Gallus gallus

<400>  252

Glu Ile Leu Ala Val Glu Ala Ser Thr Ser Arg
1               5                   10

<210>  253
<211>  14
<212>  PRT
<213>  Gallus gallus

<400>  253

Phe Leu Gly Gly Ser His Asp Asn Ser Ile Ser Leu Thr Lys
1               5                   10

<210>  254
<211>  11
<212>  PRT
<213>  Gallus gallus

<400>  254

Gly Glu Gly Gln Ile Gln Ser Leu Ser Leu Arg
1               5                   10

<210>  255
<211>  7
<212>  PRT
<213>  Gallus gallus

<400>  255

Ile Asn Thr Pro Val Phe Lys
1               5


<210>  256
<211>  17
<212>  PRT
<213>  Gallus gallus

<400>  256

Leu Ala Leu Glu Thr Leu Thr Ser Tyr Leu Ser Val Glu Thr Ser Thr
1               5                   10                  15


Lys


<210>  257
<211>  11
<212>  PRT
<213>  Gallus gallus

<400>  257

Leu Ala Thr Ala Leu Ser Leu Asn Asn Asn Lys
1               5                   10


<210>  258
<211>  9
<212>  PRT
<213>  Gallus gallus

<400>  258

Leu Asp Gly Ser Thr Ser Leu Thr Arg
1               5


<210>  259
<211>  8
<212>  PRT
<213>  Gallus gallus

<400>  259

Pro Thr Ile Ser Ser Gly Leu Lys
1               5


<210>  260
<211>  12
<212>  PRT

<213>  Gallus gallus

<400>  260

Tyr Thr Pro Leu Phe Thr Thr Thr Gly Ser Gln Lys
1               5                   10


<210>  261
<211>  741
<212>  DNA
<213>  Gallus gallus

<400>  261
accccagtgt tagctgcttt tctccatggc attagtaaca ataagaagac aggaggcctc      60

cagcttgtgg tatatgctga taccgactcg gtcaggccgc gggtgcaggt atttgtgaca     120

aacctcacag attcgagcaa gtggaagctc tgcgcagatg cttcggtccg caatgctcac     180

aaggcagtgg cctacgtgaa atggggctgg gactgccggg actacaaggt ttctactgag     240

ctggtaactg ggcggtttgc tgggcaccct gctgcacaag tgaagctgga gtggcccaag     300

gttccttcga atgtcagatc agtggttgaa tggttttacg agtttgtccc tggggctgca     360

tttatgctgg gtttctctga gagaatggac aagaatcctt ctcgacaagc caggatggtt     420

gtggctctaa cttctccgag gacatgtgat gttgttgtca agctgcctga tataatcctc     480

tatcaaaaag ccgtgaggct tcctctatca ctccctgtgg gtccaaggat cccagcttca     540

gagctgcagc ctccaatctg gaacgtcttt gctgaagccc cctctgccgt gctcgagaat     600

ttgaaagctc gctgctcagt ttcgtacaac aagatcaaaa cctttaatga agtcaagttc     660

aactactcga tgcccgcaaa ctgctatcac atcttggttc aggattgcag ctctgaactt     720

aagttcctgg tgatgatgaa a                                               741


<210>  262
<211>  247
<212>  PRT
<213>  Gallus gallus

<400>  262

Thr Pro Val Leu Ala Ala Phe Leu His Gly Ile Ser Asn Asn Lys Lys
1               5                   10                  15


Thr Gly Gly Leu Gln Leu Val Val Tyr Ala Asp Thr Asp Ser Val Arg
            20                  25                  30


Pro Arg Val Gln Val Phe Val Thr Asn Leu Thr Asp Ser Ser Lys Trp
        35                  40                  45


Lys Leu Cys Ala Asp Ala Ser Val Arg Asn Ala His Lys Ala Val Ala
    50                  55                  60

Tyr Val Lys Trp Gly Trp Asp Cys Arg Asp Tyr Lys Val Ser Thr Glu
65                  70                  75                  80

Leu Val Thr Gly Arg Phe Ala Gly His Pro Ala Ala Gln Val Lys Leu
                85                  90                  95

Glu Trp Pro Lys Val Pro Ser Asn Val Arg Ser Val Val Glu Trp Phe
            100                 105                 110

Tyr Glu Phe Val Pro Gly Ala Ala Phe Met Leu Gly Phe Ser Glu Arg
        115                 120                 125

Met Asp Lys Asn Pro Ser Arg Gln Ala Arg Met Val Val Ala Leu Thr
    130                 135                 140

Ser Pro Arg Thr Cys Asp Val Val Val Lys Leu Pro Asp Ile Ile Leu
145                 150                 155                 160

Tyr Gln Lys Ala Val Arg Leu Pro Leu Ser Leu Pro Val Gly Pro Arg
                165                 170                 175

Ile Pro Ala Ser Glu Leu Gln Pro Pro Ile Trp Asn Val Phe Ala Glu
            180                 185                 190

Ala Pro Ser Ala Val Leu Glu Asn Leu Lys Ala Arg Cys Ser Val Ser
        195                 200                 205

Tyr Asn Lys Ile Lys Thr Phe Asn Glu Val Lys Phe Asn Tyr Ser Met
    210                 215                 220

Pro Ala Asn Cys Tyr His Ile Leu Val Gln Asp Cys Ser Ser Glu Leu
225                 230                 235                 240

Lys Phe Leu Val Met Met Lys
                245

<210>  263
<211>  10
<212>  PRT
<213>  Gallus gallus

<400>  263

Phe Ala Gly His Pro Ala Ala Gln Val Lys
1               5                   10

<210>  264
<211>  19
<212>  PRT

<213>  Gallus gallus

<400>  264

Lys Thr Gly Gly Leu Gln Leu Val Val Tyr Ala Asp Thr Asp Ser Val
1               5                   10                  15

Arg Pro Arg

<210>  265
<211>  10
<212>  PRT
<213>  Gallus gallus

<400>  265

Leu Pro Leu Ser Leu Pro Val Gly Pro Arg
1               5                   10

<210>  266
<211>  9
<212>  PRT
<213>  Gallus gallus

<400>  266

Thr Gly Gly Leu Gln Leu Val Val Tyr
1               5

<210>  267
<211>  9
<212>  PRT
<213>  Gallus gallus

<400>  267

Ala Asp Thr Asp Ser Val Arg Pro Arg
1               5

<210>  268
<211>  15
<212>  PRT
<213>  Gallus gallus

<400>  268

Thr Pro Val Leu Ala Ala Phe Leu His Gly Ile Ser Asn Asn Lys
1               5                   10                  15

<210>  269
<211>  13
<212>  PRT
<213>  Gallus gallus

<400>  269

Val Gln Val Phe Val Thr Asn Leu Thr Asp Ser Ser Lys
1               5                   10

<210>  270
<211>  9
<212>  PRT
<213>  Gallus gallus

<400>  270

Val Ser Thr Glu Leu Val Thr Gly Arg
1               5

<210>  271
<211>  8
<212>  PRT
<213>  Gallus gallus

<400>  271

Val Val Ala Leu Thr Ser Pro Arg
1               5

<210>  272
<211>  15
<212>  PRT
<213>  Artificial Sequence

<220>
<223>  epitope

<400>  272

Lys Val Arg Ser Pro Gln Val Glu Glu Tyr Asn Gly Val Trp Pro
1               5                   10                  15

<210>  273
<211>  15
<212>  PRT
<213>  Artificial Sequence

<220>
<223>  epitope

<400>  273

Gln Met Thr Ile Lys Lys Ser Gln Asn Val Tyr Glu Leu Gln Glu
1               5                   10                  15

<210>  274
<211>  15
<212>  PRT
<213>  Artificial Sequence

<220>
<223>  epitope

<400>  274

Arg Gly Arg Glu Asp Lys Met Lys Leu Ala Leu Lys Cys Ile Gly
1               5                   10                  15


<210>  275
<211>  15
<212>  PRT
<213>  Artificial Sequence

<220>
<223>  epitope

<400>  275

Ala Leu Lys Lys Ile Ala Trp Lys Asp Pro Lys Thr Val Gln Gly
1               5                   10                  15


<210>  276
<211>  15
<212>  PRT
<213>  Artificial Sequence

<220>
<223>  epitope

<400>  276

Ile Ala Leu Lys Leu Leu Ser Pro Lys Leu Asp Ser Met Ser Tyr
1               5                   10                  15


<210>  277
<211>  15
<212>  PRT
<213>  Artificial Sequence

<220>
<223>  epitope

<400>  277

Val Asn Ser Pro Arg Thr Met Phe Pro Ser Ala Ile Ile Ser Lys
1               5                   10                  15


<210>  278
<211>  15
<212>  PRT
<213>  Artificial Sequence

<220>
<223>  epitope

<400>  278

Ala Ile Ile Ser Lys Leu Met Ala Asn Ser Ala Gly Ser Val Ala
1               5                   10                  15


<210>  279
<211>  15

<212>  PRT
<213>  Artificial Sequence

<220>
<223>  epitope

<400>  279

Gly Lys Ala Leu Gln Gly Trp Lys Glu Leu Pro Thr Glu Thr Pro
1               5                   10                  15


<210>  280
<211>  15
<212>  PRT
<213>  Artificial Sequence

<220>
<223>  epitope

<400>  280

Ile Asn Ile Asn Lys Glu Leu Leu Gln Gln Val Met Lys Thr Val
1               5                   10                  15


<210>  281
<211>  15
<212>  PRT
<213>  Artificial Sequence

<220>
<223>  epitope

<400>  281

Val Met Lys Thr Val Val Glu Pro Ala Asp Arg Asn Ala Ala Ile
1               5                   10                  15


<210>  282
<211>  15
<212>  PRT
<213>  Artificial Sequence

<220>
<223>  epitope

<400>  282

Lys Phe Asp Ala Lys Ile Asp Val Lys Leu Lys Asn Leu Lys Ile
1               5                   10                  15


<210>  283
<211>  15
<212>  PRT
<213>  Artificial Sequence

<220>
<223>  epitope

<400>  283

Phe Ile Gln Asn Thr Tyr Leu His Lys Leu Ile Gly Glu His Glu
1               5                   10                  15

<210>  284
<211>  15
<212>  PRT
<213>  Artificial Sequence

<220>
<223>  epitope

<400>  284

Ser Pro Tyr Glu Asp Ile Gln Ala Lys Leu Lys Arg Ile Leu Gly
1               5                   10                  15

<210>  285
<211>  15
<212>  PRT
<213>  Artificial Sequence

<220>
<223>  epitope

<400>  285

Asn Arg Pro Ser Lys Lys Gly Asn Thr Val Leu Ala Glu Phe Gly
1               5                   10                  15

<210>  286
<211>  15
<212>  PRT
<213>  Artificial Sequence

<220>
<223>  epitope

<400>  286

Ser Ser Ser Ser Ser Ser Ser Ser Ser Asn Ser Lys Ser Ser Ser
1               5                   10                  15

<210>  287
<211>  15
<212>  PRT
<213>  Artificial Sequence

<220>
<223>  epitope

<400>  287

Gln Glu Phe Pro Lys Arg Lys Leu Pro Gly Asp Arg Ala Thr Ser
1               5                   10                  15

<210>  288
<211>  15

<212>  PRT
<213>  Artificial Sequence

<220>
<223>  epitope

<400>  288

Asn Leu Thr Asp Ser Ser Lys Trp Lys Leu Cys Ala Asp Ala Ser
1               5                   10                  15


<210>  289
<211>  15
<212>  PRT
<213>  Artificial Sequence

<220>
<223>  epitope

<400>  289

Tyr Gln Lys Ala Val Arg Leu Pro Leu Ser Leu Pro Val Gly Pro
1               5                   10                  15


<210>  290
<211>  15
<212>  PRT
<213>  Artificial Sequence

<220>
<223>  epitope

<400>  290

Lys Ile Lys Thr Phe Asn Glu Val Lys Phe Asn Tyr Ser Met Pro
1               5                   10                  15


<210>  291
<211>  15
<212>  PRT
<213>  Artificial Sequence

<220>
<223>  epitope

<400>  291

Glu Ala Thr Asn Leu Lys Ala Ile Asn Ile Lys Ile Gly Ser His
1               5                   10                  15


<210>  292
<211>  15
<212>  PRT
<213>  Artificial Sequence

<220>
<223>  epitope

<400>  292

Ala Ala Pro Gly His Gly Ile Asp Lys Leu Tyr Phe Asp Gly Lys
1               5                   10                  15

<210>  293
<211>  15
<212>  PRT
<213>  Artificial Sequence

<220>
<223>  epitope

<400>  293

Glu Gln Glu Tyr Arg Met Pro Asn Gly Tyr Leu Ala Lys Asn Ala
1               5                   10                  15

<210>  294
<211>  15
<212>  PRT
<213>  Artificial Sequence

<220>
<223>  epitope

<400>  294

Gly Ala Cys Lys Leu His Arg Ser Phe Val Lys Leu Glu Lys Thr
1               5                   10                  15

<210>  295
<211>  15
<212>  PRT
<213>  Artificial Sequence

<220>
<223>  epitope

<400>  295

Thr Lys Thr Thr Pro Val Thr Val Gly Phe His Cys Leu Pro Ala
1               5                   10                  15

<210>  296
<211>  15
<212>  PRT
<213>  Artificial Sequence

<220>
<223>  epitope

<400>  296

Ser Pro Arg Thr Cys Asp Val Val Leu Lys Leu Pro Glu Leu Thr
1               5                   10                  15

<210>  297
<211>  15

<212>  PRT
<213>  Artificial Sequence

<220>
<223>  epitope

<400>  297

Ser Gly Leu Leu Ile Ser Ser Glu Lys Glu Gly Leu Ser Leu Lys
1               5                   10                  15


<210>  298
<211>  15
<212>  PRT
<213>  Artificial Sequence

<220>
<223>  epitope

<400>  298

Gly Lys Thr Cys Gly Ile Cys Gly Lys Tyr Asp Ala Glu Lys Lys
1               5                   10                  15


<210>  299
<211>  15
<212>  PRT
<213>  Artificial Sequence

<220>
<223>  epitope

<400>  299

Asp Ala Glu Lys Lys Arg Glu Tyr Gln Met Pro Ser Gly Tyr Leu
1               5                   10                  15


<210>  300
<211>  15
<212>  PRT
<213>  Artificial Sequence

<220>
<223>  epitope

<400>  300

Pro Ser Gly Tyr Leu Ala Lys Asp Ala Val Ser Phe Ala Gln Ser
1               5                   10                  15


<210>  301
<211>  15
<212>  PRT
<213>  Artificial Sequence

<220>
<223>  epitope

<400>  301

Asp Thr Cys Thr Gly Ala Cys Lys Leu Gln Arg Lys Phe Val Lys
1               5                   10                  15

<210>  302
<211>  15
<212>  PRT
<213>  Artificial Sequence

<220>
<223>  epitope

<400>  302

Arg Lys Phe Val Lys Ile Glu Lys Pro Val Ala Phe Asn Lys Lys
1               5                   10                  15

<210>  303
<211>  15
<212>  PRT
<213>  Artificial Sequence

<220>
<223>  epitope

<400>  303

Gly Cys Ser Ala Thr Arg Thr Val Pro Val Ser Val Gly Phe His
1               5                   10                  15

<210>  304
<211>  15
<212>  PRT
<213>  Artificial Sequence

<220>
<223>  epitope

<400>  304

Ala Glu Lys Tyr Asp Leu Lys Asn Trp Ala Glu Asn Leu Asn Gln
1               5                   10                  15

<210>  305
<211>  15
<212>  PRT
<213>  Artificial Sequence

<220>
<223>  epitope

<400>  305

Thr Lys Val Lys Leu Tyr Pro Glu Lys Asp Glu Pro Leu Asn Val
1               5                   10                  15

<210>  306
<211>  15

<212>  PRT
<213>  Artificial Sequence

<220>
<223>  epitope

<400>  306

Val Ser Lys Arg Val Ser Val Pro Gly Leu Asn Pro Ile Ser Ala
1               5                   10                  15


<210>  307
<211>  15
<212>  PRT
<213>  Artificial Sequence

<220>
<223>  epitope

<400>  307

Glu Leu Gly Leu Asp Gly Lys Gly Phe Glu Pro Thr Leu Glu Ala
1               5                   10                  15


<210>  308
<211>  15
<212>  PRT
<213>  Artificial Sequence

<220>
<223>  epitope

<400>  308

Ala Leu Ser Gly Ile Val Thr Pro Gly Ala Lys Val Ala Val Lys
1               5                   10                  15


<210>  309
<211>  15
<212>  PRT
<213>  Artificial Sequence

<220>
<223>  epitope

<400>  309

Val Asn Tyr Ser Pro Thr Lys Gly Tyr Leu Gln Met Ser Ser Ser
1               5                   10                  15


<210>  310
<211>  15
<212>  PRT
<213>  Artificial Sequence

<220>
<223>  epitope

<400>  310

Asn Glu Leu Leu Asp Arg Lys Val Ala Leu Thr Asp Met Thr Met
1               5                   10                  15

<210>  311
<211>  15
<212>  PRT
<213>  Artificial Sequence

<220>
<223>  epitope

<400>  311

Leu His Leu Ala Ala Val Asn Lys Leu Glu Phe Leu Leu Ser Lys
1               5                   10                  15

<210>  312
<211>  15
<212>  PRT
<213>  Artificial Sequence

<220>
<223>  epitope

<400>  312

Ser Gly Tyr Leu Lys Met Phe Gly Gln Glu Leu Leu Phe Gly Arg
1               5                   10                  15

<210>  313
<211>  15
<212>  PRT
<213>  Artificial Sequence

<220>
<223>  epitope

<400>  313

Leu Leu Phe Gly Arg Leu Asp Lys Asp Thr Leu Gln Asn Val Leu
1               5                   10                  15

<210>  314
<211>  15
<212>  PRT
<213>  Artificial Sequence

<220>
<223>  epitope

<400>  314

Pro Asp Glu Lys Ile Pro Ser Ile Arg Arg Leu Ile Ser Ser Leu
1               5                   10                  15

<210>  315
<211>  15

<212>  PRT
<213>  Artificial Sequence

<220>
<223>  epitope

<400>  315

Ile Val Ser Ser Lys Thr Phe Ala Val Thr Arg Asn Ile Glu Asp
1               5                   10                  15


<210>  316
<211>  15
<212>  PRT
<213>  Artificial Sequence

<220>
<223>  epitope

<400>  316

Arg Asn Ile Glu Asp Leu Ala Ala Ser Lys Met Thr Pro Val Leu
1               5                   10                  15


<210>  317
<211>  15
<212>  PRT
<213>  Artificial Sequence

<220>
<223>  epitope

<400>  317

Pro Asp Ile Met Lys Met Ser Phe Asp Ser Asp Ser Ala Ser Gly
1               5                   10                  15


<210>  318
<211>  15
<212>  PRT
<213>  Artificial Sequence

<220>
<223>  epitope

<400>  318

Ser Phe Lys Pro Val Tyr Ser Asp Val Pro Ile Glu Lys Ile Gln
1               5                   10                  15


<210>  319
<211>  10
<212>  PRT
<213>  Artificial Sequence

<220>
<223>  epitope

<400>  319

Leu Lys Val Arg Ser Pro Gln Val Glu Glu
1               5                   10


<210>  320
<211>  15
<212>  PRT
<213>  Artificial Sequence

<220>
<223>  epitope


<220>
<221>  misc_feature
<222>  (1)..(5)
<223>  Xaa can be any amino acid

<220>
<221>  misc_feature
<222>  (7)..(7)
<223>  Xaa can be any amino acid

<220>
<221>  misc_feature
<222>  (13)..(13)
<223>  Xaa can be any amino acid

<400>  320

Xaa Xaa Xaa Xaa Xaa Gln Xaa Glu Glu Tyr Asn Gly Xaa Trp Pro
1               5                   10                  15


<210>  321
<211>  15
<212>  PRT
<213>  Artificial Sequence

<220>
<223>  epitope


<220>
<221>  misc_feature
<222>  (1)..(3)
<223>  Xaa can be any amino acid

<400>  321

Xaa Xaa Xaa Ser Pro Gln Val Glu Glu Tyr Asn Gly Val Trp Pro
1               5                   10                  15


<210>  322
<211>  6
<212>  PRT
<213>  Artificial Sequence

<220>
<223>  epitope

<220>
<221>  misc_feature
<222>  (1)..(1)
<223>  Xaa can be any amino acid

<400>  322

Xaa Glu Glu Tyr Asn Gly
1               5


<210>  323
<211>  6
<212>  PRT
<213>  Artificial Sequence

<220>
<223>  epitope

<400>  323

Val Glu Glu Tyr Asn Gly
1               5


<210>  324
<211>  9
<212>  PRT
<213>  Artificial Sequence

<220>
<223>  epitope

<400>  324

Met Phe Gln Met Thr Ile Lys Lys Ser
1               5


<210>  325
<211>  10
<212>  PRT
<213>  Artificial Sequence

<220>
<223>  epitope


<220>
<221>  misc_feature
<222>  (1)..(8)
<223>  Xaa can be any amino acid

<400>  325

Xaa Xaa Xaa Xaa Xaa Xaa Xaa Xaa Asn Val
1               5                   10


<210>  326
<211>  10
<212>  PRT

<213>  Artificial Sequence

<220>
<223>  epitiope



<220>
<221>  misc_feature
<222>  (1)..(3)
<223>  Xaa can be any amino acid

<220>
<221>  misc_feature
<222>  (6)..(8)
<223>  Xaa can be any amino acid

<400>  326

Xaa Xaa Xaa Ile Lys Xaa Xaa Xaa Asn Val
1               5                   10


<210>  327
<211>  10
<212>  PRT
<213>  Artificial Sequence

<220>
<223>  epitope

<400>  327

Gln Met Thr Ile Lys Lys Ser Gln Asn Val
1               5                   10


<210>  328
<211>  15
<212>  PRT
<213>  Artificial Sequence

<220>
<223>  epitope



<220>
<221>  misc_feature
<222>  (1)..(8)
<223>  Xaa can be any amino acid

<220>
<221>  misc_feature
<222>  (13)..(15)
<223>  Xaa can be any amino acid

<400>  328

Xaa Xaa Xaa Xaa Xaa Xaa Xaa Xaa Asn Val Tyr Glu Xaa Xaa Xaa
1               5                   10                  15


<210>  329
<211>  8

<212>  PRT
<213>  Artificial Sequence

<220>
<223>  epitope


<220>
<221>  misc_feature
<222>  (2)..(2)
<223>  Xaa can be any amino acid

<220>
<221>  misc_feature
<222>  (4)..(5)
<223>  Xaa can be any amino acid

<220>
<221>  misc_feature
<222>  (7)..(8)
<223>  Xaa can be any amino acid

<400>  329

Arg Xaa Arg Xaa Xaa Lys Xaa Xaa
1               5


<210>  330
<211>  8
<212>  PRT
<213>  Artificial Sequence

<220>
<223>  epitope

<400>  330

Arg Gly Arg Glu Asp Lys Met Lys
1               5


<210>  331
<211>  10
<212>  PRT
<213>  Artificial Sequence

<220>
<223>  epitope

<400>  331

Lys Met Lys Leu Ala Leu Lys Cys Ile Gly
1               5                   10


<210>  332
<211>  15
<212>  PRT
<213>  Artificial Sequence

<220>
<223>  epitope

<220>
<221>  misc_feature
<222>  (2)..(2)
<223>  Xaa can be any amino acid

<220>
<221>  misc_feature
<222>  (4)..(5)
<223>  Xaa can be any amino acid

<220>
<221>  misc_feature
<222>  (7)..(15)
<223>  Xaa can be any amino acid

<400>  332

Arg Xaa Arg Xaa Xaa Lys Xaa Xaa Xaa Xaa Xaa Xaa Xaa Xaa Xaa
1               5                   10                  15


<210>  333
<211>  9
<212>  PRT
<213>  Artificial Sequence

<220>
<223>  epitope

<400>  333

Arg Glu Asp Lys Met Lys Leu Ala Leu
1               5


<210>  334
<211>  10
<212>  PRT
<213>  Artificial Sequence

<220>
<223>  epitope


<220>
<221>  misc_feature
<222>  (2)..(4)
<223>  Xaa can be any amino acid

<220>
<221>  misc_feature
<222>  (7)..(10)
<223>  Xaa can be any amino acid

<400>  334

Lys Xaa Xaa Xaa Ala Leu Xaa Xaa Xaa Xaa
1               5                   10


<210>  335

<211>  10
<212>  PRT
<213>  Artificial Sequence

<220>
<223>  epitope

<400>  335

Lys Met Lys Leu Ala Leu Lys Cys Ile Gly
1               5                   10


<210>  336
<211>  10
<212>  PRT
<213>  Artificial Sequence

<220>
<223>  epitope

<400>  336

Ala Leu Lys Cys Ile Gly Asn Met Gly Glu
1               5                   10


<210>  337
<211>  15
<212>  PRT
<213>  Artificial Sequence

<220>
<223>  epitope


<220>
<221>  misc_feature
<222>  (2)..(5)
<223>  Xaa can be any amino acid

<220>
<221>  misc_feature
<222>  (7)..(9)
<223>  Xaa can be any amino acid

<220>
<221>  misc_feature
<222>  (12)..(15)
<223>  Xaa can be any amino acid

<400>  337

Arg Xaa Xaa Xaa Xaa Lys Xaa Xaa Xaa Ala Leu Xaa Xaa Xaa Xaa
1               5                   10                  15


<210>  338
<211>  10
<212>  PRT
<213>  Artificial Sequence

<220>

<223>  epitope

<220>
<221>  misc_feature
<222>  (2)..(3)
<223>  Xaa can be any amino acid

<220>
<221>  misc_feature
<222>  (7)..(9)
<223>  Xaa can be any amino acid

<400>  338

Ala Xaa Xaa Lys Ile Ala Xaa Xaa Xaa Pro
1               5                   10

<210>  339
<211>  10
<212>  PRT
<213>  Artificial Sequence

<220>
<223>  epitope

<400>  339

Ala Leu Lys Lys Ile Ala Trp Lys Asp Pro
1               5                   10

<210>  340
<211>  10
<212>  PRT
<213>  Artificial Sequence

<220>
<223>  epitope

<400>  340

Asp Pro Lys Thr Val Gln Gly Tyr Leu Ile
1               5                   10

<210>  341
<211>  15
<212>  PRT
<213>  Artificial Sequence

<220>
<223>  epitope

<220>
<221>  misc_feature
<222>  (2)..(3)
<223>  Xaa can be any amino acid

<220>
<221>  misc_feature

<222>  (7)..(9)
<223>  Xaa can be any amino acid

<220>
<221>  misc_feature
<222>  (14)..(15)
<223>  Xaa can be any amino acid

<400>  341

Ala Xaa Xaa Lys Ile Ala Xaa Xaa Xaa Pro Lys Thr Val Xaa Xaa
1               5                   10                  15


<210>  342
<211>  10
<212>  PRT
<213>  Artificial Sequence

<220>
<223>  epitope

<400>  342

Asp Ala Ile Thr Ala Leu Lys Lys Ile Ala
1               5                   10


<210>  343
<211>  9
<212>  PRT
<213>  Artificial Sequence

<220>
<223>  epitope

<400>  343

Leu Lys Lys Ile Ala Trp Lys Asp Pro
1               5


<210>  344
<211>  10
<212>  PRT
<213>  Artificial Sequence

<220>
<223>  epitope

<400>  344

Lys Ile Ala Trp Lys Asp Pro Lys Thr Val
1               5                   10


<210>  345
<211>  10
<212>  PRT
<213>  Artificial Sequence

<220>
<223>  epitope

<220>
<221>  misc_feature
<222>  (7)..(9)
<223>  Xaa can be any amino acid

<400>  345

Ala Trp Lys Asp Pro Lys Xaa Xaa Xaa Gly
1               5                   10


<210>  346
<211>  10
<212>  PRT
<213>  Artificial Sequence

<220>
<223>  epitope

<400>  346

Ala Trp Lys Asp Pro Lys Thr Val Gln Gly
1               5                   10


<210>  347
<211>  5
<212>  PRT
<213>  Artificial Sequence

<220>
<223>  epitope

<400>  347

Leu Lys Lys Ile Ala
1               5


<210>  348
<211>  15
<212>  PRT
<213>  Artificial Sequence

<220>
<223>  epitope


<220>
<221>  misc_feature
<222>  (2)..(5)
<223>  Xaa can be any amino acid

<220>
<221>  misc_feature
<222>  (7)..(11)
<223>  Xaa can be any amino acid

<220>
<221>  misc_feature
<222>  (15)..(15)

<223>  Xaa can be any amino acid

<400>  348

Ala Xaa Xaa Xaa Xaa Ala Xaa Xaa Xaa Xaa Xaa Thr Val Gln Xaa
1               5                   10                  15


<210>  349
<211>  8
<212>  PRT
<213>  Artificial Sequence

<220>
<223>  epitope


<220>
<221>  misc_feature
<222>  (2)..(2)
<223>  Xaa can be any amino acid

<220>
<221>  misc_feature
<222>  (4)..(8)
<223>  Xaa can be any amino acid

<400>  349

Ala Xaa Lys Xaa Xaa Xaa Xaa Xaa
1               5


<210>  350
<211>  8
<212>  PRT
<213>  Artificial Sequence

<220>
<223>  epitope

<400>  350

Ala Trp Lys Asp Pro Lys Thr Val
1               5


<210>  351
<211>  8
<212>  PRT
<213>  Artificial Sequence

<220>
<223>  epitope

<400>  351

Pro Lys Thr Val Gln Gly Tyr Leu
1               5


<210>  352
<211>  15

<212>  PRT
<213>  Artificial Sequence

<220>
<223>  epitope


<220>
<221>  misc_feature
<222>  (2)..(5)
<223>  Xaa can be any amino acid

<220>
<221>  misc_feature
<222>  (7)..(7)
<223>  Xaa can be any amino acid

<220>
<221>  misc_feature
<222>  (9)..(13)
<223>  Xaa can be any amino acid

<400>  352

Ala Xaa Xaa Xaa Xaa Ala Xaa Lys Xaa Xaa Xaa Xaa Xaa Gln Gly
1               5                   10                  15


<210>  353
<211>  10
<212>  PRT
<213>  Artificial Sequence

<220>
<223>  epitope

<400>  353

Cys Asn Ile Ala Leu Lys Leu Leu Ser Pro
1               5                   10


<210>  354
<211>  8
<212>  PRT
<213>  Artificial Sequence

<220>
<223>  epitope


<220>
<221>  misc_feature
<222>  (1)..(1)
<223>  Xaa can be any amino acid

<400>  354

Xaa Leu Ser Pro Lys Leu Asp Ser
1               5


<210>  355

<211>  8
<212>  PRT
<213>  Artificial Sequence

<220>
<223>  epitope

<400>  355

Leu Leu Ser Pro Lys Leu Asp Ser
1               5


<210>  356
<211>  4
<212>  PRT
<213>  Artificial Sequence

<220>
<223>  epitope

<400>  356

Lys Leu Asp Ser
1


<210>  357
<211>  9
<212>  PRT
<213>  Artificial Sequence

<220>
<223>  epitope

<400>  357

Lys Leu Asp Ser Met Ser Tyr Arg Tyr
1               5


<210>  358
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (4)..(5)
<223>  Xaa can be any amino acid

<400>  358

Xaa Ala Leu Xaa Xaa Leu Ser Pro Lys Leu Asp Ser Met Ser Tyr

1                   5                   10                  15


<210>  359
<211>  9
<212>  PRT
<213>  Artificial Sequence

<220>
<223>  epitiope

<400>  359

Val Val Asn Ser Pro Arg Thr Met Phe
1               5


<210>  360
<211>  10
<212>  PRT
<213>  Artificial Sequence

<220>
<223>  epitope

<400>  360

Pro Ser Ala Ile Ile Ser Lys Leu Met Ala
1               5                   10


<210>  361
<211>  15
<212>  PRT
<213>  Artificial Sequence

<220>
<223>  epitope



<220>
<221>  misc_feature
<222>  (2)..(4)
<223>  Xaa can be any amino acid

<220>
<221>  misc_feature
<222>  (10)..(10)
<223>  Xaa can be any amino acid

<220>
<221>  misc_feature
<222>  (12)..(12)
<223>  Xaa can be any amino acid

<400>  361

Val Xaa Xaa Xaa Arg Thr Met Phe Pro Xaa Ala Xaa Ile Ser Lys
1               5                   10                  15


<210>  362
<211>  15

<212>  PRT
<213>  Artificial Sequence

<220>
<223>  epitope


<220>
<221>  misc_feature
<222>  (3)..(4)
<223>  Xaa can be any amino acid

<220>
<221>  misc_feature
<222>  (12)..(12)
<223>  Xaa can be any amino acid

<400>  362

Val Asn Xaa Xaa Arg Thr Met Phe Pro Ser Ala Xaa Ile Ser Lys
1               5                   10                  15


<210>  363
<211>  7
<212>  PRT
<213>  Artificial Sequence

<220>
<223>  epitope

<400>  363

Val Val Asn Ser Pro Arg Thr
1               5


<210>  364
<211>  10
<212>  PRT
<213>  Artificial Sequence

<220>
<223>  epitope


<220>
<221>  misc_feature
<222>  (2)..(4)
<223>  Xaa can be any amino acid

<220>
<221>  misc_feature
<222>  (6)..(6)
<223>  Xaa can be any amino acid

<220>
<221>  misc_feature
<222>  (8)..(8)
<223>  Xaa can be any amino acid

<220>
<221>  misc_feature

<222>  (10)..(10)
<223>  Xaa can be any amino acid

<400>  364

Ser Xaa Xaa Xaa Met Xaa Pro Xaa Ala Xaa
1               5                   10


<210>  365
<211>  10
<212>  PRT
<213>  Artificial Sequence

<220>
<223>  epitope


<220>
<221>  misc_feature
<222>  (6)..(6)
<223>  Xaa can be any amino acid

<220>
<221>  misc_feature
<222>  (8)..(8)
<223>  Xaa can be any amino acid

<220>
<221>  misc_feature
<222>  (10)..(10)
<223>  Xaa can be any amino acid

<400>  365

Ser Pro Arg Thr Met Xaa Pro Xaa Ala Xaa
1               5                   10


<210>  366
<211>  10
<212>  PRT
<213>  Artificial Sequence

<220>
<223>  epitope

<400>  366

Ser Pro Arg Thr Met Phe Pro Ser Ala Ile
1               5                   10


<210>  367
<211>  10
<212>  PRT
<213>  Artificial Sequence

<220>
<223>  epitope


<220>

<221>  misc_feature
<222>  (1)..(1)
<223>  Xaa can be any amino acid

<220>
<221>  misc_feature
<222>  (3)..(3)
<223>  Xaa can be any amino acid

<220>
<221>  misc_feature
<222>  (5)..(5)
<223>  Xaa can be any amino acid

<220>
<221>  misc_feature
<222>  (7)..(9)
<223>  Xaa can be any amino acid

<400>  367

Xaa Met Xaa Pro Xaa Ala Xaa Xaa Xaa Lys
1               5                   10


<210>  368
<211>  10
<212>  PRT
<213>  Artificial Sequence

<220>
<223>  epitope


<220>
<221>  misc_feature
<222>  (3)..(3)
<223>  Xaa can be any amino acid

<220>
<221>  misc_feature
<222>  (5)..(5)
<223>  Xaa can be any amino acid

<220>
<221>  misc_feature
<222>  (7)..(9)
<223>  Xaa can be any amino acid

<400>  368

Thr Met Xaa Pro Xaa Ala Xaa Xaa Xaa Lys
1               5                   10


<210>  369
<211>  10
<212>  PRT
<213>  Artificial Sequence

<220>
<223>  epitope

<400>  369

Thr Met Phe Pro Ser Ala Ile Ile Ser Lys
1               5                   10


<210>  370
<211>  15
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (4)..(6)
<223>  Xaa can be any amino acid

<220>
<221>  misc_feature
<222>  (8)..(8)
<223>  Xaa can be any amino acid

<220>
<221>  misc_feature
<222>  (10)..(10)
<223>  Xaa can be any amino acid

<220>
<221>  misc_feature
<222>  (12)..(14)
<223>  Xaa can be any amino acid

<400>  370

Xaa Xaa Ser Xaa Xaa Xaa Met Xaa Pro Xaa Ala Xaa Xaa Xaa Lys
1               5                   10                  15


<210>  371
<211>  15
<212>  PRT
<213>  Artificial Sequence

<220>
<223>  epitope


<220>
<221>  misc_feature
<222>  (8)..(8)
<223>  Xaa can be any amino acid

<220>
<221>  misc_feature
<222>  (10)..(10)

<223>  Xaa can be any amino acid

<220>
<221>  misc_feature
<222>  (12)..(14)
<223>  Xaa can be any amino acid

<400>  371

Val Asn Ser Pro Arg Thr Met Xaa Pro Xaa Ala Xaa Xaa Xaa Lys
1               5                   10                  15


<210>  372
<211>  9
<212>  PRT
<213>  Artificial Sequence

<220>
<223>  epitope


<220>
<221>  misc_feature
<222>  (2)..(2)
<223>  Xaa can be any amino acid

<220>
<221>  misc_feature
<222>  (5)..(5)
<223>  Xaa can be any amino acid

<220>
<221>  misc_feature
<222>  (7)..(8)
<223>  Xaa can be any amino acid

<400>  372

Ile Xaa Lys Leu Xaa Ala Xaa Xaa Ala
1               5


<210>  373
<211>  9
<212>  PRT
<213>  Artificial Sequence

<220>
<223>  epitope


<220>
<221>  misc_feature
<222>  (5)..(5)
<223>  Xaa can be any amino acid

<220>
<221>  misc_feature
<222>  (7)..(8)
<223>  Xaa can be any amino acid

<400>  373

Ile Ser Lys Leu Xaa Ala Xaa Xaa Ala
1               5

<210>  374
<211>  9
<212>  PRT
<213>  Artificial Sequence

<220>
<223>  epitope

<400>  374

Ile Ser Lys Leu Met Ala Asn Ser Ala
1               5

<210>  375
<211>  10
<212>  PRT
<213>  Artificial Sequence

<220>
<223>  epitope

<400>  375

Ser Ala Gly Ser Val Ala Asp Leu Val Glu
1               5                   10

<210>  376
<211>  15
<212>  PRT
<213>  Artificial Sequence

<220>
<223>  epitope

<220>
<221>  misc_feature
<222>  (7)..(7)
<223>  Xaa can be any amino acid

<220>
<221>  misc_feature
<222>  (9)..(10)
<223>  Xaa can be any amino acid

<220>
<221>  misc_feature
<222>  (12)..(12)
<223>  Xaa can be any amino acid

<220>
<221>  misc_feature
<222>  (14)..(14)
<223>  Xaa can be any amino acid

<400>  376

Ala Ile Ile Ser Lys Leu Xaa Ala Xaa Xaa Ala Xaa Ser Xaa Ala
1               5                   10                  15

<210>  377
<211>  10
<212>  PRT
<213>  Artificial Sequence

<220>
<223>  epitope

<400>  377

Ile Lys Glu Leu Gly Lys Ala Leu Gln Gly
1               5                   10

<210>  378
<211>  10
<212>  PRT
<213>  Artificial Sequence

<220>
<223>  epitope

<220>
<221>  misc_feature
<222>  (5)..(6)
<223>  Xaa can be any amino acid

<400>  378

Lys Ala Leu Gln Xaa Xaa Lys Glu Leu Pro
1               5                   10

<210>  379
<211>  10
<212>  PRT
<213>  Artificial Sequence

<220>
<223>  epitope

<400>  379

Lys Ala Leu Gln Gly Trp Lys Glu Leu Pro
1               5                   10

<210>  380
<211>  10
<212>  PRT
<213>  Artificial Sequence

<220>
<223>  epitope

<400>  380

Glu Leu Pro Thr Glu Thr Pro Leu Val Ser
1               5                   10


<210>  381
<211>  9
<212>  PRT
<213>  Artificial Sequence

<220>
<223>  epitope

<400>  381

Leu Pro Thr Glu Thr Pro Leu Val Ser
1               5


<210>  382
<211>  15
<212>  PRT
<213>  Artificial Sequence

<220>
<223>  epitope


<220>
<221>  misc_feature
<222>  (6)..(7)
<223>  Xaa can be any amino acid

<220>
<221>  misc_feature
<222>  (13)..(13)
<223>  Xaa can be any amino acid

<400>  382

Gly Lys Ala Leu Gln Xaa Xaa Lys Glu Leu Pro Thr Xaa Thr Pro
1               5                   10                  15


<210>  383
<211>  15
<212>  PRT
<213>  Artificial Sequence

<220>
<223>  epitope


<220>
<221>  misc_feature
<222>  (6)..(7)
<223>  Xaa can be any amino acid

<400>  383

Gly Lys Ala Leu Gln Xaa Xaa Lys Glu Leu Pro Thr Glu Thr Pro
1               5                   10                  15

<210>  384
<211>  10
<212>  PRT
<213>  Artificial Sequence

<220>
<223>  epitope

<400>  384

Ile Lys Glu Leu Gly Lys Ala Leu Gln Gly
1               5                   10


<210>  385
<211>  10
<212>  PRT
<213>  Artificial Sequence

<220>
<223>  epitope

<400>  385

Glu Leu Gly Lys Ala Leu Gln Gly Trp Lys
1               5                   10


<210>  386
<211>  9
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(7)
<223>  Xaa can be any amino acid

<220>
<221>  misc_feature
<222>  (9)..(9)
<223>  Xaa can be any amino acid

<400>  386

Xaa Ala Xaa Xaa Xaa Xaa Xaa Glu Xaa
1               5


<210>  387
<211>  9
<212>  PRT
<213>  Artificial Sequence

<220>
<223>  epitope

<220>
<221>  misc_feature
<222>  (1)..(1)
<223>  Xaa can be any amino acid

<220>
<221>  misc_feature
<222>  (4)..(7)
<223>  Xaa can be any amino acid

<220>
<221>  misc_feature
<222>  (9)..(9)
<223>  Xaa can be any amino acid

<400>  387

Xaa Ala Leu Xaa Xaa Xaa Xaa Glu Xaa
1               5

<210>  388
<211>  9
<212>  PRT
<213>  Artificial Sequence

<220>
<223>  epitope

<400>  388

Lys Ala Leu Gln Gly Trp Lys Glu Leu
1               5

<210>  389
<211>  10
<212>  PRT
<213>  Artificial Sequence

<220>
<223>  epitope

<220>
<221>  misc_feature
<222>  (1)..(3)
<223>  Xaa can be any amino acid

<220>
<221>  misc_feature
<222>  (5)..(9)
<223>  Xaa can be any amino acid

<400>  389

Xaa Xaa Xaa Glu Xaa Xaa Xaa Xaa Xaa Pro
1               5                   10

<210>  390
<211>  10
<212>  PRT
<213>  Artificial Sequence

<220>
<223>  epitope


<220>
<221>  misc_feature
<222>  (1)..(3)
<223>  Xaa can be any amino acid

<220>
<221>  misc_feature
<222>  (5)..(7)
<223>  Xaa can be any amino acid

<220>
<221>  misc_feature
<222>  (9)..(9)
<223>  Xaa can be any amino acid

<400>  390

Xaa Xaa Xaa Glu Xaa Xaa Xaa Glu Xaa Pro
1               5                   10


<210>  391
<211>  9
<212>  PRT
<213>  Artificial Sequence

<220>
<223>  epitope

<400>  391

Gly Trp Lys Glu Leu Pro Thr Glu Thr
1               5


<210>  392
<211>  10
<212>  PRT
<213>  Artificial Sequence

<220>
<223>  epitope

<400>  392

Gly Trp Lys Glu Leu Pro Thr Glu Thr Pro
1               5                   10


<210>  393
<211>  8
<212>  PRT
<213>  Artificial Sequence

<220>
<223>  epitope

<400>  393

Leu Pro Thr Glu Thr Pro Leu Val
1               5


<210>  394
<211>  15
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (4)..(8)
<223>  Xaa can be any amino acid

<220>
<221>  misc_feature
<222>  (10)..(14)
<223>  Xaa can be any amino acid

<400>  394

Xaa Xaa Ala Xaa Xaa Xaa Xaa Xaa Glu Xaa Xaa Xaa Xaa Xaa Pro
1               5                   10                  15


<210>  395
<211>  15
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (5)..(8)
<223>  Xaa can be any amino acid

<220>
<221>  misc_feature
<222>  (10)..(14)
<223>  Xaa can be any amino acid

<400>  395

Xaa Xaa Ala Leu Xaa Xaa Xaa Xaa Glu Xaa Xaa Xaa Xaa Xaa Pro
1               5                   10                  15


<210>  396
<211>  10
<212>  PRT
<213>  Artificial Sequence

<220>
<223>  epitope


<220>
<221>  misc_feature
<222>  (3)..(6)
<223>  Xaa can be any amino acid

<220>
<221>  misc_feature
<222>  (9)..(10)
<223>  Xaa can be any amino acid

<400>  396

Lys Ala Xaa Xaa Xaa Xaa Lys Glu Xaa Xaa
1               5                   10


<210>  397
<211>  10
<212>  PRT
<213>  Artificial Sequence

<220>
<223>  epitope

<400>  397

Lys Ala Leu Gln Gly Trp Lys Glu Leu Pro
1               5                   10


<210>  398
<211>  10
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (5)..(10)

<223>  Xaa can be any amino acid

<400>  398

Xaa Xaa Lys Glu Xaa Xaa Xaa Xaa Xaa Xaa
1               5                   10


<210>  399
<211>  10
<212>  PRT
<213>  Artificial Sequence

<220>
<223>  epitope


<220>
<221>  misc_feature
<222>  (2)..(2)
<223>  Xaa can be any amino acid

<220>
<221>  misc_feature
<222>  (5)..(5)
<223>  Xaa can be any amino acid

<220>
<221>  misc_feature
<222>  (7)..(8)
<223>  Xaa can be any amino acid

<220>
<221>  misc_feature
<222>  (10)..(10)
<223>  Xaa can be any amino acid

<400>  399

Gly Xaa Lys Glu Xaa Pro Xaa Xaa Thr Xaa
1               5                   10


<210>  400
<211>  10
<212>  PRT
<213>  Artificial Sequence

<220>
<223>  epitope

<400>  400

Gly Trp Lys Glu Leu Pro Thr Glu Thr Pro
1               5                   10


<210>  401
<211>  9
<212>  PRT
<213>  Artificial Sequence

<220>

<223>  epitope

<400>  401

Leu Pro Thr Glu Thr Pro Leu Val Ser
1               5


<210>  402
<211>  10
<212>  PRT
<213>  Artificial Sequence

<220>
<223>  epitope

<400>  402

Leu Pro Thr Glu Thr Pro Leu Val Ser Ala
1               5                   10


<210>  403
<211>  15
<212>  PRT
<213>  Artificial Sequence

<220>
<223>  epitope


<220>
<221>  misc_feature
<222>  (4)..(7)
<223>  Xaa can be any amino acid

<220>
<221>  misc_feature
<222>  (10)..(15)
<223>  Xaa can be any amino acid

<400>  403

Gly Lys Ala Xaa Xaa Xaa Xaa Lys Glu Xaa Xaa Xaa Xaa Xaa Xaa
1               5                   10                  15


<210>  404
<211>  10
<212>  PRT
<213>  Artificial Sequence

<220>
<223>  epitope

<400>  404

Glu Val Ala Phe Ile Asn Ile Asn Lys Glu
1               5                   10


<210>  405
<211>  10

<212>  PRT
<213>  Artificial Sequence

<220>
<223>  epitope

<400>  405

Ala Phe Ile Asn Ile Asn Lys Glu Leu Leu
1               5                   10


<210>  406
<211>  5
<212>  PRT
<213>  Artificial Sequence

<220>
<223>  epitope


<220>
<221>  misc_feature
<222>  (3)..(3)
<223>  Xaa can be any amino acid

<400>  406

Glu Leu Xaa Gln Gln
1               5


<210>  407
<211>  5
<212>  PRT
<213>  Artificial Sequence

<220>
<223>  epitope

<400>  407

Glu Leu Leu Gln Gln
1               5


<210>  408
<211>  15
<212>  PRT
<213>  Artificial Sequence

<220>
<223>  epitope


<220>
<221>  misc_feature
<222>  (1)..(3)
<223>  Xaa can be any amino acid

<220>
<221>  misc_feature
<222>  (8)..(8)

<223>  Xaa can be any amino acid

<220>
<221>  misc_feature
<222>  (13)..(14)
<223>  Xaa can be any amino acid

<400>  408

Xaa Xaa Xaa Asn Lys Glu Leu Xaa Gln Gln Val Met Xaa Xaa Val
1               5                   10                  15


<210>  409
<211>  10
<212>  PRT
<213>  Artificial Sequence

<220>
<223>  epitope

<400>  409

Leu Gln Gln Val Met Lys Thr Val Val Glu
1               5                   10


<210>  410
<211>  8
<212>  PRT
<213>  Artificial Sequence

<220>
<223>  epitope


<220>
<221>  misc_feature
<222>  (1)..(3)
<223>  Xaa can be any amino acid

<220>
<221>  misc_feature
<222>  (6)..(6)
<223>  Xaa can be any amino acid

<400>  410

Xaa Xaa Xaa Val Val Xaa Pro Ala
1               5


<210>  411
<211>  8
<212>  PRT
<213>  Artificial Sequence

<220>
<223>  epitope


<220>
<221>  misc_feature

<222>  (1)..(2)
<223>  Xaa can be any amino acid

<400>  411

Xaa Xaa Thr Val Val Glu Pro Ala
1               5


<210>  412
<211>  8
<212>  PRT
<213>  Artificial Sequence

<220>
<223>  epitope

<400>  412

Met Lys Thr Val Val Glu Pro Ala
1               5


<210>  413
<211>  5
<212>  PRT
<213>  Artificial Sequence

<220>
<223>  epitope


<220>
<221>  misc_feature
<222>  (3)..(3)
<223>  Xaa can be any amino acid

<400>  413

Ala Asp Xaa Asn Ala
1               5


<210>  414
<211>  5
<212>  PRT
<213>  Artificial Sequence

<220>
<223>  epitope

<400>  414

Ala Asp Arg Asn Ala
1               5


<210>  415
<211>  15
<212>  PRT
<213>  Artificial Sequence

<220>

<223>  epitope


<220>
<221>  misc_feature
<222>  (1)..(4)
<223>  Xaa can be any amino acid

<220>
<221>  misc_feature
<222>  (7)..(7)
<223>  Xaa can be any amino acid

<220>
<221>  misc_feature
<222>  (11)..(11)
<223>  Xaa can be any amino acid

<400>  415

Xaa Xaa Xaa Xaa Val Val Xaa Pro Ala Asp Xaa Asn Ala Ala Ile
1               5                   10                  15


<210>  416
<211>  15
<212>  PRT
<213>  Artificial Sequence

<220>
<223>  epitope


<220>
<221>  misc_feature
<222>  (1)..(3)
<223>  Xaa can be any amino acid

<220>
<221>  misc_feature
<222>  (11)..(11)
<223>  Xaa can be any amino acid

<400>  416

Xaa Xaa Xaa Thr Val Val Glu Pro Ala Asp Xaa Asn Ala Ala Ile
1               5                   10                  15


<210>  417
<211>  8
<212>  PRT
<213>  Artificial Sequence

<220>
<223>  epitope


<220>
<221>  misc_feature
<222>  (1)..(4)
<223>  Xaa can be any amino acid

<220>
<221>  misc_feature
<222>  (8)..(8)
<223>  Xaa can be any amino acid

<400>  417

Xaa Xaa Xaa Xaa Lys Asn Leu Xaa
1               5


<210>  418
<211>  8
<212>  PRT
<213>  Artificial Sequence

<220>
<223>  epitope


<220>
<221>  misc_feature
<222>  (3)..(4)
<223>  Xaa can be any amino acid

<220>
<221>  misc_feature
<222>  (8)..(8)
<223>  Xaa can be any amino acid

<400>  418

Asp Val Xaa Xaa Lys Asn Leu Xaa
1               5


<210>  419
<211>  8
<212>  PRT
<213>  Artificial Sequence

<220>
<223>  epitope

<400>  419

Asp Val Lys Leu Lys Asn Leu Lys
1               5


<210>  420
<211>  15
<212>  PRT
<213>  Artificial Sequence

<220>
<223>  epitope


<220>
<221>  misc_feature
<222>  (2)..(3)
<223>  Xaa can be any amino acid

<220>
<221>  misc_feature
<222>  (6)..(10)
<223>  Xaa can be any amino acid

<220>
<221>  misc_feature
<222>  (13)..(15)
<223>  Xaa can be any amino acid

<400>  420

Lys Xaa Xaa Ala Lys Xaa Xaa Xaa Xaa Xaa Lys Asn Xaa Xaa Xaa
1               5                   10                  15


<210>  421
<211>  15
<212>  PRT
<213>  Artificial Sequence

<220>
<223>  epitope


<220>
<221>  misc_feature
<222>  (2)..(3)
<223>  Xaa can be any amino acid

<220>
<221>  misc_feature
<222>  (6)..(6)
<223>  Xaa can be any amino acid

<220>
<221>  misc_feature
<222>  (9)..(10)
<223>  Xaa can be any amino acid

<220>
<221>  misc_feature
<222>  (14)..(15)
<223>  Xaa can be any amino acid

<400>  421

Lys Xaa Xaa Ala Lys Xaa Asp Val Xaa Xaa Lys Asn Leu Xaa Xaa
1               5                   10                  15


<210>  422
<211>  10
<212>  PRT
<213>  Artificial Sequence

<220>
<223>  epitope

<400>  422

Pro Met Lys Phe Asp Ala Lys Ile Asp Val

1                   5                   10

<210>  423
<211>  9
<212>  PRT
<213>  Artificial Sequence

<220>
<223>  epitope

<220>
<221>  misc_feature
<222>  (2)..(2)
<223>  Xaa can be any amino acid

<220>
<221>  misc_feature
<222>  (4)..(4)
<223>  Xaa can be any amino acid

<220>
<221>  misc_feature
<222>  (7)..(7)
<223>  Xaa can be any amino acid

<220>
<221>  misc_feature
<222>  (9)..(9)
<223>  Xaa can be any amino acid

<400>  423

Asp Xaa Lys Xaa Lys Asn Xaa Lys Xaa
1               5

<210>  424
<211>  9
<212>  PRT
<213>  Artificial Sequence

<220>
<223>  epitope

<220>
<221>  misc_feature
<222>  (4)..(4)
<223>  Xaa can be any amino acid

<220>
<221>  misc_feature
<222>  (7)..(7)
<223>  Xaa can be any amino acid

<220>
<221>  misc_feature
<222>  (9)..(9)
<223>  Xaa can be any amino acid

<400>  424

Asp Val Lys Xaa Lys Asn Xaa Lys Xaa
1               5

<210>  425
<211>  9
<212>  PRT
<213>  Artificial Sequence

<220>
<223>  epitope

<400>  425

Asp Val Lys Leu Lys Asn Leu Lys Ile
1               5

<210>  426
<211>  10
<212>  PRT
<213>  Artificial Sequence

<220>
<223>  epitope

<400>  426

Leu Lys Asn Leu Lys Ile Glu Thr Asn Pro
1               5                   10

<210>  427
<211>  15
<212>  PRT
<213>  Artificial Sequence

<220>
<223>  epitope

<220>
<221>  misc_feature
<222>  (3)..(3)
<223>  Xaa can be any amino acid

<220>
<221>  misc_feature
<222>  (6)..(6)
<223>  Xaa can be any amino acid

<220>
<221>  misc_feature
<222>  (8)..(8)
<223>  Xaa can be any amino acid

<220>
<221>  misc_feature
<222>  (10)..(10)
<223>  Xaa can be any amino acid

<220>

<221>  misc_feature
<222>  (13)..(13)
<223>  Xaa can be any amino acid

<220>
<221>  misc_feature
<222>  (15)..(15)
<223>  Xaa can be any amino acid

<400>  427

Lys Phe Xaa Ala Lys Xaa Asp Xaa Lys Xaa Lys Asn Xaa Lys Xaa
1               5                   10                  15


<210>  428
<211>  15
<212>  PRT
<213>  Artificial Sequence

<220>
<223>  epitope


<220>
<221>  misc_feature
<222>  (6)..(6)
<223>  Xaa can be any amino acid

<220>
<221>  misc_feature
<222>  (10)..(10)
<223>  Xaa can be any amino acid

<220>
<221>  misc_feature
<222>  (13)..(13)
<223>  Xaa can be any amino acid

<220>
<221>  misc_feature
<222>  (15)..(15)
<223>  Xaa can be any amino acid

<400>  428

Lys Phe Asp Ala Lys Xaa Asp Val Lys Xaa Lys Asn Xaa Lys Xaa
1               5                   10                  15


<210>  429
<211>  10
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (5)..(5)
<223>  Xaa can be any amino acid

<400>  429

Xaa Xaa Lys Lys Xaa Asn Thr Val Leu Ala
1               5                   10


<210>  430
<211>  10
<212>  PRT
<213>  Artificial Sequence

<220>
<223>  epitope

<400>  430

Pro Ser Lys Lys Gly Asn Thr Val Leu Ala
1               5                   10


<210>  431
<211>  15
<212>  PRT
<213>  Artificial Sequence

<220>
<223>  epitope


<220>
<221>  misc_feature
<222>  (2)..(4)
<223>  Xaa can be any amino acid

<220>
<221>  misc_feature
<222>  (7)..(7)
<223>  Xaa can be any amino acid

<220>
<221>  misc_feature
<222>  (9)..(9)
<223>  Xaa can be any amino acid

<220>
<221>  misc_feature
<222>  (11)..(11)
<223>  Xaa can be any amino acid

<220>
<221>  misc_feature
<222>  (13)..(15)
<223>  Xaa can be any amino acid

<400>  431

Asn Xaa Xaa Xaa Lys Lys Xaa Asn Xaa Val Xaa Ala Xaa Xaa Xaa

1               5                   10                  15


<210>  432
<211>  15
<212>  PRT
<213>  Artificial Sequence

<220>
<223>  epitope


<220>
<221>  misc_feature
<222>  (3)..(4)
<223>  Xaa can be any amino acid

<220>
<221>  misc_feature
<222>  (7)..(7)
<223>  Xaa can be any amino acid

<220>
<221>  misc_feature
<222>  (13)..(15)
<223>  Xaa can be any amino acid

<400>  432

Asn Arg Xaa Xaa Lys Lys Xaa Asn Thr Val Leu Ala Xaa Xaa Xaa
1               5                   10                  15


<210>  433
<211>  9
<212>  PRT
<213>  Artificial Sequence

<220>
<223>  epitope


<220>
<221>  misc_feature
<222>  (4)..(4)
<223>  Xaa can be any amino acid

<400>  433

Ser Ser Ser Xaa Ser Asn Ser Lys Ser
1               5


<210>  434
<211>  9
<212>  PRT
<213>  Artificial Sequence

<220>
<223>  epitope

<400>  434

Ser Ser Ser Ser Ser Asn Ser Lys Ser
1               5

<210>  435
<211>  10
<212>  PRT
<213>  Artificial Sequence

<220>
<223>  epitope

<220>
<221>  misc_feature
<222>  (2)..(2)
<223>  Xaa can be any amino acid

<400>  435

Ser Xaa Ser Asn Ser Lys Ser Ser Ser Ser
1               5                   10

<210>  436
<211>  10
<212>  PRT
<213>  Artificial Sequence

<220>
<223>  epitope

<400>  436

Ser Ser Ser Asn Ser Lys Ser Ser Ser Ser
1               5                   10

<210>  437
<211>  15
<212>  PRT
<213>  Artificial Sequence

<220>
<223>  epitope

<220>
<221>  misc_feature
<222>  (8)..(8)
<223>  Xaa can be any amino acid

<400>  437

Ser Ser Ser Ser Ser Ser Ser Xaa Ser Asn Ser Lys Ser Ser Ser
1               5                   10                  15

<210>  438
<211>  7
<212>  PRT
<213>  Artificial Sequence

<220>
<223>  epitope

<220>
<221>  misc_feature
<222>  (1)..(3)
<223>  Xaa can be any amino acid

<400>  438

Xaa Xaa Xaa Ser Lys Ser Ser
1               5

<210>  439
<211>  7
<212>  PRT
<213>  Artificial Sequence

<220>
<223>  epitope

<220>
<221>  misc_feature
<222>  (1)..(2)
<223>  Xaa can be any amino acid

<400>  439

Xaa Xaa Asn Ser Lys Ser Ser
1               5

<210>  440
<211>  7
<212>  PRT
<213>  Artificial Sequence

<220>
<223>  epitope

<400>  440

Ser Ser Asn Ser Lys Ser Ser
1               5

<210>  441
<211>  15
<212>  PRT
<213>  Artificial Sequence

<220>
<223>  XXSXSSSXXXSKSSS

<220>
<221>  misc_feature
<222>  (1)..(2)
<223>  Xaa can be any amino acid

<220>
<221>  misc_feature
<222>  (4)..(4)
<223>  Xaa can be any amino acid

<220>
<221>  misc_feature
<222>  (8)..(10)
<223>  Xaa can be any amino acid

<400>  441

Xaa Xaa Ser Xaa Ser Ser Ser Xaa Xaa Xaa Ser Lys Ser Ser Ser
1               5                   10                  15


<210>  442
<211>  15
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (8)..(9)
<223>  Xaa can be any amino acid

<400>  442

Xaa Xaa Ser Ser Ser Ser Ser Xaa Xaa Asn Ser Lys Ser Ser Ser
1               5                   10                  15


<210>  443
<211>  10
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(6)
<223>  Xaa can be any amino acid

<220>
<221>  misc_feature
<222>  (8)..(8)

<223>  Xaa can be any amino acid

<220>
<221>  misc_feature
<222>  (10)..(10)
<223>  Xaa can be any amino acid

<400>  443

Xaa Glu Xaa Xaa Xaa Xaa Lys Xaa Pro Xaa
1               5                   10


<210>  444
<211>  10
<212>  PRT
<213>  Artificial Sequence

<220>
<223>  epitope

<400>  444

Gln Glu Phe Pro Lys Arg Lys Leu Pro Gly
1               5                   10


<210>  445
<211>  10
<212>  PRT
<213>  Artificial Sequence

<220>
<223>  epitope



<220>
<221>  misc_feature
<222>  (1)..(3)
<223>  Xaa can be any amino acid

<220>
<221>  misc_feature
<222>  (5)..(5)
<223>  Xaa can be any amino acid

<220>
<221>  misc_feature
<222>  (7)..(9)
<223>  Xaa can be any amino acid

<400>  445

Xaa Xaa Xaa Lys Xaa Pro Xaa Xaa Xaa Ala
1               5                   10


<210>  446
<211>  10
<212>  PRT
<213>  Artificial Sequence

<220>

<223>  epitope

<400>  446

Pro Lys Arg Lys Leu Pro Gly Asp Arg Ala
1               5                   10


<210>  447
<211>  10
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(3)
<223>  Xaa can be any amino acid

<220>
<221>  misc_feature
<222>  (5)..(7)
<223>  Xaa can be any amino acid

<220>
<221>  misc_feature
<222>  (9)..(10)
<223>  Xaa can be any amino acid

<400>  447

Xaa Lys Xaa Pro Xaa Xaa Xaa Ala Xaa Xaa
1               5                   10


<210>  448
<211>  10
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(3)
<223>  Xaa can be any amino acid

<220>

<221>  misc_feature
<222>  (5)..(7)
<223>  Xaa can be any amino acid

<220>
<221>  misc_feature
<222>  (9)..(9)
<223>  Xaa can be any amino acid

<400>  448

Xaa Lys Xaa Pro Xaa Xaa Xaa Ala Xaa Ser
1               5                   10


<210>  449
<211>  10
<212>  PRT
<213>  Artificial Sequence

<220>
<223>  epitope

<400>  449

Arg Lys Leu Pro Gly Asp Arg Ala Thr Ser
1               5                   10


<210>  450
<211>  15
<212>  PRT
<213>  Artificial Sequence

<220>
<223>  epitope



<220>
<221>  misc_feature
<222>  (1)..(1)
<223>  Xaa can be any amino acid

<220>
<221>  misc_feature
<222>  (3)..(6)
<223>  Xaa can be any amino acid

<220>
<221>  misc_feature
<222>  (8)..(8)
<223>  Xaa can be any amino acid

<220>
<221>  misc_feature
<222>  (10)..(12)
<223>  Xaa can be any amino acid

<220>
<221>  misc_feature
<222>  (14)..(15)
<223>  Xaa can be any amino acid

<400>  450

Xaa Glu Xaa Xaa Xaa Xaa Lys Xaa Pro Xaa Xaa Xaa Ala Xaa Xaa
1               5                   10                  15


<210>  451
<211>  10
<212>  PRT
<213>  Artificial Sequence

<220>
<223>  epitope

<400>  451

Phe Val Thr Asn Leu Thr Asp Ser Ser Lys
1               5                   10


<210>  452
<211>  10
<212>  PRT
<213>  Artificial Sequence

<220>
<223>  epitope

<400>  452

Thr Asn Leu Thr Asp Ser Ser Lys Trp Lys
1               5                   10


<210>  453
<211>  10
<212>  PRT
<213>  Artificial Sequence

<220>
<223>  epitope


<220>
<221>  misc_feature
<222>  (2)..(4)
<223>  Xaa can be any amino acid

<220>
<221>  misc_feature
<222>  (6)..(7)
<223>  Xaa can be any amino acid

<220>
<221>  misc_feature
<222>  (9)..(9)
<223>  Xaa can be any amino acid

<400>  453

Thr Xaa Xaa Xaa Lys Xaa Xaa Leu Xaa Ala
1               5                   10

<210>  454
<211>  10
<212>  PRT
<213>  Artificial Sequence

<220>
<223>  epitope


<220>
<221>  misc_feature
<222>  (2)..(4)
<223>  Xaa can be any amino acid

<220>
<221>  misc_feature
<222>  (6)..(6)
<223>  Xaa can be any amino acid

<400>  454

Thr Xaa Xaa Xaa Lys Xaa Lys Leu Cys Ala
1               5                   10


<210>  455
<211>  10
<212>  PRT
<213>  Artificial Sequence

<220>
<223>  epitope

<400>  455

Thr Asp Ser Ser Lys Trp Lys Leu Cys Ala
1               5                   10


<210>  456
<211>  9
<212>  PRT
<213>  Artificial Sequence

<220>
<223>  epitope


<220>
<221>  misc_feature
<222>  (2)..(3)
<223>  Xaa can be any amino acid

<220>
<221>  misc_feature
<222>  (5)..(5)
<223>  Xaa can be any amino acid

<220>
<221>  misc_feature
<222>  (9)..(9)
<223>  Xaa can be any amino acid

<400>  456

Lys Xaa Xaa Leu Xaa Ala Asp Ala Xaa
1               5

<210>  457
<211>  9
<212>  PRT
<213>  Artificial Sequence

<220>
<223>  epitope

<220>
<221>  misc_feature
<222>  (2)..(2)
<223>  Xaa can be any amino acid

<220>
<221>  misc_feature
<222>  (9)..(9)
<223>  Xaa can be any amino acid

<400>  457

Lys Xaa Lys Leu Cys Ala Asp Ala Xaa
1               5

<210>  458
<211>  9
<212>  PRT
<213>  Artificial Sequence

<220>
<223>  epitope

<400>  458

Lys Trp Lys Leu Cys Ala Asp Ala Ser
1               5

<210>  459
<211>  15
<212>  PRT
<213>  Artificial Sequence

<220>
<223>  epitope

<220>
<221>  misc_feature
<222>  (1)..(2)
<223>  Xaa can be any amino acid

<220>
<221>  misc_feature
<222>  (4)..(6)

<223>  Xaa can be any amino acid

<220>
<221>  misc_feature
<222>  (8)..(9)
<223>  Xaa can be any amino acid

<220>
<221>  misc_feature
<222>  (11)..(11)
<223>  Xaa can be any amino acid

<220>
<221>  misc_feature
<222>  (15)..(15)
<223>  Xaa can be any amino acid

<400>  459

Xaa Xaa Thr Xaa Xaa Xaa Lys Xaa Xaa Leu Xaa Ala Asp Ala Xaa
1               5                   10                  15


<210>  460
<211>  5
<212>  PRT
<213>  Artificial Sequence

<220>
<223>  epitope


<220>
<221>  misc_feature
<222>  (5)..(5)
<223>  Xaa can be any amino acid

<400>  460

Lys Ile Lys Thr Xaa
1               5


<210>  461
<211>  5
<212>  PRT
<213>  Artificial Sequence

<220>
<223>  epitope

<400>  461

Lys Ile Lys Thr Phe
1               5


<210>  462
<211>  8
<212>  PRT
<213>  Artificial Sequence

<220>

<223>  epitope

<400>  462

Asn Lys Ile Lys Thr Phe Asn Glu
1               5

<210>  463
<211>  10
<212>  PRT
<213>  Artificial Sequence

<220>
<223>  epitope

<220>
<221>  misc_feature
<222>  (1)..(1)
<223>  Xaa can be any amino acid

<220>
<221>  misc_feature
<222>  (4)..(5)
<223>  Xaa can be any amino acid

<400>  463

Xaa Asn Glu Xaa Xaa Phe Asn Tyr Ser Met
1               5                   10

<210>  464
<211>  10
<212>  PRT
<213>  Artificial Sequence

<220>
<223>  epitope

<400>  464

Phe Asn Glu Val Lys Phe Asn Tyr Ser Met
1               5                   10

<210>  465
<211>  10
<212>  PRT
<213>  Artificial Sequence

<220>
<223>  epitope

<400>  465

Val Lys Phe Asn Tyr Ser Met Pro Ala Asn
1               5                   10

<210>  466
<211>  10

<212>  PRT
<213>  Artificial Sequence

<220>
<223>  epitope

<400>  466

Phe Asn Tyr Ser Met Pro Ala Asn Cys Tyr
1               5                   10


<210>  467
<211>  15
<212>  PRT
<213>  Artificial Sequence

<220>
<223>  epitope


<220>
<221>  misc_feature
<222>  (5)..(5)
<223>  Xaa can be any amino acid

<220>
<221>  misc_feature
<222>  (8)..(9)
<223>  Xaa can be any amino acid

<220>
<221>  misc_feature
<222>  (11)..(12)
<223>  Xaa can be any amino acid

<400>  467

Lys Ile Lys Thr Xaa Asn Glu Xaa Xaa Phe Xaa Xaa Ser Met Pro
1               5                   10                  15


<210>  468
<211>  9
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(3)
<223>  Xaa can be any amino acid

<220>
<221>  misc_feature

<222>  (5)..(6)
<223>  Xaa can be any amino acid

<220>
<221>  misc_feature
<222>  (8)..(9)
<223>  Xaa can be any amino acid

<400>  468

Xaa Ala Xaa Asn Xaa Xaa Ala Xaa Xaa
1               5


<210>  469
<211>  9
<212>  PRT
<213>  Artificial Sequence

<220>
<223>  epitope


<220>
<221>  misc_feature
<222>  (3)..(3)
<223>  Xaa can be any amino acid

<220>
<221>  misc_feature
<222>  (6)..(6)
<223>  Xaa can be any amino acid

<220>
<221>  misc_feature
<222>  (8)..(9)
<223>  Xaa can be any amino acid

<400>  469

Glu Ala Xaa Asn Leu Xaa Ala Xaa Xaa
1               5


<210>  470
<211>  9
<212>  PRT
<213>  Artificial Sequence

<220>
<223>  epitope

<400>  470

Glu Ala Thr Asn Leu Lys Ala Ile Asn
1               5


<210>  471
<211>  10
<212>  PRT
<213>  Artificial Sequence

<220>
<223>  epitope

<400>  471

Gly Glu Ala Thr Asn Leu Lys Ala Ile Asn
1               5                   10


<210>  472
<211>  8
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (4)..(4)
<223>  Xaa can be any amino acid

<220>
<221>  misc_feature
<222>  (7)..(7)
<223>  Xaa can be any amino acid

<400>  472

Xaa Xaa Ile Xaa Ile Gly Xaa His
1               5


<210>  473
<211>  8
<212>  PRT
<213>  Artificial Sequence

<220>
<223>  epitope

<400>  473

Ile Asn Ile Lys Ile Gly Ser His
1               5


<210>  474
<211>  15
<212>  PRT
<213>  Artificial Sequence

<220>
<223>  epitope


<220>

<221>  misc_feature
<222>  (1)..(1)
<223>  Xaa can be any amino acid

<220>
<221>  misc_feature
<222>  (3)..(6)
<223>  Xaa can be any amino acid

<220>
<221>  misc_feature
<222>  (8)..(9)
<223>  Xaa can be any amino acid

<220>
<221>  misc_feature
<222>  (11)..(11)
<223>  Xaa can be any amino acid

<220>
<221>  misc_feature
<222>  (14)..(14)
<223>  Xaa can be any amino acid

<400>  474

Xaa Ala Xaa Xaa Xaa Xaa Ala Xaa Xaa Ile Xaa Ile Gly Xaa His
1               5                   10                  15


<210>  475
<211>  15
<212>  PRT
<213>  Artificial Sequence

<220>
<223>  epitope


<220>
<221>  misc_feature
<222>  (3)..(6)
<223>  Xaa can be any amino acid

<220>
<221>  misc_feature
<222>  (8)..(9)
<223>  Xaa can be any amino acid

<220>
<221>  misc_feature
<222>  (11)..(11)
<223>  Xaa can be any amino acid

<220>
<221>  misc_feature
<222>  (14)..(14)
<223>  Xaa can be any amino acid

<400>  475

Glu Ala Xaa Xaa Xaa Xaa Ala Xaa Xaa Ile Xaa Ile Gly Xaa His
1               5                   10                  15

<210>  476
<211>  10
<212>  PRT
<213>  Artificial Sequence

<220>
<223>  epitope

<400>  476

Ala Ile Asn Ile Lys Ile Gly Ser His Glu
1               5                   10


<210>  477
<211>  15
<212>  PRT
<213>  Artificial Sequence

<220>
<223>  epitope



<220>
<221>  misc_feature
<222>  (1)..(1)
<223>  Xaa can be any amino acid

<220>
<221>  misc_feature
<222>  (3)..(3)
<223>  Xaa can be any amino acid

<220>
<221>  misc_feature
<222>  (5)..(6)
<223>  Xaa can be any amino acid

<220>
<221>  misc_feature
<222>  (8)..(10)
<223>  Xaa can be any amino acid

<220>
<221>  misc_feature
<222>  (14)..(14)
<223>  Xaa can be any amino acid

<400>  477

Xaa Ala Xaa Asn Xaa Xaa Ala Xaa Xaa Xaa Lys Ile Gly Xaa His
1               5                   10                  15


<210>  478
<211>  15
<212>  PRT
<213>  Artificial Sequence

<220>
<223>  epitope

<220>
<221>  misc_feature
<222>  (1)..(1)
<223>  Xaa can be any amino acid

<220>
<221>  misc_feature
<222>  (3)..(3)
<223>  Xaa can be any amino acid

<220>
<221>  misc_feature
<222>  (5)..(5)
<223>  Xaa can be any amino acid

<220>
<221>  misc_feature
<222>  (8)..(10)
<223>  Xaa can be any amino acid

<220>
<221>  misc_feature
<222>  (14)..(14)
<223>  Xaa can be any amino acid

<400>  478

Xaa Ala Xaa Asn Xaa Lys Ala Xaa Xaa Xaa Lys Ile Gly Xaa His
1               5                   10                  15


<210>  479
<211>  10
<212>  PRT
<213>  Artificial Sequence

<220>
<223>  epitope

<400>  479

Lys Ser Ala Gly Glu Ala Thr Asn Leu Lys
1               5                   10


<210>  480
<211>  10
<212>  PRT
<213>  Artificial Sequence

<220>
<223>  epitope

<400>  480

Ala Gly Glu Ala Thr Asn Leu Lys Ala Ile
1               5                   10


<210>  481
<211>  9

<212>  PRT
<213>  Artificial Sequence

<220>
<223>  epitope


<220>
<221>  misc_feature
<222>  (2)..(3)
<223>  Xaa can be any amino acid

<220>
<221>  misc_feature
<222>  (7)..(7)
<223>  Xaa can be any amino acid

<400>  481

Ala Xaa Xaa Leu Lys Ala Xaa Asn Ile
1               5


<210>  482
<211>  9
<212>  PRT
<213>  Artificial Sequence

<220>
<223>  epitope

<400>  482

Ala Thr Asn Leu Lys Ala Ile Asn Ile
1               5


<210>  483
<211>  10
<212>  PRT
<213>  Artificial Sequence

<220>
<223>  epitope

<400>  483

Ala Thr Asn Leu Lys Ala Ile Asn Ile Lys
1               5                   10


<210>  484
<211>  9
<212>  PRT
<213>  Artificial Sequence

<220>
<223>  epitope


<220>
<221>  misc_feature
<222>  (1)..(1)

<223>  Xaa can be any amino acid

<220>
<221>  misc_feature
<222>  (3)..(3)
<223>  Xaa can be any amino acid

<220>
<221>  misc_feature
<222>  (6)..(7)
<223>  Xaa can be any amino acid

<220>
<221>  misc_feature
<222>  (9)..(9)
<223>  Xaa can be any amino acid

<400>  484

Xaa Ala Xaa Asn Ile Xaa Xaa Gly Xaa
1               5


<210>  485
<211>  9
<212>  PRT
<213>  Artificial Sequence

<220>
<223>  epitope


<220>
<221>  misc_feature
<222>  (3)..(3)
<223>  Xaa can be any amino acid

<220>
<221>  misc_feature
<222>  (6)..(7)
<223>  Xaa can be any amino acid

<220>
<221>  misc_feature
<222>  (9)..(9)
<223>  Xaa can be any amino acid

<400>  485

Lys Ala Xaa Asn Ile Xaa Xaa Gly Xaa
1               5


<210>  486
<211>  9
<212>  PRT
<213>  Artificial Sequence

<220>
<223>  epitope

<400>  486

Lys Ala Ile Asn Ile Lys Ile Gly Ser
1               5

<210>  487
<211>  9
<212>  PRT
<213>  Artificial Sequence

<220>
<223>  epitope

<400>  487

Asn Ile Lys Ile Gly Ser His Glu Ile
1               5

<210>  488
<211>  15
<212>  PRT
<213>  Artificial Sequence

<220>
<223>  epitope

<220>
<221>  misc_feature
<222>  (1)..(1)
<223>  Xaa can be any amino acid

<220>
<221>  misc_feature
<222>  (3)..(4)
<223>  Xaa can be any amino acid

<220>
<221>  misc_feature
<222>  (6)..(6)
<223>  Xaa can be any amino acid

<220>
<221>  misc_feature
<222>  (8)..(8)
<223>  Xaa can be any amino acid

<220>
<221>  misc_feature
<222>  (11)..(12)
<223>  Xaa can be any amino acid

<220>
<221>  misc_feature
<222>  (14)..(14)
<223>  Xaa can be any amino acid

<400>  488

Xaa Ala Xaa Xaa Leu Xaa Ala Xaa Asn Ile Xaa Xaa Gly Xaa His
1               5                   10                  15

<210>  489
<211>  15
<212>  PRT
<213>  Artificial Sequence

<220>
<223>  epitope


<220>
<221>  misc_feature
<222>  (3)..(4)
<223>  Xaa can be any amino acid

<220>
<221>  misc_feature
<222>  (8)..(8)
<223>  Xaa can be any amino acid

<220>
<221>  misc_feature
<222>  (11)..(12)
<223>  Xaa can be any amino acid

<220>
<221>  misc_feature
<222>  (14)..(14)
<223>  Xaa can be any amino acid

<400>  489

Glu Ala Xaa Xaa Leu Lys Ala Xaa Asn Ile Xaa Xaa Gly Xaa His
1               5                   10                  15


<210>  490
<211>  6
<212>  PRT
<213>  Artificial Sequence

<220>
<223>  epitope


<220>
<221>  misc_feature
<222>  (4)..(5)
<223>  Xaa can be any amino acid

<400>  490

Ala Ala Pro Xaa Xaa Gly
1               5


<210>  491
<211>  6
<212>  PRT
<213>  Artificial Sequence

<220>
<223>  epitope

<400>  491

Ala Ala Pro Gly His Gly
1               5


<210>  492
<211>  7
<212>  PRT
<213>  Artificial Sequence

<220>
<223>  epitope

<400>  492

Leu Ala Ala Pro Gly His Gly
1               5


<210>  493
<211>  6
<212>  PRT
<213>  Artificial Sequence

<220>
<223>  epitope


<220>
<221>  misc_feature
<222>  (3)..(6)
<223>  Xaa can be any amino acid

<400>  493

Asp Lys Xaa Xaa Xaa Xaa
1               5


<210>  494
<211>  6
<212>  PRT
<213>  Artificial Sequence

<220>
<223>  epitope

<400>  494

Asp Lys Leu Tyr Phe Asp
1               5


<210>  495
<211>  10
<212>  PRT
<213>  Artificial Sequence

<220>
<223>  epitope

<400>  495

Tyr Phe Asp Gly Lys Thr Ile Thr Ile Gln
1               5                   10


<210>  496
<211>  15
<212>  PRT
<213>  Artificial Sequence

<220>
<223>  epitope


<220>
<221>  misc_feature
<222>  (4)..(5)
<223>  Xaa can be any amino acid

<220>
<221>  misc_feature
<222>  (7)..(7)
<223>  Xaa can be any amino acid

<220>
<221>  misc_feature
<222>  (10)..(13)
<223>  Xaa can be any amino acid

<400>  496

Ala Ala Pro Xaa Xaa Gly Xaa Asp Lys Xaa Xaa Xaa Xaa Gly Lys
1               5                   10                  15


<210>  497
<211>  10
<212>  PRT
<213>  Artificial Sequence

<220>
<223>  epitope

<400>  497

Gly Phe Ala Leu Ala Ala Pro Gly His Gly
1               5                   10


<210>  498
<211>  10
<212>  PRT
<213>  Artificial Sequence

<220>
<223>  epitope

<400>  498

Ala Leu Ala Ala Pro Gly His Gly Ile Asp
1               5                   10

<210>  499
<211>  7
<212>  PRT
<213>  Artificial Sequence

<220>
<223>  epitope


<220>
<221>  misc_feature
<222>  (4)..(4)
<223>  Xaa can be any amino acid

<400>  499

Asp Lys Leu Xaa Phe Asp Gly
1               5


<210>  500
<211>  7
<212>  PRT
<213>  Artificial Sequence

<220>
<223>  epitope

<400>  500

Asp Lys Leu Tyr Phe Asp Gly
1               5


<210>  501
<211>  15
<212>  PRT
<213>  Artificial Sequence

<220>
<223>  epitope


<220>
<221>  misc_feature
<222>  (4)..(5)
<223>  Xaa can be any amino acid

<220>
<221>  misc_feature
<222>  (11)..(11)
<223>  Xaa can be any amino acid

<400>  501

Ala Ala Pro Xaa Xaa Gly Ile Asp Lys Leu Xaa Phe Asp Gly Lys
1               5                   10                  15


<210>  502
<211>  15
<212>  PRT
<213>  Artificial Sequence

<220>
<223>  epitope


<220>
<221>  misc_feature
<222>  (4)..(5)
<223>  Xaa can be any amino acid

<400>  502

Ala Ala Pro Xaa Xaa Gly Ile Asp Lys Leu Tyr Phe Asp Gly Lys
1               5                   10                  15


<210>  503
<211>  10
<212>  PRT
<213>  Artificial Sequence

<220>
<223>  epitope

<400>  503

Gly Phe Ala Leu Ala Ala Pro Gly His Gly
1               5                   10


<210>  504
<211>  8
<212>  PRT
<213>  Artificial Sequence

<220>
<223>  epitope


<220>
<221>  misc_feature
<222>  (3)..(6)
<223>  Xaa can be any amino acid

<400>  504

Ala Ala Xaa Xaa Xaa Xaa Ile Asp
1               5


<210>  505
<211>  8
<212>  PRT
<213>  Artificial Sequence

<220>
<223>  epitope

<400>  505

Ala Ala Pro Gly His Gly Ile Asp
1               5

<210>  506
<211>  10
<212>  PRT
<213>  Artificial Sequence

<220>
<223>  epitope


<220>
<221>  misc_feature
<222>  (1)..(4)
<223>  Xaa can be any amino acid

<220>
<221>  misc_feature
<222>  (8)..(10)
<223>  Xaa can be any amino acid

<400>  506

Xaa Xaa Xaa Xaa Ile Asp Lys Xaa Xaa Xaa
1               5                   10


<210>  507
<211>  10
<212>  PRT
<213>  Artificial Sequence

<220>
<223>  epitope

<400>  507

Pro Gly His Gly Ile Asp Lys Leu Tyr Phe
1               5                   10


<210>  508
<211>  9
<212>  PRT
<213>  Artificial Sequence

<220>
<223>  epitope


<220>
<221>  misc_feature
<222>  (3)..(7)
<223>  Xaa can be any amino acid

<400>  508

Asp Lys Xaa Xaa Xaa Xaa Xaa Lys Thr
1               5


<210>  509
<211>  9
<212>  PRT

<213>  Artificial Sequence

<220>
<223>  epitope

<400>  509

Asp Lys Leu Tyr Phe Asp Gly Lys Thr
1               5


<210>  510
<211>  15
<212>  PRT
<213>  Artificial Sequence

<220>
<223>  epitope


<220>
<221>  misc_feature
<222>  (3)..(6)
<223>  Xaa can be any amino acid

<220>
<221>  misc_feature
<222>  (10)..(14)
<223>  Xaa can be any amino acid

<400>  510

Ala Ala Xaa Xaa Xaa Xaa Ile Asp Lys Xaa Xaa Xaa Xaa Xaa Lys
1               5                   10                  15


<210>  511
<211>  10
<212>  PRT
<213>  Artificial Sequence

<220>
<223>  epitope


<220>
<221>  misc_feature
<222>  (2)..(2)
<223>  Xaa can be any amino acid

<400>  511

Met Xaa Asn Gly Tyr Leu Ala Lys Asn Ala
1               5                   10


<210>  512
<211>  10
<212>  PRT
<213>  Artificial Sequence

<220>
<223>  epitope

<400>  512

Met Pro Asn Gly Tyr Leu Ala Lys Asn Ala
1               5                   10


<210>  513
<211>  15
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (4)..(5)
<223>  Xaa can be any amino acid

<220>
<221>  misc_feature
<222>  (7)..(7)
<223>  Xaa can be any amino acid

<220>
<221>  misc_feature
<222>  (11)..(11)
<223>  Xaa can be any amino acid

<400>  513

Xaa Xaa Glu Xaa Xaa Met Xaa Asn Gly Tyr Xaa Ala Lys Asn Ala
1               5                   10                  15


<210>  514
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (4)..(5)
<223>  Xaa can be any amino acid

<220>
<221>  misc_feature

<222>  (7)..(7)
<223>  Xaa can be any amino acid

<400>  514

Xaa Gln Glu Xaa Xaa Met Xaa Asn Gly Tyr Leu Ala Lys Asn Ala
1               5                   10                  15


<210>  515
<211>  10
<212>  PRT
<213>  Artificial Sequence

<220>
<223>  epitope

<400>  515

Gly Tyr Leu Ala Lys Asn Ala Val Ser Phe
1               5                   10


<210>  516
<211>  9
<212>  PRT
<213>  Artificial Sequence

<220>
<223>  epitope

<400>  516

Pro Cys Arg Gly Ala Cys Lys Leu His
1               5


<210>  517
<211>  10
<212>  PRT
<213>  Artificial Sequence

<220>
<223>  epitope

<400>  517

Ala Pro Cys Arg Gly Ala Cys Lys Leu His
1               5                   10


<210>  518
<211>  10
<212>  PRT
<213>  Artificial Sequence

<220>
<223>  epitope


<220>
<221>  misc_feature
<222>  (2)..(3)

<223>  Xaa can be any amino acid

<220>
<221>  misc_feature
<222>  (5)..(7)
<223>  Xaa can be any amino acid

<400>  518

Ala Xaa Xaa Leu Xaa Xaa Xaa Phe Val Lys
1               5                   10


<210>  519
<211>  10
<212>  PRT
<213>  Artificial Sequence

<220>
<223>  epitope


<220>
<221>  misc_feature
<222>  (2)..(3)
<223>  Xaa can be any amino acid

<220>
<221>  misc_feature
<222>  (5)..(6)
<223>  Xaa can be any amino acid

<400>  519

Ala Xaa Xaa Leu Xaa Xaa Ser Phe Val Lys
1               5                   10


<210>  520
<211>  10
<212>  PRT
<213>  Artificial Sequence

<220>
<223>  epitope

<400>  520

Ala Cys Lys Leu His Arg Ser Phe Val Lys
1               5                   10


<210>  521
<211>  7
<212>  PRT
<213>  Artificial Sequence

<220>
<223>  epitope


<220>
<221>  misc_feature

<222>  (1)..(1)
<223>  Xaa can be any amino acid

<220>
<221>  misc_feature
<222>  (7)..(7)
<223>  Xaa can be any amino acid

<400>  521

Xaa Ser Phe Val Lys Leu Xaa
1               5


<210>  522
<211>  7
<212>  PRT
<213>  Artificial Sequence

<220>
<223>  epitope

<400>  522

Arg Ser Phe Val Lys Leu Glu
1               5


<210>  523
<211>  10
<212>  PRT
<213>  Artificial Sequence

<220>
<223>  epitope

<400>  523

Phe Val Lys Leu Glu Lys Thr Val Gln Leu
1               5                   10


<210>  524
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(4)
<223>  Xaa can be any amino acid

<220>
<221>  misc_feature

<222>  (6)..(8)
<223>  Xaa can be any amino acid

<220>
<221>  misc_feature
<222>  (13)..(15)
<223>  Xaa can be any amino acid

<400>  524

Xaa Ala Xaa Xaa Leu Xaa Xaa Xaa Phe Val Lys Leu Xaa Xaa Xaa
1               5                   10                  15


<210>  525
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(4)
<223>  Xaa can be any amino acid

<220>
<221>  misc_feature
<222>  (6)..(7)
<223>  Xaa can be any amino acid

<220>
<221>  misc_feature
<222>  (13)..(15)
<223>  Xaa can be any amino acid

<400>  525

Xaa Ala Xaa Xaa Leu Xaa Xaa Ser Phe Val Lys Leu Xaa Xaa Xaa
1               5                   10                  15


<210>  526
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (7)..(9)
<223>  Xaa can be any amino acid

<220>
<221>  misc_feature
<222>  (11)..(15)
<223>  Xaa can be any amino acid

<400>  526

Xaa Pro Arg Thr Cys Asp Xaa Xaa Xaa Lys Xaa Xaa Xaa Xaa Xaa
1               5                   10                  15


<210>  527
<211>  15
<212>  PRT
<213>  Artificial Sequence

<220>
<223>  epitope


<220>
<221>  misc_feature
<222>  (4)..(8)
<223>  Xaa can be any amino acid

<220>
<221>  misc_feature
<222>  (10)..(10)
<223>  Xaa can be any amino acid

<220>
<221>  misc_feature
<222>  (13)..(15)
<223>  Xaa can be any amino acid

<400>  527

Ser Pro Arg Xaa Xaa Xaa Xaa Xaa Leu Xaa Leu Pro Xaa Xaa Xaa
1               5                   10                  15


<210>  528
<211>  5
<212>  PRT
<213>  Artificial Sequence

<220>
<223>  epitope

<400>  528

Gly Leu Leu Ile Ser
1               5


<210>  529
<211>  15
<212>  PRT

<213>  Artificial Sequence

<220>
<223>  epitope


<220>
<221>  misc_feature
<222>  (2)..(7)
<223>  Xaa can be any amino acid

<220>
<221>  misc_feature
<222>  (9)..(15)
<223>  Xaa can be any amino acid

<400>  529

Ser Xaa Xaa Xaa Xaa Xaa Xaa Glu Xaa Xaa Xaa Xaa Xaa Xaa Xaa
1               5                   10                  15


<210>  530
<211>  15
<212>  PRT
<213>  Artificial Sequence

<220>
<223>  epitope


<220>
<221>  misc_feature
<222>  (2)..(5)
<223>  Xaa can be any amino acid

<220>
<221>  misc_feature
<222>  (7)..(7)
<223>  Xaa can be any amino acid

<220>
<221>  misc_feature
<222>  (9)..(15)
<223>  Xaa can be any amino acid

<400>  530

Ser Xaa Xaa Xaa Xaa Ser Xaa Glu Xaa Xaa Xaa Xaa Xaa Xaa Xaa
1               5                   10                  15


<210>  531
<211>  5
<212>  PRT
<213>  Artificial Sequence

<220>
<223>  epitope

<400>  531

Gly Leu Leu Ile Ser

1                5


<210>  532
<211>  15
<212>  PRT
<213>  Artificial Sequence

<220>
<223>  epitope


<220>
<221>  misc_feature
<222>  (1)..(3)
<223>  Xaa can be any amino acid

<220>
<221>  misc_feature
<222>  (5)..(5)
<223>  Xaa can be any amino acid

<220>
<221>  misc_feature
<222>  (7)..(15)
<223>  Xaa can be any amino acid

<400>  532

Xaa Xaa Xaa Leu Xaa Ser Xaa Xaa Xaa Xaa Xaa Xaa Xaa Xaa Xaa
1               5                   10                  15


<210>  533
<211>  8
<212>  PRT
<213>  Artificial Sequence

<220>
<223>  epitope

<400>  533

Ile Cys Gly Lys Tyr Asp Ala Glu
1               5


<210>  534
<211>  9
<212>  PRT
<213>  Artificial Sequence

<220>
<223>  epitope

<400>  534

Gly Ile Cys Gly Lys Tyr Asp Ala Glu
1               5


<210>  535
<211>  9

<212>  PRT
<213>  Artificial Sequence

<220>
<223>  epitope

<400>  535

Tyr Asp Ala Glu Lys Lys Arg Glu Tyr
1               5


<210>  536
<211>  15
<212>  PRT
<213>  Artificial Sequence

<220>
<223>  epitope


<220>
<221>  misc_feature
<222>  (3)..(13)
<223>  Xaa can be any amino acid

<400>  536

Gly Lys Xaa Xaa Xaa Xaa Xaa Xaa Xaa Xaa Xaa Xaa Xaa Lys Lys
1               5                   10                  15


<210>  537
<211>  15
<212>  PRT
<213>  Artificial Sequence

<220>
<223>  epitope


<220>
<221>  misc_feature
<222>  (3)..(12)
<223>  Xaa can be any amino acid

<400>  537

Gly Lys Xaa Xaa Xaa Xaa Xaa Xaa Xaa Xaa Xaa Xaa Glu Lys Lys
1               5                   10                  15


<210>  538
<211>  9
<212>  PRT
<213>  Artificial Sequence

<220>
<223>  epitope


<220>
<221>  misc_feature

<222>  (2)..(2)
<223>  Xaa can be any amino acid

<220>
<221>  misc_feature
<222>  (4)..(9)
<223>  Xaa can be any amino acid

<400>  538

Cys Xaa Ile Xaa Xaa Xaa Xaa Xaa Xaa
1               5


<210>  539
<211>  9
<212>  PRT
<213>  Artificial Sequence

<220>
<223>  epitope

<400>  539

Cys Gly Ile Cys Gly Lys Tyr Asp Ala
1               5


<210>  540
<211>  10
<212>  PRT
<213>  Artificial Sequence

<220>
<223>  epitope


<220>
<221>  misc_feature
<222>  (2)..(7)
<223>  Xaa can be any amino acid

<400>  540

Ile Xaa Xaa Xaa Xaa Xaa Xaa Glu Lys Lys
1               5                   10


<210>  541
<211>  10
<212>  PRT
<213>  Artificial Sequence

<220>
<223>  epitope

<400>  541

Ile Cys Gly Lys Tyr Asp Ala Glu Lys Lys
1               5                   10


<210>  542

<211>  8
<212>  PRT
<213>  Artificial Sequence

<220>
<223>  epitope

<400>  542

Tyr Asp Ala Glu Lys Lys Arg Glu
1               5


<210>  543
<211>  6
<212>  PRT
<213>  Artificial Sequence

<220>
<223>  epitope


<220>
<221>  misc_feature
<222>  (1)..(3)
<223>  Xaa can be any amino acid

<400>  543

Xaa Xaa Xaa Glu Lys Lys
1               5


<210>  544
<211>  6
<212>  PRT
<213>  Artificial Sequence

<220>
<223>  epitope

<400>  544

Tyr Asp Ala Glu Lys Lys
1               5


<210>  545
<211>  15
<212>  PRT
<213>  Artificial Sequence

<220>
<223>  epitope


<220>
<221>  misc_feature
<222>  (1)..(2)
<223>  Xaa can be any amino acid

<220>
<221>  misc_feature

<222>  (5)..(12)
<223>  Xaa can be any amino acid

<400>  545

Xaa Xaa Thr Cys Xaa Xaa Xaa Xaa Xaa Xaa Xaa Xaa Glu Lys Lys
1               5                   10                  15


<210>  546
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (5)..(5)
<223>  Xaa can be any amino acid

<220>
<221>  misc_feature
<222>  (7)..(12)
<223>  Xaa can be any amino acid

<400>  546

Xaa Lys Thr Cys Xaa Ile Xaa Xaa Xaa Xaa Xaa Xaa Glu Lys Lys
1               5                   10                  15


<210>  547
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(5)
<223>  Xaa can be any amino acid

<400>  547

Xaa Ala Xaa Xaa Xaa Arg Glu Tyr Gln Met Pro Ser Gly Tyr Leu
1               5                   10                  15

<210>  548
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(8)
<223>  Xaa can be any amino acid

<220>
<221>  misc_feature
<222>  (13)..(14)
<223>  Xaa can be any amino acid

<400>  548

Xaa Ala Xaa Xaa Xaa Xaa Xaa Xaa Gln Met Pro Ser Xaa Xaa Leu
1               5                   10                  15


<210>  549
<211>  15
<212>  PRT
<213>  Artificial Sequence

<220>
<223>  epitope


<220>
<221>  misc_feature
<222>  (3)..(8)
<223>  Xaa can be any amino acid

<220>
<221>  misc_feature
<222>  (10)..(13)
<223>  Xaa can be any amino acid

<400>  549

Asp Ala Xaa Xaa Xaa Xaa Xaa Xaa Gln Xaa Xaa Xaa Xaa Tyr Leu
1               5                   10                  15


<210>  550
<211>  9
<212>  PRT
<213>  Artificial Sequence

<220>
<223>  epitope

<400>  550

Glu Asp Thr Cys Thr Gly Ala Cys Lys
1               5


<210>  551
<211>  8
<212>  PRT
<213>  Artificial Sequence

<220>
<223>  epitope

<400>  551

Gln Arg Lys Phe Val Lys Ile Glu
1               5


<210>  552
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(5)
<223>  Xaa can be any amino acid

<220>
<221>  misc_feature
<222>  (10)..(15)
<223>  Xaa can be any amino acid

<400>  552

Xaa Thr Xaa Xaa Xaa Ala Cys Lys Leu Xaa Xaa Xaa Xaa Xaa Xaa
1               5                   10                  15


<210>  553
<211>  10
<212>  PRT
<213>  Artificial Sequence

<220>
<223>  epitope

<400>  553

Ile Val Thr Leu Ala Glu Lys Tyr Asp Leu
1               5                   10

<210>  554
<211>  8
<212>  PRT
<213>  Artificial Sequence

<220>
<223>  epitope


<220>
<221>  misc_feature
<222>  (3)..(8)
<223>  Xaa can be any amino acid

<400>  554

Ala Glu Xaa Xaa Xaa Xaa Xaa Xaa
1               5


<210>  555
<211>  8
<212>  PRT
<213>  Artificial Sequence

<220>
<223>  epitope

<400>  555

Ala Glu Lys Tyr Asp Leu Lys Asn
1               5


<210>  556
<211>  10
<212>  PRT
<213>  Artificial Sequence

<220>
<223>  epitope

<400>  556

Asn Trp Ala Glu Asn Leu Asn Gln Phe Ile
1               5                   10


<210>  557
<211>  10
<212>  PRT
<213>  Artificial Sequence

<220>
<223>  epitope

<400>  557

Ala Glu Asn Leu Asn Gln Phe Ile Glu Thr
1               5                   10

<210>  558
<211>  15
<212>  PRT
<213>  Artificial Sequence

<220>
<223>  epitope


<220>
<221>  misc_feature
<222>  (3)..(12)
<223>  Xaa can be any amino acid

<220>
<221>  misc_feature
<222>  (15)..(15)
<223>  Xaa can be any amino acid

<400>  558

Ala Glu Xaa Xaa Xaa Xaa Xaa Xaa Xaa Xaa Xaa Xaa Leu Asn Xaa
1               5                   10                  15


<210>  559
<211>  5
<212>  PRT
<213>  Artificial Sequence

<220>
<223>  epitope


<220>
<221>  misc_feature
<222>  (1)..(1)
<223>  Xaa can be any amino acid

<400>  559

Xaa Glu Pro Leu Asn
1               5


<210>  560
<211>  6
<212>  PRT
<213>  Artificial Sequence

<220>
<223>  epitope


<220>
<221>  misc_feature
<222>  (1)..(1)
<223>  Xaa can be any amino acid

<400>  560

Xaa Glu Pro Leu Asn Val

1               5


<210>  561
<211>  5
<212>  PRT
<213>  Artificial Sequence

<220>
<223>  epitope

<400>  561

Asp Glu Pro Leu Asn
1               5


<210>  562
<211>  6
<212>  PRT
<213>  Artificial Sequence

<220>
<223>  epitope

<400>  562

Asp Glu Pro Leu Asn Val
1               5


<210>  563
<211>  15
<212>  PRT
<213>  Artificial Sequence

<220>
<223>  epitope


<220>
<221>  misc_feature
<222>  (1)..(13)
<223>  Xaa can be any amino acid

<400>  563

Xaa Xaa Xaa Xaa Xaa Xaa Xaa Xaa Xaa Xaa Xaa Xaa Xaa Asn Val
1               5                   10                  15


<210>  564
<211>  15
<212>  PRT
<213>  Artificial Sequence

<220>
<223>  epitope


<220>
<221>  misc_feature
<222>  (1)..(9)

<223>  Xaa can be any amino acid

<400>  564

Xaa Xaa Xaa Xaa Xaa Xaa Xaa Xaa Xaa Asp Glu Pro Leu Asn Val
1               5                   10                  15


<210>  565
<211>  9
<212>  PRT
<213>  Artificial Sequence

<220>
<223>  epitope

<400>  565

Gln Asp Gly Thr Lys Val Lys Leu Tyr
1               5


<210>  566
<211>  9
<212>  PRT
<213>  Artificial Sequence

<220>
<223>  epitope

<400>  566

Gln Asp Gly Thr Lys Val Lys Leu Tyr
1               5


<210>  567
<211>  10
<212>  PRT
<213>  Artificial Sequence

<220>
<223>  epitope


<220>
<221>  misc_feature
<222>  (1)..(6)
<223>  Xaa can be any amino acid

<220>
<221>  misc_feature
<222>  (8)..(8)
<223>  Xaa can be any amino acid

<220>
<221>  misc_feature
<222>  (10)..(10)
<223>  Xaa can be any amino acid

<400>  567

Xaa Xaa Xaa Xaa Xaa Xaa Lys Xaa Glu Xaa

1                   5                   10


<210>  568
<211>  10
<212>  PRT
<213>  Artificial Sequence

<220>
<223>  epitope



<220>
<221>  misc_feature
<222>  (1)..(1)
<223>  Xaa can be any amino acid

<220>
<221>  misc_feature
<222>  (6)..(6)
<223>  Xaa can be any amino acid

<220>
<221>  misc_feature
<222>  (8)..(10)
<223>  Xaa can be any amino acid

<400>  568

Xaa Lys Leu Tyr Pro Xaa Lys Xaa Xaa Xaa
1               5                   10


<210>  569
<211>  10
<212>  PRT
<213>  Artificial Sequence

<220>
<223>  epitope

<400>  569

Val Lys Leu Tyr Pro Glu Lys Asp Glu Pro
1               5                   10


<210>  570
<211>  7
<212>  PRT
<213>  Artificial Sequence

<220>
<223>  epitope



<220>
<221>  misc_feature
<222>  (2)..(2)
<223>  Xaa can be any amino acid

<220>
<221>  misc_feature

<222>  (4)..(5)
<223>  Xaa can be any amino acid

<220>
<221>  misc_feature
<222>  (7)..(7)
<223>  Xaa can be any amino acid

<400>  570

Lys Xaa Glu Xaa Xaa Asn Xaa
1               5


<210>  571
<211>  7
<212>  PRT
<213>  Artificial Sequence

<220>
<223>  epitope

<400>  571

Lys Asp Glu Pro Leu Asn Val
1               5


<210>  572
<211>  4
<212>  PRT
<213>  Artificial Sequence

<220>
<223>  epitope


<220>
<221>  misc_feature
<222>  (2)..(2)
<223>  Xaa can be any amino acid

<220>
<221>  misc_feature
<222>  (4)..(4)
<223>  Xaa can be any amino acid

<400>  572

Lys Xaa Glu Xaa
1


<210>  573
<211>  4
<212>  PRT
<213>  Artificial Sequence

<220>
<223>  epitope

<400>  573

Lys Asp Glu Pro
1


<210> 574
<211> 15
<212> PRT
<213> Artificial Sequence

<220>
<223> epitope



<220>
<221> misc_feature
<222> (1)..(8)
<223> Xaa can be any amino acid

<220>
<221> misc_feature
<222> (10)..(10)
<223> Xaa can be any amino acid

<220>
<221> misc_feature
<222> (12)..(13)
<223> Xaa can be any amino acid

<220>
<221> misc_feature
<222> (15)..(15)
<223> Xaa can be any amino acid

<400> 574

Xaa Xaa Xaa Xaa Xaa Xaa Xaa Xaa Lys Xaa Glu Xaa Xaa Asn Xaa
1               5                   10                  15


<210> 575
<211> 15
<212> PRT
<213> Artificial Sequence

<220>
<223> epitope



<220>
<221> misc_feature
<222> (1)..(3)
<223> Xaa can be any amino acid

<220>
<221> misc_feature
<222> (8)..(8)
<223> Xaa can be any amino acid

<220>
<221> misc_feature
<222> (10)..(10)
<223> Xaa can be any amino acid

<220>
<221>  misc_feature
<222>  (12)..(13)
<223>  Xaa can be any amino acid

<220>
<221>  misc_feature
<222>  (15)..(15)
<223>  Xaa can be any amino acid

<400>  575

Xaa Xaa Xaa Lys Leu Tyr Pro Xaa Lys Xaa Glu Xaa Xaa Asn Xaa
1               5                   10                  15


<210>  576
<211>  8
<212>  PRT
<213>  Artificial Sequence

<220>
<223>  epitope

<400>  576

Tyr Gln Val Ser Lys Arg Val Ser
1               5


<210>  577
<211>  9
<212>  PRT
<213>  Artificial Sequence

<220>
<223>  epitope

<400>  577

Leu Asn Pro Ile Ser Ala Lys Val Glu
1               5


<210>  578
<211>  6
<212>  PRT
<213>  Artificial Sequence

<220>
<223>  epitope


<220>
<221>  misc_feature
<222>  (5)..(6)
<223>  Xaa can be any amino acid

<400>  578

Val Ser Lys Arg Xaa Xaa
1               5

<210>  579
<211>  6
<212>  PRT
<213>  Artificial Sequence

<220>
<223>  epitope

<400>  579

Val Ser Lys Arg Val Ser
1               5


<210>  580
<211>  7
<212>  PRT
<213>  Artificial Sequence

<220>
<223>  epitope


<220>
<221>  misc_feature
<222>  (4)..(7)
<223>  Xaa can be any amino acid

<400>  580

Ser Val Pro Xaa Xaa Xaa Xaa
1               5


<210>  581
<211>  7
<212>  PRT
<213>  Artificial Sequence

<220>
<223>  epitope

<400>  581

Ser Val Pro Gly Leu Asn Pro
1               5


<210>  582
<211>  15
<212>  PRT
<213>  Artificial Sequence

<220>
<223>  epitope


<220>
<221>  misc_feature
<222>  (5)..(6)
<223>  Xaa can be any amino acid

<220>
<221>  misc_feature
<222>  (8)..(15)
<223>  Xaa can be any amino acid

<400>  582

Val Ser Lys Arg Xaa Xaa Val Xaa Xaa Xaa Xaa Xaa Xaa Xaa Xaa
1               5                   10                  15


<210>  583
<211>  9
<212>  PRT
<213>  Artificial Sequence

<220>
<223>  epitope

<400>  583

Asp Ile Phe Glu Leu Gly Leu Asp Gly
1               5


<210>  584
<211>  9
<212>  PRT
<213>  Artificial Sequence

<220>
<223>  epitope

<400>  584

Glu Pro Thr Leu Glu Ala Leu Phe Gly
1               5


<210>  585
<211>  5
<212>  PRT
<213>  Artificial Sequence

<220>
<223>  epitope

<400>  585

Gly Lys Gly Phe Glu
1               5


<210>  586
<211>  4
<212>  PRT
<213>  Artificial Sequence

<220>
<223>  epitope


<220>

<221>  misc_feature
<222>  (3)..(4)
<223>  Xaa can be any amino acid

<400>  586

Ser Gly Xaa Xaa
1


<210>  587
<211>  4
<212>  PRT
<213>  Artificial Sequence

<220>
<223>  epitope

<400>  587

Ser Gly Ile Val
1


<210>  588
<211>  10
<212>  PRT
<213>  Artificial Sequence

<220>
<223>  epitope


<220>
<221>  misc_feature
<222>  (1)..(3)
<223>  Xaa can be any amino acid

<220>
<221>  misc_feature
<222>  (5)..(5)
<223>  Xaa can be any amino acid

<220>
<221>  misc_feature
<222>  (7)..(8)
<223>  Xaa can be any amino acid

<220>
<221>  misc_feature
<222>  (10)..(10)
<223>  Xaa can be any amino acid

<400>  588

Xaa Xaa Xaa Pro Xaa Ala Xaa Xaa Ala Xaa
1               5                   10


<210>  589
<211>  10
<212>  PRT
<213>  Artificial Sequence

<220>
<223>  epitope

<400>  589

Ile Val Thr Pro Gly Ala Lys Val Ala Val
1               5                   10


<210>  590
<211>  15
<212>  PRT
<213>  Artificial Sequence

<220>
<223>  epitope


<220>
<221>  misc_feature
<222>  (1)..(3)
<223>  Xaa can be any amino acid

<220>
<221>  misc_feature
<222>  (5)..(7)
<223>  Xaa can be any amino acid

<220>
<221>  misc_feature
<222>  (9)..(9)
<223>  Xaa can be any amino acid

<220>
<221>  misc_feature
<222>  (11)..(12)
<223>  Xaa can be any amino acid

<220>
<221>  misc_feature
<222>  (14)..(15)
<223>  Xaa can be any amino acid

<400>  590

Xaa Xaa Xaa Gly Xaa Xaa Xaa Pro Xaa Ala Xaa Xaa Ala Xaa Xaa
1               5                   10                  15


<210>  591
<211>  9
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(6)
<223>  Xaa can be any amino acid

<400>  591

Xaa Gly Xaa Xaa Xaa Xaa Gly Ala Lys
1               5


<210>  592
<211>  9
<212>  PRT
<213>  Artificial Sequence

<220>
<223>  epitope

<400>  592

Ser Gly Ile Val Thr Pro Gly Ala Lys
1               5


<210>  593
<211>  10
<212>  PRT
<213>  Artificial Sequence

<220>
<223>  epitope


<220>
<221>  misc_feature
<222>  (1)..(4)
<223>  Xaa can be any amino acid

<400>  593

Xaa Xaa Xaa Xaa Gly Ala Lys Val Ala Val
1               5                   10


<210>  594
<211>  10
<212>  PRT
<213>  Artificial Sequence

<220>
<223>  epitope

<400>  594

Ile Val Thr Pro Gly Ala Lys Val Ala Val
1               5                   10


<210>  595
<211>  10
<212>  PRT

<213>  Artificial Sequence

<220>
<223>  epitope

<400>  595

Ala Lys Val Ala Val Lys Leu His Gln Lys
1               5                   10


<210>  596
<211>  15
<212>  PRT
<213>  Artificial Sequence

<220>
<223>  epitope


<220>
<221>  misc_feature
<222>  (1)..(3)
<223>  Xaa can be any amino acid

<220>
<221>  misc_feature
<222>  (5)..(8)
<223>  Xaa can be any amino acid

<220>
<221>  misc_feature
<222>  (15)..(15)
<223>  Xaa can be any amino acid

<400>  596

Xaa Xaa Xaa Gly Xaa Xaa Xaa Xaa Gly Ala Lys Val Ala Val Xaa
1               5                   10                  15


<210>  597
<211>  8
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (4)..(5)
<223>  Xaa can be any amino acid

<220>
<221>  misc_feature
<222>  (7)..(7)

<223>  Xaa can be any amino acid

<400>  597

Xaa Xaa Ile Xaa Xaa Pro Xaa Ala
1               5


<210>  598
<211>  8
<212>  PRT
<213>  Artificial Sequence

<220>
<223>  epitope

<400>  598

Ser Gly Ile Val Thr Pro Gly Ala
1               5


<210>  599
<211>  10
<212>  PRT
<213>  Artificial Sequence

<220>
<223>  epitope


<220>
<221>  misc_feature
<222>  (2)..(3)
<223>  Xaa can be any amino acid

<220>
<221>  misc_feature
<222>  (5)..(5)
<223>  Xaa can be any amino acid

<220>
<221>  misc_feature
<222>  (7)..(8)
<223>  Xaa can be any amino acid

<220>
<221>  misc_feature
<222>  (10)..(10)
<223>  Xaa can be any amino acid

<400>  599

Ile Xaa Xaa Pro Xaa Ala Xaa Xaa Ala Xaa
1               5                   10


<210>  600
<211>  10
<212>  PRT
<213>  Artificial Sequence

<220>

<223>  epitope

<400>  600

Ile Val Thr Pro Gly Ala Lys Val Ala Val
1               5                   10


<210>  601
<211>  8
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(3)
<223>  Xaa can be any amino acid

<220>
<221>  misc_feature
<222>  (5)..(6)
<223>  Xaa can be any amino acid

<220>
<221>  misc_feature
<222>  (8)..(8)
<223>  Xaa can be any amino acid

<400>  601

Xaa Pro Xaa Ala Xaa Xaa Ala Xaa
1               5


<210>  602
<211>  8
<212>  PRT
<213>  Artificial Sequence

<220>
<223>  epitope

<400>  602

Thr Pro Gly Ala Lys Val Ala Val
1               5


<210>  603
<211>  15
<212>  PRT
<213>  Artificial Sequence

<220>

<223>  epitope

<220>
<221>  misc_feature
<222>  (1)..(4)
<223>  Xaa can be any amino acid

<220>
<221>  misc_feature
<222>  (6)..(7)
<223>  Xaa can be any amino acid

<220>
<221>  misc_feature
<222>  (9)..(9)
<223>  Xaa can be any amino acid

<220>
<221>  misc_feature
<222>  (11)..(12)
<223>  Xaa can be any amino acid

<220>
<221>  misc_feature
<222>  (14)..(14)
<223>  Xaa can be any amino acid

<400>  603

Xaa Xaa Xaa Xaa Ile Xaa Xaa Pro Xaa Ala Xaa Xaa Ala Xaa Lys
1               5                   10                  15


<210>  604
<211>  8
<212>  PRT
<213>  Artificial Sequence

<220>
<223>  epitope

<400>  604

Leu Val Asn Tyr Ser Pro Thr Lys
1               5


<210>  605
<211>  9
<212>  PRT
<213>  Artificial Sequence

<220>
<223>  epitope

<400>  605

Leu Val Asn Tyr Ser Pro Thr Lys Gly
1               5


<210>  606

<211>  10
<212>  PRT
<213>  Artificial Sequence

<220>
<223>  epitope


<220>
<221>  misc_feature
<222>  (2)..(4)
<223>  Xaa can be any amino acid

<220>
<221>  misc_feature
<222>  (6)..(7)
<223>  Xaa can be any amino acid

<400>  606

Pro Xaa Xaa Xaa Tyr Xaa Xaa Met Ser Ser
1               5                   10


<210>  607
<211>  10
<212>  PRT
<213>  Artificial Sequence

<220>
<223>  epitope


<220>
<221>  misc_feature
<222>  (2)..(4)
<223>  Xaa can be any amino acid

<220>
<221>  misc_feature
<222>  (7)..(7)
<223>  Xaa can be any amino acid

<400>  607

Pro Xaa Xaa Xaa Tyr Leu Xaa Met Ser Ser
1               5                   10


<210>  608
<211>  10
<212>  PRT
<213>  Artificial Sequence

<220>
<223>  epitope

<400>  608

Pro Thr Lys Gly Tyr Leu Gln Met Ser Ser
1               5                   10

<210>  609
<211>  7
<212>  PRT
<213>  Artificial Sequence

<220>
<223>  epitope

<400>  609

Leu Gln Met Ser Ser Ser Val
1               5


<210>  610
<211>  15
<212>  PRT
<213>  Artificial Sequence

<220>
<223>  epitope


<220>
<221>  misc_feature
<222>  (1)..(4)
<223>  Xaa can be any amino acid

<220>
<221>  misc_feature
<222>  (6)..(8)
<223>  Xaa can be any amino acid

<220>
<221>  misc_feature
<222>  (10)..(11)
<223>  Xaa can be any amino acid

<400>  610

Xaa Xaa Xaa Xaa Pro Xaa Xaa Xaa Tyr Xaa Xaa Met Ser Ser Ser
1               5                   10                  15


<210>  611
<211>  15
<212>  PRT
<213>  Artificial Sequence

<220>
<223>  epitope


<220>
<221>  misc_feature
<222>  (1)..(4)
<223>  Xaa can be any amino acid

<220>
<221>  misc_feature
<222>  (6)..(8)
<223>  Xaa can be any amino acid

<220>
<221>  misc_feature
<222>  (11)..(11)
<223>  Xaa can be any amino acid

<400>  611

Xaa Xaa Xaa Xaa Pro Xaa Xaa Xaa Tyr Leu Xaa Met Ser Ser Ser
1               5                   10                  15


<210>  612
<211>  15
<212>  PRT
<213>  Artificial Sequence

<220>
<223>  epitope


<220>
<221>  misc_feature
<222>  (1)..(6)
<223>  Xaa can be any amino acid

<220>
<221>  misc_feature
<222>  (8)..(8)
<223>  Xaa can be any amino acid

<220>
<221>  misc_feature
<222>  (10)..(10)
<223>  Xaa can be any amino acid

<220>
<221>  misc_feature
<222>  (15)..(15)
<223>  Xaa can be any amino acid

<400>  612

Xaa Xaa Xaa Xaa Xaa Xaa Lys Xaa Tyr Xaa Gln Met Ser Ser Xaa
1               5                   10                  15


<210>  613
<211>  7
<212>  PRT
<213>  Artificial Sequence

<220>
<223>  epitope

<400>  613

Ala Asn Glu Leu Leu Asp Arg
1               5


<210>  614
<211>  9
<212>  PRT

<213>  Artificial Sequence

<220>
<223>  epitope


<220>
<221>  misc_feature
<222>  (2)..(2)
<223>  Xaa can be any amino acid

<220>
<221>  misc_feature
<222>  (4)..(9)
<223>  Xaa can be any amino acid

<400>  614

Leu Xaa Asp Xaa Xaa Xaa Xaa Xaa Xaa
1               5


<210>  615
<211>  9
<212>  PRT
<213>  Artificial Sequence

<220>
<223>  epitope


<220>
<221>  misc_feature
<222>  (2)..(2)
<223>  Xaa can be any amino acid

<220>
<221>  misc_feature
<222>  (4)..(7)
<223>  Xaa can be any amino acid

<220>
<221>  misc_feature
<222>  (9)..(9)
<223>  Xaa can be any amino acid

<400>  615

Leu Xaa Asp Xaa Xaa Xaa Xaa Leu Xaa
1               5


<210>  616
<211>  9
<212>  PRT
<213>  Artificial Sequence

<220>
<223>  epitope

<400>  616

Leu Leu Asp Arg Lys Val Ala Leu Thr

1               5


<210>  617
<211>  4
<212>  PRT
<213>  Artificial Sequence

<220>
<223>  epitope

<400>  617

Leu Leu Asp Arg
1


<210>  618
<211>  10
<212>  PRT
<213>  Artificial Sequence

<220>
<223>  epitope


<220>
<221>  misc_feature
<222>  (2)..(5)
<223>  Xaa can be any amino acid

<220>
<221>  misc_feature
<222>  (7)..(8)
<223>  Xaa can be any amino acid

<220>
<221>  misc_feature
<222>  (10)..(10)
<223>  Xaa can be any amino acid

<400>  618

Asp Xaa Xaa Xaa Xaa Leu Xaa Xaa Met Xaa
1               5                   10


<210>  619
<211>  10
<212>  PRT
<213>  Artificial Sequence

<220>
<223>  epitope

<400>  619

Asp Arg Lys Val Ala Leu Thr Asp Met Thr
1               5                   10


<210>  620
<211>  15

<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (4)..(4)
<223>  Xaa can be any amino acid

<220>
<221>  misc_feature
<222>  (6)..(15)
<223>  Xaa can be any amino acid

<400>  620

Xaa Xaa Leu Xaa Asp Xaa Xaa Xaa Xaa Xaa Xaa Xaa Xaa Xaa Xaa
1               5                   10                  15


<210>  621
<211>  15
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (4)..(4)
<223>  Xaa can be any amino acid

<220>
<221>  misc_feature
<222>  (6)..(9)
<223>  Xaa can be any amino acid

<220>
<221>  misc_feature
<222>  (11)..(12)
<223>  Xaa can be any amino acid

<220>
<221>  misc_feature
<222>  (14)..(15)
<223>  Xaa can be any amino acid

<400>  621

Xaa Xaa Leu Xaa Asp Xaa Xaa Xaa Xaa Leu Xaa Xaa Met Xaa Xaa
1               5                   10                  15


<210>  622
<211>  9
<212>  PRT
<213>  Artificial Sequence

<220>
<223>  epitope

<400>  622

Gly Arg Tyr Leu His Leu Ala Ala Val
1               5


<210>  623
<211>  10
<212>  PRT
<213>  Artificial Sequence

<220>
<223>  epitope

<400>  623

Gly Arg Tyr Leu His Leu Ala Ala Val Asn
1               5                   10


<210>  624
<211>  9
<212>  PRT
<213>  Artificial Sequence

<220>
<223>  epitope

<400>  624

Lys Leu Glu Phe Leu Leu Ser Lys Lys
1               5


<210>  625
<211>  15
<212>  PRT
<213>  Artificial Sequence

<220>
<223>  epitope


<220>
<221>  misc_feature
<222>  (4)..(15)
<223>  Xaa can be any amino acid

<400>  625

Leu His Leu Xaa Xaa Xaa Xaa Xaa Xaa Xaa Xaa Xaa Xaa Xaa Xaa
1               5                   10                  15

<210>  626
<211>  8
<212>  PRT
<213>  Artificial Sequence

<220>
<223>  epitope

<220>
<221>  misc_feature
<222>  (1)..(5)
<223>  Xaa can be any amino acid

<400>  626

Xaa Xaa Xaa Xaa Xaa Met Phe Gly
1               5

<210>  627
<211>  8
<212>  PRT
<213>  Artificial Sequence

<220>
<223>  epitope

<400>  627

Ser Gly Tyr Leu Lys Met Phe Gly
1               5

<210>  628
<211>  10
<212>  PRT
<213>  Artificial Sequence

<220>
<223>  epitope

<220>
<221>  misc_feature
<222>  (1)..(3)
<223>  Xaa can be any amino acid

<220>
<221>  misc_feature
<222>  (6)..(6)
<223>  Xaa can be any amino acid

<400>  628

Xaa Xaa Xaa Met Phe Xaa Gln Glu Leu Leu
1               5                   10

<210>  629
<211>  10
<212>  PRT
<213>  Artificial Sequence

<220>
<223>  epitope


<220>
<221>  misc_feature
<222>  (1)..(3)
<223>  Xaa can be any amino acid

<400>  629

Xaa Xaa Xaa Met Phe Gly Gln Glu Leu Leu
1               5                   10


<210>  630
<211>  10
<212>  PRT
<213>  Artificial Sequence

<220>
<223>  epitope

<400>  630

Tyr Leu Lys Met Phe Gly Gln Glu Leu Leu
1               5                   10


<210>  631
<211>  9
<212>  PRT
<213>  Artificial Sequence

<220>
<223>  epitope

<400>  631

Gln Glu Leu Leu Phe Gly Arg Leu Asp
1               5


<210>  632
<211>  10
<212>  PRT
<213>  Artificial Sequence

<220>
<223>  epitope

<400>  632

Gln Glu Leu Leu Phe Gly Arg Leu Asp Lys
1               5                   10


<210>  633

<211>  15
<212>  PRT
<213>  Artificial Sequence

<220>
<223>  epitope


<220>
<221>  misc_feature
<222>  (1)..(5)
<223>  Xaa can be any amino acid

<220>
<221>  misc_feature
<222>  (8)..(8)
<223>  Xaa can be any amino acid

<220>
<221>  misc_feature
<222>  (15)..(15)
<223>  Xaa can be any amino acid

<400>  633

Xaa Xaa Xaa Xaa Xaa Met Phe Xaa Gln Glu Leu Leu Phe Gly Xaa
1               5                   10                  15


<210>  634
<211>  15
<212>  PRT
<213>  Artificial Sequence

<220>
<223>  epitope


<220>
<221>  misc_feature
<222>  (1)..(5)
<223>  Xaa can be any amino acid

<220>
<221>  misc_feature
<222>  (15)..(15)
<223>  Xaa can be any amino acid

<400>  634

Xaa Xaa Xaa Xaa Xaa Met Phe Gly Gln Glu Leu Leu Phe Gly Xaa
1               5                   10                  15


<210>  635
<211>  8
<212>  PRT
<213>  Artificial Sequence

<220>
<223>  epitope

<220>
<221>  misc_feature
<222>  (2)..(5)
<223>  Xaa can be any amino acid

<220>
<221>  misc_feature
<222>  (8)..(8)
<223>  Xaa can be any amino acid

<400>  635

Ser Xaa Xaa Xaa Xaa Met Phe Xaa
1               5


<210>  636
<211>  8
<212>  PRT
<213>  Artificial Sequence

<220>
<223>  epitope

<400>  636

Ser Gly Tyr Leu Lys Met Phe Gly
1               5


<210>  637
<211>  3
<212>  PRT
<213>  Artificial Sequence

<220>
<223>  epitope

<400>  637

Glu Leu Leu
1


<210>  638
<211>  15
<212>  PRT
<213>  Artificial Sequence

<220>
<223>  epitope


<220>
<221>  misc_feature
<222>  (2)..(5)
<223>  Xaa can be any amino acid

<220>
<221>  misc_feature
<222>  (8)..(11)
<223>  Xaa can be any amino acid

<220>
<221>  misc_feature
<222>  (13)..(15)
<223>  Xaa can be any amino acid

<400>  638

Ser Xaa Xaa Xaa Xaa Met Phe Xaa Xaa Xaa Xaa Leu Xaa Xaa Xaa
1               5                   10                  15


<210>  639
<211>  15
<212>  PRT
<213>  Artificial Sequence

<220>
<223>  epitope


<220>
<221>  misc_feature
<222>  (1)..(5)
<223>  Xaa can be any amino acid

<220>
<221>  misc_feature
<222>  (7)..(11)
<223>  Xaa can be any amino acid

<220>
<221>  misc_feature
<222>  (13)..(15)
<223>  Xaa can be any amino acid

<400>  639

Xaa Xaa Xaa Xaa Xaa Met Xaa Xaa Xaa Xaa Xaa Leu Xaa Xaa Xaa
1               5                   10                  15


<210>  640
<211>  7
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (4)..(5)
<223>  Xaa can be any amino acid

<400>  640

Xaa Xaa Tyr Xaa Xaa Met Phe

1               5


<210>  641
<211>  7
<212>  PRT
<213>  Artificial Sequence

<220>
<223>  epitope

<400>  641

Ser Gly Tyr Leu Lys Met Phe
1               5


<210>  642
<211>  5
<212>  PRT
<213>  Artificial Sequence

<220>
<223>  epitope

<400>  642

Gly Gln Glu Leu Leu
1               5


<210>  643
<211>  15
<212>  PRT
<213>  Artificial Sequence

<220>
<223>  epitope



<220>
<221>  misc_feature
<222>  (1)..(2)
<223>  Xaa can be any amino acid

<220>
<221>  misc_feature
<222>  (4)..(5)
<223>  Xaa can be any amino acid

<220>
<221>  misc_feature
<222>  (8)..(12)
<223>  Xaa can be any amino acid

<220>
<221>  misc_feature
<222>  (14)..(15)
<223>  Xaa can be any amino acid

<400>  643

Xaa Xaa Tyr Xaa Xaa Met Phe Xaa Xaa Xaa Xaa Xaa Phe Xaa Xaa

1               5                   10                  15


<210>  644
<211>  8
<212>  PRT
<213>  Artificial Sequence

<220>
<223>  epitope

<400>  644

Gln Glu Leu Leu Phe Gly Arg Leu
1               5


<210>  645
<211>  8
<212>  PRT
<213>  Artificial Sequence

<220>
<223>  epitope


<220>
<221>  misc_feature
<222>  (1)..(4)
<223>  Xaa can be any amino acid

<220>
<221>  misc_feature
<222>  (8)..(8)
<223>  Xaa can be any amino acid

<400>  645

Xaa Xaa Xaa Xaa Asp Thr Leu Xaa
1               5


<210>  646
<211>  8
<212>  PRT
<213>  Artificial Sequence

<220>
<223>  epitope


<220>
<221>  misc_feature
<222>  (2)..(4)
<223>  Xaa can be any amino acid

<220>
<221>  misc_feature
<222>  (8)..(8)
<223>  Xaa can be any amino acid

<400>  646

Arg Xaa Xaa Xaa Asp Thr Leu Xaa
1               5

<210>  647
<211>  8
<212>  PRT
<213>  Artificial Sequence

<220>
<223>  epitope

<400>  647

Arg Leu Asp Lys Asp Thr Leu Gln
1               5

<210>  648
<211>  8
<212>  PRT
<213>  Artificial Sequence

<220>
<223>  epitope

<220>
<221>  misc_feature
<222>  (4)..(8)
<223>  Xaa can be any amino acid

<400>  648

Asp Thr Leu Xaa Xaa Xaa Xaa Xaa
1               5

<210>  649
<211>  8
<212>  PRT
<213>  Artificial Sequence

<220>
<223>  epitope

<400>  649

Asp Thr Leu Gln Asn Val Leu Gln
1               5

<210>  650
<211>  15
<212>  PRT
<213>  Artificial Sequence

<220>
<223>  epitope

<220>
<221>  misc_feature

<222>  (1)..(8)
<223>  Xaa can be any amino acid

<220>
<221>  misc_feature
<222>  (12)..(15)
<223>  Xaa can be any amino acid

<400>  650

Xaa Xaa Xaa Xaa Xaa Xaa Xaa Xaa Asp Thr Leu Xaa Xaa Xaa Xaa
1               5                   10                  15


<210>  651
<211>  15
<212>  PRT
<213>  Artificial Sequence

<220>
<223>  epitope


<220>
<221>  misc_feature
<222>  (1)..(4)
<223>  Xaa can be any amino acid

<220>
<221>  misc_feature
<222>  (6)..(8)
<223>  Xaa can be any amino acid

<220>
<221>  misc_feature
<222>  (12)..(15)
<223>  Xaa can be any amino acid

<400>  651

Xaa Xaa Xaa Xaa Arg Xaa Xaa Xaa Asp Thr Leu Xaa Xaa Xaa Xaa
1               5                   10                  15


<210>  652
<211>  15
<212>  PRT
<213>  Artificial Sequence

<220>
<223>  epitope


<220>
<221>  misc_feature
<222>  (1)..(3)
<223>  Xaa can be any amino acid

<220>
<221>  misc_feature
<222>  (8)..(8)
<223>  Xaa can be any amino acid

<220>
<221>  misc_feature
<222>  (10)..(15)
<223>  Xaa can be any amino acid

<400>  652

Xaa Xaa Xaa Ser Lys Thr Ala Xaa Val Xaa Xaa Xaa Xaa Xaa Xaa
1               5                   10                  15


<210>  653
<211>  15
<212>  PRT
<213>  Artificial Sequence

<220>
<223>  epitope



<220>
<221>  misc_feature
<222>  (1)..(3)
<223>  Xaa can be any amino acid

<220>
<221>  misc_feature
<222>  (5)..(6)
<223>  Xaa can be any amino acid

<220>
<221>  misc_feature
<222>  (9)..(10)
<223>  Xaa can be any amino acid

<220>
<221>  misc_feature
<222>  (12)..(15)
<223>  Xaa can be any amino acid

<400>  653

Xaa Xaa Xaa Ser Xaa Xaa Phe Ala Xaa Xaa Arg Xaa Xaa Xaa Xaa
1               5                   10                  15


<210>  654
<211>  15
<212>  PRT
<213>  Artificial Sequence

<220>
<223>  epitope



<220>
<221>  misc_feature
<222>  (1)..(3)
<223>  Xaa can be any amino acid

<220>
<221>  misc_feature
<222>  (5)..(6)

<223>  Xaa can be any amino acid

<220>
<221>  misc_feature
<222>  (9)..(12)
<223>  Xaa can be any amino acid

<220>
<221>  misc_feature
<222>  (14)..(14)
<223>  Xaa can be any amino acid

<400>  654

Xaa Xaa Xaa Ser Xaa Xaa Phe Ala Xaa Xaa Xaa Xaa Ile Xaa Asp
1               5                   10                  15


<210>  655
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (4)..(6)
<223>  Xaa can be any amino acid

<220>
<221>  misc_feature
<222>  (9)..(12)
<223>  Xaa can be any amino acid

<220>
<221>  misc_feature
<222>  (14)..(15)
<223>  Xaa can be any amino acid

<400>  655

Xaa Asn Ile Xaa Xaa Xaa Ala Ala Xaa Xaa Xaa Xaa Pro Xaa Xaa
1               5                   10                  15


<210>  656
<211>  15
<212>  PRT
<213>  Artificial Sequence

<220>
<223>  epitope


<220>

<221>  misc_feature
<222>  (1)..(6)
<223>  Xaa can be any amino acid

<220>
<221>  misc_feature
<222>  (10)..(10)
<223>  Xaa can be any amino acid

<220>
<221>  misc_feature
<222>  (12)..(12)
<223>  Xaa can be any amino acid

<220>
<221>  misc_feature
<222>  (14)..(15)
<223>  Xaa can be any amino acid

<400>  656

Xaa Xaa Xaa Xaa Xaa Xaa Ala Ala Ser Xaa Met Xaa Pro Xaa Xaa
1               5                   10                  15


<210>  657
<211>  10
<212>  PRT
<213>  Artificial Sequence

<220>
<223>  epitope

<400>  657

Val Pro Asp Ile Met Lys Met Ser Phe Asp
1               5                   10


<210>  658
<211>  9
<212>  PRT
<213>  Artificial Sequence

<220>
<223>  epitope

<400>  658

Met Lys Met Ser Phe Asp Ser Asp Ser
1               5


<210>  659
<211>  10
<212>  PRT
<213>  Artificial Sequence

<220>
<223>  epitope

<400>  659

Met Ser Phe Asp Ser Asp Ser Ala Ser Gly
1               5                   10

<210>  660
<211>  9
<212>  PRT
<213>  Artificial Sequence

<220>
<223>  epitope

<400>  660

Asp Ser Asp Ser Ala Ser Gly Glu Thr
1               5

<210>  661
<211>  15
<212>  PRT
<213>  Artificial Sequence

<220>
<223>  epitope

<220>
<221>  misc_feature
<222>  (2)..(5)
<223>  Xaa can be any amino acid

<220>
<221>  misc_feature
<222>  (7)..(8)
<223>  Xaa can be any amino acid

<220>
<221>  misc_feature
<222>  (10)..(12)
<223>  Xaa can be any amino acid

<400>  661

Pro Xaa Xaa Xaa Xaa Met Xaa Xaa Asp Xaa Xaa Xaa Ala Ser Gly
1               5                   10                  15

<210>  662
<211>  15
<212>  PRT
<213>  Artificial Sequence

<220>
<223>  epitope

<220>
<221>  misc_feature
<222>  (2)..(2)
<223>  Xaa can be any amino acid

<220>

<221>  misc_feature
<222>  (5)..(5)
<223>  Xaa can be any amino acid

<220>
<221>  misc_feature
<222>  (7)..(7)
<223>  Xaa can be any amino acid

<220>
<221>  misc_feature
<222>  (11)..(11)
<223>  Xaa can be any amino acid

<400>  662

Pro Xaa Ile Met Xaa Met Xaa Phe Asp Ser Xaa Ser Ala Ser Gly
1               5                   10                  15


<210>  663
<211>  10
<212>  PRT
<213>  Artificial Sequence

<220>
<223>  epitope

<400>  663

Pro Asp Ile Met Lys Met Ser Phe Asp Ser
1               5                   10


<210>  664
<211>  7
<212>  PRT
<213>  Artificial Sequence

<220>
<223>  epitope

<400>  664

Met Ser Phe Asp Ser Asp Ser
1               5


<210>  665
<211>  8
<212>  PRT
<213>  Artificial Sequence

<220>
<223>  epitope

<400>  665

Ser Asp Ser Ala Ser Gly Glu Thr
1               5


<210>  666

<211>  15
<212>  PRT
<213>  Artificial Sequence

<220>
<223>  epitope


<220>
<221>  misc_feature
<222>  (1)..(11)
<223>  Xaa can be any amino acid

<400>  666

Xaa Xaa Xaa Xaa Xaa Xaa Xaa Xaa Xaa Xaa Xaa Ser Ala Ser Gly
1               5                   10                  15


<210>  667
<211>  8
<212>  PRT
<213>  Artificial Sequence

<220>
<223>  epitope


<220>
<221>  misc_feature
<222>  (1)..(2)
<223>  Xaa can be any amino acid

<400>  667

Xaa Xaa Asp Val Pro Ile Glu Lys
1               5


<210>  668
<211>  8
<212>  PRT
<213>  Artificial Sequence

<220>
<223>  epitope

<400>  668

Tyr Ser Asp Val Pro Ile Glu Lys
1               5


<210>  669
<211>  9
<212>  PRT
<213>  Artificial Sequence

<220>
<223>  epitope

<400>  669

Tyr Ser Asp Val Pro Ile Glu Lys Ile
1               5

<210>  670
<211>  15
<212>  PRT
<213>  Artificial Sequence

<220>
<223>  epitope

<220>
<221>  misc_feature
<222>  (1)..(7)
<223>  Xaa can be any amino acid

<400>  670

Xaa Xaa Xaa Xaa Xaa Xaa Xaa Asp Val Pro Ile Glu Lys Ile Gln
1               5                   10                  15

<210>  671
<211>  15
<212>  PRT
<213>  Artificial Sequence

<220>
<223>  epitope

<220>
<221>  misc_feature
<222>  (1)..(5)
<223>  Xaa can be any amino acid

<400>  671

Xaa Xaa Xaa Xaa Xaa Tyr Ser Asp Val Pro Ile Glu Lys Ile Gln
1               5                   10                  15

<210>  672
<211>  10
<212>  PRT
<213>  Artificial Sequence

<220>
<223>  epitope

<400>  672

Met Ser Phe Lys Pro Val Tyr Ser Asp Val
1               5                   10

<210>  673
<211>  8
<212>  PRT
<213>  Artificial Sequence

<220>
<223>  epitope

<220>
<221>  misc_feature
<222>  (1)..(1)
<223>  Xaa can be any amino acid

<220>
<221>  misc_feature
<222>  (3)..(3)
<223>  Xaa can be any amino acid

<220>
<221>  misc_feature
<222>  (5)..(6)
<223>  Xaa can be any amino acid

<220>
<221>  misc_feature
<222>  (8)..(8)
<223>  Xaa can be any amino acid

<400>  673

Xaa Ser Xaa Val Xaa Xaa Glu Xaa
1               5

<210>  674
<211>  8
<212>  PRT
<213>  Artificial Sequence

<220>
<223>  epitope

<400>  674

Tyr Ser Asp Val Pro Ile Glu Lys
1               5

<210>  675
<211>  15
<212>  PRT
<213>  Artificial Sequence

<220>
<223>  epitope

<220>
<221>  misc_feature
<222>  (1)..(1)
<223>  Xaa can be any amino acid

<220>
<221>  misc_feature
<222>  (3)..(4)
<223>  Xaa can be any amino acid

<220>
<221>  misc_feature
<222>  (6)..(6)
<223>  Xaa can be any amino acid

<220>
<221>  misc_feature
<222>  (8)..(14)
<223>  Xaa can be any amino acid

<400>  675

Xaa Phe Xaa Xaa Val Xaa Ser Xaa Xaa Xaa Xaa Xaa Xaa Xaa Gln
1               5                   10                  15


<210>  676
<211>  9
<212>  PRT
<213>  Artificial Sequence

<220>
<223>  epitope

<400>  676

Pro Ile Glu Lys Ile Gln Val Thr Ile
1               5


<210>  677
<211>  15
<212>  PRT
<213>  Artificial Sequence

<220>
<223>  epitope



<220>
<221>  misc_feature
<222>  (1)..(6)
<223>  Xaa can be any amino acid

<220>
<221>  misc_feature
<222>  (8)..(8)
<223>  Xaa can be any amino acid

<220>
<221>  misc_feature
<222>  (10)..(10)
<223>  Xaa can be any amino acid

<220>
<221>  misc_feature
<222>  (12)..(12)
<223>  Xaa can be any amino acid

<220>
<221>  misc_feature
<222>  (14)..(14)
<223>  Xaa can be any amino acid

<400>  677

Xaa Xaa Xaa Xaa Xaa Xaa Ser Xaa Val Xaa Ile Xaa Lys Xaa Gln
1               5                   10                  15


<210>  678
<211>  15
<212>  PRT
<213>  Artificial Sequence

<220>
<223>  epitope


<220>
<221>  misc_feature
<222>  (1)..(6)
<223>  Xaa can be any amino acid

<220>
<221>  misc_feature
<222>  (10)..(10)
<223>  Xaa can be any amino acid

<220>
<221>  misc_feature
<222>  (12)..(12)
<223>  Xaa can be any amino acid

<400>  678

Xaa Xaa Xaa Xaa Xaa Xaa Ser Asp Val Xaa Ile Xaa Lys Ile Gln
1               5                   10                  15


<210>  679
<211>  6
<212>  PRT
<213>  Artificial Sequence

<220>
<223>  epitope


<220>
<221>  misc_feature
<222>  (6)..(6)
<223>  Xaa can be any amino acid

<400>  679

Ser Phe Lys Pro Val Xaa
1               5


<210>  680
<211>  6
<212>  PRT
<213>  Artificial Sequence

<220>

<223>  epitope

<400>  680

Ser Phe Lys Pro Val Tyr
1               5


<210>  681
<211>  10
<212>  PRT
<213>  Artificial Sequence

<220>
<223>  epitope

<400>  681

Val Tyr Ser Asp Val Pro Ile Glu Lys Ile
1               5                   10


<210>  682
<211>  15
<212>  PRT
<213>  Artificial Sequence

<220>
<223>  epitope


<220>
<221>  misc_feature
<222>  (6)..(15)
<223>  Xaa can be any amino acid

<400>  682

Ser Phe Lys Pro Val Xaa Xaa Xaa Xaa Xaa Xaa Xaa Xaa Xaa Xaa
1               5                   10                  15